# FASCICULE DE BREVET EUROPEEN

(11) **EP 3 063 300 B1**
(45) Date de publication et mention de la délivrance du brevet: **06.03.2019**
(21) Numéro de dépôt: 14809449.3
(22) Date de dépôt: 31.10.2014
(51) Int. Cl.: C12Q 1/68, C07K 7/06, C07K 7/08, C12N 15/11, C07K 14/415, C12N 15/82

(54) **MICROPEPTIDES ET LEUR UTILISATION POUR MODULER L'EXPRESSION DE GÈNES**
MIKROPEPTIDE UND VERWENDUNG DAVON ZUR MODULATION DER GENEXPRESSION
MICROPEPTIDES AND USE OF SAME FOR MODULATING GENE EXPRESSION

(30) Priorité: 31.10.2013 FR 1360727; 03.06.2014 FR 1455044
(43) Date de publication de la demande: 07.09.2016
(60) Demande divisionnaire: 19152673.0
(73) Titulaire: Université Paul Sabatier Toulouse III, 31062 Toulouse Cedex 9 (FR); Centre National de la Recherche Scientifique, 75794 Paris Cedex 16 (FR)
(72) Inventeur: COMBIER, Jean-Philippe, F-31320 Castanet Tolosan (FR); LAURESSERGUES, Dominique, F-31000 Toulouse (FR); BECARD, Guillaume, F-31450 Odars (FR); PAYRE, François, F-31450 Pompertuzat (FR); PLAZA, Serge, F-31520 Ramonville Saint Agne (FR); CAVAILLE, Jérôme, F-31520 Ramonville Saint Agne (FR)
(74) Mandataire: Grosset-Fournier, Chantal Catherine
(86) Numéro de dépôt international: PCT/FR2014/052781
(87) Numéro de publication internationale: WO 2015/063431

(56) Documents cités:
- AXEL BRENNICKE: "Micropeptide als Gen-Regulatoren", BIOL. UNSERER ZEIT, vol. 40, 1 juin 2010 (2010-06-01), page 371, XP055168530,
- JEROEN CRAPPÉ ET AL: "Micro peptides as a new class of bio-active peptides in Eukaryotes", BENELUX BIOINFORMATICS CONFERENCE: PROCEEDINGS OF BBC11, 9 décembre 2011 (2011-12-09), page 77, XP055168531,
- MÁXIMO IBO GALINDO ET AL: "Peptides Encoded by Short ORFs Control Development and Define a New Eukaryotic Gene Family", CURRENT SCIENCE, vol. 83, no. 5, 1 janvier 2007 (2007-01-01), page 426, XP055168533, ISSN: 0011-3891, DOI: 0011-3891(2002)083[0426:COAEDF]2.0.CO;2
- C. LELANDAIS-BRIERE ET AL: "Genome-Wide Medicago truncatula Small RNA Analysis Revealed Novel MicroRNAs and Isoforms Differentially Regulated in Roots and Nodules", THE PLANT CELL ONLINE, vol. 21, no. 9, 1 septembre 2009 (2009-09-01), pages 2780-2796, XP055127996, ISSN: 1040-4651, DOI: 10.1105/tpc.109.068130
- DOMINIQUE LAURESSERGUES ET AL: "The microRNA miR171h modulates arbuscular mycorrhizal colonization of Medicago truncatula by targeting NSP2", THE PLANT JOURNAL, vol. 72, no. 3, 30 août 2012 (2012-08-30), pages 512-522, XP055128000, ISSN: 0960-7412, DOI: 10.1111/j.1365-313X.2012.05099.x
- BOUTIN J A ET AL: "Combinatorial peptide synthesis: statistical evaluation of peptide distribution", TRENDS IN PHARMACOLOGICAL SCIENCES, ELSEVIER, HAYWARTH, GB, vol. 17, no. 1, 1 January 1996 (1996-01-01), pages 8-12, XP004034505, ISSN: 0165-6147, DOI: 10.1016/0165-6147(96)81563-X
- H Weinberger ET AL: "Small peptide libraries: combinatorial split-mix synthesis followed by combinatorial amino acid analysis of selected variants", Archiv der Pharmazie, 1 April 1997 (1997-04-01), pages 109-111, XP055461851, GERMANY DOI: 10.1002/ardp.19973300406 Retrieved from the Internet: URL:https://onlinelibrary.wiley.com/doi/pd f/10.1002/ardp.19973300406
- Man Zhou ET AL: "MicroRNA-mediated gene regulation: potential applications for plant genetic engineering", PLANT MOLECULAR BIOLOGY, vol. 83, no. 1-2, 1 September 2013 (2013-09-01), pages 59-75, XP055462531, NL ISSN: 0167-4412, DOI: 10.1007/s11103-013-0089-1
- WANG V ET AL: "MicroRNA-Based Therapeutics for Cancer", BIOD, ADIS INTERNATIONAL LTD, NZ, vol. 23, no. 1, 1 January 2009 (2009-01-01), pages 15-23, XP008113416, ISSN: 1173-8804, DOI: 10.2165/00063030-200923010-00002

## Description

La présente invention concerne des micropeptides (peptides codés par des microARNs ou « miPEPs ») et leur utilisation pour moduler l'expression de gènes.

Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont rencontrés chez les plantes et les animaux.

Les gènes cibles sont souvent des gènes clés de processus développementaux. Ils codent par exemple des facteurs de transcription ou des protéines du protéasome.

La régulation de l'expression des miRs est très peu connue, mais on sait notamment que celle-ci fait intervenir, à l'instar de la plupart des gènes codants, une ARN polymerase II : cette enzyme produit un transcrit primaire, appelé *« pri-miR »*, qui est ensuite maturé par un complexe protéique contenant notamment les enzymes type Dicer. Cette maturation conduit tout d'abord à la formation d'un précurseur de miR appelé *« pre-miR »*, ayant une structure secondaire en forme tige-boucle contenant le *miR* et sa séquence *miR** complémentaire. Puis le précurseur est maturé, ce qui conduit à la formation d'un ARN double brin plus court contenant le miR et le miR*. Le miR est ensuite pris en charge par le complexe RISC qui clive l'ARNm du gène cible ou bien inhibe sa traduction.

Par ailleurs, il a été montré que la présence d'introns dans le transcrit primaire du microARN augmente l'expression du microARN mature (Schwab et al., EMBO Rep., 14(7):615-21, 2013). Cependant, du fait de difficultés expérimentales, les transcrits primaires de microARNs, ou pri-miRs, sont très peu étudiés.

Environ 50% des gènes eucaryotes possèdent, au sein de leur région 5'UTR (5' UnTranslated Region) en amont de la séquence codante, de petits cadres ouverts de lecture. Ces petits cadres ouverts de lecture (ou « uORFs » pour upstream ORFs) peuvent jouer un rôle de régulateur de la traduction, principalement en *cis*, en modulant la fixation et la vitesse des ribosomes sur l'ARNm, mais également en *trans* selon un mécanisme encore inconnu, par l'intermédiaire de peptides codés par lesdits uORFs (Combier et al., Gene Dev, 22:1549-1559, 2008). Par définition, les uORFS sont présents en amont de gènes codants.

Récemment, il a également été découvert de petits ORFs dans de longs ARN non codants intergéniques (lincRNAs) dont la fonction putative, si elle existe, n'est pas connue (Ingolia et al., Cell, 147(4):789-802, 2011 ; Guttman & Rinn, Nature, 482(7385):339-46, 2012).

Toutefois, aucun exemple n'a encore été rapporté concernant l'existence d'ORFs codant des peptides au sein de microARNs non codants. Jusqu'à présent, les microARNs, et par extension leur transcrit primaire, ont toujours été considérés, de par leur mode d'action particulier, comme des ARNs régulateurs non codants ne produisant aucun peptide.

L'un des aspects de l'invention est de proposer des peptides capables de moduler l'expression des microARNs.

Un autre aspect de l'invention est de proposer un moyen permettant de moduler l'expression d'un ou plusieurs gènes cibles d'un microARN.

La présente invention présente l'avantage de permettre un contrôle plus facile et plus efficace de l'expression de gènes ciblés par les microARNs, à l'aide d'un moyen autre que le microARN.

L'invention est décrite par les revendications attenantes.

L'invention concerne ainsi un procédé de détection et d'identification d'un micropeptide (miPEP) codé par une séquence nucléotidique contenue dans la séquence du transcrit primaire d'un microARN,
comprenant :
- a) une étape de détection d'un cadre ouvert de lecture de 12 à 303 nucléotides contenu dans la séquence du transcrit primaire dudit microARN, puis
- b) une étape de comparaison entre :
   - l'accumulation dudit microARN dans une cellule eucaryote déterminée exprimant ledit microARN,
      en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
   - l'accumulation dudit microARN dans une cellule eucaryote de même type que la susdite cellule eucaryote déterminée exprimant ledit microARN,
      en absence dudit peptide,
dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.

L'invention concerne en particulier un procédé de détection et d'identification d'un micropeptide (miPEP) codé par une séquence nucléotidique contenue dans la séquence du transcrit primaire d'un microARN,
comprenant :
- a) une étape de détection d'un cadre ouvert de lecture de 15 à 303 nucléotides contenu dans la séquence du transcrit primaire dudit microARN, puis
- b) une étape de comparaison entre :
   - l'accumulation dudit microARN dans une cellule eucaryote déterminée exprimant ledit microARN,
      en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
   - l'accumulation dudit microARN dans une cellule eucaryote de même type que la susdite cellule eucaryote déterminée exprimant ledit microARN,
      en absence dudit peptide,
dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.

La demande décrit aussi un procédé de détection et d'identification d'un micropeptide (miPEP) codé par une séquence nucléotidique contenue dans la séquence du transcrit primaire d'un microARN,
comprenant :
a) une étape de détection d'un cadre ouvert de lecture de 15 à 303 nucléotides, ou de 12 nucléotides, contenu dans la séquence du transcrit primaire dudit microARN, puis
b) une étape de comparaison entre :
   - l'accumulation dudit microARN dans une cellule eucaryote déterminée exprimant ledit microARN,
      en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
   - l'accumulation dudit microARN dans une cellule eucaryote de même type que la susdite cellule eucaryote déterminée exprimant ledit microARN,
      en absence dudit peptide,
dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.

La demande décrit aussi un procédé de détection et d'identification d'un micropeptide (miPEP) codé par une séquence nucléotidique contenue dans la séquence du transcrit primaire d'un microARN,
comprenant :
a) une étape de détection d'un cadre ouvert de lecture de 12 nucléotides contenu dans la séquence du transcrit primaire dudit microARN, puis
b) une étape de comparaison entre :
   - l'accumulation dudit microARN dans une cellule eucaryote déterminée exprimant ledit microARN,
      en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
   - l'accumulation dudit microARN dans une cellule eucaryote de même type que la susdite cellule eucaryote déterminée exprimant ledit microARN,
      en absence dudit peptide,
dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.

Dans une première étape, le procédé de détection et d'identification d'un micropeptide consiste donc à détecter sur le transcrit primaire d'un microARN l'existence d'un cadre ouvert de lecture codant potentiellement un peptide.

La deuxième étape permet, quant à elle, de caractériser ledit peptide, c'est-à-dire de déterminer si ledit peptide correspond a un peptide réellement produit dans la cellule, en recherchant un effet dudit peptide sur l'accumulation dudit microARN.

Pour mettre en évidence un effet du peptide sur l'accumulation du microARN, une quantité importante de peptide est introduite dans une première cellule exprimant ledit microARN. L'accumulation du microARN dans cette première cellule est ensuite mesurée et comparée avec l'accumulation du microARN dans une deuxième cellule identique à la première, mais ne contenant pas ledit peptide.

L'observation d'une variation des quantités de microARN entre les cellules en présence et en absence du peptide indique ainsi (i) qu'il existe un peptide codé sur le transcrit primaire dudit microARN, (ii) que la séquence de ce peptide est codée par le cadre ouvert de lecture identifié sur le transcrit primaire dudit microARN, et (iii) que ledit peptide agit sur l'accumulation dudit microARN.

L'invention repose donc sur la double observation inattendue faite par les Inventeurs que d'une part, il existe des cadres ouverts de lecture susceptibles de coder des micropeptides présents sur les transcrits primaires de microARNs, et d'autre part que lesdits micropeptides sont capables de moduler l'accumulation desdits microARNs.

Dans l'invention, les termes *« microARN », « microARN non codant »* et *« miR »* sont équivalents et peuvent être utilisés l'un pour l'autre. Ils définissent des petites molécules d'ARN d'environ 21 nucléotides, qui ne sont pas traduites et ne conduisent pas à un peptide ou une protéine.

Cependant, sous cette forme mature, les microARNs assurent une fonction de régulation de certains gènes *via* des mécanismes post-transcriptionnels, comme par exemple par l'intermédiaire du complexe RISC.

Le transcrit primaire du microARN ou *« pri-miR »* correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du microARN ou *« pre-miR »*, puis à la forme mature du microARN ou *« miR ».*

Les termes *« micropeptides »* et *« miPEPs* » (*microRNA encoded PEPtides*) sont équivalents et peuvent être utilisés l'un pour l'autre. Ils définissent un peptide qui est codé par un cadre ouvert de lecture présent sur le transcrit primaire d'un microARN, et qui est capable de moduler l'accumulation dudit microARN. Les micropeptides au sens de la présente invention ne doivent être entendus comme étant nécessairement des peptides de petite taille, dans la mesure où « micro » ne correspond pas à la taille du peptide.

Selon l'invention, un miPEP peut être également assimilé à un modulateur de transcription, et notamment un activateur transcriptionnel. Un tel modulateur de transcription peut agir au niveau de la transcription pour moduler l'accumulation du pri-miR, du pre-miR et du miR. Compte tenu de la dégénérescence du code génétique, un même micropeptide peut être codé par plusieurs séquences nucléotidiques. De telles séquences nucléotidiques, différentes entre elles d'au moins un nucléotide mais codant un même peptide, sont appelées *« séquences dégénérées ».*

Les termes *« cadre ouvert de lecture »* ou *« ORF »* (*open reading frame*) sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start (le codon start codant généralement une méthionine), suivi d'une série de codons (chaque codon codant un acide aminé), et se termine par un codon stop (le codon stop n'étant pas traduit).

Dans l'invention, les ORFs peuvent être dénommés de manière spécifique *« miORFs »* lorsque ceux-ci sont présents sur les transcrits primaires de microARN.

Les miORFs tels que définis dans l'invention particulier peuvent avoir une taille de 12 à 303 nucléotides et coder des peptides de 3 à 100 acides aminés.

En particulier, les miORFs tels que définis dans l'invention peuvent avoir une taille de 15 à 303 nucléotides. Un acide aminé étant codé par un codon de 3 nucléotides, les miORFs de 15 à 303 nucléotides codent des miPEPS de 4 à 100 acides aminés.

En particulier, les miORFs ont une taille de :
15, 18, 21, 24, 27, 30, 33, 36, 39, 42, 45, 47, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171, 174, 177, 180, 183, 186, 189, 192, 195, 198, 201, 204, 207, 210, 213, 216, 219, 222, 225, 228, 231, 234, 237, 240, 243, 246, 249, 252, 255, 258, 261, 264, 267, 270, 273, 276, 279, 282, 285, 288, 291, 294, 297, 300 ou 303 nucléotides, et codent respectivement des miPEPs ayant une taille de :
4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 acides aminés.

Dans l'invention, par *« accumulation »*, on entend la production d'une molécule, telle qu'un microARN ou un micropeptide, dans la cellule.

Ainsi, la *« modulation »* de l'accumulation d'une molécule dans une cellule correspond à une modification de la quantité de cette molécule présente dans la cellule.

Par ailleurs, l'effet d'un miPEP peut être observé via la modulation de l'accumulation du miR, mais également à travers la modulation de l'accumulation du pri-miR ou du pre-miR correspondant.

Dans un mode de réalisation, l'invention concerne un procédé de détection et d'identification d'un miPEP tel que défini ci-dessus, dans lequel la modulation de l'accumulation dudit microARN est une diminution ou une augmentation de l'accumulation dudit microARN, en particulier une augmentation.

Une *« diminution de l'accumulation »* correspond à une baisse de la quantité de ladite molécule dans la cellule.

A l'inverse, une *« augmentation de l'accumulation »* correspond à une hausse de la quantité de ladite molécule dans la cellule.

Dans un mode de réalisation avantageux, l'invention concerne un procédé de détection et d'identification d'un miPEP tel que défini ci-dessus, dans lequel la modulation de l'accumulation dudit microARN est une augmentation de l'accumulation dudit microARN. Dans un mode de réalisation, l'invention concerne un procédé de détection et d'identification d'un miPEP tel que défini ci-dessus, dans lequel la présence dudit peptide dans la cellule résulte :
- de l'introduction dans la cellule d'un acide nucléique codant ledit peptide, ou
- de l'introduction dans la cellule dudit peptide.

De manière à caractériser un miPEP, il est nécessaire de disposer d'un modèle cellulaire exprimant un microARN et dans lequel ledit peptide à tester est présent. Pour cela, il est possible d'introduire un peptide dans la cellule, soit en mettant la cellule en contact avec le dit peptide, soit en introduisant dans la cellule un acide nucléique codant ledit peptide, lequel acide nucléique sera alors traduit en peptide à l'intérieur de la cellule.

Dans un mode de réalisation, l'invention concerne un procédé de détection et d'identification d'un miPEP tel que défini ci-dessus, dans lequel le dit cadre ouvert de lecture de l'étape a) est contenu dans la partie 5' ou 3' dudit transcrit primaire du microARN, de préférence dans la partie 5'.

Les parties 5' ou 3' du transcrit primaire du microARN correspondent aux parties terminales de la molécule de l'ARN qui sont clivées au cours de la maturation du microARN.

Dans un mode de réalisation, l'invention concerne un procédé de détection et d'identification d'un miPEP tel que défini ci-dessus, dans lequel ledit microARN est présent dans une cellule végétale sauvage.

Dans l'invention, une cellule végétale sauvage correspond à une cellule végétale qui n'a pas été modifiée génétiquement par l'homme.

Dans un mode de réalisation, l'invention concerne un procédé de détection et d'identification d'un miPEP tel que défini ci-dessus, dans lequel ledit microARN est présent dans une cellule animale sauvage, et notamment une cellule humaine sauvage ou une cellule de drosophile sauvage.

Dans l'invention, une cellule animale sauvage correspond à une cellule animale, et notamment humaine, qui n'a pas été modifiée génétiquement par l'homme.

Dans un mode de réalisation, l'invention concerne un procédé de détection et d'identification d'un miPEP tel que défini ci-dessus, dans lequel ladite cellule eucaryote déterminée et ladite cellule eucaryote de même type que la susdite cellule eucaryote déterminée, utilisées à l'étape b, sont des cellules végétales d'une plante crucifère telle *qu'Arabidopsis thaliana*, d'une plante légumineuse telle que *Glycine max* (soja), *Medicago truncatula* et *Medicago sativa* (luzerne) ou d'une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé de détection et d'identification d'un miPEP tel que défini ci-dessus, dans lequel ladite cellule eucaryote déterminée et ladite cellule eucaryote de même type que la susdite cellule eucaryote déterminée, utilisées à l'étape b, sont des cellules végétales, de préférence des cellules de *Medicago truncatula, de Nicotiana benthamiana* ou *d'Arabidopsis thaliana.*

Dans un mode de réalisation, l'invention concerne un procédé de détection et d'identification d'un miPEP tel que défini ci-dessus, dans lequel ladite cellule eucaryote déterminée et ladite cellule eucaryote de même type que la susdite cellule eucaryote déterminée, utilisées à l'étape b, sont des cellules animales, de préférence des cellules humaines ou de drosophile. Dans le procédé de détection et d'identification d'un micropeptide tel que défini ci-dessus, après avoir identifié un ORF susceptible de coder un peptide sur le transcrit primaire d'un microARN, il est nécessaire de disposer d'un modèle cellulaire possédant ledit microARN et ledit peptide, pour pouvoir démontrer un effet éventuel du peptide sur ledit microARN.

Deux options sont donc envisageables :
- le modèle cellulaire dans lequel a été identifié le miORF et celui dans lequel est mis en évidence l'effet du peptide sur le miR sont identiques, ou
- le modèle cellulaire dans lequel a été identifié le miORF et celui dans lequel est mis en évidence l'effet du peptide sur le miR sont différents.

Dans la première option, le modèle cellulaire utilisé pour observer un effet du peptide est le même que celui dans lequel le transcrit primaire dudit microARN a été isolé. Dans ce modèle cellulaire, les cellules eucaryotes déterminées contiennent naturellement ledit microARN et seul le peptide à tester doit être introduit dans ces cellules. Dans ce contexte, ledit microARN est qualifié « d'origine endogène » car celui-ci existe naturellement dans les cellules. Néanmoins, dans une cellule, d'autres copies d'un microARN d'origine endogène peuvent être ajoutées, comme par exemple en introduisant dans la cellule un vecteur codant ledit microARN d'origine endogène.

Dans la deuxième option, le modèle cellulaire utilisé pour observer un effet du peptide est différent de celui dans lequel le transcrit primaire dudit microARN a été isolé. Dans ce modèle cellulaire, les cellules eucaryotes déterminées ne contiennent ni le microARN, ni le peptide à tester. Ces deux éléments doivent donc être introduits dans ces cellules. Dans ce contexte, ledit microARN est qualifié « d'origine exogène » car celui-ci n'existe pas naturellement dans les cellules.

Dans un mode de réalisation, l'invention concerne un procédé de détection et d'identification d'un miPEP tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).

Dans un mode de réalisation, l'invention concerne un procédé de détection et d'identification d'un miPEP tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.

Dans un mode de réalisation, l'invention concerne un procédé de détection et d'identification d'un miPEP tel que défini ci-dessus, dans lequel l'accumulation dudit microARN est déterminée en mettant en oeuvre une RT-PCR quantitative ou un Northern blot.

Dans un mode de réalisation, l'invention concerne un procédé de détection et d'identification d'un miPEP tel que défini ci-dessus, dans lequel l'accumulation dudit microARN est déterminée en mettant en oeuvre une puce à ADN ou à ARN.

L'accumulation dudit microARN peut être déterminée à l'aide des techniques de biologie moléculaire permettant le dosage de molécules d'acides nucléiques spécifiques.

La demande décrit aussi un procédé de détection et d'identification d'un microARN dont la séquence du transcrit primaire contient une séquence nucléotidique codant un miPEP,
comprenant :
- a) une étape de détection d'un cadre ouvert de lecture de 15 à 303 nucléotides contenu dans la séquence du transcrit primaire dudit microARN, puis
- b) une étape de comparaison entre :
   - l'accumulation dudit microARN dans une cellule eucaryote déterminée exprimant ledit microARN,
      en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
   - l'accumulation dudit microARN dans une cellule eucaryote, de même type que la susdite cellule eucaryote déterminée exprimant ledit microARN,
      en absence dudit peptide,
dans lequel, une modulation de l'accumulation dudit microARN en présence dudit peptide par rapport à l'accumulation dudit microARN en absence dudit peptide indique l'existence d'un microARN dont le transcrit primaire contient une séquence nucléotidique codant un micropeptide.

En particulier, la demande décrit aussi un procédé de détection et d'identification d'un microARN dont la séquence du transcrit primaire contient une séquence nucléotidique codant un miPEP,
comprenant :
- a) une étape de détection d'un cadre ouvert de lecture de 15 à 303 nucléotides contenu dans la séquence du transcrit primaire dudit microARN, puis
- b) une étape de comparaison entre :
   - l'accumulation dudit microARN dans une cellule eucaryote déterminée exprimant le transcrit primaire dudit microARN,
      en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
   - l'accumulation dudit microARN dans une cellule eucaryote, de même type que la susdite cellule eucaryote déterminée exprimant le transcrit primaire dudit microARN, en absence dudit peptide,
dans lequel, une modulation de l'accumulation dudit microARN en présence dudit peptide par rapport à l'accumulation dudit microARN en absence dudit peptide indique l'existence d'un microARN dont le transcrit primaire contient une séquence nucléotidique codant un micropeptide.

Dans un mode de réalisation, la demande décrit aussi un procédé de détection et d'identification d'un microARN tel que défini ci-dessus, dans lequel la modulation de l'accumulation dudit microARN est une diminution ou une augmentation de l'accumulation dudit microARN, en particulier une augmentation.

Dans un mode de réalisation, la demande décrit aussi un procédé de détection et d'identification d'un microARN tel que défini ci-dessus, dans lequel la présence dudit peptide dans la cellule résulte :
- de l'introduction dans la cellule d'un acide nucléique codant ledit peptide, ou
- de l'introduction dans la cellule de ledit peptide.

Dans un mode de réalisation, la demande décrit aussi un procédé de détection et d'identification d'un microARN tel que défini ci-dessus, dans lequel le dit cadre ouvert de lecture de l'étape a) est contenu dans la partie 5' ou 3' dudit transcrit primaire du microARN, de préférence dans la partie 5'.

Dans un mode de réalisation, l'invention concerne un procédé de détection et d'identification d'un microARN tel que défini ci-dessus, dans lequel ledit microARN est présent dans une cellule végétale sauvage.

Dans un mode de réalisation, la demande décrit aussi un procédé de détection et d'identification d'un microARN tel que défini ci-dessus, dans lequel ledit microARN est présent dans une cellule animale, et notamment humaine, sauvage.

Dans un mode de réalisation, la demande décrit aussi un procédé de détection et d'identification d'un microARN tel que défini ci-dessus, dans lequel ladite cellule eucaryote, et ladite cellule eucaryote de même type que la susdite cellule eucaryote déterminée, utilisées à l'étape b) sont des cellules végétales, de préférence des cellules de *Medicago truncatula.*

Dans un mode de réalisation, la demande décrit aussi un procédé de détection et d'identification d'un microARN tel que défini ci-dessus, dans lequel ladite cellule eucaryote, et ladite cellule eucaryote de même type que la susdite cellule eucaryote déterminée, utilisées à l'étape b) sont des cellules animales, de préférence des cellules de drosophile.

Dans un mode de réalisation, la demande décrit aussi un procédé de détection et d'identification d'un microARN tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).

Dans un mode de réalisation, la demande décrit aussi un procédé de détection et d'identification d'un microARN tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.

Dans un mode de réalisation, la demande décrit aussi un procédé de détection et d'identification d'un microARN tel que défini ci-dessus, dans lequel l'accumulation dudit microARN est déterminée en mettant en oeuvre une RT-PCR quantitative ou un Northern blot.

Dans un mode de réalisation, la demande décrit aussi un procédé de détection et d'identification d'un microARN tel que défini ci-dessus, dans lequel l'accumulation dudit microARN est déterminée en mettant en oeuvre une puce à ADN ou à ARN.

Dans un autre aspect, la description concerne un miPEP tel qu'obtenu par la mise en oeuvre du procédé tel que défini ci-dessus.

Plus particulièrement, la description concerne un miPEP codé par une séquence nucléotidique telle qu'obtenue par la mise en oeuvre du procédé tel que défini ci-dessus. En d'autres termes, l'invention concerne un miPEP codé par une séquence nucléotidique détectée et identifiée par la mise en oeuvre du procédé tel que défini ci-dessus.

Un autre aspect concerne également un miPEP de 3 à 100 acides aminés, en particulier de 4 à 100 acides aminés, notamment de 4 à 60 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote.

Un autre aspect concerne également un miPEP de 4 à 100 acides aminés, ou de 3 acides aminés, codé par une séquence nucléotidique contenue dans le transcrit primaire d'un microARN, ledit miPEP étant capable de moduler l'accumulation dudit microARN dans une cellule eucaryote.

Un autre aspect concerne également un miPEP de 3 acides aminés, codé par une séquence nucléotidique contenue dans le transcrit primaire d'un microARN, ledit miPEP étant capable de moduler l'accumulation dudit microARN dans une cellule eucaryote.

En particulier, le miPEP tel que défini est codé par un miORF de 15 à 303 nucléotides et a une taille de 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 acides aminés, en particulier 5, 8, 10, 18, 19, 23, 37, 50 ou 59 acides aminés.

En particulier, le miPEP a une taille allant de 4 à 10 acides aminés, 4 à 20 acides aminés, 4 à 30 acides aminés, 4 à 40 acides aminés, 4 à 50 acides aminés, 4 à 60 acides aminés, 4 à 70 acides aminés, 4 à 80 acides aminés, 4 à 90 acides aminés, ou 4 à 100 acides aminés.

Par ailleurs, il convient de noter que plusieurs miORFS peuvent être identifiés sur le transcrit primaire d'un microARN, indiquant qu'un transcrit primaire de microARN peut potentiellement coder plusieurs miPEPs.

Il convient également de noter que l'effet d'un miPEP est généralement spécifique d'un seul microARN, à savoir celui résultant du transcrit primaire codant ledit miPEP.

La modulation du microARN par ledit miPEP peut être mise en évidence après observation d'une variation de quantités de microARN entre les cellules en présence et en absence de miPEP.

Dans un mode de réalisation, la description concerne un miPEP tel que défini ci-dessus, ladite séquence nucléotidique étant contenue dans la partie 5' ou 3' dudit transcrit primaire d'un microARN, de préférence dans la partie 5'.

Dans un mode de réalisation, la description concerne un miPEP tel que défini ci-dessus, ladite séquence nucléotidique correspondant au premier cadre ouvert de lecture présent sur ledit transcrit primaire d'un microARN.

Dans un mode de réalisation, la description concerne un miPEP tel que défini ci-dessus, ledit miPEP possédant un point isoélectrique basique, de préférence supérieur à 8.

Dans un mode de réalisation, la description concerne un miPEP tel que défini ci-dessus, ledit miPEP possédant un point isoélectrique acide.

Dans un mode de réalisation, l'invention concerne un miPEP tel que défini ci-dessus, ledit miPEP étant choisi parmi le groupe de peptides consistant en SEQ ID NO : 1 à SEQ ID NO : 104, SEQ ID NO : 375 à SEQ ID NO : 386, et SEQ ID NO : 355 (Table 1).

Dans un mode de réalisation, l'invention concerne un miPEP tel que défini ci-dessus, ledit miPEP étant choisi parmi le groupe de peptides consistant en :
- SEQ ID NO : 1 à SEQ ID NO : 104 et SEQ ID NO : 355,
- SEQ ID NO : 375 à SEQ ID NO : 386, ou
- SEQ ID NO : 424.

Dans un mode de réalisation, l'invention concerne un miPEP tel que défini ci-dessus, consistant en la séquence d'acides aminés MVT.

Dans un autre aspect, l'invention concerne une molécule d'acide nucléique codant un miPEP tel que défini ci-dessus.

Dans un mode de réalisation, l'invention concerne une molécule d'acide nucléique telle que définie ci-dessus, ladite molécule étant choisie parmi le groupe d'acides nucléiques consistant en SEQ ID NO : 105 à SEQ ID NO 208, SEQ ID NO : 387 à SEQ ID NO : 399 et SEQ ID NO : 356 (Table 2).

Dans un mode de réalisation, l'invention concerne une molécule d'acide nucléique telle que définie ci-dessus, ladite molécule étant choisie parmi le groupe d'acides nucléiques consistant en :
- SEQ ID NO : 105 à SEQ ID NO 208 et SEQ ID NO : 356,
- SEQ ID NO : 387 à SEQ ID NO : 399, ou
- SEQ ID NO : 425.

Dans un mode de réalisation particulier, l'invention concerne le MtmiPEP171b1 (SEQ ID NO : 59) codé par la séquence nucléotidique (SEQ ID NO : 163) contenue dans le transcrit primaire du miR171b (SEQ ID NO : 319), ledit MtmiPEP171b1 étant capable de moduler l'accumulation dudit miR171b dans une cellule eucaryote.

Dans un mode de réalisation particulier, l'invention concerne le AtmiPEP164a1 (SEQ ID NO : 24) codé par la séquence nucléotidique (SEQ ID NO : 128) contenue dans le transcrit primaire du miR164a (SEQ ID NO : 297), ledit AtmiPEP164a1 étant capable de moduler l'accumulation dudit miR164a dans une cellule eucaryote.

Dans un mode de réalisation particulier, l'invention concerne le AtmiPEP165a (SEQ ID NO : 43) codé par la séquence nucléotidique (SEQ ID NO : 147) contenue dans le transcrit primaire du miR165a (SEQ ID NO : 305), ledit miPEP165a étant capable de moduler l'accumulation dudit miR165a dans une cellule eucaryote.

Dans un mode de réalisation particulier, l'invention concerne le AtmiPEP319a1 (SEQ ID NO : 76) codé par la séquence nucléotidique (SEQ ID NO : 180) contenue dans le transcrit primaire du miR319a (SEQ ID NO : 331), ledit AtmiPEP319a1 étant capable de moduler l'accumulation dudit miR319a dans une cellule eucaryote.

Dans un mode de réalisation particulier, l'invention concerne le AtmiPEP319a2 (SEQ ID NO : 77) codé par la séquence nucléotidique (SEQ ID NO : 181) contenue dans le transcrit primaire du miR319a (SEQ ID NO : 331), ledit AtmiPEP319a2 étant capable de moduler l'accumulation dudit miR319a dans une cellule eucaryote.

Dans un mode de réalisation particulier, l'invention concerne le AtmiPEP160b1 (SEQ ID NO : 14) codé par la séquence nucléotidique (SEQ ID NO : 118) contenue dans le transcrit primaire du miR160b (SEQ ID NO : 291), ledit AtmiPEP160b1 étant capable de moduler l'accumulation dudit miR160b dans une cellule eucaryote.

Dans un mode de réalisation particulier, l'invention concerne le MtmiPEP169d (SEQ ID NO : 424) codé par la séquence nucléotidique (SEQ ID NO : 425) contenue dans le transcrit primaire du miR169d (SEQ ID NO : 427), ledit MtmiPEP169d étant capable de moduler l'accumulation dudit miR169d dans une cellule eucaryote.

Dans un mode de réalisation particulier, l'invention concerne le MtmiPEP171e (SEQ ID NO : 63) codé par la séquence nucléotidique (SEQ ID NO : 167) contenue dans le transcrit primaire du miR171e (SEQ ID NO : 322), ledit MtmiPEP171e étant capable de moduler l'accumulation dudit miR171e dans une cellule eucaryote.

Dans un mode de réalisation particulier, l'invention concerne le DmmiPEPla (SEQ ID NO : 102) codé par la séquence nucléotidique (SEQ ID NO : 206) contenue dans le transcrit primaire du miR1 (SEQ ID NO: 353), ledit dmmiPEP1a étant capable de moduler l'accumulation dudit miR1 dans une cellule eucaryote.

Dans un mode de réalisation particulier, l'invention concerne le DmmiPEP1b (SEQ ID NO : 103) codé par la séquence nucléotidique (SEQ ID NO : 207) contenue dans le transcrit primaire du miR1 (SEQ ID NO: 353), ledit dmmiPEP1b étant capable de moduler l'accumulation dudit miR1 dans une cellule eucaryote.

Dans un mode de réalisation particulier, l'invention concerne le dmmiPEP8 (SEQ ID NO : 104) codé par la séquence nucléotidique (SEQ ID NO : 208) contenue dans le transcrit primaire du miR8 (SEQ ID NO : 354), ledit dmmiPEP8 étant capable de moduler l'accumulation dudit miR8 dans une cellule eucaryote.

Dans un mode de réalisation particulier, l'invention concerne le HsmiPEP155 (SEQ ID NO : 355) codé par la séquence nucléotidique (SEQ ID NO : 356) contenue dans le transcrit primaire du miR155 (SEQ ID NO : 358), ledit HsmiPEP155 étant capable de moduler l'accumulation dudit miR155 dans une cellule eucaryote.

Dans un autre aspect, la description concerne un peptide isolé, ou un peptide isolé et purifié, ou un peptide synthétique ou un peptide recombinant, comprenant ou consistant en une séquence identique à celle d'un miPEP, ledit miPEP étant notamment présent naturellement chez une plante, ou chez un animal, tel que l'Homme.

Dans autre aspect, l'invention concerne un vecteur comprenant au moins une molécule d'acide nucléique tel que définie ci-dessus.

Dans un autre aspect, l'invention concerne l'utilisation d'au moins :
- un miPEP tel que défini ci dessus ,
- un acide nucléique codant ledit miPEP, ou
- un vecteur contenant ledit acide nucléique,
pour moduler l'expression d'un gène dans une cellule eucaryote végétale déterminée, ladite cellule eucaryote végétale déterminée étant capable d'exprimer un microARN, dont le transcrit primaire contient au moins une séquence nucléotidique codant ledit au moins un miPEP et dont l'accumulation est modulée par ledit au moins un miPEP,
l'expression dudit gène étant régulée par ledit microARN, ledit miPEP étant choisi parmi le groupe de peptides consistant en :
SEQ ID NO : 59, SEQ ID NO : 60, SEQ ID NO : 24, SEQ ID NO : 25, SEQ ID NO : 26, SEQ ID NO : 43, SEQ ID NO : 76,
ledit acide nucléique étant choisi parmi le groupe d'acides nucléiques consistant en :
SEQ ID NO : 163, SEQ ID NO : 164, SEQ ID NO : 128, SEQ ID NO : 129, SEQ ID NO : 130, SEQ ID NO : 147, SEQ ID NO : 180,
ledit microARN étant choisi parmi le groupe d'acides nucléiques consistant en :
SEQ ID NO : 319, SEQ ID NO : 297, SEQ ID NO : 305, SEQ ID NO : 331.

La description concerne également l'utilisation d'au moins :
- un miPEP tel que défini ci-dessus,
- un acide nucléique codant ledit miPEP, ou
- un vecteur contenant ledit acide nucléique,
pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP,
l'expression dudit au moins un gène étant régulée par ledit microARN.

Dans un autre aspect, la description concerne également l'utilisation d'au moins :
- un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote,
- un acide nucléique codant ledit miPEP, ou
- un vecteur contenant ledit acide nucléique,
pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP,
l'expression dudit au moins un gène étant régulée par ledit microARN.

Dans un autre aspect, la description concerne également l'utilisation d'au moins :
- un miPEP de 4 à 100 acides aminés, ou de 3 acides aminés, codé par une séquence nucléotidique contenue dans le transcrit primaire d'un microARN, ledit miPEP étant capable de moduler l'accumulation dudit microARN dans une cellule eucaryote,
- un acide nucléique codant ledit miPEP, ou
- un vecteur contenant ledit acide nucléique,
pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP,
l'expression dudit au moins un gène étant régulée par ledit microARN.

Dans un autre aspect, la description concerne également l'utilisation d'au moins :
- un miPEP de 3 acides aminés, codé par une séquence nucléotidique contenue dans le transcrit primaire d'un microARN, ledit miPEP étant capable de moduler l'accumulation dudit microARN dans une cellule eucaryote,
- un acide nucléique codant ledit miPEP, ou
- un vecteur contenant ledit acide nucléique,
pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP,
l'expression dudit au moins un gène étant régulée par ledit microARN.

L'invention repose sur l'observation surprenante faite par les Inventeurs qu'il est possible de moduler l'expression d'un ou plusieurs gènes cibles d'un même microARN en modulant l'accumulation dudit microARN à l'aide d'un miPEP.

Dans un mode de réalisation, l'invention concerne l'utilisation telle que définie ci-dessus dans laquelle ladite cellule eucaryote déterminée est une cellule végétale.

Dans un mode de réalisation, l'invention concerne l'utilisation telle que définie ci-dessus dans laquelle ladite cellule eucaryote déterminée est une cellule végétale d'une plante crucifère telle *qu'Arabidopsis thaliana*, d'une plante légumineuse telle que *Glycine max* (soja), *Medicago truncatula* et *Medicago sativa* (luzerne) ou d'une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, la description concerne l'utilisation telle que définie ci-dessus dans laquelle ladite cellule eucaryote déterminée est une cellule animale, notamment humaine.

Dans un mode de réalisation, la description concerne l'utilisation telle que définie ci-dessus dans laquelle ladite cellule eucaryote déterminée est une cellule animale, notamment humaine, ledit miPEP n'étant pas utilisé pour le traitement chirurgical ou thérapeutique du corps humain ou animal, ni pour modifier l'identité génétique de l'être humain.

Dans un mode de réalisation, la description concerne l'utilisation telle que définie ci-dessus dans laquelle ladite cellule eucaryote déterminée est une cellule animale, ledit miPEP étant utilisé pour le traitement chirurgical ou thérapeutique du corps humain ou animal.

Dans un mode de réalisation, l'invention concerne l'utilisation telle que définie ci-dessus dans laquelle ledit microARN et ledit gène sont d'origine endogène dans ladite cellule eucaryote déterminée.

Dans un mode de réalisation, l'invention concerne l'utilisation telle que définie ci-dessus dans laquelle ledit microARN et ledit gène sont d'origine exogène dans ladite cellule eucaryote déterminée, ladite cellule eucaryote déterminée contenant au moins un vecteur permettant l'expression dudit microARN et dudit gène.

Dans l'invention, les expressions *« d'origine endogène »* et *« d'origine exogène »* sont utilisées pour distinguer lesdits microARNs et/ou les gènes d'espèces différentes, étant donné la conservation des séquences entre espèces.

Ainsi, le terme *« d'origine endogène »* indique que le microARN et/ou le gène peuvent être présents de manière naturelle dans la cellule en question. De manière artificielle, d'autres copies du microARN et/ou du gène d'origine endogène peuvent néanmoins être ajoutées dans la cellule en question, comme par exemple par clonage.

A l'inverse, le terme *« d'origine exogène »* indique que le microARN et/ou le gène ne sont jamais présents naturellement dans la cellule en question. Il s'agit d'un microARN et/ou d'un gène identifié dans un autre type cellulaire ou dans un organisme d'une autre espèce, ce microARN et/ou ce gène sont donc nécessairement introduits artificiellement dans la cellule en question.

Dans l'invention, une cellule transformée génétiquement peut donc contenir 2 groupes de microARNs et/ou de gènes potentiellement proches en termes de séquence, l'un d'origine endogène et l'autre d'origine exogène.

Dans un mode de réalisation, l'invention concerne l'utilisation telle que définie ci-dessus dans laquelle le transcrit primaire du miARN et ledit gène sont d'origine exogène dans ladite cellule eucaryote déterminée, ladite cellule eucaryote déterminée contenant au moins un vecteur permettant l'expression du transcrit primaire du microARN.

Dans un mode de réalisation, l'invention concerne l'utilisation telle que définie ci-dessus dans laquelle le transcrit primaire du miARN est codé par un vecteur introduit articiellement dans la cellule.

Dans un mode de réalisation, la description concerne l'utilisation telle que définie ci-dessus dans laquelle ledit miPEP est choisi parmi le groupe de peptides consistant en SEQ ID NO : 1 à SEQ ID NO : 104, SEQ ID NO : 375 à SEQ ID NO : 386 et SEQ ID NO : 355 (Table 1).

Dans un mode de réalisation, la description concerne l'utilisation telle que définie ci-dessus, dans laquelle ledit miPEP est choisi parmi le groupe de peptides consistant en :
- SEQ ID NO : 1 à SEQ ID NO : 104 et SEQ ID NO : 355,
- SEQ ID NO : 375 à SEQ ID NO : 386, ou
- SEQ ID NO : 424.

Dans un mode de réalisation, la description concerne l'utilisation telle que définie ci-dessus dans laquelle ledit miPEP est choisi parmi le MtmiPEP171b1 (SEQ ID NO: 59), le AtmiPEP164a1 (SEQ ID NO : 24), le AtmiPEP165a (SEQ ID NO : 43), le AtmiPEP319a1 (SEQ ID NO : 76) et le AtmiPEP319a2 (SEQ ID NO : 77).

Dans un mode de réalisation, la description concerne l'utilisation telle que définie ci-dessus dans laquelle ledit miPEP est choisi parmi : le MtmiPEP171b1 (SEQ ID NO : 59), le AtmiPEP164a1 (SEQ ID NO : 24), le AtmiPEP165a (SEQ ID NO : 43), le AtmiPEP319a1 (SEQ ID NO : 76), le AtmiPEP319a2 (SEQ ID NO : 77), le AtmiPEP160b1 (SEQ ID NO : 14), le MtmiPEP171e (SEQ ID NO : 63) et le MtmiPEP169d (SEQ ID NO : 424).

Dans un mode de réalisation, l'invention concerne l'utilisation telle que définie ci-dessus dans laquelle ledit miPEP est choisi parmi le MtmiPEP171b1 (SEQ ID NO : 59), le AtmiPEP164a1 (SEQ ID NO : 24), le AtmiPEP165a (SEQ ID NO : 43) et le AtmiPEP319a1 (SEQ ID NO : 76).

Dans un mode de réalisation, la description concerne l'utilisation telle que définie ci-dessus dans laquelle ledit miPEP est choisi parmi le DmmiPEP1a (SEQ ID NO : 102), le DmmiPEP1b (SEQ ID NO : 103) et le DmmiPEP8 (SEQ ID NO : 104).

Dans un mode de réalisation, la description concerne l'utilisation telle que définie ci-dessus dans laquelle ledit miPEP est le HsmiPEP155a (SEQ ID NO : 355).

Dans un mode de réalisation, la description concerne l'utilisation telle que définie ci-dessus dans laquelle ledit acide nucléique est choisi parmi le groupe d'acides nucléiques consistant en SEQ ID NO : 105 à SEQ ID NO : 208 et SEQ ID NO : 356 (Table 2).

Dans un mode de réalisation, la description concerne l'utilisation telle que définie ci-dessus dans laquelle ledit acide nucléique est choisi parmi le groupe d'acides nucléiques consistant en :
- SEQ ID NO : 105 à SEQ ID NO : 208 et SEQ ID NO : 356,
- SEQ ID NO : 387 à SEQ ID NO : 399, ou
- SEQ ID NO : 425.

Dans un mode de réalisation, la description concerne l'utilisation telle que définie ci-dessus dans laquelle ledit acide nucléique est choisi parmi le miORF171b (SEQ ID NO : 163), le miORF164a1 (SEQ ID NO : 128), le miORF165a (SEQ ID NO : 147), le miORF319a1 (SEQ ID NO : 180) et le miORF319a2 (SEQ ID NO : 181).

Dans un mode de réalisation, la description concerne l'utilisation telle que définie ci-dessus dans laquelle ledit acide nucléique est choisi parmi le miORF171b (SEQ ID NO : 163), le miORF164a1 (SEQ ID NO : 128), le miORF165a (SEQ ID NO : 147), le miORF319a1 (SEQ ID NO : 180), le miORF319a2 (SEQ ID NO : 181), le miORF160b1 (SEQ ID NO : 118), le miORF169d (SEQ ID NO : 425) et le miORF171e (SEQ ID NO : 167).

Dans un mode de réalisation, l'invention concerne l'utilisation telle que définie ci-dessus dans laquelle ledit acide nucléique est choisi parmi le miORF171b (SEQ ID NO : 163), le miORF164a1 (SEQ ID NO : 128), le miORF165a (SEQ ID NO : 147) et le miORF319a1 (SEQ ID NO : 180).

Dans un mode de réalisation, la description concerne l'utilisation telle que définie ci-dessus dans laquelle ledit acide nucléique est choisi parmi le miORF1a (SEQ ID NO : 206), le miORF1b (SEQ ID NO : 207) et le miORF8 (SEQ ID NO : 208).

Dans un mode de réalisation, la description concerne l'utilisation telle que définie ci-dessus dans laquelle ledit acide nucléique est choisi parmi le miORF155 (SEQ ID NO : 356).

Dans un mode de réalisation, la description concerne l'utilisation telle que définie ci-dessus dans laquelle ledit microARN est choisi parmi le groupe d'acides nucléiques consistant en SEQ ID NO : 282 à SEQ ID NO : 354 et SEQ ID NO : 358.

Dans un mode de réalisation, la description concerne l'utilisation telle que définie ci-dessus dans laquelle ledit microARN est choisi parmi le groupe d'acides nucléiques consistant en :
- SEQ ID NO : 282 à SEQ ID NO : 354 et SEQ ID NO : 358,
- SEQ ID NO : 412 à SEQ ID NO : 423, ou
- SEQ ID NO : 427.

Dans un mode de réalisation, l'invention concerne l'utilisation telle que définie ci-dessus dans laquelle ledit microARN est choisi parmi le miR171b (SEQ ID NO : 319), le miR165a (SEQ ID NO : 305) et le miR319a (SEQ ID NO : 331).

Dans un mode de réalisation, la description concerne l'utilisation telle que définie ci-dessus dans laquelle ledit microARN est choisi parmi le miR171b (SEQ ID NO : 319), le miR164a (SEQ ID NO : 297), le miR165a (SEQ ID NO : 305), le miR319a (SEQ ID NO : 331), le miR160b (SEQ ID NO : 291), le miR171e (SEQ ID NO : 322) et le miR169d (SEQ ID NO : 427).

Dans un mode de réalisation, l'invention concerne l'utilisation telle que définie ci-dessus dans laquelle ledit microARN est choisi parmi le miR171b (SEQ ID NO : 319), le miR164a (SEQ ID NO : 297), le miR165a (SEQ ID NO : 305) et le miR319a (SEQ ID NO : 331).

Dans un mode de réalisation, la description concerne l'utilisation telle que définie ci-dessus dans laquelle ledit microARN est choisi parmi le miR1a (SEQ ID NO : 353) et le miR8 (SEQ ID NO : 354).

Dans un mode de réalisation, la description concerne l'utilisation telle que définie ci-dessus dans laquelle ledit microARN est choisi parmi le miR155 (SEQ ID NO : 358).

Dans un autre aspect, l'invention concerne un procédé de modulation de l'expression d'un gène régulée par un microARN dans une cellule eucaryote végétale,
comprenant la mise en oeuvre d'une étape d'accumulation d'un miPEP dans ladite cellule eucaryote,
ledit miPEP étant choisi parmi le groupe de peptides consistant en : SEQ ID NO : 59, SEQ ID NO : 60, SEQ ID NO : 24, SEQ ID NO : 25, SEQ ID NO : 26, SEQ ID NO : 43, SEQ ID NO : 76
dans lequel, l'accumulation dudit miPEP dans ladite cellule eucaryote induit une modulation de l'expression dudit gène par rapport à l'expression dudit gène sans accumulation dudit miPEP.

La description concerne en particulier un procédé de modulation de l'expression d'un gène régulée par un microARN dans une cellule eucaryote,
comprenant la mise en oeuvre d'une étape d'accumulation d'un miPEP dans ladite cellule eucaryote,
ledit miPEP ayant :
- une taille de 3 à 100 acides aminés, de préférence 4 à 20 acides aminés, et
- une séquence peptidique identique à celle codée par une séquence nucléotidique contenue dans le transcrit primaire d'un microARN régulant l'expression dudit gène, et
- étant capable de moduler l'accumulation dudit microARN,
dans lequel, l'accumulation dudit miPEP dans ladite cellule eucaryote induit une modulation de l'expression dudit gène par rapport à l'expression dudit gène sans accumulation dudit miPEP.

Dans un autre aspect, la description concerne en particulier un procédé de modulation de l'expression d'un gène régulée par un microARN dans une cellule eucaryote,
comprenant la mise en oeuvre d'une étape d'accumulation d'un miPEP dans ladite cellule eucaryote,
ledit miPEP ayant :
- une taille de 4 à 100 acides aminés, ou de 3 acides aminés, et
- une séquence peptidique identique à celle codée par une séquence nucléotidique contenue dans le transcrit primaire d'un microARN régulant l'expression dudit gène, et
- étant capable de moduler l'accumulation dudit microARN,
dans lequel, l'accumulation dudit miPEP dans ladite cellule eucaryote induit une modulation de l'expression dudit gène par rapport à l'expression dudit gène sans accumulation dudit miPEP.

Dans un autre aspect, la description concerne en particulier un procédé de modulation de l'expression d'un gène régulée par un microARN dans une cellule eucaryote,
comprenant la mise en oeuvre d'une étape d'accumulation d'un miPEP dans ladite cellule eucaryote,
ledit miPEP ayant :
- une taille de 3 acides aminés, et
- une séquence peptidique identique à celle codée par une séquence nucléotidique contenue dans le transcrit primaire d'un microARN régulant l'expression dudit gène, et
- étant capable de moduler l'accumulation dudit microARN,
dans lequel, l'accumulation dudit miPEP dans ladite cellule eucaryote induit une modulation de l'expression dudit gène par rapport à l'expression dudit gène sans accumulation dudit miPEP.

En particulier, la description concerne un procédé de modulation de l'expression d'un gène régulée par un microARN dans une cellule eucaryote,
comprenant la mise en oeuvre d'une étape d'accumulation d'un miPEP dans ladite cellule eucaryote,
ledit miPEP ayant :
- une taille de 4 à 100 acides aminés, de préférence 4 à 20 acides aminés, et
- une séquence peptidique identique à celle codée par une séquence nucléotidique contenue dans le transcrit primaire d'un microARN régulant l'expression dudit gène, et
- étant capable de moduler l'accumulation dudit microARN,
dans lequel, l'accumulation dudit miPEP dans ladite cellule eucaryote induit une modulation de l'expression dudit gène par rapport à l'expression dudit gène sans accumulation dudit miPEP.

Dans un mode de réalisation, la demande décrit aussi un procédé de modulation de l'expression d'un gène tel que défini ci-dessus, dans lequel l'accumulation dudit miPEP dans la cellule résulte :
- de l'introduction dans la cellule d'un acide nucléique codant ledit miPEP, ou
- de l'introduction dans la cellule dudit miPEP.

Dans un mode de réalisation, l'invention concerne un procédé de modulation de l'expression d'un gène tel que défini ci-dessus dans lequel ladite cellule eucaryote est une cellule végétale.

Dans un mode de réalisation, la description concerne un procédé de modulation de l'expression d'un gène tel que défini ci-dessus dans lequel ladite cellule eucaryote est une cellule animale et notamment humaine.

Dans un mode de réalisation, la description concerne un procédé de modulation de l'expression d'un gène tel que défini ci-dessus dans lequel ladite cellule eucaryote est une cellule animale et notamment humaine, ledit procédé n'étant pas utilisé pour le traitement chirurgical ou thérapeutique du corps humain ou animal, ni pour modifier l'identité génétique de l'être humain.

Dans un mode de réalisation, la demande décrit aussi un procédé de modulation de l'expression d'un gène tel que défini ci-dessus dans lequel ledit microARN et ledit gène sont d'origine endogène dans ladite cellule eucaryote.

Dans un mode de réalisation, la demande décrit aussi un procédé de modulation de l'expression d'un gène tel que défini ci-dessus dans lequel ledit microARN et ledit gène sont d'origine exogène dans ladite cellule eucaryote, ladite cellule eucaryote contenant au moins un vecteur permettant l'expression dudit microARN et dudit gène.

Dans un mode de réalisation, la description concerne un procédé de modulation de l'expression d'un gène tel que défini ci-dessus dans lequel ledit miPEP est choisi parmi le groupe de peptides consistant en SEQ ID NO : 1 à SEQ ID NO : 104, SEQ ID NO : 375 à SEQ ID NO : 386 et SEQ ID NO : 355.

Dans un mode de réalisation, la description concerne un procédé de modulation de l'expression d'un gène tel que défini ci-dessus dans lequel ledit miPEP est choisi parmi le groupe de peptides consistant en:
- SEQ ID NO : 1 à SEQ ID NO : 104 et SEQ ID NO : 355,
- SEQ ID NO : 375 à SEQ ID NO : 386, ou
- SEQ ID NO : 424.

Dans un mode de réalisation particulier, l'invention concerne un procédé de modulation de l'expression d'un gène régulée par le miR171b (SEQ ID NO : 319) dans une cellule eucaryote,
comprenant la mise en oeuvre d'une étape d'accumulation du MtmiPEP171b1 (SEQ ID NO : 59) dans ladite cellule eucaryote,
dans lequel, l'accumulation dudit MtmiPEP171b1 dans ladite cellule eucaryote induit une modulation de l'expression dudit gène par rapport à l'expression dudit gène sans accumulation dudit MtmiPEP171b1.

Dans un mode de réalisation particulier, l'invention concerne un procédé de modulation de l'expression d'un gène régulée par le miR171b (SEQ ID NO : 319) dans une cellule eucaryote,
comprenant la mise en oeuvre d'une étape d'accumulation du MtmiPEP171b1 (SEQ ID NO : 59) dans ladite cellule eucaryote,
dans lequel, l'accumulation dudit MtmiPEP171b1 dans ladite cellule eucaryote induit une modulation de l'expression dudit gène par rapport à l'expression dudit gène sans accumulation dudit MtmiPEP171b1,
dans lequel ledit gène est choisi parmi les gènes *HAM1* (accession n° MtGI9- TC114268) et *HAM2* (accession n° MtGI9- TC120850) (numéros d'accession selon la banque de donnees *Medicago truncatula* Gene Expression Atlas « MtGEA »).

Dans un mode de réalisation particulier, l'invention concerne un procédé de modulation de l'expression d'un gène régulée par le miR164a (SEQ ID NO : 297) dans une cellule eucaryote,
comprenant la mise en oeuvre d'une étape d'accumulation du AtmiPEP165a1 (SEQ ID NO : 24) dans ladite cellule eucaryote,
dans lequel, l'accumulation dudit AtmiPEP164a1 dans ladite cellule eucaryote induit une modulation de l'expression dudit gène par rapport à l'expression dudit gène sans accumulation dudit AtmiPEP164a1.

Dans un mode de réalisation particulier, l'invention concerne un procédé de modulation de l'expression d'un gène régulée par le miR165a (SEQ ID NO : 305) dans une cellule eucaryote,
comprenant la mise en oeuvre d'une étape d'accumulation du AtmiPEP165a (SEQ ID NO : 43) dans ladite cellule eucaryote,
dans lequel, l'accumulation dudit AtmiPEP165a dans ladite cellule eucaryote induit une modulation de l'expression dudit gène par rapport à l'expression dudit gène sans accumulation dudit AtmiPEP165a.

Dans un mode de réalisation particulier, l'invention concerne un procédé de modulation de l'expression d'un gène régulée par le miR319a (SEQ ID NO : 331) dans une cellule eucaryote,
comprenant la mise en oeuvre d'une étape d'accumulation du AtmiPEP319a1 (SEQ ID NO : 76) dans ladite cellule eucaryote,
dans lequel, l'accumulation dudit AtmiPEP319a1 dans ladite cellule eucaryote induit une modulation de l'expression dudit gène par rapport à l'expression dudit gène sans accumulation dudit AtmiPEP319a1.

Dans un mode de réalisation particulier, la description concerne un procédé de modulation de l'expression d'un gène régulée par le miR319a (SEQ ID NO : 331) dans une cellule eucaryote,
comprenant la mise en oeuvre d'une étape d'accumulation du AtmiPEP319a2 (SEQ ID NO : 77) dans ladite cellule eucaryote,
dans lequel, l'accumulation dudit AtmiPEP319a2 dans ladite cellule eucaryote induit une modulation de l'expression dudit gène par rapport à l'expression dudit gène sans accumulation dudit AtmiPEP319a2.

Dans un mode de réalisation particulier, la description concerne un procédé de modulation de l'expression d'un gène régulée par le miR160b (SEQ ID NO : 291) dans une cellule eucaryote,
comprenant la mise en oeuvre d'une étape d'accumulation du AtmiPEP160b1 (SEQ ID NO : 14) dans ladite cellule eucaryote,
dans lequel, l'accumulation dudit AtmiPEP160b1 dans ladite cellule eucaryote induit une modulation de l'expression dudit gène par rapport à l'expression dudit gène sans accumulation dudit AtmiPEP160b1.

Dans un mode de réalisation particulier, la description concerne un procédé de modulation de l'expression d'un gène régulée par le miR169d (SEQ ID NO : 427) dans une cellule eucaryote,
comprenant la mise en oeuvre d'une étape d'accumulation du MtmiPEP169d (SEQ ID NO : 424) dans ladite cellule eucaryote,
dans lequel, l'accumulation dudit MtmiPEP169d dans ladite cellule eucaryote induit une modulation de l'expression dudit gène par rapport à l'expression dudit gène sans accumulation dudit MtmiPEP169d.

Dans un mode de réalisation particulier, la description concerne un procédé de modulation de l'expression d'un gène régulée par le miR171e (SEQ ID NO : 322) dans une cellule eucaryote,
comprenant la mise en oeuvre d'une étape d'accumulation du MtmiPEP171e (SEQ ID NO : 63) dans ladite cellule eucaryote,
dans lequel, l'accumulation dudit MtmiPEP171e dans ladite cellule eucaryote induit une modulation de l'expression dudit gène par rapport à l'expression dudit gène sans accumulation dudit MtmiPEP171e.

Dans un mode de réalisation particulier, la description concerne un procédé de modulation de l'expression d'un gène régulée par le miR1 (SEQ ID NO : 353) dans une cellule eucaryote,
comprenant la mise en oeuvre d'une étape d'accumulation du DmmiPEP1a (SEQ ID NO : 102) dans ladite cellule eucaryote,
dans lequel, l'accumulation dudit DmmiPEP1a dans ladite cellule eucaryote induit une modulation de l'expression dudit gène par rapport à l'expression dudit gène sans accumulation dudit DmmiPEP1a.

Dans un mode de réalisation particulier, la description concerne un procédé de modulation de l'expression d'un gène régulée par le miR1 (SEQ ID NO : 353) dans une cellule eucaryote,
comprenant la mise en oeuvre d'une étape d'accumulation du DmmiPEP1b (SEQ ID NO : 103) dans ladite cellule eucaryote,
dans lequel, l'accumulation dudit DmmiPEP1b dans ladite cellule eucaryote induit une modulation de l'expression dudit gène par rapport à l'expression dudit gène sans accumulation dudit DmmiPEP1b.

Dans un mode de réalisation particulier, la description concerne un procédé de modulation de l'expression d'un gène régulée par le miR8 (SEQ ID NO : 354) dans une cellule eucaryote,
comprenant la mise en oeuvre d'une étape d'accumulation du DmmiPEP8 (SEQ ID NO : 104) dans ladite cellule eucaryote,
dans lequel, l'accumulation dudit DmmiPEP8 dans ladite cellule eucaryote induit une modulation de l'expression dudit gène par rapport à l'expression dudit gène sans accumulation dudit DmmiPEP8.

Dans un mode de réalisation particulier, la description concerne un procédé de modulation de l'expression d'un gène régulée par le miR155 (SEQ ID NO : 358) dans une cellule eucaryote,
comprenant la mise en oeuvre d'une étape d'accumulation du hsmiPEP155 (SEQ ID NO : 355) dans ladite cellule eucaryote,
dans lequel, l'accumulation dudit hsmiPEP155 dans ladite cellule eucaryote induit une modulation de l'expression dudit gène par rapport à l'expression dudit gène sans accumulation dudit hsmiPEP155.

Dans un autre aspect, la demande décrit aussi une cellule eucaryote modifiée contenant un peptide identique à un miPEP tel que défini ci-dessus, lequel peptide est présent dans ladite cellule eucaryote indépendamment de la transcription du transcrit primaire du microARN portant la séquence nucléotidique codant ledit miPEP.

Dans l'invention, le terme *« cellule eucaryote modifiée »* signifie que ladite cellule eucaryote contient un miPEP introduit artificiellement dans la cellule, que ce soit en tant que peptide, ou par l'intermédiaire d'un vecteur codant ledit miPEP.

En plus d'un miPEP introduit artificiellement dans la cellule, une cellule eucaryote modifiée selon l'invention contient également au moins un acide nucléique correspondant au miORF codant ledit miPEP.

En plus d'un miPEP introduit artificiellement dans la cellule et du miORF, une cellule eucaryote modifiée selon l'invention contient également au moins un miR, dont le transcrit primaire contient ledit miORF.

Dans un mode de réalisation, la demande décrit aussi une cellule eucaryote modifiée telle que définie ci-dessus, dans laquelle ledit microARN est d'origine endogène.

Dans un autre mode de réalisation, la demande décrit aussi une cellule eucaryote modifiée telle que définie ci-dessus dans laquelle ledit microARN est d'origine exogène, ladite cellule eucaryote modifiée contenant un vecteur permettant l'expression dudit microARN.

Dans un mode de réalisation, la demande décrit aussi une cellule eucaryote modifiée telle que définie ci-dessus, ladite cellule étant une cellule végétale.

Dans un mode de réalisation, la demande décrit aussi une cellule eucaryote modifiée telle que définie ci-dessus, dans laquelle la dite cellule végétale est une cellule de *Medicago truncatula* ou *d'Arabidopsis thaliana*, ledit peptide est choisi parmi le groupe de peptides consistant en :
- SEQ ID NO : 1 à SEQ ID NO: 101,
- SEQ ID NO : 375 à SEQ ID NO : 386, ou
- SEQ ID NO : 424.

Dans un mode de réalisation, la demande décrit aussi une cellule eucaryote modifiée telle que définie ci-dessus, dans laquelle la dite cellule végétale est une cellule de *Medicago truncatula* ou *d'Arabidopsis thaliana*, ledit peptide est choisi parmi le groupe de peptides consistant en SEQ ID NO : 43, SEQ ID NO : 59 et SEQ ID NO : 77.

Dans un mode de réalisation, la demande décrit aussi une cellule eucaryote modifiée telle que définie ci-dessus, dans laquelle la dite cellule végétale est une cellule de *Medicago truncatula* ou *d'Arabidopsis thaliana*, ledit peptide est choisi parmi le groupe de peptides consistant en SEQ ID NO : 59, SEQ ID NO : 24, SEQ ID NO : 43, SEQ ID NO : 76, SEQ ID NO : 77, SEQ ID NO : 14, SEQ ID NO : 424 et SEQ ID NO : 63 Dans un mode de réalisation, la demande décrit aussi une cellule eucaryote modifiée telle que définie ci-dessus, ladite cellule étant une cellule animale.

Dans un mode de réalisation, la demande décrit aussi une cellule eucaryote modifiée telle que définie ci-dessus, dans laquelle la dite cellule animale est une cellule de drosophile et ledit peptide est choisi parmi le groupe de peptides consistant en SEQ ID NO : 102, SEQ ID NO : 103 et SEQ ID NO : 104.

Dans un mode de réalisation, la demande décrit aussi une cellule eucaryote modifiée telle que définie ci-dessus, dans laquelle la dite cellule animale est une cellule humaine et ledit peptide consiste en SEQ ID NO : 355.

Dans un autre aspect, la demande décrit aussi une plante comprenant au moins une cellule eucaryote modifiée telle que définie ci-dessus.

Dans un autre aspect, la demande décrit aussi également un organisme animal non humain comprenant au moins une cellule eucaryote modifiée telle que définie ci-dessus.

Dans un autre aspect, la demande décrit aussi une composition comprenant au moins :
- un miPEP tel que défini ci-dessus,
- un acide nucléique codant ledit miPEP, ou
- un vecteur contenant ledit acide nucléique.

Dans un autre aspect, l'invention concerne une composition comprenant au moins un miPEP choisi parmi le groupe consistant en :
- SEQ ID NO : 1 à SEQ ID NO: 101,
- SEQ ID NO : 375 à SEQ ID NO : 386, ou
- SEQ ID NO : 424.

Dans un autre aspect, l'invention concerne une composition pesticide comprenant au moins :
- un miPEP tel que défini ci-dessus,
- un acide nucléique codant ledit miPEP, ou
- un vecteur contenant ledit acide nucléique.

Dans un autre aspect, l'invention concerne une composition phytopharmaceutique comprenant au moins :
- un miPEP tel que défini ci-dessus,
- un acide nucléique codant ledit miPEP, ou
- un vecteur contenant ledit acide nucléique.

Dans un autre aspect, l'invention concerne une composition élicitrice comprenant au moins :
- un miPEP tel que défini ci-dessus,
- un acide nucléique codant ledit miPEP, ou
- un vecteur contenant ledit acide nucléique.

Par « composition élicitrice », on désigne une composition capable de conférer à la plante une meilleure aptitude à la symbiose ou une meilleure résistance à différents stress, qu'ils soient de nature thermiques, hydriques ou chimiques.

A cet effet, l'invention concerne également des compositions agissant sur la croissance (inhibition de la croissance ou au contraire augmentation de la croissance) et la physiologie (meilleure aptitude à mycorhizer, noduler, meilleure tolérance à différents stress) de la plante.

En particulier, l'invention concerne des compositions pour favoriser la croissance des plantes.

Dans un autre aspect, l'invention concerne une composition herbicide comprenant au moins :
- un miPEP tel que défini ci-dessus,
- un acide nucléique codant ledit miPEP, ou
- un vecteur contenant ledit acide nucléique.

Dans un autre aspect, l'invention concerne une composition insecticide comprenant au moins :
- un miPEP tel que défini ci-dessus,
- un acide nucléique codant ledit miPEP, ou
- un vecteur contenant ledit acide nucléique.

Dans un autre aspect, l'invention concerne une composition, en particulier une composition phytosanitaire, comprenant le miPEP164a en tant que substance active, ledit miPEP164a consistant de préférence en SEQ ID NO : 24.

Dans un autre aspect, l'invention concerne une composition, en particulier une composition phytosanitaire, comprenant le miPEP319a en tant que substance active, ledit miPEP319a consistant de préférence en SEQ ID NO : 76.

Dans un autre aspect, l'invention concerne une composition, en particulier une composition phytosanitaire, comprenant le miPEP171b en tant que substance active, ledit miPEP171b consistant de préférence en SEQ ID NO : 59.

Dans un autre aspect, l'invention concerne une composition, en particulier une composition phytosanitaire, comprenant le miPEP165a en tant que substance active, ledit miPEP165a consistant de préférence en SEQ ID NO : 43.

Dans un autre aspect, l'invention concerne une composition, en particulier une composition phytosanitaire, comprenant le miPEP319a2 en tant que substance active, ledit miPEP319a2 consistant de préférence en SEQ ID NO : 77.

Dans un autre aspect, l'invention concerne une composition, en particulier une composition phytosanitaire, comprenant le miPEP160b1 en tant que substance active, ledit miPEP160b1 consistant de préférence en SEQ ID NO : 14.

Dans un autre aspect, l'invention concerne une composition, en particulier une composition phytosanitaire, comprenant le miPEP169d en tant que substance active, ledit miPEP169d consistant de préférence en SEQ ID NO : 424.

Dans un autre aspect, l'invention concerne une composition, en particulier une composition phytosanitaire, comprenant le miPEP171e en tant que substance active, ledit miPEP171e consistant de préférence en SEQ ID NO : 63.

Les propriétés solubles des miPEPs sont déterminées notamment par leur composition en acides aminés. Les miPEPs hydrophiles peuvent être solubilisés et conditionnés dans des solutions aqueuses, telles que l'eau. Les miPEPs hydrophobes peuvent être solubilisés et conditionnés dans des solvants, tels que les solvants organiques.

Pour un traitement des plantes par les miPEPS, les solvants organiques sont des solvants non toxiques pour les plantes en faibles quantités, c'est-à-dire qu'ils n'ont pas d'effet délétères sur le développement de la plante. De manière non limitative, les solvants organiques peuvent être choisis parmi l'acétonitrile et l'acide acétique.

Les miPEPs peuvent également être solubilisés et conditionnés dans des mélanges de solvants organiques, comme par exemple un mélange d'acétonitrile et d'acide acétique. En particulier, les miPEPs peuvent être solubilisés dans une solution comprenant 50 % d'acétonitrile, 10% d'acide acétique et 40% d'eau (volume/volume/volume).

De préférence, les miPEPs 164a et 165a sont dissous dans de l'eau, et les miPEPs 171b et 319a sont dissous dans une solution comprenant 50 % d'acétonitrile, 10% d'acide acétique et 40% d'eau (volume/volume/volume).

De manière non limitative, les compositions, les compositions pesticides, les compositions phytopharmaceutiques, les compositions herbicides et les compositions insecticides telles que définies ci-dessus peuvent comprendre 10⁻⁹ M à 10⁻⁴ M de miPEP, notamment 10⁻⁹ M, 10⁻⁸ M, 10⁻⁷ M, 10⁻⁶ M, 10⁻⁵ M ou 10⁻⁴ M de miPEP.

Des compositions plus ou moins concentrées peuvent également être prévues selon les applications envisagées. Par exemple, des compositions comprenant 10⁻¹ M à 10⁻³ M de miPEP, notamment 10⁻¹ M, 10⁻² M ou 10⁻³ M de miPEP, peuvent être envisagées dans le cas où le miPEP doit être administré à la plante par épandage.

Dans un autre aspect, l'invention concerne l'utilisation d'une composition telle que définie ci-dessus, en tant qu'herbicide pour éliminer les plantes ou ralentir leur croissance, de préférence en tant qu'herbicide spécifique d'une espèce ou d'un genre de plantes.

Dans un mode de réalisation particulier, l'invention concerne l'utilisation d'une composition comprenant au moins :
- un miPEP tel que défini selon la revendication 8 à l'exception des miPEP de séquences SEQ ID NO : 102, SEQ ID NO : 103, SEQ ID NO : 104 et SEQ ID NO : 355,
- un acide nucléique selon la revendication 9 codant ledit miPEP, à l'exception des acides nucléiques de séquences SEQ ID NO : 206, SEQ ID NO : 207, SEQ ID NO : 208 et SEQ ID NO : 356, ou
- un vecteur contenant ledit acide nucléique,
en tant qu'herbicide, pour éliminer les plantes ou ralentir leur croissance, ou
en tant que pesticide pour éliminer les organismes nuisibles des plantes ou susceptibles d'être classés comme tels.

Dans un autre aspect, la description concerne l'utilisation d'une composition telle que définie ci-dessus, en tant qu'agent phytopharmaceutique,
- pour favoriser la croissance et/ou le développement des plantes,
notamment pour la modulation des paramètres physiologiques d'une plante, en particulier la biomasse, la surface foliaire, la floraison, le calibre du fruit, la production et/ou la sélection de semence végétales, en particulier pour contrôler la parthénocarpie ou la monoecie d'une plante, ou pour la modification des paramètres physiologiques de semences végétales, en particulier la germination, l'implantation racinaire et la résistance au stress hydrique,
- ou pour prévenir ou traiter les maladies des plantes,
en particulier pour favoriser la résistance aux maladies infectieuses.

Dans un mode de réalisation particulier, l'invention concerne l'utilisation d'une composition comprenant au moins :
- un miPEP tel que défini selon la revendication 8, à l'exception des miPEP de séquences SEQ ID NO : 102, SEQ ID NO : 103, SEQ ID NO : 104 et SEQ ID NO : 355,
- un acide nucléique selon la revendication 9 codant ledit miPEP, à l'exception des acides nucléiques de séquences SEQ ID NO : 206, SEQ ID NO : 207, SEQ ID NO : 208 et SEQ ID NO : 356, ou
- un vecteur contenant ledit acide nucléique,
en tant qu'agent phytopharmaceutique,
- pour favoriser la croissance et/ou le développement des plantes,
   - ou pour la modification des paramètres physiologiques de semences végétalesou pour prévenir ou traiter les maladies des plantes,
ladite composition étant appliquée par enrobage ou pelliculage sur lesdites semences végétales.

Dans un autre aspect, l'invention concerne l'utilisation d'une composition telle que définie ci-dessus, pour moduler les paramètres physiologiques d'une plante, en particulier la biomasse, la surface foliaire, ou le calibre du fruit.

Dans un mode de réalisation, l'invention concerne l'utilisation d'une composition telle que définie ci-dessus, pour l'éclaircissage de vergers afin d'augmenter le calibre des fruits.
Dans un mode de réalisation, la description concerne l'utilisation d'une composition telle que définie ci-dessus, pour la production et/ou la sélection de semences végétales, ladite composition étant utilisée pour contrôler la parthénocarpie ou la monoecie d'une plante.

Dans un mode de réalisation, l'invention concerne l'utilisation d'une composition telle que définie ci-dessus, ladite composition étant administrée à ladite plante par la voie foliaire ou par la voie racinaire.
Dans un mode de réalisation, la description concerne l'utilisation d'une composition telle que définie ci-dessus, pour la production et/ou la sélection de semences végétales.
Dans un mode de réalisation, l'invention concerne l'utilisation d'une composition telle que définie ci-dessus, dans laquelle ladite composition est utilisée pour modifier les paramètres physiologiques desdites semences végétales, en particulier l'implantation racinaire, la germination et la résistance au stress hydrique.
Dans un mode de réalisation, l'invention concerne l'utilisation d'une composition telle que décrite ci-dessus, pour favoriser la résistance aux maladies infectieuses.
Dans un mode de réalisation, l'invention concerne l'utilisation d'une composition telle que définie ci-dessus, dans laquelle ladite composition est appliquée par enrobage ou pelliculage sur lesdites semences végétales.

Dans un autre aspect, l'invention concerne l'utilisation d'une composition telle que définie ci-dessus, en tant que pesticide, pour éliminer les organismes nuisibles des plantes ou susceptibles d'être classés comme tels, notamment en tant qu'insecticide, arachnicide, limacide ou rodonticide.
Dans un mode de réalisation, l'invention concerne l'utilisation d'une composition telle que définie ci-dessus, en tant qu'insecticide.
Dans un mode de réalisation, l'invention concerne l'utilisation d'une composition telle que définie ci-dessus, pour éliminer les insectes ravageurs.
Dans un mode de réalisation, l'invention concerne l'utilisation d'une composition telle que définie ci-dessus, pour éliminer les espèces animales classées nuisibles ou susceptibles d'être classées comme telles, en particulier les muridae, notamment le rat.

Dans un mode de réalisation, l'invention concerne l'utilisation d'une composition telle que définie ci-dessus, en tant que pesticide pour éliminer les organismes nuisibles des plantes ou susceptibles d'être classés comme tels,
notamment en tant qu'insecticide, arachnicide, limacide, ou rodonticide,
en particulier par application de ladite composition sur une plante ou sur un support en contact avec la plante.

Dans un autre aspect, l'invention concerne l'utilisation d'une composition telle que définie ci-dessus, dans laquelle ladite composition est appliquée sur une plante pour la protéger d'insectes ravageurs.

Dans un autre aspect, l'invention concerne l'utilisation d'un peptide pour favoriser la croissance d'une plante, ledit peptide étant introduit dans la plante, ledit peptide ayant une séquence d'acides aminés comprenant ou consistant en une séquence identique à celle d'un miPEP naturellement présent dans ladite plante,
ledit miPEP naturellement présent dans ladite plante étant un peptide de 4 à 100 acides aminés dont la séquence est codée par un cadre ouvert de lecture situé sur le transcrit primaire d'un miR, ledit miPEP étant capable de moduler l'accumulation dudit miR dans ladite plante, lequel miR régule l'expression d'au moins un gène impliqué dans le développement des parties végétatives ou reproductrices de la plante, notamment les racines, la tige, les feuilles ou les fleurs.

Les Inventeurs ont constaté de manière surprenante que l'utilisation de peptides dont la séquence comprend ou consiste en une séquence identique à celle de miPEPs codés sur les transcrits primaires de miRs, permet de favoriser la croissance des plantes.

Dans l'invention, le terme *« plante »* fait référence de manière générale à tout ou partie d'une plante quel que soit son stade de développement (y compris la plante sous forme de graine ou de jeune pousse), à un ou plusieurs organes de la plante (comme par exemple les feuilles, les racines, la tige, les fleurs), à une ou plusieurs cellules de la plante, ou encore à un amas de cellules de la plante.

Dans l'invention, le terme *« croissance »* fait référence au développement de tout ou partie d'une plante au cours du temps. La croissance de la plante peut ainsi être déterminée et quantifiée par le suivi de paramètres évolutifs observables pour certaines parties, cellules ou organes de la plante, tels que les feuilles, les racines, les tiges ou encore les fleurs.

De manière non limitative, les paramètres permettant de déterminer et quantifier la croissance d'une plante peuvent être notamment :
- la taille, la surface, le volume, la masse et le nombre de feuilles,
- la taille et le nombre de fleurs,
- la taille de la tige (ou de la hampe florale),
- la longueur et le nombre de racines,
- la précocité de la germination,
- la précocité du bourgeonnement,
- la précocité de l'induction florale (ou transition florale),
- ou encore le nombre de cellules.

Dans le cas des plantes légumineuses, la croissance de la plante peut également être liée à la vitesse de nodulation, ou encore à la taille et au nombre de nodules sur les racines.
Par ailleurs, dans l'invention, l'expression *« favoriser la croissance de la plante »*, ou *« améliorer la croissance de la plante »*, indique :
- soit une accélération du développement (comme par exemple une taille des feuilles plus importante pour une plante à un instant donné par rapport à une plante de référence),
- soit à un accroissement du développement (comme par exemple une taille de feuilles plus importante pour une plante qui ne peut être atteinte par une plante de référence),
- soit à une accélération et un accroissement du développement de la plante.
Il est important de noter que l'utilisation selon l'invention possède l'avantage d'être écologique, en comparaison des méthodes chimiques classiquement utilisées dans l'industrie botanique ou dans l'agriculture, car le miPEP est un peptide qui est présent naturellement chez la plante.
L'invention concerne également l'utilisation d'un miPEP introduit dans une plante pour en favoriser la croissance,
ledit miPEP introduit étant un peptide comprenant, ou consistant en, une séquence identique à celle d'un miPEP naturellement présent dans ladite plante,
lequel miPEP naturellement présent est un peptide de 4 à 100 acides aminés, dont la séquence est codée par un cadre ouvert de lecture situé en 5' sur le transcrit primaire d'un miR,
ledit miPEP étant capable de moduler l'accumulation dudit miR dans ladite plante, lequel miR régule l'expression d'au moins un gène impliqué dans le développement des parties végétatives ou reproductrices de la plante, notamment les racines, la tige, les feuilles ou les fleurs,
la somme de la quantité dudit miPEP introduit et de celle dudit miPEP naturellement présent étant strictement supérieure à la quantité dudit miPEP naturellement présent.

Dans l'invention, l'expression *« miPEP introduit»* fait référence à un miPEP introduit artificiellement dans la plante par opposition au *« miPEP présent naturellement dans la plante »*. L'introduction d'un miPEP dans la plante implique donc une étape technique, laquelle étape n'est pas un phénomène naturel et ne correspond ni à un croisement, ni à une sélection.

Le miPEP introduit peut être soit un peptide produit hors de la plante (comme par exemple un peptide isolé et/ou purifié, un peptide synthétique ou un peptide recombinant), soit un peptide produit dans la plante suite à l'introduction non naturelle d'un acide nucléique codant ledit miPEP dans ladite plante.

La plante dans laquelle le miPEP n'a pas été introduit possède une quantité basale dudit miPEP, qui correspond à celle dudit miPEP naturellement présent. L'utilisation d'un miPEP comprenant, ou consistant en, une séquence identique à celle dudit miPEP entraîne une augmentation de la quantité totale de miPEP, ce qui module l'accumulation du miR dont le transcrit primaire contient la séquence codant ledit miPEP.

Par ailleurs, le miPEP introduit se retrouve dans la plante et son introduction n'a pas d'impact sur sa stabilité.

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1), *CUC2* (Accession n° AT5G53950.1), *TCP3* (Accession n°AT1G53230.1) et *TCP4* (Accession n°AT3G15030.1) (numéros d'accession selon la banque de données *The Arabidopsis Information Resource « TAIR »).*

En particulier, l'invention concerne l'utilisation telle que définie ci-dessus, dans laquelle ledit gène, impliqué dans le développement des parties végétatives ou reproductrices de la plante, est choisi parmi le groupe consistant en : *NAC1, NAC4, NAC5, CUC1* et *CUC2.*

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en : *TCP3* et *TCP4.*

Dans un mode de réalisation, l'invention concerne l'utilisation telle que définie ci-dessus, dans laquelle ledit miARN est choisi parmi le miR164a et le mir319a.

Dans un mode de réalisation, l'invention concerne l'utilisation telle que définie ci-dessus, dans laquelle ledit miR est choisi parmi le miR164a, le miR319a, le miR171b, le miR165a, le miR160b, le miR169d et le miR171e.

Dans un mode de réalisation, l'invention concerne l'utilisation telle que définie ci-dessus, dans laquelle ledit miPEP est choisi parmi le AtmiPEP164a1, le AtmiPEP319a1, le AtmiPEP319a2, le MtmiPEP171b1, le AtmiPEP165a1, le AtmiPEP160b1, le MtmiPEP169d, le MtmiPEP171e.

En particulier, l'invention concerne l'utilisation telle que définie ci-dessus, dans laquelle, ledit miR164a possède une séquence nucléotidique consistant en SEQ ID NO : 297.

En particulier, l'invention concerne l'utilisation telle que définie ci-dessus, dans laquelle ledit ledit miR164a possède une séquence nucléotidique présentant au moins 80% d'identité, de préférence au moins 90 % d'identité, avec la séquence nucléotidique SEQ ID NO : 297.

Dans un mode de réalisation, l'invention concerne l'utilisation telle que définie ci-dessus, dans laquelle ledit miPEP est le AtmiPEP164a1, en particulier, dans laquelle ledit AtmiPEP164a1 possède une séquence d'acides aminés consistant en SEQ ID NO : 24.

En particulier, l'invention concerne l'utilisation telle que définie ci-dessus, dans laquelle, ledit miR319a possède une séquence nucléotidique consistant en SEQ ID NO : 331.

En particulier, l'invention concerne l'utilisation telle que définie ci-dessus, dans laquelle ledit ledit miR319a possède une séquence nucléotidique présentant au moins 80% d'identité, de préférence au moins 90 % d'identité, avec la séquence nucléotidique SEQ ID NO : 331.

Dans un mode de réalisation, l'invention concerne l'utilisation telle que définie ci-dessus, dans laquelle ledit miPEP est le AtmiPEP319a1, en particulier, dans laquelle ledit AtmiPEP319a1 possède une séquence d'acides aminés consistant en SEQ ID NO : 76.

Dans un mode de réalisation, l'invention concerne l'utilisation telle que définie ci-dessus, dans laquelle ladite plante est une plante crucifère telle qu'*Arabidopsis thaliana*, une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne l'utilisation telle que définie ci-dessus, dans laquelle ladite plante est une plante crucifère.

Dans un mode de réalisation, l'invention concerne l'utilisation telle que définie ci-dessus, dans laquelle ladite plante est *Arabidopsis thaliana.*

Dans un mode de réalisation, l'invention concerne l'utilisation telle que définie ci-dessus, pour favoriser la croissance d'une plante *Arabidopsis thaliana*, dans laquelle le AtmiPEP164a1 est introduit dans ladite plante *Arabidopsis thaliana*, ledit AtmiPEP164a1 étant également naturellement présent dans ladite plante *Arabidopsis thaliana*,

ledit AtmiPEP164a1 introduit étant un peptide dont la séquence comprend ou consiste en une séquence identique *à* celle dudit AtmiPEP164a1 naturellement présent, ladite séquence du AtmiPEP164a1 naturellement présent étant codée par un cadre ouvert de lecture situé en 5' sur le transcrit primaire du miR164a, lequel miR164a régule l'expression d'au moins un gène impliqué dans le développement des parties végétatives ou reproductrices *d'Arabidopsis thaliana*,

la somme de la quantité dudit AtmiPEP164a1 introduit et de celle dudit AtmiPEP164a1 naturellement présent étant strictement supérieure à la quantité dudit AtmiPEP164a1 naturellement présent chez ladite plante *Arabidopsis thaliana.*

Dans un mode de réalisation, l'invention concerne l'utilisation telle que définie ci-dessus, pour favoriser la croissance d'une plante *Arabidopsis thaliana*, dans laquelle le AtmiPEP319a1 est introduit dans ladite plante *Arabidopsis thaliana*, ledit AtmiPEP319a1 étant également naturellement présent dans ladite plante *Arabidopsis thaliana*,

ledit AtmiPEP319a1 introduit étant un peptide dont la séquence comprend ou consiste en une séquence identique *à* celle dudit AtmiPEP319a1 naturellement présent, ladite séquence du AtmiPEP319a1 naturellement présent étant codée par un cadre ouvert de lecture situé en 5' sur le transcrit primaire du miR319a, lequel miR319a régule l'expression d'au moins un gène impliqué dans le développement des parties végétatives ou reproductrices *d'Arabidopsis thaliana*,
la somme de la quantité dudit AtmiPEP319a1 introduit et de celle dudit AtmiPEP319a1 naturellement présent étant strictement supérieure à la quantité dudit AtmiPEP319a1 naturellement présent chez ladite plante *Arabidopsis thaliana.*

Dans un mode de réalisation, l'invention concerne l'utilisation telle que définie ci-dessus, dans laquelle ledit miPEP est introduit par voie externe dans la plante, de préférence par arrosage, par pulvérisation ou par l'ajout d'un engrais, d'un terreau, d'un substrat de culture ou d'un support inerte.

Dans un mode de réalisation, l'invention concerne l'utilisation telle que définie ci-dessus, dans laquelle ledit miPEP est introduit par arrosage et par pulvérisation.

Dans un mode de réalisation, l'invention concerne l'utilisation telle que définie ci-dessus, dans laquelle ledit miPEP est introduit par arrosage et par l'ajout d'un engrais.

Dans un mode de réalisation, l'invention concerne l'utilisation telle que définie ci-dessus, dans laquelle ledit miPEP est introduit par pulvérisation et par l'ajout d'un engrais.

Dans un mode de réalisation, l'invention concerne l'utilisation telle que définie ci-dessus, dans laquelle ledit miPEP est introduit, par arrosage, par pulvérisation et par l'ajout d'un engrais.

Les Inventeurs ont en effet constaté de manière inattendue qu'il est possible d'appliquer directement une composition comprenant un miPEP sur la plante pour moduler l'accumulation du miR correspondant dans la plante, ce qui indique que le miPEP est capté par la plante.

Dans un mode de réalisation, l'invention concerne l'utilisation telle que définie ci-dessus, dans laquelle la plante est traitée avec une composition comprenant 10⁻⁹ M à 10⁻⁴ M dudit miPEP, notamment 10⁻⁹ M, 10⁻⁸ M, 10⁻⁷ M, 10⁻⁶ M, 10⁻⁵ M ou 10⁻⁴ M dudit miPEP.

De préférence, les compositions ont une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Dans un mode de réalisation, l'invention concerne l'utilisation telle que définie ci-dessus, dans laquelle ledit miPEP est introduit dans la plante par le biais d'un acide nucléique codant ledit miPEP, ledit acide nucléique étant introduit dans la plante.

Dans un mode de réalisation, l'invention concerne l'utilisation telle que définie ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne l'utilisation telle que définie ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, l'invention concerne l'utilisation telle que définie ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, l'invention concerne l'utilisation telle que définie ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, l'invention concerne l'utilisation telle que définie ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, l'invention concerne l'utilisation telle que définie ci-dessus, dans laquelle la vitesse de nodulation est augmentée chez la plante dans laquelle a été introduit ledit miPEP par rapport à la vitesse de nodulation d'une plante identique et de même âge dans laquelle aucun miPEP n'a été introduit, ou bien par rapport à la vitesse de nodulation d'une plante identique et de même âge dans laquelle ledit miPEP n'a pas été introduit.

Dans un mode de réalisation, l'invention concerne l'utilisation telle que définie ci-dessus, dans laquelle le nombre de nodules est augmenté chez la plante dans laquelle a été introduit ledit miPEP par rapport au nombre de nodules d'une plante identique et de même âge dans laquelle aucun miPEP n'a été introduit, ou bien par rapport au nombre de nodules d'une plante identique et de même âge dans laquelle ledit miPEP n'a pas été introduit.

L'augmentation des paramètres permettant de déterminer et quantifier la croissance chez la plante dans laquelle a été introduit le miPEP (tels que la taille de la tige, le nombre et la taille des feuilles, le nombre et la longueur des racines, la vitesse de nodulation ou encore le nombre de nodules sur les racines) est de préférence mis en évidence par comparaison avec une plante identique (c'est-à-dire une plante de la même espèce et/ou variété), de même âge et cultivée dans les mêmes conditions mais dans laquelle aucun miPEP n'a été introduit.

Dans un autre aspect, l'invention concerne un procédé pour favoriser la croissance d'une plante, comprenant une étape d'introduction d'un miPEP dans une plante, ledit miPEP étant également présent naturellement dans ladite plante,

ledit miPEP introduit étant un peptide de 4 à 100 acides aminés dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans le développement des parties végétatives ou reproductrices de la plante, notamment les racines, la tige, les feuilles ou les fleurs,

la somme de la quantité dudit miPEP introduit et de celle dudit miPEP naturellement présent étant strictement supérieure à la quantité dudit miPEP naturellement présent.

Dans un mode de réalisation, l'invention concerne un procédé tel que défini ci-dessus, dans lequel ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1), *CUC2* (Accession n° AT5G53950.1), *TCP3* (Accession n°AT1G53230.1) et *TCP4* (Accession n°AT3G15030.1).

En particulier, l'invention concerne un procédé tel que défini ci-dessus, dans lequel ledit gène, impliqué dans le développement des parties végétatives ou reproductrices de la plante, est choisi parmi le groupe consistant en : *NAC1, NAC4, NAC5, CUC1* et *CUC2.*

Dans un mode de réalisation, l'invention concerne un procédé tel que défini ci-dessus, dans lequel ledit gène, impliqué dans le développement des parties végétatives ou reproductrices de la plante, est choisi parmi le groupe consistant en : *TCP3* et *TCP4.*

Dans un mode de réalisation, l'invention concerne un procédé tel que défini ci-dessus, dans lequel ledit miR est choisi parmi le miR164a, le miR319a, le miR171b, le miR165a, le miR160b, le miR169d et le miR171e.

Dans un mode de réalisation, l'invention concerne un procédé tel que défini ci-dessus, dans lequel ledit miPEP est choisi parmi le AtmiPEP164a1, le AtmiPEP319a1, le AtmiPEP319a2, le MtmiPEP171b1, le AtmiPEP165a1, le AtmiPEP160b1, le MtmiPEP169d, le MtmiPEP171e.

Dans un mode de réalisation, l'invention concerne un procédé tel que défini ci-dessus, dans lequel ledit miARN est le miR164a, en particulier, dans lequel ledit miR164a possède une séquence nucléotidique consistant en SEQ ID NO : 297.

Dans un mode de réalisation, l'invention concerne un procédé tel que défini ci-dessus, dans lequel ledit miPEP est le AtmiPEP164a1, en particulier, dans lequel ledit AtmiPEP164a1 possède une séquence d'acides aminés consistant en SEQ ID NO : 24.

Dans un mode de réalisation, l'invention concerne un procédé tel que défini ci-dessus, dans lequel ledit miARN est le miR319a, en particulier, dans lequel ledit miR319a possède une séquence nucléotidique consistant en SEQ ID NO : 331.

Dans un mode de réalisation, l'invention concerne un procédé tel que défini ci-dessus, dans lequel ledit miPEP est le AtmiPEP319a1, en particulier, dans lequel ledit AtmiPEP319a1 possède une séquence d'acides aminés consistant en SEQ ID NO : 76.

Dans un mode de réalisation, l'invention concerne un procédé tel que défini ci-dessus, dans lequel ladite plante est une plante crucifère telle qu'*Arabidopsis thaliana*, une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé tel que défini ci-dessus, dans lequel ladite plante est une plante crucifère.

Dans un mode de réalisation, l'invention concerne un procédé tel que défini ci-dessus, dans lequel ladite plante est *Arabidopsis thaliana.*

Dans un mode de réalisation, l'invention concerne un procédé tel que défini ci-dessus, pour favoriser la croissance d'une plante *Arabidopsis thaliana*, dans lequel le AtmiPEP164a1 est introduit dans ladite plante *Arabidopsis thaliana*, ledit AtmiPEP164a1 étant également naturellement présent dans ladite plante *Arabidopsis thaliana*,

ledit AtmiPEP164a1 introduit étant un peptide comprenant ou consistant en une séquence identique à celle dudit AtmiPEP164a1 naturellement présent, lequel le AtmiPEP164a1 naturellement présent est un peptide de 4 à 100 acides aminés dont la séquence est codée par un cadre ouvert de lecture situé en 5' sur le transcrit primaire du miR164a,

ledit AtmiPEP164a1 étant capable d'augmenter l'accumulation dudit miR164a, lequel miR164a régule l'expression d'au moins un gène impliqué dans le développement des parties végétatives ou reproductrices *d'Arabidopsis thaliana*,

la somme de la quantité dudit AtmiPEP164a1 introduit et de celle dudit AtmiPEP164a1 naturellement présent étant strictement supérieure à la quantité dudit AtmiPEP164a1 naturellement présent.

Dans un mode de réalisation, l'invention concerne un procédé tel que défini ci-dessus, pour favoriser la croissance d'une plante *Arabidopsis thaliana*, dans lequel le AtmiPEP319a1 est introduit dans ladite plante *Arabidopsis thaliana*, ledit AtmiPEP319a1 étant également naturellement présent dans ladite plante *Arabidopsis thaliana*,

ledit AtmiPEP319a1 introduit étant un peptide comprenant ou consistant en une séquence identique à celle dudit AtmiPEP319a1 naturellement présent, lequel le AtmiPEP319a1 naturellement présent est un peptide de 4 à 100 acides aminés dont la séquence est codée par un cadre ouvert de lecture situé en 5' sur le transcrit primaire du miR319a,

ledit AtmiPEP319a1 étant capable d'augmenter l'accumulation dudit miR319a, lequel miR319a régule l'expression d'au moins un gène impliqué dans le développement des parties végétatives ou reproductrices *d'Arabidopsis thaliana*,

la somme de la quantité dudit AtmiPEP319a1 introduit et de celle dudit AtmiPEP319a1 naturellement présent étant strictement supérieure à la quantité dudit AtmiPEP319a1 naturellement présent.

Dans un mode de réalisation, l'invention concerne un procédé tel que défini ci-dessus, dans lequel ledit miPEP est introduit par voie externe dans la plante, de préférence par arrosage, par pulvérisation ou par l'ajout d'un engrais, d'un terreau, d'un substrat de culture ou d'un support inerte.

Dans un mode de réalisation, l'invention concerne un procédé tel que défini ci-dessus, dans lequel ledit miPEP est administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP. Dans un mode de réalisation, l'invention concerne un procédé tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé tel que défini ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne un procédé tel que défini ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, l'invention concerne un procédé tel que défini ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, l'invention concerne un procédé tel que défini ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, l'invention concerne un procédé tel que défini ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, l'invention concerne un procédé tel que défini ci-dessus, dans laquelle la vitesse de nodulation est augmentée chez la plante dans laquelle a été introduit ledit miPEP par rapport à la vitesse de nodulation d'une plante identique et de même âge dans laquelle aucun miPEP n'a été introduit, ou bien par rapport à la vitesse de nodulation d'une plante identique et de même âge dans laquelle ledit miPEP n'a pas été introduit.

Dans un mode de réalisation, l'invention concerne un procédé tel que défini ci-dessus, dans laquelle le nombre de nodules est augmenté chez la plante dans laquelle a été introduit ledit miPEP par rapport au nombre de nodules d'une plante identique et de même âge dans laquelle aucun miPEP n'a été introduit, ou bien par rapport au nombre de nodules d'une plante identique et de même âge dans laquelle ledit miPEP n'a pas été introduit.

Dans un autre aspect, l'invention concerne une plante dans laquelle a été introduit un miPEP selon l'utilisation ou le procédé pour favoriser la croissance d'une plante décrits ci-dessus.

Dans un autre aspect, la demande décrit aussi un procédé de production d'une plante transgénique comprenant:
a) une étape d'introduction d'un acide nucléique codant un miPEP de 4 à 100 acides aminés dans une plante, ou dans au moins une cellule de ladite plante, dans des conditions permettant l'expression dudit miPEP,
ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans le développement des parties végétatives ou reproductrices de la plante, notamment les racines, la tige, les feuilles ou les fleurs, et
b) une étape de culture de la plante, ou d'au moins une cellule de ladite plante, obtenues à l'étape a) dans des conditions permettant l'obtention d'une plante transgénique.

Dans un autre aspect, la demande décrit aussi un procédé de production d'une plante transgénique comprenant:
**a)** une étape d'introduction d'un acide nucléique codant un miPEP de 4 à 100 acides aminés, ou de 3 acides aminés, dans une plante, ou dans au moins une cellule de ladite plante, dans des conditions permettant l'expression dudit miPEP,
ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans le développement des parties végétatives ou reproductrices de la plante, notamment les racines, la tige, les feuilles ou les fleurs, et
**b)** une étape de culture de la plante, ou d'au moins une cellule de ladite plante, obtenues à l'étape a) dans des conditions permettant l'obtention d'une plante transgénique.

Dans un autre aspect, la demande décrit aussi un procédé de production d'une plante transgénique comprenant:
**a)** une étape d'introduction d'un acide nucléique codant un miPEP de 3 acides aminés, dans une plante, ou dans au moins une cellule de ladite plante, dans des conditions permettant l'expression dudit miPEP,
ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans le développement des parties végétatives ou reproductrices de la plante, notamment les racines, la tige, les feuilles ou les fleurs, et
**b)** une étape de culture de la plante, ou d'au moins une cellule de ladite plante, obtenues à l'étape a) dans des conditions permettant l'obtention d'une plante transgénique.

Dans un mode de réalisation, la demande décrit aussi un procédé de production d'une plante transgénique tel que défini ci-dessus, dans lequel ladite plante transgénique obtenue à l'étape b) a une croissance améliorée par rapport à une plante identique dans laquelle ledit acide nucléique n'a pas été introduit.

Dans un mode de réalisation, la demande décrit aussi un procédé de production d'une plante transgénique tel que défini ci-dessus, dans lequel l'expression dudit miPEP codé par l'acide nucléique introduit dans la plante entraîne une croissance améliorée par rapport à une plante identique dans laquelle ledit acide nucléique n'a pas été introduit.

Dans un mode de réalisation, la demande décrit aussi un procédé de production d'une plante transgénique tel que défini ci-dessus, dans lequel l'étape a) est réalisée à l'aide d'un vecteur contenant ledit acide nucléique, de préférence un plasmide.

Dans un mode de réalisation, la demande décrit aussi un procédé de production d'une plante transgénique tel que défini ci-dessus, dans lequel l'expression dudit acide nucléique de l'étape a) est placée sous le contrôle d'un promoteur fort, de préférence un promoteur fort constitutif tel que le promoteur 35S.

Dans un mode de réalisation, la demande décrit aussi un procédé de production d'une plante transgénique tel que défini ci-dessus, dans lequel ledit gène, impliqué dans le développement des parties végétatives ou reproductrices de la plante, est choisi parmi le groupe consistant en: *NAC1* (Accession n°AT1G56010.1), *NAC4* (Accession n° AT5G07680.1), *NAC5* (Accession n° AT5G61430.1), *CUC1* (Accession n° AT3G15170.1), *CUC2* (Accession n° AT5G53950.1), *TCP3* (Accession n°AT1G53230.1) et *TCP4* (Accession n°AT3G15030.1). Dans un mode de réalisation, la demande décrit aussi un procédé de production d'une plante transgénique tel que défini ci-dessus, dans lequel ledit miPEPpossède une séquence d'acides aminés comprenant ou consistant en une séquence choisie parmi le groupe constitué de SEQ ID NO : 1 à SEQ ID NO : 101 et SEQ ID NO : 375 à SEQ ID NO : 386.

Dans un mode de réalisation, la demande décrit aussi un procédé de production d'une plante transgénique tel que défini ci-dessus, dans lequel ledit miPEPpossède une séquence d'acides aminés comprenant ou consistant en une séquence choisie parmi le groupe constitué de :
- SEQ ID NO : 1 à SEQ ID NO: 101,
- SEQ ID NO : 375 à SEQ ID NO : 386, ou
- SEQ ID NO : 424.

Dans un mode de réalisation, la demande décrit aussi un procédé de production d'une plante transgénique tel que défini ci-dessus, dans lequel ledit miARN est le miR164a, en particulier, dans lequel ledit miR164a possède une séquence nucléotidique consistant en SEQ ID NO : 297.

Dans un mode de réalisation, la demande décrit aussi un procédé de production d'une plante transgénique tel que défini ci-dessus, dans lequel ledit miPEP est le AtmiPEP164a1, en particulier, dans lequel ledit AtmiPEP164a1 possède une séquence d'acides aminés consistant en SEQ ID NO : 24.

Dans un mode de réalisation, la demande décrit aussi un procédé de production d'une plante transgénique tel que défini ci-dessus, dans lequel ledit miARN est le miR319a, en particulier, dans lequel ledit miR319a possède une séquence nucléotidique consistant en SEQ ID NO : 331.

Dans un mode de réalisation, la demande décrit aussi un procédé de production d'une plante transgénique tel que défini ci-dessus, dans lequel ledit miPEP est le AtmiPEP319a1, en particulier, dans lequel ledit AtmiPEP319a1 possède une séquence d'acides aminés consistant en SEQ ID NO : 76.

Dans un mode de réalisation, la demande décrit aussi un procédé de production d'une plante transgénique tel que défini ci-dessus, dans lequel ledit acide nucléique introduit à l'étape a) comprend une séquence nucléotidique choisie parmi SEQ ID NO : 128 et SEQ ID NO : 180.

Dans un mode de réalisation, la demande décrit aussi un procédé de production d'une plante transgénique tel que défini ci-dessus, dans lequel ladite plante est une plante crucifère telle qu'*Arabidopsis thaliana*, une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, la demande décrit aussi un procédé de production d'une plante transgénique tel que défini ci-dessus, dans lequel ladite plante transgénique est une plante crucifère.

Dans un mode de réalisation, la demande décrit aussi un procédé de production d'une plante transgénique tel que défini ci-dessus, dans lequel ladite plante transgénique est *Arabidopsis thaliana.*

Dans un mode de réalisation, la demande décrit aussi un procédé de production d'une plante transgénique tel que défini ci-dessus, comprenant :
**a)** une étape d'introduction d'un acide nucléique contenant la séquence nucléotidique SEQ ID NO : 128, codant le AtmiPEP164a1 consistant en la séquence d'acides aminés SEQ ID NO : 24, dans une plante *Arabidopsis thaliana*, ou dans au moins une cellule de ladite plante *Arabidopsis thaliana*, dans des conditions permettant l'expression du AtmiPEP164a1,
ledit AtmiPEP164a1 étant également naturellement présent dans ladite plante *Arabidopsis thaliana*, ledit miPEP naturellement présent étant un peptide dont la séquence est codée par un cadre ouvert de lecture situé en 5' sur le transcrit primaire du miR164a, ledit AtmiPEP164a1 étant capable de moduler l'accumulation dudit miR164, lequel miR164a régule l'expression d'au moins un gène impliqué dans le développement des parties végétatives ou reproductrices *d'Arabidopsis thaliana*, et
**b)** une étape de culture de la plante, ou d'au moins une cellule de ladite plante, obtenues à l'étape a) dans des conditions permettant l'obtention d'une plante *Arabidopsis thaliana* transgénique.

Dans un mode de réalisation, la demande décrit aussi un procédé de production d'une plante transgénique tel que défini ci-dessus, comprenant :
**a)** une étape d'introduction d'un acide nucléique contenant la séquence nucléotidique SEQ ID NO : 180, codant le AtmiPEP319a1 consistant en la séquence d'acides aminés SEQ ID NO : 76, dans une plante *Arabidopsis thaliana*, ou dans au moins une cellule de ladite plante *Arabidopsis thaliana*, dans des conditions permettant l'expression du AtmiPEP319a1,
ledit AtmiPEP319a1 étant également naturellement présent dans ladite plante *Arabidopsis thaliana*, ledit miPEP naturellement présent étant un peptide dont la séquence est codée par un cadre ouvert de lecture situé en 5' sur le transcrit primaire du miR319a, ledit AtmiPEP319a1 étant capable de moduler l'accumulation dudit miR319, lequel miR319a régule l'expression d'au moins un gène impliqué dans le développement des parties végétatives ou reproductrices d'*Arabidopsis thaliana,* et
**b)** une étape de culture de la plante, ou d'au moins une cellule de ladite plante, obtenues à l'étape a) dans des conditions permettant l'obtention d'une plante *Arabidopsis thaliana* transgénique.

Dans un mode de réalisation, la demande décrit aussi un procédé de production tel que défini ci-dessus, dans lequel ledit miPEP est introduit dans la plante par le biais d'un acide nucléique codant ledit miPEP, ledit acide nucléique étant introduit dans la plante.

Dans un mode de réalisation, la demande décrit aussi un procédé de production d'une plante transgénique tel que défini ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne un procédé de production d'une plante transgénique tel que défini ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, la demande décrit aussi un procédé de production d'une plante transgénique tel que défini ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, la demande décrit aussi un procédé de production d'une plante transgénique tel que défini ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, la demande décrit aussi un procédé de production d'une plante transgénique tel que défini ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, la demande décrit aussi un procédé de production d'une plante transgénique tel que défini ci-dessus, dans laquelle la vitesse de nodulation est augmentée chez la plante dans laquelle a été introduit ledit miPEP par rapport à la vitesse de nodulation d'une plante identique et de même âge dans laquelle aucun miPEP n'a pas été introduit, ou bien par rapport à la vitesse de nodulation d'une plante identique et de même âge dans laquelle ledit miPEP n'a pas été introduit.

Dans un mode de réalisation, la demande décrit aussi un procédé de production d'une plante transgénique tel que défini ci-dessus, dans laquelle le nombre de nodules est augmenté chez la plante dans laquelle a été introduit ledit miPEP par rapport au nombre de nodules d'une plante identique et de même âge dans laquelle aucun miPEP n'a été introduit, ou bien par rapport au nombre de nodules d'une plante identique et de même âge dans laquelle ledit miPEP n'a pas été introduit.

Dans un aspect, la demande décrit également une plante transgénique telle qu'obtenue par le procédé de production tel que défini ci-dessus.

Dans un autre aspect, la demande décrit aussi une plante transgénique contenant un vecteur codant un miPEP, ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène,
l'expression dudit miPEP codé par le vecteur introduit dans la plante entraînant une modulation de l'expression dudit gène par rapport à une plante identique dans laquelle ledit acide nucléique n'a pas été introduit.

Dans un mode de réalisation, la demande décrit aussi une plante transgénique telle que définie ci-dessus dans laquelle ledit gène est impliqué dans le développement des parties végétatives ou reproductrices de la plante, notamment les racines, la tige, les feuilles ou les fleurs, et l'expression dudit miPEP codé par l'acide nucléique introduit dans la plante entraîne une croissance améliorée de la plante transgénique par rapport à une plante identique dans laquelle ledit acide nucléique n'a pas été introduit.

Dans un mode de réalisation, la demande décrit aussi une plante transgénique telle que définie ci-dessus dans laquelle ledit miPEP est choisi parmi le groupe consistant en :
- SEQ ID NO : 1 à SEQ ID NO: 101,
- SEQ ID NO : 375 à SEQ ID NO : 386, ou
- SEQ ID NO : 424.

Dans un autre aspect, la demande décrit aussi une plante transgénique comprenant un vecteur,
ledit vecteur contenant un acide nucléique permettant l'expression d'un transcrit primaire de miR,
ledit transcrit primaire de miR contenant un cadre ouvert de lecture codant un miPEP, ledit miPEP étant capable de moduler l'accumulation dudit miR dans ladite plante, lequel miR régule l'expression d'au moins un gène dans ladite plante,
l'expression dudit miPEP codé par le vecteur introduit dans la plante entraînant une modulation de l'expression dudit gène par rapport à une plante identique dans laquelle ledit vecteur n'a pas été introduit.

Dans un mode de réalisation, la demande décrit aussi une plante transgénique telle que définie ci-dessus dans laquelle ledit acide nucléique est d'origine endogène.

Si ledit acide nucléique est d'origine endogène, au moins une copie dudit acide nucléique est déjà présente naturellement dans la plante, indépendamment du vecteur introduit dans la plante.

Dans un mode de réalisation, la demande décrit aussi une plante transgénique telle que définie ci-dessus dans laquelle ledit acide nucléique est d'origine exogène.

Si ledit acide nucléique est d'origine exogène, aucune copie dudit acide nucléique n'est présente naturellement dans la plante.

Dans un mode de réalisation, la demande décrit aussi une plante transgénique telle que définie ci-dessus dans laquelle ledit gène est impliqué dans le développement des parties végétatives ou reproductrices de la plante, notamment les racines, la tige, les feuilles ou les fleurs, et l'expression dudit miPEP codé par l'acide nucléique introduit dans la plante entraîne une croissance améliorée de la plante transgénique par rapport à une plante identique dans laquelle ledit acide nucléique n'a pas été introduit.

Dans un mode de réalisation, la demande décrit aussi une plante transgénique telle que définie ci-dessus dans laquelle ledit miPEP est choisi parmi le groupe consistant en :
- SEQ ID NO : 1 à SEQ ID NO: 101,
- SEQ ID NO : 375 à SEQ ID NO : 386, ou
- SEQ ID NO : 424.

Dans un autre aspect, la description concerne une composition comprenant en combinaison une quantité de semences d'une plante et une quantité d'un peptide dont la séquence comprend ou consiste en une séquence identique à celle d'un miPEP naturellement présent chez ladite plante.

Dans un mode de réalisation, la description concerne une composition telle que définie ci-dessus, dans laquelle ledit miPEP est choisi parmi le groupe consistant en :
- SEQ ID NO : 1 à SEQ ID NO: 101,
- SEQ ID NO : 375 à SEQ ID NO : 386, ou
- SEQ ID NO : 424.

Dans un mode de réalisation, l'invention concerne une composition comprenant en combinaison une quantité de semences d'une plante et une quantité d'un peptide dont la séquence comprend ou consiste en une séquence identique à celle d'un miPEP naturellement présent chez ladite plante
ledit miPEP étant choisi parmi le groupe de peptides consistant en : SEQ ID NO : 59, SEQ ID NO : 60, SEQ ID NO : 24, SEQ ID NO : 25, SEQ ID NO : 26, SEQ ID NO : 43, SEQ ID NO : 76.

Dans un mode de réalisation, l'invention concerne une composition comprenant en combinaison une quantité de semences d'une plante, notamment *A. thaliana,* et une quantité d'un peptide dont la séquence comprend ou consiste en une séquence identique à celle du AtmiPEP164a1.

Dans un mode de réalisation, l'invention concerne une composition comprenant en combinaison une quantité de semences d'une plante, notamment *A. thaliana,* et une quantité d'un peptide dont la séquence comprend ou consiste en une séquence identique à celle du AtmiPEP319a1.

Dans un mode de réalisation, l'invention concerne une composition comprenant en combinaison une quantité de semences d'une plante, notamment *M. truncatula,* et une quantité d'un peptide dont la séquence comprend ou consiste en une séquence identique à celle du MtmiPEP171b.

Dans un mode de réalisation, l'invention concerne une composition telle que définie ci-dessus formulée de façon à former une semence enrobée.

L'enrobage peut être réalisé selon les procédés classiquement utilisées dans l'industrie agroalimentaire et peut être obtenu en utilisant un matériau apte à se désagréger dans un solvant ou dans la terre, tel qu'un liant ou de l'argile.

Selon l'invention, l'enrobage peut être utilisé pour par exemple conférer des propriétés particulières à une composition de miPEP, ou encore à une composition de semences en combinaison avec un miPEP.

Dans un mode de réalisation, l'invention concerne une composition telle que définie ci-dessus formulée de façon à former une semence enrobée comprenant le MtmiPEP171b.

Dans un mode de réalisation, l'invention concerne une composition telle que définie ci-dessus formulée de façon à former une semence enrobée comprenant le AtmiPEP164a1.

Dans un mode de réalisation, l'invention concerne une composition telle que définie ci-dessus formulée de façon à former une semence enrobée comprenant le AtmiPEP319a1.

Dans un autre aspect, la description concerne une composition pharmaceutique comprenant au moins :
- un miPEP tel que défini ci-dessus,
- un acide nucléique codant ledit miPEP, ou
- un vecteur contenant ledit acide nucléique,
et un excipient pharmaceutiquement acceptable.

Dans un autre aspect, la description concerne une composition comprenant au moins :
- un miPEP tel que défini ci-dessus
- un acide nucléique codant ledit miPEP, ou
- un vecteur contenant ledit acide nucléique.
pour son utilisation en tant que médicament, notamment pour l'Homme ou pour les animaux. L'utilisation des compositions de l'invention est applicable en médecine humaine et en médecine vétérinaire.

Dans un autre aspect, la description concerne une composition comprenant au moins :
- un miPEP tel que défini ci-dessus
- un acide nucléique codant ledit miPEP, ou
- un vecteur contenant ledit acide nucléique.
pour son utilisation dans la prévention et/ou le traitement d'une pathologie impliquant la dérégulation de l'expression d'un gène du patient,
l'expression dudit gène étant régulée par un microARN dont l'accumulation est modulée par ledit miPEP.

Dans un mode de réalisation, la description concerne la composition telle que définie ci-dessus dans laquelle ladite pathologie est choisie parmi le cancer, le diabète, l'obésité, les maldies infectieuses et les maladies neurodégénératives.

Dans un mode de réalisation, la description concerne la composition telle que définie ci-dessus dans laquelle ladite pathologie est choisie parmi : le cancer, le diabète, l'obésité, les maladies infectieuses, les maladies neurodégénératives, les maladies cardiovasculaires et les maladies autoimmunes.

Dans un mode de réalisation, la description concerne la composition telle que définie ci-dessus dans laquelle ladite pathologie est choisie parmi : le cancer, le diabète, l'obésité, les maladies infectieuses et les maladies neurodégénératives, ou choisie parmi les maladies cardiovasculaires et les maladies autoimmunes.

Dans un mode de réalisation, la description concerne la composition telle que définie ci-dessus dans laquelle ladite pathologie est choisie parmi les maladies cardiovasculaires et les maladies autoimmunes.

Dans un autre aspect, la description concerne une composition comprenant au moins :
- un miPEP tel que défini ci-dessus,
- un acide nucléique codant ledit miPEP, ou
- un vecteur contenant ledit acide nucléique.
pour son utilisation dans la prévention et/ou le traitement d'une infection d'un animal ou d'un humain par un organisme parasitaire,
ledit organisme parasitaire possédant un gène dont l'expression est régulée par un microARN dont l'accumulation est modulée par ledit miPEP.

Dans un autre aspect, la description concerne un miPEP de 4 à 100 acides aminés, ou de 3 acides aminés, codé par une séquence nucléotidique contenue dans le transcrit primaire d'un microARN, ledit miPEP étant capable de moduler l'accumulation dudit microARN dans une cellule eucaryote, pour son utilisation comme médicament.

Dans un autre aspect, la demande décrit aussi un anticorps reconnaissant spécifiquement un miPEP.

En particulier, la demande décrit aussi un anticorps reconnaissant spécifiquement le AtmiPEP165a.

En particulier, la demande décrit aussi un anticorps reconnaissant spécifiquement le MtmiPEP171b.

En particulier, la demande décrit aussi un anticorps reconnaissant spécifiquement le AtmiPEP164a1.

En particulier, la demande décrit aussi un anticorps reconnaissant spécifiquement le AtmiPEP319a1.

Un tel anticorps peut être obtenu à partir d'un procédé connu de l'homme du métier, comme par exemple en injectant ledit miPEP à un animal non humain pour déclencher une réaction d'immunisation et la production d'anticorps par ledit animal.

Dans un autre aspect, la demande décrit aussi un procédé d'immunolocalisation d'un miPEP comprenant une étape de marquage d'un échantillon biologique d'une plante avec un anticorps reconnaissant spécifiquement un miPEP.

En particulier, la demande décrit aussi
un procédé d'immunolocalisation du AtmiPEP165a à l'aide d'un anticorps reconnaissant spécifiquement le AtmiPEP165a.

En particulier, la demande décrit aussi un procédé d'immunolocalisation du MtmiPEP171b à l'aide d'un anticorps reconnaissant spécifiquement le MtmiPEP171b.

En particulier, la demande décrit aussi un procédé d'immunolocalisation du AtmiPEP164a1 à l'aide d'un anticorps reconnaissant spécifiquement le AtmiPEP164a1.

En particulier, la demande décrit aussi un procédé d'immunolocalisation du MtmiPEP319a1 à l'aide d'un anticorps reconnaissant spécifiquement le AtmiPEP319a1.

Dans un autre aspect, la description concerne un anticorps reconnaissant spécifiquement un miPEP selon l'invention, pour son utilisation comme médicament.

Dans un autre aspect, la description concerne une composition pharmaceutique comprenant un anticorps reconnaissant spécifiquement un miPEP selon l'invention et un excipient pharmaceutiquement acceptable.

Dans un autre aspect, la demande décrit aussi un protocole de production d'un peptide recombinant, dont la séquence comprend ou consiste en une séquence identique à celle d'un miPEP tel que défini ci-dessus, comprenant une étape de transformation d'un organisme avec un vecteur d'expression codant ledit peptide recombinant.

Dans un mode de réalisation, ledit organisme est choisi parmi le groupe comprenant les bactéries, les levures, les champignons (autre que levures), les cellules animales, les plantes et les animaux.

Dans un mode de réalisation, ledit organisme est *Escherichia coli.*

En particulier, la demande décrit aussi un protocole de production d'un peptide recombinant tel que défini ci-dessus, comprenant les étapes suivantes :
- l'acide nucléique codant ledit peptide recombinant est lié à un acide nucléique codant une étiquette, telle que la GST,
- le vecteur d'expression contenant ledit acide nucléique codant ledit peptide recombinant est introduit dans la bactérie *E. coli,*
- la bactérie *E. coli* contenant le vecteur d'expression est cultivée dans du milieu LB de préférence jusqu'à une DO comprise entre 0.2 et 0.4,
- la production du peptide recombinant est induite avec de l'IPTG, de préférence pendant 4 à 5 heures,
- les bactéries *E. coli* sont centrifugées et lysées,
- le surnageant est filtré,
- ledit peptide recombinant est purifié sur une colonne d'affinité glutathion sepharose,
- si nécessaire, cliver la GST avec une protéase.

L'ensemble des séquences des miPEPs, des miORFs, des miRs et des transcrits primaires de miRs est indiqué dans les tables 1, 2, 3, 4, 5 et 6.

La table 7 présente une analyse du polymorphisme de la séquence d'ADN des différentes régions du pri-miR171b (un haplotype est défini lorsqu'il diffère d'au moins un acide aminé des autres haplotypes).

**Table 1. Liste des miPEPs (miPEPs) potentiels**

| **miPEP (pI / taille MW)** | **miR** | **Organisme** | **Gènes cibles du miR** | **Séquence du miPEP** | **SEQ ID** |
|---|---|---|---|---|---|
| AtmiPEP156a1 (10,57 / 3824) | miR156a | *Arabidopsis thaliana* | | | SEQ ID NO: 1 |
| AtmiPEP156a2 | miR156a | *Arabidopsis thaliana* | | MRRQTSVPFACKRDKESDKSHKER | SEQ ID NO:2 |
| AtmiPEP156a3 | miR156a | *Arabidopsis thaliana* | Famille du gène *SPL*, | | SEQ ID NO:3 |
| AtmiPEP156cl (8,5 / 1359) | miR156c | *Arabidopsis thaliana* | impliqué dans le développement de la | MKDNFPLLLRL | SEQ ID NO:4 |
| AtmiPEP156c2 | miR156c | *Arabidopsis thaliana* | tige et la floraison | MSDD | SEQ ID NO:5 |
| AtmiPEP156e1 (4.03 / 1818) | miR156e | *Arabidopsis thaliana* | | MIYINKYGSISAVEDD | SEQ ID NO:6 |
| AtmiPEP156f1 (9,5 / 520) | miR156f | *Arabidopsis thaliana* | | MSQR | SEQ ID NO:7 |
| AlmiPEP159a | miR159a | *Arabidopsis lyrata* | | MTCPLLSLSFLLSKYI | SEQ ID NO:8 |
| AtmiPEP159a1 (8,34 / 1898) | miR159a | *Arabidopsis thaliana* | Famille du gène *MYB*, | MTWPLLSLSFLLSKYV | SEQ ID NO:9 |
| CrmiPEP159a | miR159a | *Capsella rubella* | impliqué dans la germination et la | MTCTLSALSLSLNMFRVN | SEQ ID NO: 10 |
| AtmiPEP159b1 (5,27 / 659) | miR159b | *Arabidopsis thaliana* | floraison | MFYLS | SEQ ID NO:1 1 |
| AtmiPEP159b2 | miR159b | *Arabidopsis thaliana* | | | SEQ ID NO:12 |
| AtmiPEP160al (8,02 / 2936) | miR160a | *Arabidopsis thaliana* | Famille du gène *ARF*, impliqué dans la germination, le développement et la floraison | MFCLLIPIFSFVFSPNRHLRLQEQ | SEQ ID NO: 13 |
| AtmiPEP160b1 (5,28 / 608) | miR160b | *Arabidopsis thaliana* | | MFSPQ | SEQ ID NO:14 |
| AtmiPEP160b2 | miR160b | *Arabidopsis thaliana* | | MKYIHILILFKSRSTYKLSTNHI | SEQ ID NO:15 |
| AtmiPEP161 (10 / 1199) | miR161 | *Arabidopsis thaliana* | Famille du gène *PPR* | MKIPLFLPKL | SEQ ID NO:16 |
| AtmiPEP162a1 (4,03 / 3045) | miR162a | *Arabidopsis thaliana* | Gène *DCL1*, impliqué dans le développement | MVSGQEDSWLKLSSLCFLFLSLLDSLI | SEQ ID NO:17 |
| (5,71 / 4114) | AtmiPEP162b1 miR162b | *Arabidopsis thaliana* | | | SEQ ID NO:18 |
| AtmiPEP163-1 (6,5 / 1076) | miR163 | *Arabidopsis thaliana* | Famille du gène *SAMT*, impliqué dans la production de métabolites secondaires | MSTTQEHRS | SEQ ID NO:19 |
| AtmiPEP163-2 | miR163 | *Arabidopsis thaliana* | | MILKCWSSRFLRVSPYQNAHSLSLG | SEQ ID NO:20 |
| AlmiPEP164a1 | miR164a | *Arabidopsis lyrata* | | MPLAVIRQGIVWP | SEQ ID NO:21 |
| AlmiPEP164a2 | miR164a | *Arabidopsis lyrata* | | MPSWHDMVLLPYVKHTHANTRHIT | SEQ ID NO:22 |
| AlmiPEP 164a3 | miR164a | *Arabidopsis lyrata* | | MTWFFCLT | SEQ ID NO:23 |
| AtmiPEP164a1 (7,05 / 4256) | miR164a | *Arabidopsis thaliana* | | | SEQ ID NO:24 |
| AtmiPEP164a2 | miR164a | *Arabidopsis thaliana* | | MAWYGSFALRKTHSRQHTHTHT | SEQ ID NO:25 |
| AtmiPEP164a3 | miR164a | *Arabidopsis thaliana* | | MVWFFCLT | SEQ ID NO:26 |
| BrmiPEP164a1 | miR164a | *Brassica rapa* | Famille du gène *NAC*, impliqué dans le développement racinaire, foliaire et floral | MMIILWK | SEQ ID NO:27 |
| BrmiPEP164a2 | miR164a | *Brassica rapa* | | MLWAKLVSFSTLHSLVFLLSPSFA | SEQ ID NO:28 |
| BrmiPEP164a3 | miR164a | *Brassica rapa* | | | SEQ ID NO:29 |
| CpmiPEP164a1 | miR164a | *Carica papaya* | | | SEQ ID NO:30 |
| CpmiPEP164a2 | miR164a | *Carica papaya* | | | SEQ ID NO:31 |
| CrmiPEP164a1 | miR164a | *Capsella rubella* | | MELKGLRTWQLLDKV | SEQ ID NO:32 |
| CrmiPEP164a2 | miR164a | *Capsella rubella* | | MPSWHGMACFYCLT | SEQ ID NO:33 |
| CrmiPEP164a3 | miR164a | *Capsella rubella* | | MAWHGMFLLPYVKHTHANTYSLYM | SEQ ID NO:34 |
| GrmiPEP164a1 | miR164a | *Gossypium raimondii* | | | SEQ ID NO:35 |
| GrmiPEP164a2 | miR164a | *Gossypium raimondii* | | MSNSRSYQLK | SEQ ID NO:36 |
| GrmiPEP164a3 | miR164a | *Gossypium raimondii* | | MNEDLEISTRKRTPQLC | SEQ ID NO:37 |
| MtmiPEP164a1 | miR164a | *Medicago truncatula* | | MPKFDIFFYIFV | SEQ ID NO:38 |
| MtmiPEP164a2 | miR164a | *Medicago truncatula* | | | SEQ ID NO:39 |
| OsmiPEP164a1 | miR164a | *Oryza sativa* | | MQTHSNTPQSTYSLSLSLSE | SEQ ID NO:40 |
| OsmiPEP164a2 | miR164a | *Oryza sativa* | | MCVCDINMHSMLMLL | SEQ ID NO:41 |
| AlmiPEP165a | miR165a | *Arabidopsis lyrata* | | MRIKLFQLRGMLSGSRILYIYTCVC | SEQ ID NO:42 |
| AtmiPEP165a (12,3 / 2105) | miR165a | *Arabidopsis thaliana* | | MRVKLFQLRGMLSGSRIL | SEQ ID NO:43 |
| BcmiPEP165a | miR165a | *Brassica carinata* | | | SEQ ID NO:44 |
| BjmiPEP165a | miR165a | *Brassica juncea* | Famille du gène *HD-ZIPIII*, impliqué dans développement vasculaire, racinaire, foliaire, floral, nodulation | | SEQ ID NO:45 |
| BnmiPEP165a | miR165a | *Brassica napus* | | | SEQ ID NO:46 |
| BomiPEP165a | miR165a | *Brassica oleracea* | | | SEQ ID NO:47 |
| BrmiPEP165a | miR165a | *Brassica rapa* | | | SEQ ID NO:48 |
| AtmiPEP166a (4, 68 / 2372) | miR166a | *Arabidopsis thaliana* | | MLDLFRSNNRIEPSDFRFD | SEQ ID NO:49 |
| AtmiPEP166b (9,35 / 576) | miR166b | *Arabidopsis thaliana* | | MRDR | SEQ ID NO:50 |
| AtmiPEP167a (11 / 1148) | miR167a | *Arabidopsis thaliana* | Famille du gène *ARF*, impliqué dans le développement racinaire et floral | MNRKISLSLS | SEQ ID NO:51 |
| AtmiPEP167b1 (5,27 / 891) | miR167b | *Arabidopsis thaliana* | | MMGCFVGF | SEQ ID NO:52 |
| AtmiPEP167b2 | miR167b | *Arabidopsis thaliana* | | MQEETYEG | SEQ ID NO:53 |
| AtmiPEP169c1 (9,3 / 7110) | miR169c | *Arabidopsis thaliana* | Famille du gène du facteur CCAAT-bing, impliqué dans la nodulation, la résistance à la sécheresse, la résistance à la carence azotée | | SEQ ID NO:54 |
| AtmiPEP 169c2 | miR169c | *Arabidopsis thaliana* | | | SEQ ID NO:55 |
| AtmiPEP1691 (8,52 / 786) | miR1691 | *Arabidopsis thaliana* | | MRHKES | SEQ ID NO:56 |
| AtmiPEP171a1 (11,05 / 4057) | miR171a | *Arabidopsis thaliana* | | | SEQ ID NO:57 |
| AtmiPEP171b (8,5 / 995) | miR171b | *Arabidopsis thaliana* | | MVLSGKLTF | SEQ ID NO:58 |
| MtmiPEP171b1 | miR171b | *Medicago truncatula* | Famille du gène | MLLHRLSKFCKIERDIVYIS | SEQ ID NO:59 |
| MtmiPEP171b2 | miR171b | *Medicago truncatula* | *GRAS*, impliqué dans le développement | MKIEE | SEQ ID NO:60 |
| ZmmiPEP171b | miR171b | *Zea mays* | floral, foliaire, racinaire, la | | SEQ ID NO:61 |
| AtmiPEP171c1 (6,68 / 1187) | miR171c | *Arabidopsis thaliana* | mycorhization, la nodulation | MLSLSHFHIC | SEQ ID NO:62 |
| MtmiPEP171e | miR171e | *Medicago truncatula* | | MMVFGKPKKAMLVRFNPKTDLHV | SEQ ID NO:63 |
| MtmiPEP171h | miR171h | *Medicago truncatula* | | MASAAKVYMA | SEQ ID NO:64 |
| AtmiPEP172a1 (8,5 / 734) | miR172a | *Arabidopsis thaliana* | Famille du gène *AP2*, impliqué dans le développement floral | MASKIW | SEQ ID NO:65 |
| AtmiPEP172a3 | miR172a | *Arabidopsis thaliana* | | MVRFQLSIRD | SEQ ID NO:66 |
| AtmiPEP172b1 (7,9 / 1621) | miR172b | *Arabidopsis thaliana* | | MCTYYYLINKYF | SEQ ID NO:67 |
| AtmiPEP172c1 (7,98 / 1367) | miR172c | *Arabidopsis thaliana* | | MFPAKWCRLES | SEQ ID NO:68 |
| AtmiPEP172e1 (8,35 / 2452) | miR172e | *Arabidopsis thaliana* | | MGSLSLFKSQLEILMLLLSLSK | SEQ ID NO:69 |
| AtmiPEP172e2 | miR172e | *Arabidopsis thaliana* | | MSVYIHVPISLNCFSPKSSC | SEQ ID NO:70 |
| AtmiPEP172e3 | miR172e | *Arabidopsis thaliana* | | MGVPNFRPRNR | SEQ ID NO:71 |
| AcmiPEP319a1 | miR319a | *Arabidopsis cebennensis* | | MRSRVSFFFFKIMLFRLLGYRSM | SEQ ID NO:72 |
| AcmiPEP319a2 | miR319a | *Arabidopsis cebennensis* | | | SEQ ID NO:73 |
| AhmiPEP319a | miR319a | *Arabidopsis halleri* | | MRSRVSLFLSFSSNFAAYSPRS | SEQ ID NO:74 |
| AlmiPEP319a | miR319a | *Arabidospis lyrata* | | | SEQ ID NO:75 |
| AtmiPEP319a1 (6,56 / 5917) | miR319a | *Arabidopsis thaliana* | | | SEQ ID NO:76 |
| AtmiPEP319a2 | miR319a | *Arabidopsis thaliana* | | MFQTLYLFIYIHTNILLLS | SEQ ID NO:77 |
| BrmiPEP319a | miR319a | *Brassica rapa* | | | SEQ ID NO:78 |
| CpmiPEP319a | miR319a | *Carica papaya* | Famille du gène *TCP*, | MKIKLGFSLIKIIILLDKNS | SEQ ID NO:79 |
| CrmiPEP319a | miR319a | *Capsella rubella* | impliqué dans le | MHPHTYIHIPSSSFLISSFCL | SEQ ID NO:80 |
| EgmiPEP319a | miR319a | *Eucalyptus grandis* | développement floral et foliaire | | SEQ ID NO:81 |
| GrmiPEP319a | miR319a | *Gossypium raimondii* | | | SEQ ID NO:82 |
| MtmiPEP319a | miR319a | *Medicago truncatula* | | MHVYLELFMVIKGLGFLLLVK | SEQ ID NO:83 |
| OsmiPEP319a | miR319a | *Oryza sativa* | | MEMIQRPCLILKFFFKLSTLYIP | SEQ ID NO:84 |
| PpmiPEP319a | miR319a | *Physcomitrella patens* | | | SEQ ID NO:85 |
| ThmiPEP319a1 | miR319a | *Thellungiella halophila* | | | SEQ ID NO:86 |
| ThmiPEP319a2 | miR319a | *Thellungiella halophila* | | | SEQ ID NO:87 |
| AtmiPEP319b1 (8,04 / 5120) | miR319b | *Arabidopsis thaliana* | | | SEQ ID NO:88 |
| AtmiPEP394a1 (9,7 / 1977) | miR394a | *Arabidopsis thaliana* | Famille du gène F-*box*, impliqué dans le développement foliaire et résistance à la sécheresse | MSLQFYERVSFKNTVK | SEQ ID NO:89 |
| AtmiPEP395c1 (3,58 / 1429) | miR395c | *Arabidopsis thaliana* | Famille des gènes *APS* et AST, impliqués dans la germination et le métabolisme du soufre | MTEQEEESQMST | SEQ ID NO:90 |
| AtmiPEP395e1 (9,98 / 4700) | miR395e | *Arabidopsis thaliana* | | | SEQ ID NO:91 |
| AtmiPEP397b1 (4,53 / 1418) | miR397b | *Arabidopsis thaliana* | Famille des gènes de laccases, impliqués dans le métabolisme du cuivre, leur surexpression améliore la croissance | MSKEIFFSPGFE | SEQ ID NO:92 |
| AtmiPEP398c1 | miR398c | *Arabidopsis thaliana* | Famille du gène *CSD*, impliqué dans le métabolisme du cuivre, sa surexpression améliore la croissance | | SEQ ID NO:93 |
| AtmiPEP399b (11 /678) | miR399b | *Arabidopsis thaliana* | Famille du gène *PHO2*, impliqué dans le métabolisme du phosphore | MKRNM | SEQ ID NO:94 |
| AtmiPEP399d1 (4 / 622) | miR399d | *Arabidopsis thaliana* | | MQCEI | SEQ ID NO:95 |
| AtmiPEP403 (5,27470) | miR403 | *Arabidopsis thaliana* | Famille du gène *AGO* | MFCA | SEQ ID NO:96 |
| AtmiPEP447a1 (6,69 / 724) | miR447a | *Arabidopsis thaliana* | Famille des gènes de phosphoglycérate kinase | MVMAHH | SEQ ID NO:97 |
| AtmiPEP447a2 | miR447a | *Arabidopsis thaliana* | | | SEQ ID NO:98 |
| AtmiPEP447b1 (4/1155) | miR447b | *Arabidopsis thaliana* | | MLLIIVELVL | SEQ ID NO:99 |
| AtmiPEP447b2 | miR447b | *Arabidopsis thaliana* | | MLCFNFRCVRRFAE | SEQ ID NO:100 |
| AtmiPEP447c | miR447c | *Arabidopsis thaliana* | | | SEQ ID NO:101 |
| DmmiPEP1 a | miR1 | *Drosophila melanogaster* | *Differenciation musculaire* | | SEQ ID NO:102 |
| DmmiPEP1b | miR1 | *Drosophila melanogaster* | *Differenciation musculaire* | | SEQ ID NO:103 |
| DmmiPEP8 | miR8 | *Drosophila melanogaster* | Croissance | | SEQ ID NO:104 |
| HsmiPEP155 | miR155 | *Homo sapiens* | inflammation | MEMALMVAQTRKGKSVV | SEQ ID NO:355 |
| AtmiPEP157c (5,95 / 1776) | miR157c | *Arabidopsis thaliana* | Famille du gène *SPL*, impliqué dans le développement de la tige et la floraison | MMLHITHRFESDVGC | SEQ ID NO: 375 |
| AtmiPEP157d (5,27 / 524) | miR157d | *Arabidopsis thaliana* | | MLYV | SEQ ID NO: 376 |
| AtmiPEP160c (9,98 / 1790) | miR160c | *Arabidopsis thaliana* | Famille du gène ARF, impliqué dans la germination, le développement et la floraison | MFMRRGLVYNNIYI | SEQ ID NO: 377 |
| AtmiPEP164b (4,72 / 1949) | miR164b | *Arabidopsis thaliana* | Famille du gène NAC, impliqué dans le développement racinaire, foliaire et floral | MMKVCDEQDGEAGHVHY | SEQ ID NO: 378 |
| AtmiPEP166c (10,42 / 3407) | miR166c | *Arabidopsis thaliana* | Famille du gène HD-ZIPIII, impliqué dans développement vasculaire, racinaire, foliaire, floral, nodulation | | SEQ ID NO: 379 |
| AtmiPEP166d (8,35 / 1125) | miR166d | *Arabidopsis thaliana* | | MKKIGSIDSF | SEQ ID NO: 380 |
| AtmiPEP169a (9,5 / 784) | miR169a | *Arabidopsis thaliana* | Famille du gène du facteur CCAAT-bing, impliqué dans la nodulation, la résistance à la sécheresse, la résistance à la carence azotée | MTCRFK | SEQ ID NO: 381 |
| AtmiPEP169h1 (5,28 / 349) | miR169h | *Arabidopsis thaliana* | | MVT | |
| AtmiPEP169h2 | miR169h | *Arabidopsis thaliana* | | MKNENLCGSQG | SEQ ID NO: 382 |
| AtmiPEP169n (8,96 / 5315) | miR169n | *Arabidopsis thaliana* | | | SEQ ID NO: 383 |
| AtmiPEP170 (5,75 / 879) | miR170 | *Arabidopsis thaliana* | Famille du gène GRAS, impliqué dans le développement floral, foliaire, racinaire, la mycorhization, la nodulation | MFPRESL | SEQ ID NO: 384 |
| AtmiPEP396a (5,3 / 3636) | miR396a | *Arabidopsis thaliana* | Famille des genes GRF impliqués dans le développement racinaire et la prolifération cellulaire, la mycorhization | | SEQ ID NO: 385 |
| AtmiPEP399c (8,66/2703) | miR399c | *Arabidopsis thaliana* | Famille du gène PHO2, impliqué dans le métabolisme du phosphore | MSLAKGELPCHCFRLNTVYNRFC | SEQ ID NO: 386 |
| MtmiPEP169d | miR169d | *Medicago tuncatula* | Famille des gènes NF-YA (ou HAP2) | MVKESFMERLKVR | SEQ ID NO: 424 |

**Table 2. Liste des miORFs**

| **miPEP** | **Organisme** | **Séquence du miORF** | **SEQ ID** |
|---|---|---|---|
| AtmiPEP156a1 | *Arabidopsis thaliana* | | SEQ ID NO:105 |
| AtmiPEP156a2 | *Arabidopsis thaliana* | | SEQ ID NO:106 |
| AtmiPEP156a3 | *Arabidopsis thaliana* | | SEQ ID NO:107 |
| AtmiPEP156c1 | *Arabidopsis thaliana* | ATGAAGGACAACTTTCCTCTTCTCCTTCGGTTATAA | SEQ ID NO:108 |
| AtmiPEP156c2 | *Arabidopsis thaliana* | ATGAGTGATGACTGA | SEQ ID NO:109 |
| AtmiPEP156e1 | *Arabidopsis thaliana* | | SEQ ID NO:110 |
| AtmiPEP156f1 | *Arabidopsis thaliana* | ATGAGCCAAAGATAA | SEQ ID NO:111 |
| AlmiPEP159a | *Arabidopsis lyrata* | | SEQ ID NO:112 |
| AtmiPEP159a1 | *Arabidopsis thaliana* | | SEQ ID NO:113 |
| CrmiPEP159a | *Capsella rubella* | | SEQ ID NO:114 |
| AtmiPEP159b1 | *Arabidopsis thaliana* | ATGTTTTATCTTTCATAA | SEQ ID NO:115 |
| AtmiPEP159b2 | *Arabidopsis thaliana* | | SEQ ID NO:116 |
| AtmiPEP160a1 | *Arabidopsis thaliana* | | SEQ ID NO:117 |
| AtmiPEP160b1 | *Arabidopsis thaliana* | ATGTTTTCCCCTCAATGA | SEQ ID NO:118 |
| AtmiPEP160b2 | *Arabidopsis thaliana* | | SEQ ID NO:119 |
| AtmiPEP161 | *Arabidopsis thaliana* | | SEQ ID NO:120 |
| AtmiPEP162a1 | *Arabidopsis thaliana* | | SEQ ID NO:121 |
| AtmiPEP162b1 | *Arabidopsis thaliana* | | SEQ ID NO:122 |
| AtmiPEP163-1 | *Arabidopsis thaliana* | ATGTCCACTACTCAAGAGCATAGGTCTTGA | SEQ ID NO:123 |
| AtmiPEP163-2 | *Arabidopsis thaliana* | | SEQ ID NO:124 |
| AlmiPEP164a1 | *Arabidopsis lyrata* | ATGCCCTTAGCAGTTATTAGACAAGGGATTGTTTGGCCCTAG | SEQ ID NO:125 |
| AlmiPEP164a2 | *Arabidopsis lyrata* | | SEQ ID NO:126 |
| AlmiPEP164a3 | *Arabidopsis lyrata* | ATGACATGGTTCTTTTGCCTTACGTAA | SEQ ID NO:127 |
| AtmiPEP164a1 | *Arabidopsis thaliana* | | SEQ ID NO:128 |
| AtmiPEP164a2 | *Arabidopsis thaliana* | | SEQ ID NO:129 |
| AtmiPEP164a3 | *Arabidopsis thaliana* | ATGGTATGGTTCTTTTGCCTTACGTAA | SEQ ID NO:130 |
| BrmiPEP164a1 | *Brassica rapa* | ATGATGATAATTTTGTGGAAATAA | SEQ ID NO:131 |
| BrmiPEP164a2 | *Brassica rapa* | | SEQ ID NO:132 |
| BrmiPEP164a3 | *Brassica rapa* | | SEQ ID NO:133 |
| CpmiPEP164a1 | *Carica papaya* | | SEQ ID NO:134 |
| CpmiPEP164a2 | *Carica papaya* | | SEQ ID NO:135 |
| CrmiPEP164a1 | *Capsella rubella* | | SEQ ID NO:136 |
| CrmiPEP164a2 | *Capsella rubella* | ATGCCATCATGGCATGGCATGGCATGTTTCTATTGCCTTACGTAA | SEQ ID NO:137 |
| CrmiPEP164a3 | *Capsella rubella* | | SEQ ID NO:138 |
| GrmiPEP164a1 | *Gossypium raimondii* | | SEQ ID NO:139 |
| GrmiPEP164a2 | *Gossypium raimondii* | ATGTCAAATTCAAGGTCGTATCAGTTAAAATGA | SEQ ID NO:140 |
| GrmiPEP164a3 | *Gossypium raimondii* | | SEQ ID NO:141 |
| MtmiPEP164a1 | *Medicago truncatula* | | SEQ ID NO:142 |
| MtmiPEP164a2 | *Medicago truncatula* | | SEQ ID NO:143 |
| OsmiPEP164a1 | *Oryza sativa* | | SEQ ID NO:144 |
| OsmiPEP164a2 | *Oryza sativa* | | SEQ ID NO:145 |
| AlmiPEP165a | *Arabidopsis lyrata* | | SEQ ID NO:146 |
| AtmiPEP165a | *Arabidopsis thaliana* | | SEQ ID NO:147 |
| BcmiPEP165a | *Brassica carinata* | | SEQ ID NO:148 |
| BjmiPEP165a | *Brassica juncea* | | SEQ ID NO:149 |
| BnmiPEP165a | *Brassica napus* | | SEQ ID NO:150 |
| BomiPEP165a | *Brassica oleracea* | | SEQ ID NO:151 |
| BrmiPEP165a | *Brassica rapa* | | SEQ ID NO:152 |
| AtmiPEP166a | *Arabidopsis thaliana* | | SEQ ID NO:153 |
| AtmiPEP166b | *Arabidopsis thaliana* | ATGAGAGATAGATAA | SEQ ID NO:154 |
| AtmiPEP167a | *Arabidopsis thaliana* | ATGAACAGAAAAATCTCTCTTTCTCTTTCTTGA | SEQ ID NO:155 |
| AtnuPEP167b1 | *Arabidopsis thaliana* | ATGATGGGTTGTTTTGTGGGATTTTAA | SEQ ID NO:156 |
| AtmiPEP167b2 | *Arabidopsis thaliana* | ATGCAGGAGGAAACATATGAGGGGTGA | SEQ ID NO:157 |
| AtmiPEP169c1 | *Arabidopsis thaliana* | | SEQ ID NO:158 |
| AtmiPEP169c2 | *Arabidopsis thaliana* | | SEQ ID NO:159 |
| AtmiPEP169l1 | *Arabidopsis thaliana* | ATGAGACATAAAGAGAGTTAA | SEQ ID NO:160 |
| AtmiPEP171a1 | *Arabidopsis thaliana* | | SEQ ID NO:161 |
| AtmiPEP171b | *Arabidopsis thaliana* | ATGGTTCTCTCCGGTAAATTAACATTTTAG | SEQ ID NO:162 |
| MtmiPEP171b1 | *Medicago truncatula* | | SEQ ID NO:163 |
| MtmiPEP171b2 | *Medicago truncatula* | ATGAAGATTGAAGAGTAA | SEQ ID NO:164 |
| ZmmiPEP171b | *Zea mays* | | SEQ ID NO:165 |
| AtmiPEP171c1 | *Arabidopsis thaliana* | ATGTTGTCTCTTTCTCATTTTCATATCTGCTAA | SEQ ID NO:166 |
| MtmiPEP171e | *Medicago truncatula* | | SEQ ID NO:167 |
| MtmiPEP171h | *Medicago truncatula* | ATGGCTTCAGCTGCAAAAGTATACATGGCGTGA | SEQ ID NO:168 |
| AtmiPEP172a1 | *Arabidopsis thaliana* | ATGGCTTCCAAGATCTGGTAA | SEQ ID NO:169 |
| AtmiPEP172a3 | *Arabidopsis thaliana* | ATGGTTAGGTTCCAACTAAGTATACGAGATTAA | SEQ ID NO:170 |
| AtmiPEP172b1 | *Arabidopsis thaliana* | ATGTGTACGTACTATTATCTCATAAATAAATATTTTTAA | SEQ ID NO:171 |
| AtmiPEP172c1 | *Arabidopsis thaliana* | ATGTTTCCAGCAAAATGGTGCCGTCTTGAGTCTTGA | SEQ ID NO:172 |
| AtmiPEP172e1 | *Arabidopsis thaliana* | | SEQ ID NO:173 |
| AtmiPEP172e2 | *Arabidopsis thaliana* | | SEQ ID NO:174 |
| AtmiPEP172e3 | *Arabidopsis thaliana* | ATGGGAGTTCCCAACTTTAGACCTCGAAACCGATAA | SEQ ID NO:175 |
| AcmiPEP319a1 | *Arabidopsis cebennensis* | | SEQ ID NO:176 |
| AcmiPEP319a2 | *Arabidopsis cebennensis* | | SEQ ID NO:177 |
| AhmiPEP319a | *Arabidopsis halleri* | | SEQ ID NO:178 |
| AlmiPEP319a | *Arabidospis lyrata* | | SEQ ID NO:179 |
| AtmiPEP319a1 | *Arabidopsis thaliana* | | SEQ ID NO:180 |
| AtmiPEP319a2 | *Arabidopsis thaliana* | | SEQ ID NO:181 |
| BrmiPEP319a | *Brassica rapa* | | SEQ ID NO:182 |
| CpmiPEP319a | *Carica papaya* | | SEQ ID NO:183 |
| CrmiPEP319a | *Capsella rubella* | | SEQ ID NO:184 |
| EgmiPEP319a | *Eucalyptus grandis* | | SEQ ID NO:185 |
| GrmiPEP319a | *Gossypium raimondii* | | SEQ ID NO:186 |
| MtmiPEP319a | *Medicago truncatula* | | SEQ ID NO:187 |
| OsmiPEP319a | *Oryza sativa* | | SEQ ID NO:188 |
| PpmiPEP319a | *Physcomitrella patens* | | SEQ ID NO:189 |
| ThmiPEP319a1 | *Thellungiella halophila* | | SEQ ID NO:190 |
| ThmiPEP319a2 | *Thellungiella halophila* | | SEQ ID NO:191 |
| AtmiPEP319b1 | *Arabidopsis thaliana* | | SEQ ID NO:192 |
| AtmiPEP394a1 | *Arabidopsis thaliana* | | SEQ ID NO:193 |
| AtmiPEP395c1 | *Arabidopsis thaliana* | ATGACAGAGCAAGAAGAAGAAAGTCAAATGTCCACATGA | SEQ ID NO:194 |
| AtmiPEP395e1 | *Arabidopsis thaliana* | | SEQ ID NO:195 |
| AtmiPEP397b1 | *Arabidopsis thaliana* | ATGAGCAAGGAGATATTTTTTTCCCCTGGGTTTGAATGA | SEQ ID NO:196 |
| AtmiPEP398c1 | *Arabidopsis thaliana* | | SEQ ID NO:197 |
| AtmiPEP399b | *Arabidopsis thaliana* | ATGAAGAGAAACATGTAA | SEQ ID NO:198 |
| AtmiPEP399d1 | *Arabidopsis thaliana* | ATGCAATGTGAAATATGA | SEQ ID NO:199 |
| AtmiPEP403 | *Arabidopsis thaliana* | ATGTTTTGTGCTTGA | SEQ ID NO:200 |
| AtmiPEP447a1 | *Arabidopsis thaliana* | ATGGTCATGGCTCATCATTAG | SEQ ID NO:201 |
| AtmiPEP447a2 | *Arabidopsis thaliana* | | SEQ ID NO:202 |
| AtmiPEP447b1 | *Arabidopsis thaliana* | ATGCTGCTTATCATCGTGGAGTTGGTTCTGTAA | SEQ ID NO:203 |
| AtmiPEP447b2 | *Arabidopsis thaliana* | ATGCTTTGTTTCAATTTCAGGTGCGTTAGAAGGTTTGCAGAGTAG | SEQ ID NO:204 |
| AtmiPEP447 c | *Arabidopsis thaliana* | | SEQ ID NO:205 |
| dmmiPEPla | *Drosophila melanogaster* | | SEQ ID NO:206 |
| DmmiPEP1b | *Drosophila melanogaster* | | SEQ ID NO:207 |
| DmmiPEP8 | *Drosophila melanogaster* | | SEQ ID NO:208 |
| HsmiPEP155 | *Homo sapiens* | | SEQ ID NO:356 |
| AtmiPEP157c | *Arabidopsis thaliana* | | SEQ ID NO : 387 |
| AtmiPEP157d | *Arabidopsis thaliana* | ATGCTGTATGTATAG | SEQ ID NO : 388 |
| AtmiPEP160c | *Arabidopsis thaliana* | ATGTTCATGCGTAGAGGTTTGGTATACAACAATATATACATATAA | SEQ ID NO : 389 |
| AtmiPEP164b | *Arabidopsis thaliana* | | SEQ ID NO : 390 |
| AtmiPEP166c | *Arabidopsis thaliana* | | SEQ ID NO : 391 |
| AtmiPEP166d | *Arabidopsis thaliana* | ATGAAGAAGATCGGTAGtATTGATTCATTTTAA | SEQ ID NO : 392 |
| AtmiPEP169a | *Arabidopsis thaliana* | ATGACTTGCCGATTTAAATGA | SEQ ID NO : 393 |
| AtmiPEP169h1 | *Arabidopsis thaliana* | ATGGTGACATGA | SEQ ID NO : 394 |
| AtmiPEP169h2 | *Arabidopsis thaliana* | ATGAAGAATGAGAACTTGTGTGGTAGCCAAGGATGA | SEQ ID NO : 395 |
| AtmiPEP169n | *Arabidopsis thaliana* | | SEQ ID NO : 396 |
| AtmiPEP170 | *Arabidopsis thaliana* | ATGTTTCCGAGAGAGTCCCTCTGA | SEQ ID NO : 397 |
| AtmiPEP396a | *Arabidopsis thaliana* | | SEQ ID NO : 398 |
| AtmiPEP399c | *Arabidopsis thaliana* | | SEQ ID NO : 399 |
| MtmiPEP169d | *Medicago truncatula* | | SEQ ID NO : 425 |

**Table 3. Liste des transcrits primaires (pri-miRs)**

| **miPEP** | **Organisme** | **Séquence du Pri-miR** | **SEQ ID** |
|---|---|---|---|
| AtmiPEP156a1 | *Arabidopsis thaliana* | | SEQ ID NO:209 |
| AtmiPEP156a2 | | | |
| AtmiPEP156a3 | | | |
| AtmiPEP156c1 | *Arabidopsis thaliana* | | SEQ ID NO:210 |
| AtmiPEP156c2 | | | |
| AtmiPEP156e1 | *Arabidopsis thaliana* | | SEQ ID NO:211 |
| AtmiPEP156f1 | *Arabidopsis thaliana* | | SEQ ID NO:212 |
| AlmiPEP159a | *Arabidopsis lyrata* | | SEQ ID NO:213 |
| AtmiPEP159a1 | *Arabidopsis thaliana* | | SEQ ID NO:214 |
| CrmiPEP159a | *Capsella rubella* | | SEQ ID NO:215 |
| | | | |
| AtmiPEP159b1 | *Arabidopsis thaliana* | | SEQ ID NO:216 |
| AtmiPEP159b2 | | | |
| AtmiPEP160a1 | *Arabidopsis thaliana* | | SEQ ID NO:217 |
| AtmiPEP160b1 | *Arabidopsis thaliana* | | SEQ ID NO:218 |
| AtmiPEP160b2 | | | |
| AtmiPEP161 | *Arabidopsis thaliana* | | SEQ ID NO:219 |
| | | | |
| AtmiPEP162a1 | *Arabidopsis thaliana* | | SEQ ID NO:220 |
| AtmiPEP162b1 | *Arabidopsis thaliana* | | SEQ ID NO:221 |
| AtmiPEP163-1 | *Arabidopsis thaliana* | | SEQ ID NO:222 |
| AtmiPEP163-2 | | | |
| AlmiPEP164a1 | *Arabidopsis lyrata* | | SEQ ID NO:223 |
| AlmiPEP164a2 | | | |
| AlmiPEP164a3 | | | |
| | | | |
| AtmiPEP164a1 | *Arabidopsis thaliana* | | SEQ ID NO:224 |
| AtmiPEP164a2 | | | |
| AtmiPEP164a3 | | | |
| BrmiPEP164a1 | *Brassica rapa* | | SEQ ID NO:225 |
| BrmiPEP164a2 | | | |
| BrmiPEP164a3 | | | |
| CpmiPEP164a1 | *Carica papaya* | | SEQ ID NO:226 |
| CpmiPEP164a2 | | | |
| CrmiPEP164a1 | *Capsella rubella* | | SEQ ID NO:227 |
| CrmiPEP164a2 | | | |
| CrmiPEP164a3 | | | |
| | | | |
| GrmiPEP164a1 | *Gossypium raimondii* | | SEQ ID NO:228 |
| GrmiPEP164a2 | | | |
| GrmiPEP164a3 | | | |
| MtmiPEP164a1 | *Medicago truncatula* | | SEQ ID NO:229 |
| MtmiPEP164a2 | | | |
| OsmiPEP164a1 | *Oryza sativa* | | SEQ ID NO:230 |
| OsmiPEP164a2 | | | |
| | | | |
| AlmiPEP165a | *Arabidopsis lyrata* | | SEQ ID NO:231 |
| AtmiPEP165a | *Arabidopsis thaliana* | | SEQ ID NO:232 |
| BcmiPEP165a | *Brassica carinata* | | SEQ ID NO:233 |
| | | | |
| BjmiPEP165a | *Brassica juncea* | | SEQ ID NO:234 |
| BnmiPEP165a | *Brassica napus* | | SEQ ID NO:235 |
| BomiPEP165a | *Brassica oleracea* | | SEQ ID NO:236 |
| | | | |
| BrmiPEP165a | *Brassica rapa* | | SEQ ID NO:237 |
| AtmiPEP166a | *Arabidopsis thaliana* | | SEQ ID NO:238 |
| AtmiPEP166b | *Arabidopsis thaliana* | | SEQ ID NO:239 |
| AtmiPEP167a | *Arabidopsis thaliana* | | SEQ ID NO:240 |
| AtmiPEP167b1 | *Arabidopsis thaliana* | | SEQ ID NO:241 |
| AtmiPEP167b2 | | | |
| | | | |
| AtmiPEP169c1 | *Arabidopsis thaliana* | | SEQ ID NO:242 |
| AtmiPEP169c2 | | | |
| AtmiPEP169l1 | *Arabidopsis thaliana* | | SEQ ID NO:243 |
| AtmiPEP171a1 | *Arabidopsis thaliana* | | SEQ ID NO:244 |
| | | | |
| AtmiPEP171b | *Arabidopsis thaliana* | | SEQ ID NO:245 |
| MtmiPEP171b1 | *Medicago truncatula* | | SEQ ID NO:246 |
| MtmiPEP171b2 | | | |
| ZmmiPEP171b | *Zea mays* | | SEQ ID NO:247 |
| AtmiPEP171c1 | *Arabidopsis thaliana* | | SEQ ID NO:248 |
| MtmiPEP171e | *Medicago truncatula* | | SEQ ID NO:249 |
| MtmiPEP171h | *Medicago truncatula* | | SEQ ID NO:250 |
| AtmiPEP172a1 | *Arabidopsis thaliana* | | SEQ ID NO:251 |
| AtmiPEP172a3 | | | |
| AtmiPEP172b1 | *Arabidopsis thaliana* | | SEQ ID NO:252 |
| AtmiPEP172c1 | *Arabidopsis thaliana* | | SEQ ID NO:253 |
| AtmiPEP172e1 | *Arabidopsis thaliana* | | SEQ ID NO:254 |
| AtmiPEP172e2 | | | |
| AtmiPEP172e3 | | | |
| AcmiPEP319a1 | *Arabidopsis cebennensis* | | SEQ ID NO:255 |
| AcmiPEP319a2 | | | |
| AhmiPEP319a | *Arabidopsis halleri* | | SEQ ID NO:256 |
| | | | |
| AlmiPEP319a | *Arabidospis lyrata* | | SEQ ID NO:257 |
| AtmiPEP319a1 | *Arabidopsis thaliana* | | SEQ ID NO:258 |
| AtmiPEP319a2 | | | |
| BrmiPEP319a | *Brassica rapa* | | SEQ ID NO:259 |
| CpmiPEP319a | *Carica papaya* | | SEQ ID NO:260 |
| CrmiPEP319a | *Capsella rubella* | | SEQ ID NO:261 |
| | | | |
| EgmiPEP319a | *Eucalyptus grandis* | | SEQ ID NO:262 |
| GrmiPEP319a | *Gossypium raimondii* | | SEQ ID NO:263 |
| | | | |
| MtmiPEP319a | *Medicago truncatula* | | SEQ ID NO:264 |
| OsmiPEP319a | *Oryza sativa* | | SEQ ID NO:265 |
| PpmiPEP319a | *Physcomitrella patens* | | SEQ ID NO:266 |
| | | | |
| ThmiPEP319a1 | *Thellungiella halophila* | | SEQ ID NO:267 |
| ThmiPEP319a2 | | | |
| AtmiPEP319b1 | *Arabidopsis thaliana* | | SEQ ID NO:268 |
| | | | |
| AtmiPEP394a1 | *Arabidopsis thaliana* | | SEQ ID NO:269 |
| AtmiPEP395c1 | *Arabidopsis thaliana* | | SEQ ID NO:270 |
| AtmiPEP395e1 | *Arabidopsis thaliana* | | SEQ ID NO:271 |
| AtmiPEP397b1 | *Arabidopsis thaliana* | | SEQ ID NO:272 |
| AtmiPEP398c1 | *Arabidopsis thaliana* | | SEQ ID NO:273 |
| AtmiPEP399b | *Arabidopsis thaliana* | | SEQ ID NO:274 |
| AtmiPEP399d1 | *Arabidopsis thaliana* | | SEQ ID NO:275 |
| AtmiPEP403 | *Arabidopsis thaliana* | | SEQ ID NO:276 |
| AtmiPEP447a1 | *Arabidopsis thaliana* | | SEQ ID NO:277 |
| AtmiPEP447a2 | | | |
| AtmiPEP447b1 | *Arabidopsis thaliana* | | SEQ ID NO:278 |
| AtmiPEP447b2 | | | |
| | | | |
| AtmiPEP447c | *Arabidopsis thaliana* | | SEQ ID NO:279 |
| DmmiPEP1a | *Drosophila melanogaster* | | SEQ ID NO:280 |
| DmmiPEP1b | | | |
| | | | |
| DmmiPEP8 | *Drosophila melanogaster* | | SEQ ID NO:281 |
| HsmiPEP155 | *Homo sapiens* | | SEQ ID NO:357 |
| | | | |
| AtmiPEP157c | *Arabidopsis thaliana* | | SEQ ID NO: 400 |
| AtmiPEP157d | *Arabidopsis thaliana* | | SEQ ID NO: 401 |
| AtmiPEP160c | *Arabidopsis thaliana* | | SEQ ID NO: 402 |
| AtmiPEP164b | *Arabidopsis thaliana* | | SEQ ID NO: 403 |
| AtmiPEP166c | *Arabidopsis thaliana* | | SEQ ID NO: 404 |
| | | | |
| AtmiPEP166d | *Arabidopsis thaliana* | | SEQ ID NO: 405 |
| AtmiPEP169a | *Arabidopsis thaliana* | | SEQ ID NO: 406 |
| AtmiPEP169h1 | *Arabidopsis thaliana* | | SEQ ID NO: 407 |
| AtmiPEP169h2 | | | |
| AtmiPEP169n | *Arabidopsis thaliana* | | SEQ ID NO: 408 |
| AtmiPEP170 | *Arabidopsis thaliana* | | SEQ ID NO: 409 |
| AtmiPEP396a | *Arabidopsis thaliana* | | SEQ ID NO: 410 |
| | | | |
| AtmiPEP399c | *Arabidopsis thaliana* | | SEQ ID NO: 411 |
| MtmiPEP169d | *Medicago truncatula* | | SEQ ID NO: 426 |

**Table 4. Liste des microARNs (miRs)**

| **miPEP** | **Organisme** | **Séquence du miR** | **SEQ ID** |
|---|---|---|---|
| AtmiPEP156a1 | *Arabidopsis thaliana* | ugacagaagagagugagcac | SEQ ID NO:282 |
| AtmiPEP156a2 | | | |
| AtmiPEP156a3 | | | |
| AtmiPEP156c1 | *Arabidopsis thaliana* | ugacagaagagagugagcac | SEQ ID NO:283 |
| AtmiPEP156c2 | | | |
| AtmiPEP156e1 | *Arabidopsis thaliana* | ugacagaagagagugagcac | SEQ ID NO:284 |
| AtmiPEP156f1 | *Arabidopsis thaliana* | ugacagaagagagugagcac | SEQ ID NO:285 |
| AlmiPEP159a | *Arabidopsis lyrata* | uuuggauugaagggagcucua | SEQ ID NO:286 |
| AtmiPEP159a1 | *Arabidopsis thaliana* | uuuggauugaagggagcucua | SEQ ID NO:287 |
| CrmiPEP159a | *Capsella rubella* | uuuggauugaagggagcucua | SEQ ID NO:288 |
| AtmiPEP159b1 | *Arabidopsis thaliana* | uuuggauugaagggagcucuu | SEQ ID NO:289 |
| AtmiPEP159b2 | | | |
| AtmiPEP160a1 | *Arabidopsis thaliana* | ugccuggcucccuguaugcca | SEQ ID NO:290 |
| AtmiPEP160b1 | *Arabidopsis thaliana* | ugccuggcucccuguaugcca | SEQ ID NO:291 |
| AtmiPEP160b2 | | | |
| AtmiPEP161 | *Arabidopsis thaliana* | ucaaugcauugaaagugacua | SEQ ID NO:292 |
| AtmiPEP162a1 | *Arabidopsis thaliana* | ucgauaaaccucugcauccag | SEQ ID NO:293 |
| AtmiPEP162b1 | *Arabidopsis thaliana* | ucgauaaaccucugcauccag | SEQ ID NO:294 |
| AtmiPEP163-1 | *Arabidopsis thaliana* | uugaagaggacuuggaacuucgau | SEQ ID NO:295 |
| AtmiPEP163-2 | | | |
| AlmiPEP164a1 | *Arabidopsis lyrata* | uggagaagcagggcacgugca | SEQ ID NO:296 |
| AlmiPEP164a2 | | | |
| AlmiPEP164a3 | | | |
| AtmiPEP164a1 | *Arabidopsis thaliana* | uggagaagcagggcacgugca | SEQ ID NO:297 |
| AtmiPEP164a2 | | | |
| AtmiPEP164a3 | | | |
| BrmiPEP164a1 | *Brassica rapa* | uggagaagcagggcacgugca | SEQ ID NO:298 |
| BrmiPEP164a2 | | | |
| BrmiPEP164a3 | | | |
| CpmiPEP164a1 | *Carica papaya* | uggagaagcagggcacgugca | SEQ ID NO:299 |
| CpmiPEP164a2 | | | |
| CrmiPEP164a1 | *Capsella rubella* | uggagaagcagggcacgugca | SEQ ID NO:300 |
| CrmiPEP164a2 | | | |
| CrmiPEP164a3 | | | |
| GrmiPEP164a1 | *Gossypium raimondii* | uggagaagcagggcacgugca | SEQ ID NO:301 |
| GrmiPEP164a2 | | | |
| GrmiPEP164a3 | | | |
| MtmiPEP164a1 | *Medicago truncatula* | uggagaagcagggcacgugca | SEQ ID NO:302 |
| MtmiPEP164a2 | | | |
| OsmiPEP164a1 | *Oryza sativa* | uggagaagcaggguacgugca | SEQ ID NO:303 |
| OsmiPEP164a2 | | | |
| AlmiPEP165a | *Arabidopsis lyrata* | ucggaccaggcuucauccccc | SEQ ID NO:304 |
| AtmiPEP165a | *Arabidopsis thaliana* | ucggaccaggcuucauccccc | SEQ ID NO:305 |
| BcmiPEP165a | *Brassica carinata* | ucggaccaggcuucauccccc | SEQ ID NO:306 |
| BjmiPEP165a | *Brassica juncea* | ucggaccaggcuucauccccc | SEQ ID NO:307 |
| BnmiPEP165a | *Brassica napus* | ucggaccaggcuucauccccc | SEQ ID NO:308 |
| BomiPEP165a | *Brassica oleracea* | ucggaccaggcuucauccccc | SEQ ID NO:309 |
| BrmiPEP165a | *Brassica rapa* | ucggaccaggcuucauccccc | SEQ ID NO:310 |
| AtmiPEP166a | *Arabidopsis thaliana* | ucggaccaggcuucauucccc | SEQ ID NO:311 |
| AtmiPEP166b | *Arabidopsis thaliana* | ucggaccaggcuucauucccc | SEQ ID NO:312 |
| AtmiPEP167a | *Arabidopsis thaliana* | ugaagcugccagcaugaucua | SEQ ID NO:313 |
| AtmiPEP167b1 | *Arabidopsis thaliana* | ugaagcugccagcaugaucua | SEQ ID NO:314 |
| AtmiPEP167b2 | | | |
| AtmiPEP169c1 | *Arabidopsis thaliana* | cagccaaggaugacuugccgg | SEQ ID NO:315 |
| AtmiPEP169c2 | | | |
| AtmiPEP169l1 | *Arabidopsis thaliana* | uagccaaggaugacuugccug | SEQ ID NO:316 |
| AtmiPEP171a1 | *Arabidopsis thaliana* | ugauugagccgcgccaauauc | SEQ ID NO:317 |
| AtmiPEP171b | *Arabidopsis thaliana* | uugagccgugccaauaucacg | SEQ ID NO:318 |
| MtmiPEP171b1 | *Medicago truncatula* | ugauugagccgcgucaauauc | SEQ ID NO:319 |
| MtmiPEP171b2 | | | |
| ZmmiPEP171b | *Zea mays* | ggauugagccgcgucaauauc | SEQ ID NO:320 |
| AtmiPEP171c1 | *Arabidopsis thaliana* | uugagccgugccaauaucacg | SEQ ID NO:321 |
| MtmiPEP171e | *Medicago truncatula* | agauugagccgcgccaauauc | SEQ ID NO:322 |
| MtmiPEP171h | *Medicago truncatula* | cgagccgaaucaauaucacuc | SEQ ID NO:323 |
| AtmiPEP172a1 | *Arabidopsis thaliana* | agaaucuugaugaugcugcau | SEQ ID NO:324 |
| AtmiPEP172a3 | | | |
| AtmiPEP172b1 | *Arabidopsis thaliana* | gcagcaccauuaagauucac | SEQ ID NO:325 |
| AtmiPEP172c1 | *Arabidopsis thaliana* | agaaucuugaugaugcugcag | SEQ ID NO:326 |
| AtmiPEP172e1 | *Arabidopsis thaliana* | ggaaucuugaugaugcugcau | SEQ ID NO:327 |
| AtmiPEP172e2 | | | |
| AtmiPEP172e3 | | | |
| AcmiPEP319a1 | *Arabidopsis cebennensis* | uuggacugaagggagcucccu | SEQ ID NO:328 |
| AcmiPEP319a2 | | | |
| AhmiPEP319a | *Arabidopsis halleri* | uuggacugaagggagcucccu | SEQ ID NO:329 |
| AlmiPEP319a | *Arabidospis lyrata* | uuggacugaagggagcucccu | SEQ ID NO:330 |
| AtmiPEP319a1 | *Arabidopsis thaliana* | uuggacugaagggagcucccu | SEQ ID NO:331 |
| AtmiPEP319a2 | | | |
| BrmiPEP319a | *Brassica rapa* | uuggacugaagggagcucccu | SEQ ID NO:332 |
| CpmiPEP319a | *Carica papaya* | uuggacugaagggagcuccuu | SEQ ID NO:333 |
| CrmiPEP319a | *Capsella rubella* | uuggacugaagggagcucc | SEQ ID NO:334 |
| EgmiPEP319a | *Eucalyptus grandis* | uuggacugaagggagcucccu | SEQ ID NO:335 |
| GrmiPEP319a | *Gossypium raimondii* | uuggacugaagggagcucccu | SEQ ID NO:336 |
| MtmiPEP319a | *Medicago truncatula* | uuggacugaagggagucucccu | SEQ ID NO:337 |
| OsmiPEP319a | *Oryza sativa* | uuggacugaagggugcucccu | SEQ ID NO:338 |
| PpmiPEP319a | *Physcomitrella patens* | cuuggacugaagggagcucc | SEQ ID NO:339 |
| ThmiPEP319a1 | *Thellungiella halophila* | uggacucaaggaagcucucu | SEQ ID NO:340 |
| ThmiPEP319a2 | | | |
| AtmiPEP319b1 | *Arabidopsis thaliana* | uuggacugaagggagcucccu | SEQ ID NO:341 |
| AtmiPEP394a1 | *Arabidopsis thaliana* | uuggcauucuguccaccucc | SEQ ID NO:342 |
| AtmiPEP395c1 | *Arabidopsis thaliana* | cugaaguguuuggggggacuc | SEQ ID NO:343 |
| AtmiPEP395e1 | *Arabidopsis thaliana* | cugaaguguuugggggaacuc | SEQ ID NO:344 |
| AtmiPEP397b1 | *Arabidopsis thaliana* | ucauugagugcaucguugaug | SEQ ID NO:345 |
| AtmiPEP398c1 | *Arabidopsis thaliana* | uguguucucaggucaccccug | SEQ ID NO:346 |
| AtmiPEP399b | *Arabidopsis thaliana* | ugccaaaggagaguugcccug | SEQ ID NO:347 |
| AtmiPEP399d1 | *Arabidopsis thaliana* | ugccaaaggagauuugccccg | SEQ ID NO:348 |
| AtmiPEP403 | *Arabidopsis thaliana* | uuagauucacgcacaaacucg | SEQ ID NO:349 |
| AtmiPEP447a1 | *Arabidopsis thaliana* | uuggggacgagauguuuuguug | SEQ ID NO:350 |
| AtmiPEP447a2 | *Arabidopsis thaliana* | uuggggacgagauguuuuguug | |
| AtmiPEP447b1 | *Arabidopsis thaliana* | uuggggacgagauguuuuguug | SEQ ID NO:351 |
| AtmiPEP447b2 | *Arabidopsis thaliana* | uuggggacgagauguuuuguug | |
| AtmiPEP447c | *Arabidopsis thaliana* | ccccuuacaaugucgaguaaa | SEQ ID NO:352 |
| DmmiPEP1a | *Drosophila melanogaster* | uggaauguaaagaaguauggag | SEQ ID NO:353 |
| DmmiPEP1b | | | |
| DmmiPEP8 | *Drosophila melanogaster* | uaauacugucagguaaagauguc | SEQ ID NO:354 |
| HsmiPEP155 | *Homo sapiens* | uuaaugcuaaucgugauaggggu | SEQ ID NO:358 |
| AtmiPEP157c | *Arabidopsis thaliana* | uugacagaagauagagagcac | SEQ ID NO : 412 |
| AtmiPEP157d | *Arabidopsis thaliana* | ugacagaagauagagagcac | SEQ ID NO : 413 |
| AtmiPEP160c | *Arabidopsis thaliana* | ugccuggcucccuguaugcca | SEQ ID NO : 414 |
| AtmiPEP164b | *Arabidopsis thaliana* | uggagaagcagggcacgugca | SEQ ID NO : 415 |
| AtmiPEP166c | *Arabidopsis thaliana* | ucggaccaggcuucauucccc | SEQ ID NO : 416 |
| AtmiPEP166d | *Arabidopsis thaliana* | ucggaccaggcuucauucccc | SEQ ID NO : 417 |
| AtmiPEP169a | *Arabidopsis thaliana* | cagccaaggaugacuugccga | SEQ ID NO : 418 |
| AtmiPEP169h | *Arabidopsis thaliana* | uagccaaggaugacuugccug | SEQ ID NO : 419 |
| AtmiPEP169n | *Arabidopsis thaliana* | uagccaaggaugacuugccug | SEQ ID NO : 420 |
| AtmiPEP170 | *Arabidopsis thaliana* | ugauugagccgugucaauauc | SEQ ID NO : 421 |
| AtmiPEP396a | *Arabidopsis thaliana* | uuccacagcuuucuugaacug | SEQ ID NO : 422 |
| AtmiPEP399c | *Arabidopsis thaliana* | ugccaaaggagaguugcccug | SEQ ID NO : 423 |
| AtmiPEP169d | *Medicago truncatula* | aagccaaggaugacuugccgg | SEQ ID NO : 427 |

**Table 5. Liste des Pre-miRs témoins**

| **Pre-miR** | **Organisme** | **Séquence du Pre-miR** | **SEQ ID** |
|---|---|---|---|
| Pre-miR169 | *Medicago truncatula* | | SEQ ID NO:359 |
| Pre-miR169a | *Medicago truncatula* | | SEQ ID NO:360 |
| Pre-miR171a MI 0001753 | *Medicago truncatula* | | SEQ ID NO:361 |
| Pre-miR171h | *Medicago truncatula* | | SEQ ID NO:362 |
| Pre-miR393a | *Medicago truncatula* | | SEQ ID NO:363 |
| Pre-miR393b | *Medicago truncatula* | | SEQ ID NO:364 |
| Pre-miR396a | *Medicago truncatula* | | SEQ ID NO:365 |
| Pre-miR396b | *Medicago truncatula* | | SEQ ID NO:366 |

**Table 6. Liste des miRs témoins**

| **miR** | **Organisme** | **Séquence du miR** | **SEQ ID** |
|---|---|---|---|
| miR169 | *Medicago truncatula* | CAGCCAAGGAUGACUUGCCGG | SEQ ID NO:367 |
| miR169a | *Medicago truncatula* | CAGCCAAGGAUGACUUGCCGA | SEQ ID NO:368 |
| miR171a | *Medicago truncatula* | UGAUUGAGUCGUGCCAAUAUC | SEQ ID NO:369 |
| miR171h | *Medicago truncatula* | GAGCCGAAUCAAUAUCACUC | SEQ ID NO:370 |
| miR393a | *Medicago truncatula* | UCCAAAGGGAUCGCAUUGAUC | SEQ ID NO:371 |
| miR393b | *Medicago truncatula* | UCCAAAGGGAUCGCAUUGAUC | SEQ ID NO:372 |
| miR396a | *Medicago truncatula* | UUCCACAGCUUUCUUGAACUU | SEQ ID NO:373 |
| miR396b | *Medicago truncatula* | UUCCACAGCUUUCUUGAACUG | SEQ ID NO:374 |

**Table 7. Polymorphisme de la séquence d'ADN des différentes régions du pri-miR171b**

| | Taille | # SNPs | # mutations | %SNP | # haplotypes |
|---|---|---|---|---|---|
| pri-mir171b | 1127 | 91 | 100 | 8,07 | 161 |
| 5' pri-miR171b | 129 | 4 | 4 | 3,1 | 5 |
| miPEP171b | 62 | 2 | 2 | 3,22 | 3 |
| Pre-miR171b | 118 | 1 | 1 | 0,85 | 2 |
| miR171b+miR171b* | 42 | 0 | 0 | 0 | 1 |
| 3' pri-miR171b | 259 | 39 | 42 | 15,06 | 89 |

Les figures et les exemples suivants illustreront mieux l'invention, sans pour autant en limiter sa portée.

### LEGENDES DES FIGURES

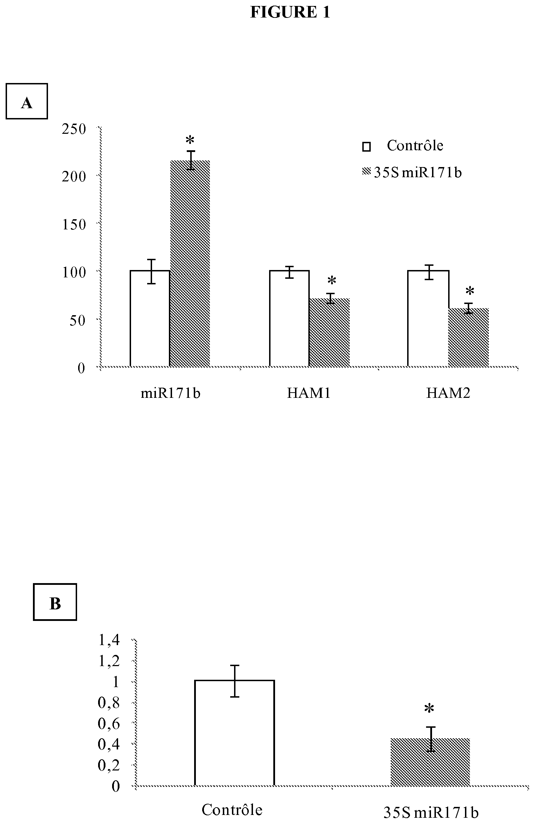

**FIGURE 1****. Effets de la surexpression du MtmiR171b (miR171b identifié chez *Medicago truncatula*) sur l'expression des gènes *HAM1* et *HAM2* (A) ou sur le nombre de racines latérales (B) chez *M. truncatula.***
   (A) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante dans laquelle est surexprimé MtmiR171b (colonnes noires). La barre d'erreur correspond à l'erreur standard à la moyenne (nombre d'individus = 10). La surexpression de MtmiR171b induit une diminution de l'expression des gènes *HAM1* et *HAM2.*
   (B) L'axe des ordonnées indique le nombre moyen de racines latérales observées chez une plante témoin (colonne blanche) ou chez une plante dans laquelle est surexprimé MtmiR171b (colonne noire). La barre d'erreur correspond à l'erreur standard à la moyenne (nombre d'individus = 100). La surexpression de MtmiR171b entraîne une réduction du nombre de racines latérales.

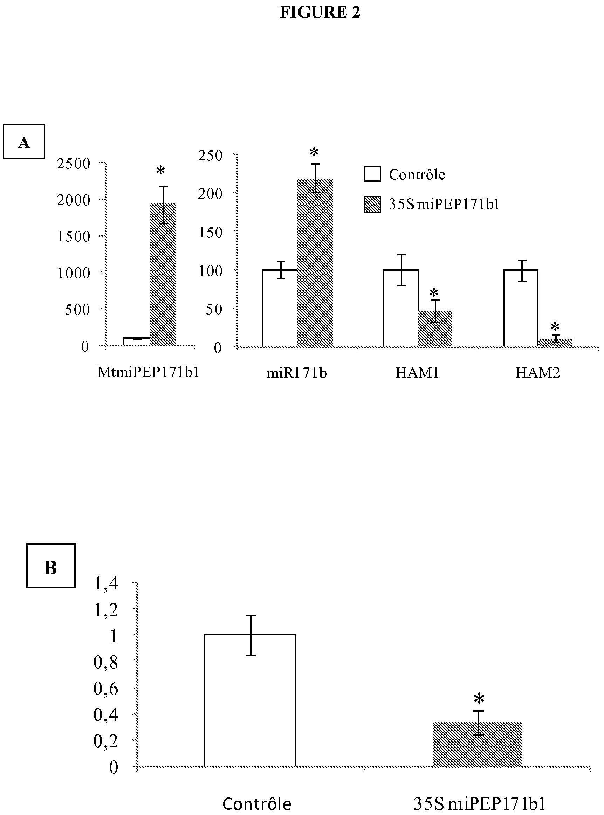

**FIGURE 2****. Effets de la surexpression du MtmiPEP171b1 sur l'expression du MtmiR171b et des gènes *HAM1* et *HAM2* (A) ou sur le nombre racines latérales (B) chez *M. truncatula.***
   (A) L'axe des ordonnées indique l'expression relative du MtmiPEP171b1 (graphique de gauche), du miR171b (graphique de droite, colonnes de gauche), de *HAM1* (accession n°MtGI9- TC114268) (graphique de droite, colonnes du milieu) ou de *HAM2* (accession n° MtGI9- TC120850) (graphique de droite, colonnes de droite) chez une plante témoin (colonnes blanches) ou chez une plante dans laquelle est surexprimé MtmiPEP171b1 (colonnes noires). La barre d'erreur correspond à l'erreur standard à la moyenne (nombre d'individus = 10). La surexpression de MtmiPEP171b1 induit une augmentation de l'accumulation de MtmiR171b, ainsi qu'une diminution de l'expression des gènes *HAM1* et *HAM2.*
   (B) L'axe des ordonnées indique le nombre moyen de racines latérales observées chez une plante témoin (colonne blanche) ou chez une plante dans laquelle est surexprimé MtmiPEP171b1 (colonne noire). La barre d'erreur correspond à l'erreur standard à la moyenne (nombre d'individus = 100). La surexpression de MtmiPEP171b1 entraîne une réduction du nombre de racines latérales.

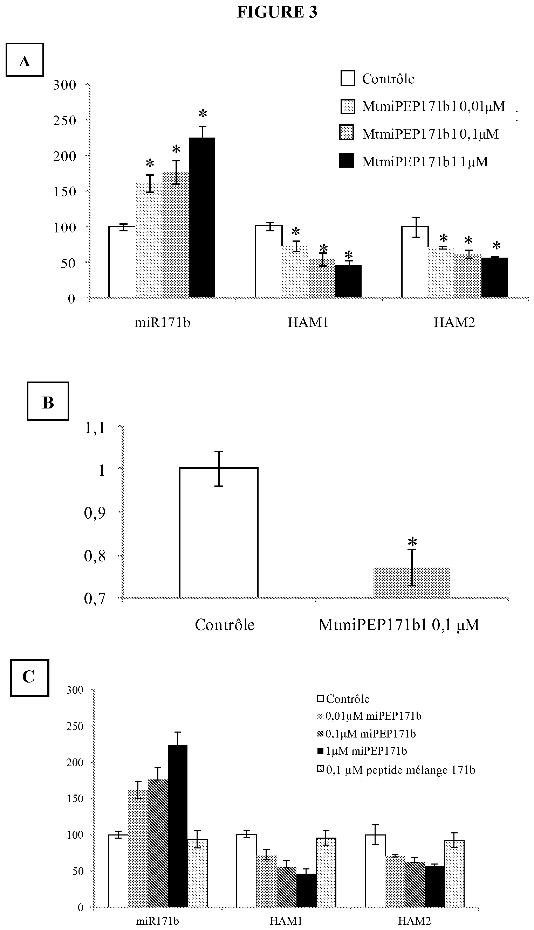

**FIGURE 3****. Effets du MtmiPEP171b1 sur l'expression du MtmiR171b et des gènes *HAM1* et *HAM2* (A) et sur le nombre racines latérales (B) chez *M. truncatula.***
   (A) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante traitée par arrosage pendant 5 jours et 1 fois par jour avec le MtmiPEP171b1 à 0,01 µM (colonnes gris clair), 0,1 µM (colonnes gris foncé) ou 1µM (colonnes noires). La barre d'erreur correspond à l'erreur standard à la moyenne (nombre d'individus = 10). L'application du MtmiPEP171b1 à différentes concentrations induit une augmentation de l'accumulation de MtmiR171b, ainsi qu'une diminution de l'expression des gènes *HAM1* et *HAM2.*
   (B) L'axe des ordonnées indique le nombre moyen de racines latérales observées chez une plante témoin (colonne blanche) ou chez une plante traitée par arrosage avec le MtmiPEP171b1 à 0,1 µM pendant 5 jours et 1 fois par jour (colonne noire). La barre d'erreur correspond à l'erreur standard à la moyenne (nombre d'individus = 100). L'application du MtmiPEP171b1 à 0,1 µM entraîne une réduction du nombre de racines latérales.
   (C) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante traitée par arrosage pendant 5 jours et 1 fois par jour avec le MtmiPEP171b1 à 0,01 µM (colonnes gris), 0,1 µM (colonnes gris foncé) ou 1µM (colonnes noires) ou avec 0,01 µM d'un peptide mélange (LIVSHLYSEKFDCMRKILRI, SEQ ID NO : 428)(colonnes gris clair) dont la composition en acides aminés est identique au miPEP171b mais dont la séquence est différente. La barre d'erreur correspond à l'erreur standard à la moyenne (nombre d'individus = 10).

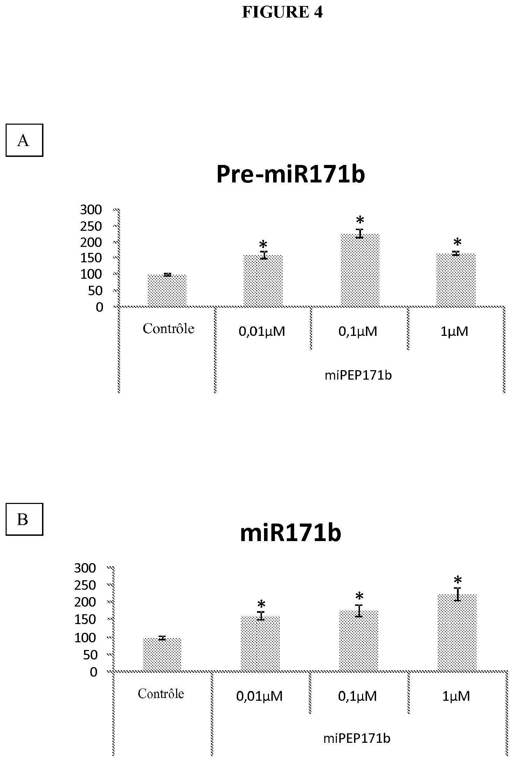

**FIGURE 4****. Effets du MtmiPEP171b1 sur l'expression du pre-MtmiR171b (A) et du MtmiR171b (B) chez *M. truncatula.***
   L'axe des ordonnées indique l'expression relative des précurseurs des différentes formes du microARN chez des plantes contrôles (colonne de gauche) ou chez des plantes traitées par arrosage pendant 5 jours et 1 fois par jour avec le MtmiPEP171b1 à 0,01 µM, 0,1 µM ou 1 µM (colonnes de droite). La barre d'erreur correspond à l'erreur standard à la moyenne (nombre d'individus = 200). L'application du MtmiPEP171b1 à différentes concentrations entraîne une augmentation de l'accumulation du pre-MtmiR171b (A) et du MtmiR171b (B).

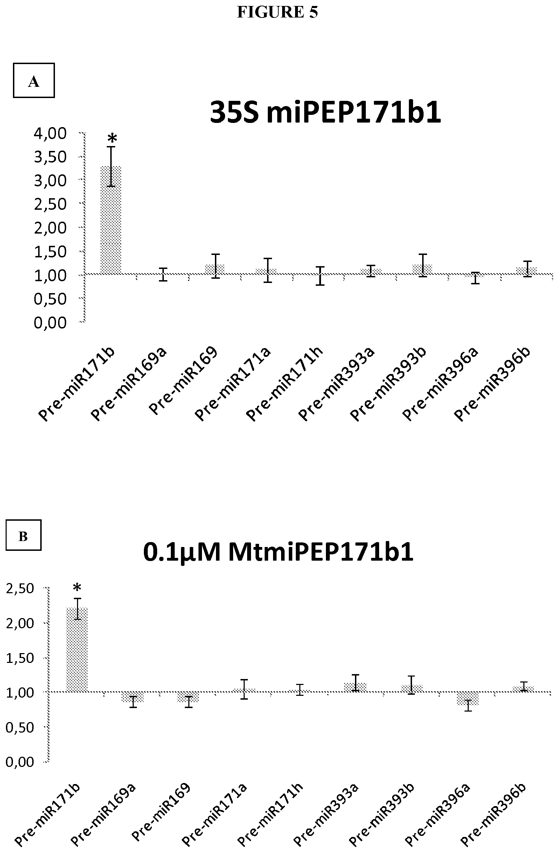

**FIGURE 5****. Effets de la surexpression du MtmiPEP171b1 (A) et effets du MtmiPEP171b1 (B) sur l'expression de différents précurseurs de microARNs chez *M*. *truncatula.***
   L'axe des ordonnées indique le ratio de l'expression des précurseurs de microARNs dans des plantes surexprimant le MtmiPEP171b1 sur l'expression de ces mêmes précurseurs dans des racines contrôle (A), ou le ratio de l'expression des précurseurs de microARNs dans des plantes traitées avec le MtmiPEP171b1 (0,1 µM) sur l'expression de ces mêmes précurseurs dans des racines contrôle (B). Les différents précurseurs de microARNs testés sont indiqués de gauche à droite sur l'axe des abscisses, à savoir pre-MtmiR171b (SEQ ID NO : 246), pre-MtmiR169 (SEQ ID NO : 359), pre-MtmiR169a (SEQ ID NO : 360), pre-MtmiR171a (SEQ ID NO : 361), pre-MtmiR171h (SEQ ID NO : 362), pre-MtmiR393a (SEQ ID NO : 363), pre-MtmiR393b (SEQ ID NO: 364), pre-MtmiR396a (SEQ ID NO: 365) et pre-MtmiR396b (SEQ ID NO : 366). La barre d'erreur correspond à l'erreur standard à la moyenne (nombre d'individus = 10). On constate que le MtmiPEP171b1 n'entraîne un effet que sur l'accumulation du MtmiR171b et pas sur les autres miRs.

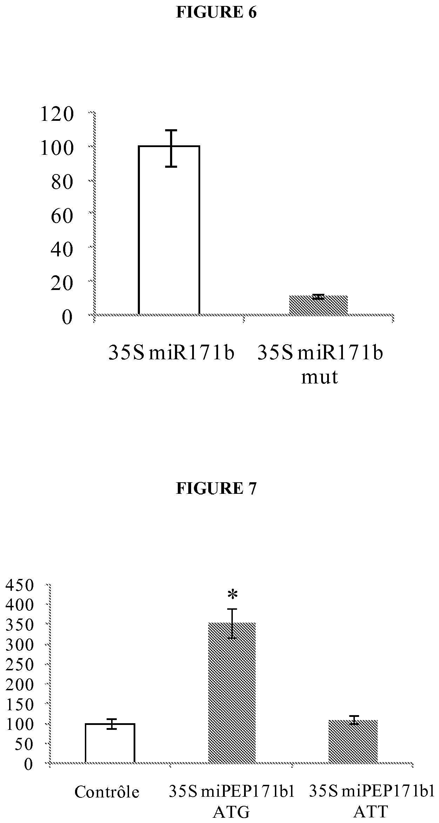

**FIGURE 6****. Effets de la traduction du MtmiPEP171b1 sur l'expression du MtmiR171b mis en évidence chez la plante modèle** ***Nicotiana benthamiana.*** L'axe des ordonnées indique l'expression relative du MtmiR171b chez des plantes de tabac transformées pour exprimer le pri-MtmiR171b (colonne blanche) ou un pri-MtmiR171b muté dans lequel le codon ATG a été remplacé par ATT (colonne noire). Le pri-MtmiR171b muté est donc incapable de produire le MtmiPEP171bl. La barre d'erreur correspond à l'erreur standard à la moyenne (nombre d'individus = 30). On constate que l'absence de la traduction du MtmiPEP171b1 entraîne une forte diminution de l'accumulation du miR171b.
**FIGURE 7****. Effets de la surexpression du MtmiPEP171b1 sur l'expression du pre-MtmiR171b mis en évidence chez la plante modèle *Nicotiana benthamiana.***
   L'axe des ordonnées indique l'expression relative du pre-MtmiR171b chez des plantes de tabac qui ont été transformées pour exprimer le MtmiR171b (colonne de gauche), le MtmiR171b et le MtmiPEP171b1 (colonne du centre), ou le MtmiR171b et une version mutée du MtmiORF171b dans laquelle le codon d'initiation ATG a été remplacé ATT (colonne de droite). La barre d'erreur correspond à l'erreur standard à la moyenne (nombre d'individus = 30). On constate que l'expression du MtmiPEP171b1 augmente l'expression du MtmiR171b, et cet effet est dépendant de la traduction du MtmiORF171b en MtmiPEP171b1.

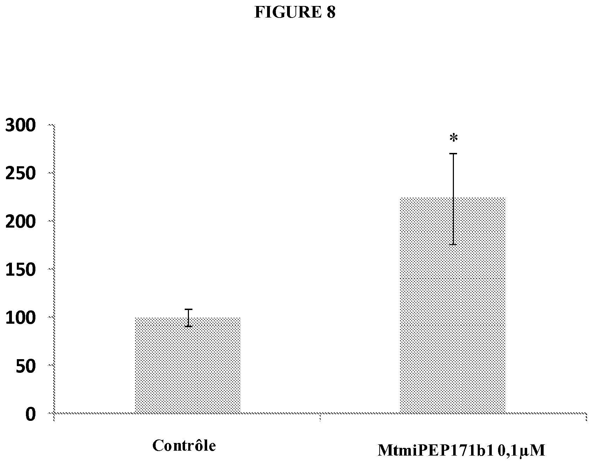

**FIGURE 8****. Effets du MtmiPEP171b1 sur l'expression du pre-MtmiR171b mis en évidence chez la plante modèle *Nicotiana benthamiana.***
   L'axe des ordonnées indique l'expression relative du MtmiR171b chez des plantes de tabac transformées pour exprimer le MtmiR171b sur lesquelles le MtmiPEP171b1 a été pulvérisé (0,1 µM) 2 fois, 12h puis 30min avant prélèvement (colonne de droite) ou non (colonne de gauche). La barre d'erreur correspond à l'erreur standard à la moyenne (nombre d'individus = 6). Le peptide MtmiPEP171b1 utilisé par pulvérisation induit une augmentation de l'accumulation du MtmiR171b.

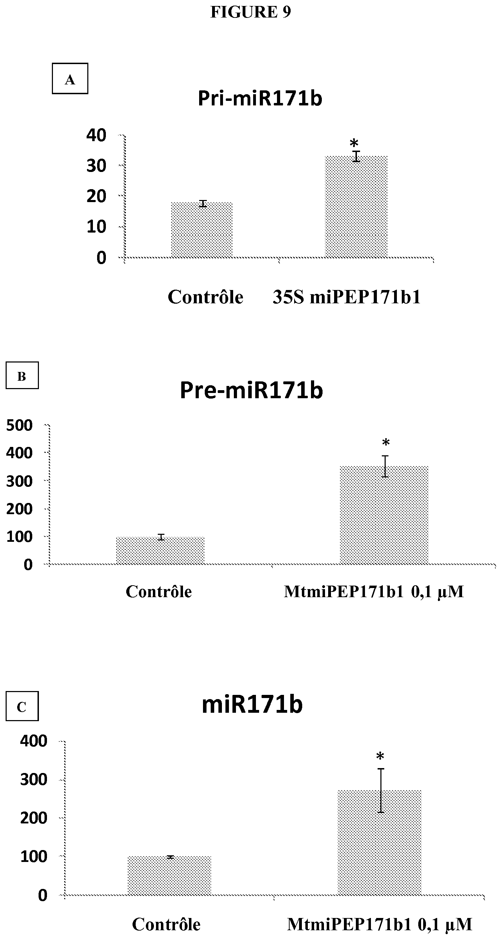

**FIGURE 9****. Effets du MtmiPEP171b1 sur l'expression du pri-miR171b (A), du pre-MtmiR171b (B) et du MtmiR171b (C) mis en évidence chez la plante modèle *Nicotiana benthamiana.***
   L'axe des ordonnées indique l'expression relative des précurseurs des différentes formes du microARN chez des plantes de tabac modifiées pour exprimer le MtmiR171b (colonne de gauche) ou modifiées pour exprimer le MtmiR171b et surexprimer le MtmiPEP171b1 (colonnes de droite, fig. 9A) ou traitées par 0.1µM de miPEP171b1 (Fig. 9B et C). La barre d'erreur correspond à l'erreur standard à la moyenne (nombre d'individus = 30). La surexpression du MtmiPEP171b1 ou l'application du miPEP171b1 augmente l'accumulation du pri-MtmiR171b (A), du pre-MtmiR171b (B) et du MtmiR171b (C).
**FIGURE 10****. Localisation du MtmiPEP171b1 dans les cellules de feuilles de tabac qui ont été modifiées pour exprimer le MtmiPEP171b1.**
   Les photographies représentent des cellules de feuilles de tabac modifiées pour exprimer la protéine GFP seule (cadre de gauche) ou la protéine GFP fusionnée au MtmiPEP171b1 (cadre de droite). Ces observations indiquent que le MtmiPEP171b1 est localisé dans des petits corps nucléaires.
**FIGURE 11****. Effets de l'expression du AtmiPEP165a (identifié chez *Arabidopsis thaliana*) sur l'expression du AtmiR165a (A) et de l'expression du AtmiPEP319a2 (identifié chez *Arabidopsis thaliana*) sur le AtmiR319a (B), mis en évidence chez la plante modèle du tabac.**
   (A) L'axe des ordonnées indique l'expression relative du AtmiR165a chez des plantes de tabac modifiées pour exprimer le AtmiR165a (colonne de gauche) ou pour exprimer le AtmiR165a et le AtmiPEP165a (colonne de droite).
   (B) L'axe des ordonnées indique l'expression relative du AtmiR319a chez des plantes de tabac modifiées pour exprimer le AtmiR319a (colonne de gauche) ou pour exprimer le AtmiR319a et le AtmiPEP319a (colonne de droite).

   La barre d'erreur correspond à l'erreur standard à la moyenne (nombre d'individus = 30). Dans les deux cas, on constate que l'expression du miORF, et donc la production du miPEP, entraîne une augmentation de l'accumulation du pre-miR.

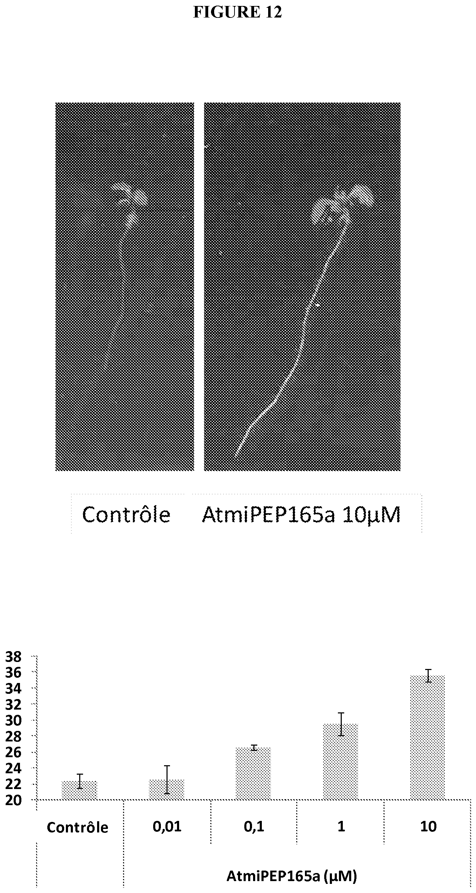

**FIGURE 12****. Effets du traitement par le AtmiPEP165a sur la croissance des racines d*'Arabidopsis thaliana.***
   La photographie présente deux plantes de même âge : une plante contrôle (plante de gauche) et une plante traitée par le AtmiPEP165a (plante de droite). Le traitement par le AtmiPEP165a entraine un phénotype de croissance racinaire très accélérée chez *Arabidopsis thaliana.* Le graphique montre l'expression du pre-miR165 en réponse au traitement par des doses croissantes de AtmiPEP165a.

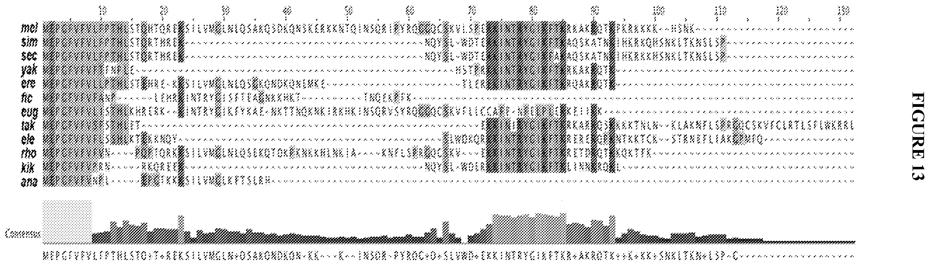

**FIGURE 13****. Conservation de la séquence du miPEP8 identifié chez la drosophile.**
   Les séquences du miPEP8 (SEQ ID NO : 104) ont été déduites à partir des séquences du miORF8 (SEQ ID NO : 208) de 12 espèces de drosophile différentes et ont été alignées. Un histogramme représente la conservation de chaque acide aminé entre les séquences du miORF8 chez les 12 espèces analysées.

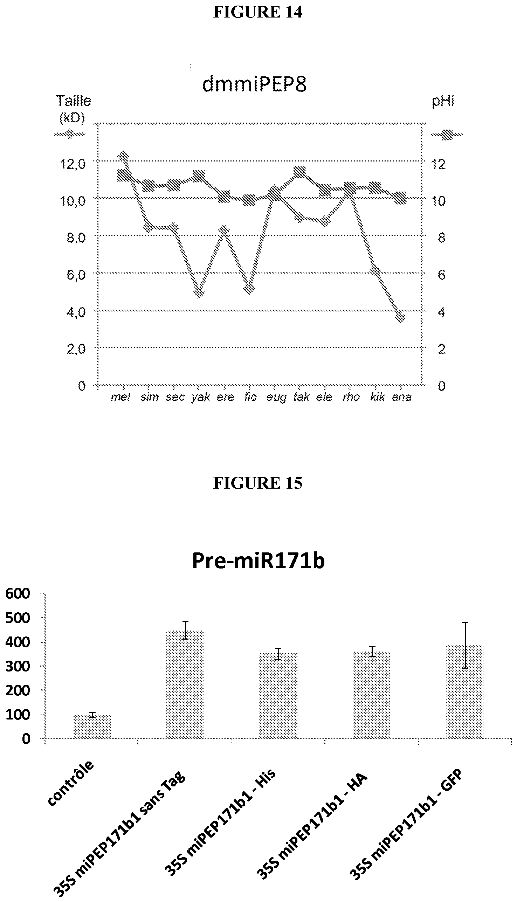

**FIGURE 14****. Evolution de la masse (kDa) et du point isoélectrique (pI) du miPEP8 chez les espèces de drosophile.**
   L'axe des ordonnées de gauche indique la taille du miPEP8 (en kD). L'axe des ordonnées de droit indique le point isoélectrique du miPEP. L'axe des abscisses indique l'origine du miPEP, c'est-à-dire l'espèce de drosophile. On constate que malgré une modification importante de leur taille (plus d'un facteur 3), la charge des miPEPs reste très basique (>9,8) chez les 12 espèces étudiées.
**FIGURE 15****. Effet de l'ajout de séquences sur la fonction du miPEP.**
   Les feuilles de tabac ont été transformées pour surexprimer le miPEP171b. Ces graphiques montrent que l'ajout de séquences (tag His, HA ou GFP) n'altère pas la fonction du miPEP. L'axe des ordonnées indique l'expression relative du pre-MtmiR171b chez des plantes de tabac qui ont été transformées pour exprimer le MtmiR171b (colonne de gauche), le MtmiR171b et le MtmiPEP171b1 avec ou sans ajout de tags proteiques (colonnes de droite) La barre d'erreur correspond à l'erreur standard à la moyenne (nombre d'individus = 6). On constate que l'expression du MtmiPEP171b1 augmente l'expression du MtmiR171b, et cet effet est indépendant de la présence de tags.

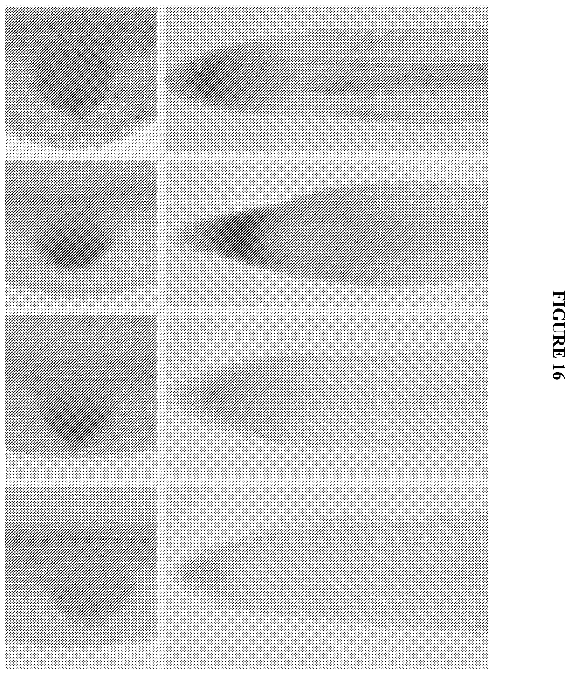

**FIGURE 16****. Expression du MtmiPEP171b1 dans le système racinaire de *Medicago truncatula.***
   Les racines de *Medicago truncatula* ont été transformées pour exprimer des fusions entre la protéine GUS (en bleu) et le promoteur du miR17b (A, E), l'ATG du miPEP171b1 (B, F), le miPEP171b1 entier (C, G) ou bien l'ATG2 (deuxième ATG se trouvant sur le précurseur, après le miPEP) (D, H). On voit bien une expression du miR dans les pointes racinaires (A) ainsi que les racines latérales (E). Les fusions transcriptionnelles (B, F) et traductionnelles (C, G) montrent une expression du miPEP171b dans les mêmes tissus, alors que l'ATG suivant n'est pas actif (D, H).

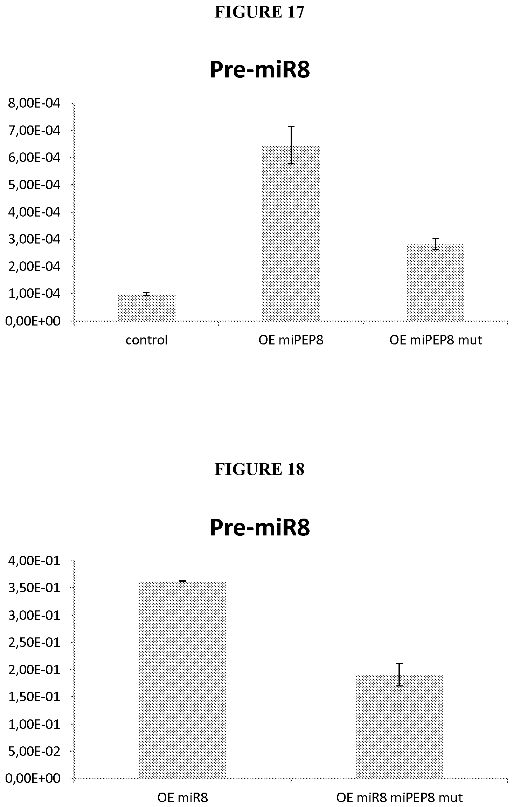

**FIGURE 17****. Expression du DmmiPEP8 dans des cellules de *Drosophila melanogaster***
   Les cellules de *Drosphila melanogaster* ont été transfectées pour surexprimer le DmmiPEP8 (OE miPEP8) ou bien le miPEP8 dont les codons d'initiation de traduction ont été mutés (OE miPEP8 mut). L'axe des ordonnées indique l'expression relative du Pre-miR8. La barre d'erreur correspond à l'erreur standard à la moyenne (nombre d'expériences indépendantes = 6). On constate que l'expression du DmmiPEP8 augmente l'expression du DmmiR8, et cet effet est lié à la traduction de l'ARNm.
**FIGURE 18****. Impact du DmmiPEP8 sur l'accumulation du DmmiR8 dans des cellules de *D. melanogaster***
   Les cellules de *Drosphila melanogaster* ont été transfectées pour surexprimer le DmmiR8 sauvage (OE miR8) ou bien le DmmiR8 dans lequel les codons d'intitiation de la traduction ont été mutés (OE miR8 miPEP8 mut) L'axe des ordonnées indique l'expression relative du Pre-miR8. La barre d'erreur correspond à l'erreur standard à la moyenne (nombre d'expériences indépendantes = 2). On constate que la présence du DmmiPEP8 augmente l'expression du DmmiR8.

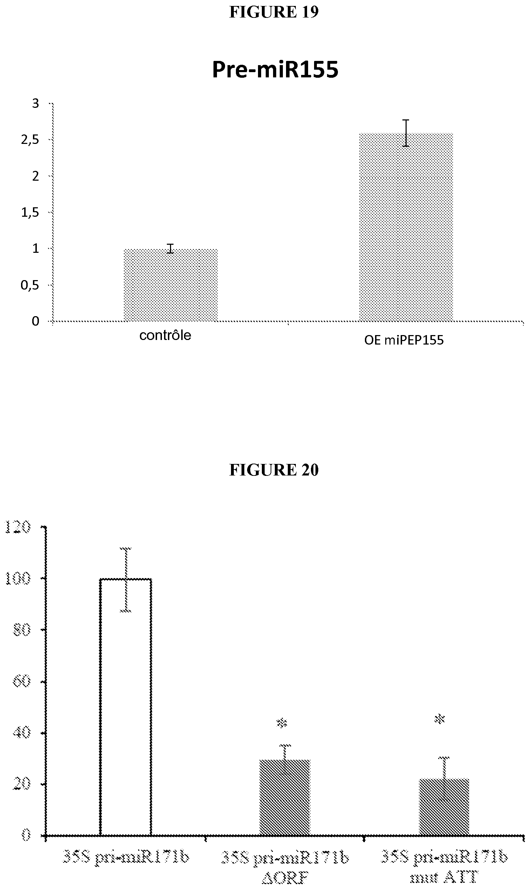

**FIGURE 19****. Impact du HsmiPEP155 sur l'accumulation du HsmiR155 dans des cellules d'*Homo sapiens***
   Les cellules HeLA d'Homo sapiens ont été transfectées pour surexprimer le HsmiPEP155 (OE miPEP155). L'axe des ordonnées indique l'expression relative du Pre-miR155. La barre d'erreur correspond à l'erreur standard à la moyenne (nombre d'expériences indépendantes = 2). On constate que l'expression du HsmiPEP155 augmente l'expression du HsmiR155.
**FIGURE 20****. Effets de la traduction du MtmiPEP171bl sur l'expression du MtmiR171b mis en évidence chez la plante modèle *Nicotiana benthamiana.***
   L'axe des ordonnées indique l'expression relative du MtmiR171b chez des plantes de tabac transformées pour exprimer le pri-miR171b (colonne de gauche), un pri-miR171b dans lequel le miORF171b a été délété (colonne du milieu) ou un pri-miR171b muté dans lequel le codon ATG a été remplacé par ATT (colonne de droite). Le pri-miR171b muté est donc incapable de produire le miPEP171b1. La barre d'erreur correspond à l'erreur standard à la moyenne (nombre d'individus = 30). On constate que l'absence de la traduction du miPEP171b1 entraîne une forte diminution de l'accumulation du miR171b.

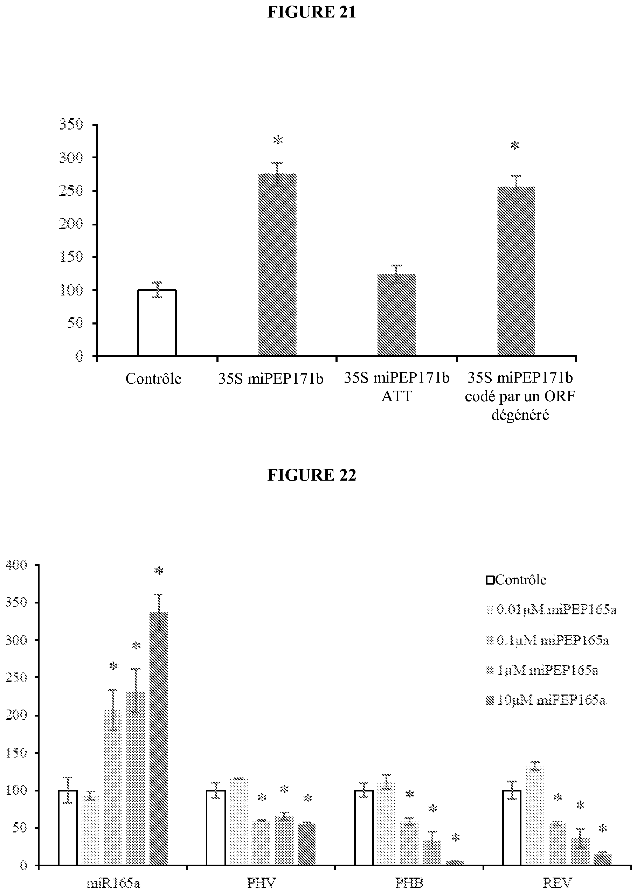

**FIGURE 21****. Effets de la surexpression du MtmiPEP171bl sur l'expression du MtmiR171b mis en évidence chez la plante modèle *Nicotiana benthamiana.***
   L'axe des ordonnées indique l'expression relative du MtmiR171b chez des plantes de tabac qui ont été transformées avec un vecteur permettant l'expression du miPEP171b et soit un second vecteur vide (colonne blanche), soit un vecteur permettant l'expression du mtmiPEP171b (colonne noire de gauche), soit un vecteur dans lequel le codon ATG de l'ORF codant le mtmiPEP171b a été remplacé par ATT (colonne noire du milieu), soit un vecteur dans lequel la séquence nucléotidique de l'ORF a été mutée sans modifier la séquence d'acides aminés du peptide traduit (miPEP codé par un ORF dégénéré) (colonne noire de droite). La barre d'erreur correspond à l'erreur standard à la moyenne (nombre d'individus = 30). On constate que l'expression du MtmiPEP171b1 augmente l'expression du MtmiR171b, et cet effet est dépendant de la traduction du MtmiORF171b en MtmiPEP171b1.
**FIGURE 22****. Effets du AtmiPEP165a sur l'accumulation du AtmiR165a et de ses gènes cibles (*PHAVOLUTA : PHV, PHABOLUSA* : *PHB* et *REVOLUTA* : *REV*)*.***
   L'axe des ordonnées indique l'expression relative de AtmiR165a, PHV, PHB et REV dans des racines d'*Arabidopsis thaliana* traitées avec de l'eau (contrôle) ou différentes concentrations de AtmiPEP165a (0,01 µM, 0,1 µM, 1 µM ou 10 µM). La barre d'erreur correspond à l'erreur standard à la moyenne (nombre d'individus = 10).
   Le traitement des plantes avec des concentrations de plus en plus importantes de AtmiPEP165a met en évidence un effet dose-dépendant de l'accumulation du AtmiR165a et de la régulation négative de ses gènes cibles en fonction de la quantité de AtmiPEP165a.

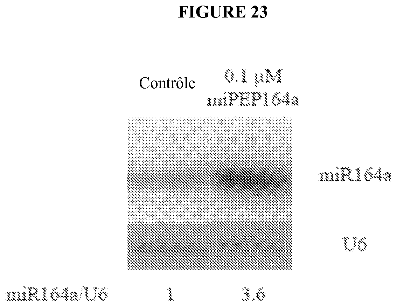

**FIGURE 23****. Effets d'un traitement avec le AtmiPEP164a sur l'expression du AtmiR164a chez *A*. *thaliana.***
   Les photographies représentent les résultats d'une analyse par Northern blot de l'accumulation du AtmiR164a dans des racines traitées avec de l'eau (contrôle, photographie de gauche) ou avec 0,1 µM d'un peptide synthétique, ayant une séquence identique à celle du AtmiPEP164a, dissout dans de l'eau (0,1 µM miPEP164a). L'ARN U6 est utilisé comme témoin de charge permettant de quantifier la quantité de AtmiR164a.
   Cette expérience a été répétée 4 fois de manière indépendante et a abouti à des résultats similaires.
   Le traitement des pousses d'*A*. *thaliana* avec 0,1 µM de miPEP164a entraîne une augmentation de l'accumulation du miR164a.

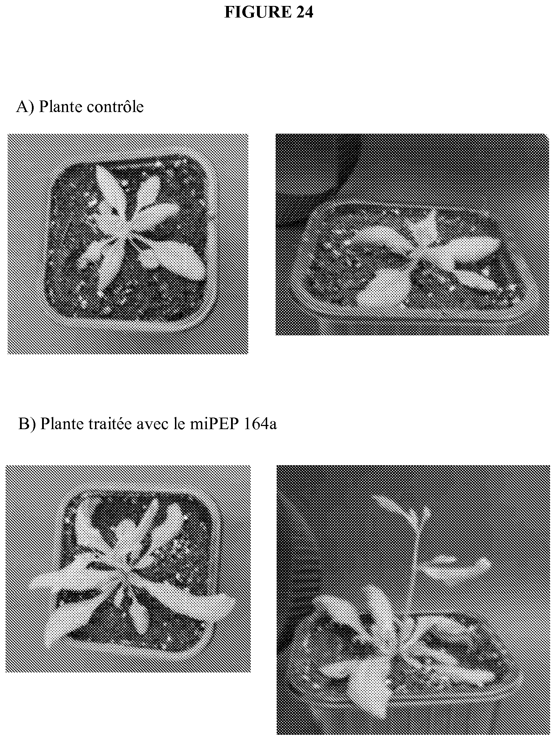

**FIGURE 24****. Effets du traitement par le AtmiPEP164a sur la croissance d'*Arabidopsis thaliana.***
   Les photographies présentent deux plantes (vues de dessus et vues de côté) après 3 semaines de croissance: une plante contrôle arrosée avec de l'eau (A), et une plante arrosée avec une composition de 0,1 µM de peptide synthétique correspondant à AtmiPEP164a (B). L'arrosage des plantes d'*Arabidopsis thaliana avec* AtmiPEP164a augmente significativement la croissance de la plante.

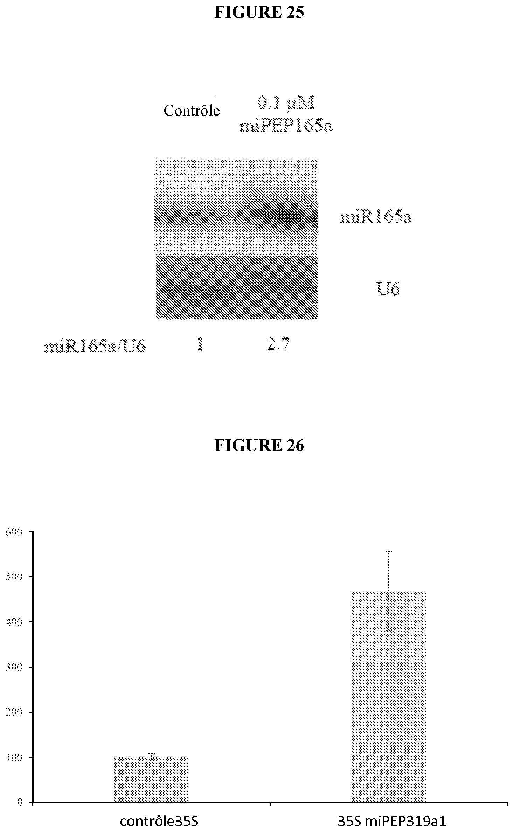

**FIGURE 25****. Effets d'un traitement avec le AtmiPEP165a sur l'expression du AtmiR165a chez *A*. *thaliana.***
   Les photographies représentent les résultats d'une analyse par Northern blot de l'accumulation du AtmiR165a dans des racines traitées avec de l'eau (contrôle, photographie de gauche) ou avec 0,1 µM d'un peptide synthétique, ayant une séquence identique à celle du AtmiPEP165a, dissout dans de l'eau (0,1 µM miPEP165a). L'ARN U6 est utilisé comme témoin de charge permettant de quantifier la quantité de AtmiR165a.
   Cette expérience a été répétée 4 fois de manière indépendante et a abouti à des résultats similaires.
   Le traitement des pousses d'*A*. *thaliana* avec 0,1 µM de miPEP165a entraîne une augmentation de l'accumulation du miR165a.
**FIGURE 26****. Effets de la surexpression du AtmiPEP319a1 sur l'expression du AtmiR319a chez *A*. *thaliana.***
   L'axe des ordonnées indique l'expression relative du AtmiR319a chez une plante témoin (colonne de gauche) ou chez une plante dans laquelle est surexprimé AtmiPEP319a1 (colonne de droite). La barre d'erreur correspond à l'erreur standard à la moyenne (nombre d'individus = 10). La surexpression de AtmiPEP319a1 induit une augmentation de l'accumulation de Atmir319a.

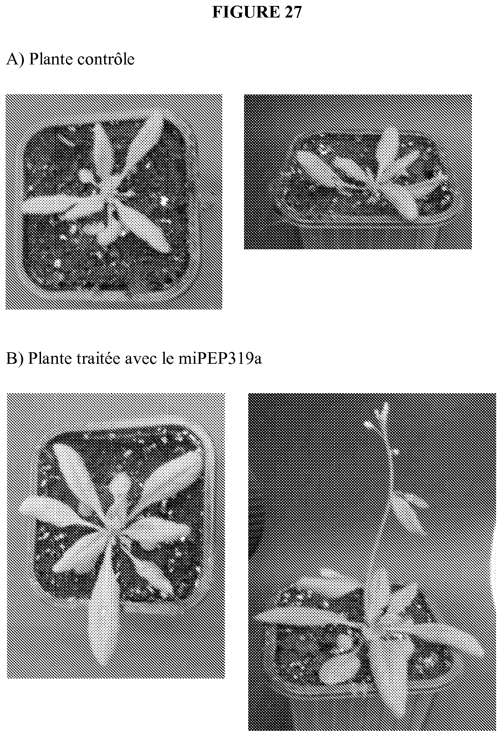

**FIGURE 27****. Effets du traitement par le AtmiPEP319a sur la croissance d'*Arabidopsis thaliana.***
   Les photographies présentent deux plantes (vues de dessus et vues de côté) après 3 semaines de croissance: une plante contrôle arrosée avec de l'eau (A), et une plante arrosée avec une composition de 0,1 µM de peptide synthétique correspondant à AtmiPEP319a1 (B). L'arrosage des plantes d'*Arabidopsis thaliana avec* AtmiPEP319a1 augmente significativement la croissance de la plante.

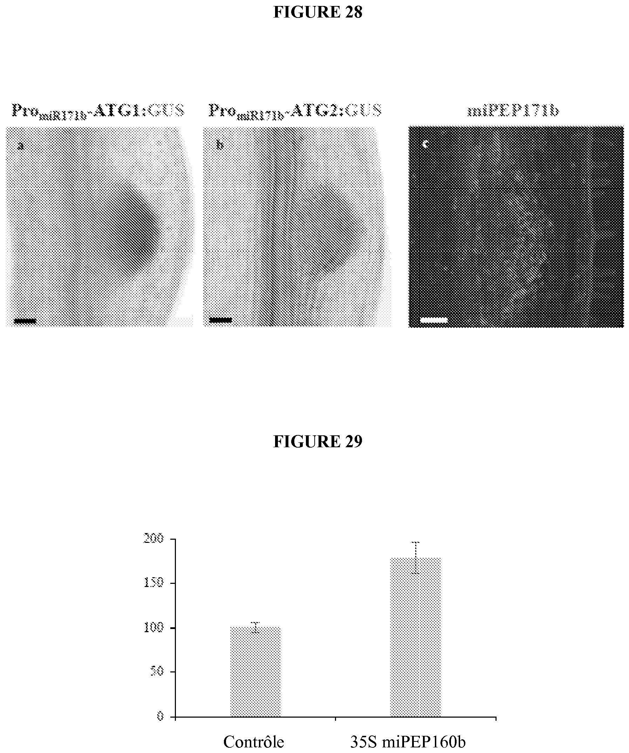

**FIGURE 28****. Immunolocalisation.**
   Les racines de *Medicago truncatula* ont été transformées pour exprimer des fusions entre la protéine GUS (en bleu) et l'ATG du miPEP171b (Pro_{miR171b}-ATG1:GUS) ou bien l'ATG2 (deuxième ATG se trouvant sur le précurseur, après le miPEP) (Pro_{miR171b}-ATG2:GUS). Un marquage a été également réalisé avec un anticorps anti-miPEP171b (miPEP171b). L'immunolocalisation du miPEP171b dans les racines de *M. truncatula* révèle la présence du miPEP171b dans les sites d'initiation des racines latérales, montrant une co-localisation entre le microARN et le miPEP correspondant.
**FIGURE 29****. Effets de la surexpression du AtmiPEP160b1 sur l'expression du AtmiR160b chez *A. thaliana.***
   L'axe des ordonnées indique l'expression relative du AtmiR160b chez une plante témoin (barre de gauche) ou chez une plante dans laquelle est surexprimé AtmiPEP160b1 (barre de droite). La barre d'erreur correspond à l'erreur standard à la moyenne (nombre d'individus = 10).
   La surexpression de AtmiPEP160b1 entraîne une augmentation de l'expression relative du AtmiR160b.

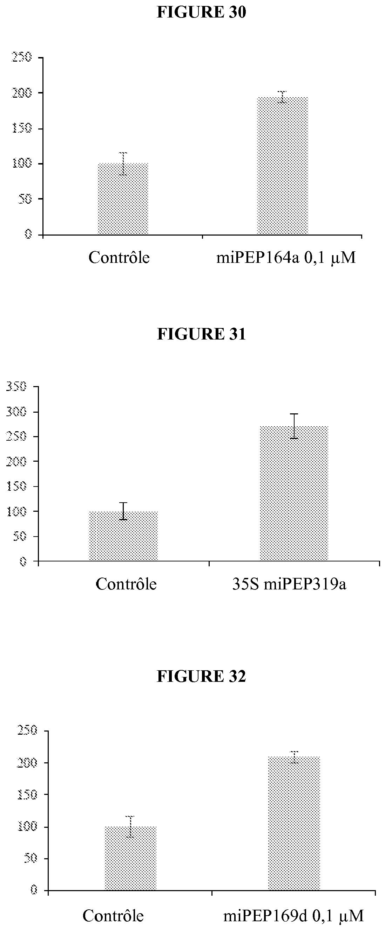

**FIGURE 30****. Effets du AtmiPEP164a1 sur l'expression du AtmiR164a.**
   L'axe des ordonnées indique l'expression relative du AtmiR164a chez une plante témoin (barre de gauche) ou chez une plante traitée par arrosage 1 fois par jour avec le AtmiPEP164a1 à 0,1 µM (barre de droite). La barre d'erreur correspond à l'erreur standard à la moyenne (nombre d'individus = 10).
   L'ajout de AtmiPEP164a1 entraîne une augmentation de l'expression relative du AtmiR164a.
**FIGURE 31****. Effets de la surexpression du AtmiPEP319a1 sur l'expression de AtmiR319a chez *A. thaliana.***
   L'axe des ordonnées indique l'expression relative de AtmiR319a chez une plante témoin (barre de gauche) ou chez une plante dans laquelle est surexprimé AtmiPEP319a1 (barre de droite). La barre d'erreur correspond à l'erreur standard à la moyenne (nombre d'individus = 10).
   La surexpression de AtmiPEP319a1 entraîne une augmentation de l'expression relative de AtmiR319a.
**FIGURE 32****. Effets du MtmiPEP169d sur l'expression du MtmiR169d chez *M. truncatula.***
   L'axe des ordonnées indique l'expression relative du MtmiR169d chez une plante témoin (barre de gauche) ou chez une plante traitée par arrosage 1 fois par jour avec le MtmiPEP169d à 0,1 µM (barre de droite). La barre d'erreur correspond à l'erreur standard à la moyenne (nombre d'individus = 10).
   L'ajout de MtmiPEP169d entraîne une augmentation de l'expression relative du MtmiR169d.

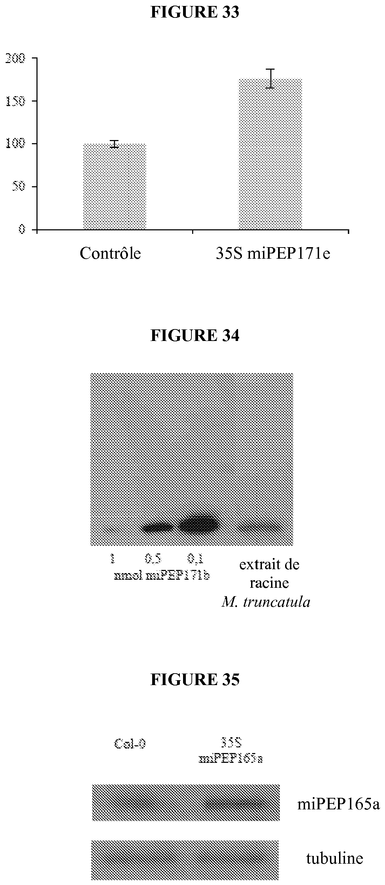

**FIGURE 33****. Effets de la surexpression du MtmiPEP171e sur l'expression de MtmiR171e chez *M. truncatula***
   L'axe des ordonnées indique l'expression relative de MtmiR171e chez une plante témoin (barre de gauche) ou chez une plante dans laquelle est surexprimé MtmiPEP171e (barre de droite). La barre d'erreur correspond à l'erreur standard à la moyenne (nombre d'individus = 10).
   La surexpression de MtmiPEP171e entraîne une augmentation de l'expression relative de MtmiR171e.
**FIGURE 34****. Localisation du MtmiPEP171b1 dans une plante *M. truncatula* sauvage.**
   Différentes quantités de peptides synthétiques MtmiPEP171b1 (1, 0,5, ou 0,1 nmol) et un extrait total de racines de *M. truncatula* ont été analysés par immunoblot avec un anticorps spécifique du MtmiPEP171b1.
   Le MtmiPEP171b1 est naturellement produit dans les racines de *M. truncatula.*
**FIGURE 35****. Présence du AtmiPEP165a dans une plante *A*. *thaliana* sauvage.**
   La photographie du haut correpond à une analyse par western blot de la quantité de AtmiPEP165a chez un jeune plant d'*A*. *thaliana* sauvage Col-0 (gauche) et un jeune plant d'*A*. *thaliana* surexprimant AtmiPEP165a (droite).
   La photographie du bas correspond à l'analyse par western blot de la quantité d'une protéine contrôle, la tubuline, chez un jeune plant d'*A*. *thaliana* sauvage Col-0 (gauche) et un jeune plant d'*A*. *thaliana* surexprimant AtmiPEP165a (droite).
   Le AtmiPEP165a est naturellement produit chez *A*. *thaliana* et est présent en plus grande quantité dans les plantes surexprimant AtmiPEP165a.

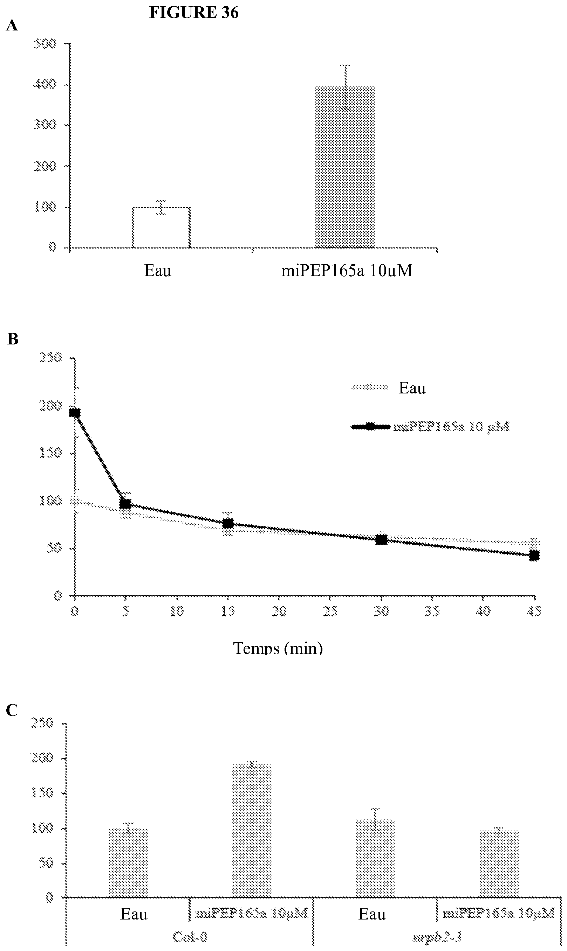

**FIGURE 36****. Mode d'action du AtmiPEP165a chez *A*. *thaliana.***
   (A) L'axe des ordonnées indique l'expression relative du pri-miR165a dans des racines témoins (barre de gauche) ou traitées par arrosage 1 fois par jour avec le AtmiPEP165a à 10 µM (barre de droite). La barre d'erreur correspond à l'erreur standard à la moyenne (nombre d'individus = 10).
      L'ajout de AtmiPEP165a entraîne une augmentation de l'expression relative du AtmiR165a.
   (B) L'axe des ordonnées indique l'expression relative du pri-miR165a, en présence de cordycepine (inhibiteur de synthèse de l'ARN), dans des racines témoins (courbe grise) ou traitées par arrosage 1 fois par jour avec le AtmiPEP165a à 10 µM (courbe noire). L'axe des abscisses indique la durée du traitement à la cordycepine.
      L'ajout de AtmiPEP165a n'entraîne pas une meilleure stabilité du pri-miR165a.
   (C) L'axe des ordonnées indique l'expression relative du pri-miR165a, chez des plantes sauvages (Col-0) ou mutées avec un allèle faible de la seconde grande sous-unité de l'ARN polymerase II (*nrpb2-3*). L'axe des abscisses indique si les plantes ont été arrosées avec de l'eau ou avec le AtmiPEP165a à 10 µM.

   Les plantes mutées, contrairement aux plantes sauvages, ne sont pas capables d'accumuler le AtmiR165a en réponse au AtmiPEP165a.

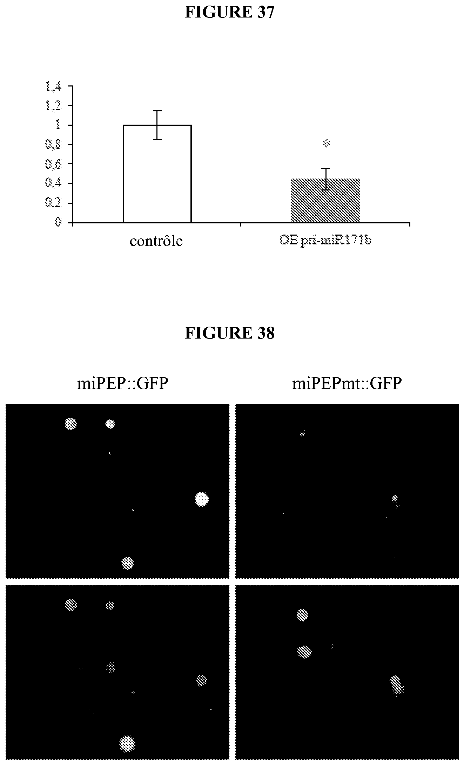

**FIGURE 37****. Effets du pri-miR171b sur le nombre de racines latérales chez *M*. *truncatula.***
   L'axe des ordonnées indique le nomre moyen de racines latérales observées chez une plante témoin (barre blanche) ou chez une plante dans laquelle est surexprimé le pri-miR171b (barre noire). La barre d'erreur correspond à l'erreur standard à la moyenne (nombre d'individus = 100).
   La surexpression du pri-miR171b entraîne une réduction du nombre de racines latérales.
**FIGURE 38****. Traductibilité du DmmiPEP8 dans les cellules de *Drosophila melanogaster.***
   Des cellules de *Drosphila melanogaster* ont été transfectées pour surexprimer le DmmiPEP8 (miPEP8::GFP) ou bien le DmmiPEP8 mt (dont les codons d'initiation de traduction ont été mutés (miPEP8 mt::GFP)). Comme contrôle de transfection, un plasmide produisant la protéine moesin-RFP (images du bas) a été co-transfecté avec les plasmides codants les DmmiPEP8 (image de gauche) et DmmiPEP 8 mt (image de droite). On constate que les codons initiateurs présents dans la séquence DmmiPEP8 sont fonctionnels puisqu'ils permettent la synthèse de la GFP (image du haut a gauche) alors que les constructions mutées sur les ATG ne produisent quasiment plus de GFP (image du haut a droite).
   Ces résultats suggèrent donc que l'ORF du miPEP est fonctionnel.

### EXEMPLES

### A : Analyse des miPEPS chez les plantes

### EXEMPLE 1 - Caractérisation chez la plante modèle Medicago truncatula

### Exemple 1A - MtmiPEP171b1

### a) Identification et caractérisation du MtmiPEP171b1 (miPEP171b1 identifié chez Medicago truncatula)

Le microARN MtmiR171b est exprimé dans la région méristématique des racines. La surexpression de ce microARN conduit notamment à une réduction de l'expression des gènes *HAM1* (Accession n°. MtGI9- TC114268) et *HAM2* (Accession n°. MtGI9-TC120850) (Figure 1A), ainsi qu'à une réduction du nombre de racines latérales (Figures 1B). La surexpression du pri-miR171b entraîne également une réduction du nombre de racines latérales (Figure 37).

La séquence du transcrit primaire du MtmiR171b a été déterminée en utilisant la technique de RACE-PCR. L'analyse de la séquence du transcrit primaire a permis d'identifier la présence de plusieurs petits cadres ouverts de lecture (ORF) complètement inattendus. Ces ORFs ont été dénommés miORFs pour *« microRNA ORF »*. Ces miORFs codent potentiellement des peptides courts, d'environ 4 à 100 acides aminés. Aucune homologie significative concernant ces miORFS n'a été trouvée dans les bases de données.

La surexpression du premier miORF, nommé MtmiORF171b, conduit à une augmentation de l'accumulation de MtmiR171b et une réduction de l'expression des gènes *HAM1* et *HAM2* (voir Figure 2A), ainsi qu'à une réduction du nombre de racines latérales (Figure 2B), comme cela a déjà été observé lors de la surexpression du MtmiR171b.

Pour déterminer si le MtmiORF171b conduit à la production réelle d'un peptide et si la fonction de régulation observée ci-dessus est bien portée par ledit peptide, un peptide synthétique, dont la séquence est identique à celle potentiellement codée par MtmiORF171b, a été appliqué sur les racines de *Medicago truncatula.* L'application de ce peptide entraîne le phénotype déjà observé plus haut lors de la surexpression du MtmiORF171b, c'est-à-dire qu'il entraîne une augmentation de l'accumulation de MtmiR171b et une réduction de l'expression des gènes *HAM1* et *HAM2* (voir Figure 3A), ainsi qu'une réduction du nombre de racines latérales (Figure 3B).

Les résultats de ces expériences démontrent que le MtmiORF171b code un peptide capable de moduler l'accumulation du MtmiR171b, et l'expression des gènes cibles de MtmiR171b: *HAM1* et *HAM2.* Ledit peptide a été dénommé MtmiPEP171b1 (« miPEP » correspondant à *microRNA encoded PEPtide*).

Par ailleurs, le MtPEP171b1 entraîne une augmentation de l'accumulation du MtmiR171b (Figure 4A) et du pre-MtmiR171b (figure 4B).

### b) Spécificité du miPEP171b1

L'expression de différents précurseurs microARNs de *Medicago truncatula* (MtmiR171b SEQ ID NO: 319, MtmiR169 SEQ ID NO: 367, MtmiR169a SEQ ID NO: 368, MtmiR171a SEQ ID NO : 369, MtmiR171h SEQ ID NO : 370, MtmiR393a SEQ ID NO : 371, MtmiR393b SEQ ID NO : 372, MtmiR396a SEQ ID NO : 373 et MtmiR396b SEQ ID NO : 374) a été déterminée et comparée entre des plantes témoins et des plantes dans lesquelles a été surexprimé le MtmiORF171b codant le MtmiPEP171b1 (Figure 5A), ou entre des plantes témoins et des plantes cultivées sur milieu de culture contenant le MtmiPEP171b1 (Figure 5B).

Les résultats obtenus indiquent que le MtmiPEP171b1 n'entraîne une augmentation de l'accumulation que du MtmiR171b et pas des autres miRs, ce qui indique qu'un miPEP n'a d'effet que sur le microARN dont il est issu.

### c) Localisation du miPEP171b1

Par ailleurs, l'immunolocalisation du miPEP171b1 dans les racines de *M. truncatula* révèle la présence du miPEP171b1 dans les sites d'initiation des racines latérales, montrant une co-localisation entre le microARN et le miPEP correspondant (Figures 28 et 34).

### Exemple 1B - MtmiPEP169d

Concernant MtmiPEP169d, il a été démontré *in vivo* chez *M. truncatula* que l'ajout de MtmiPEP169d entraine une accumulation du MtmiR169d (Figure 32).

### Exemple 1C - MtmiPEP171e

Concernant MtmiPEP171e, il a été démontré *in vivo* chez *M. truncatula* que la surexpression de ce miPEP entraine une accumulation du MtmiR171e (Figure 33).

### EXEMPLE 2 - Caractérisation chez la plante modèle du tabac

### a) Conservation du mécanisme chez le tabac

Pour déterminer si le mécanisme de régulation des microARNs est conservé chez d'autres espèces de plantes, la régulation du MtmiR171b par le MtmiPEP171b1 a été testée dans un modèle cellulaire différent. Pour cela, le MtmiR171b et le MtmiPEP171b1 ont été introduits dans des feuilles de tabac.

L'accumulation du MtmiR171b été mesurée chez des feuilles de tabac transformées pour exprimer le MtmiR171b de *Medicago truncatula* à partir du pri-miR sauvage capable de produire le MtmiPEP171b1, ou à partir d'une version mutée du pri-miR incapable de produire le MtmiPEP171b1 (dans laquelle le codon d'initiation ATG du MtmiORF171b a été remplacé ATT) (Figure 6 et Figure 20). L'absence de traduction du MtmiPEP171b1 conduit à une forte diminution de l'accumulation du MtmiR171b.

L'accumulation de pre-MtmiR171b a été mesurée chez des feuilles de tabac transformées pour exprimer le MtmiR171b de *Medicago truncatula* seul (témoin), ou exprimant en plus le MtmiORF171b sauvage de *Medicago truncatula* (35SmiPEP171b1 ATG), ou une version mutée de MtmiORF171b dans laquelle le codon d'initiation ATG a été remplacé ATT (35SmiPEP171b1 ATT) (Figure 7 et Figure 21). L'expression de MtmiORF171b entraîne une augmentation de l'accumulation du pre-miR171b, et cette accumulation du pre-miR171b dépend de la traduction du MtmiORF171b en micropeptide.

De même, chez les feuilles de tabac transformées pour exprimer le MtmiR171b de *Medicago truncatula*, traitées ou non par pulvérisation avec le MtmiPEP171b1 (0,1 µM) une première fois 12h avant prélevement puis une seconde fois 30 minutes avant le prélevement, il a été observé que le MtmiPEP171b1 peut être utilisé directement sous forme de peptide par le biais de pulvérisations foliaires (Figure 8).

Par ailleurs, il a été observé chez le tabac (comme chez *Medicago truncatula*) que le MtmiPEP171b1 entraîne une augmentation de l'accumulation du mtmiR171b (Figure 9A)
et du pre-MtmiR171b (Figure 9B), mais diminue l'accumulation du pri-MtmiR171b (Figure 9C).

L'ensemble de ces résultats indique que le mécanisme de régulation des microARNs et de leurs gènes cibles sous le contrôle de miPEPs est conservé entre les espèces.

### b) Localisation intracellulaire du MtmiPEP171b1

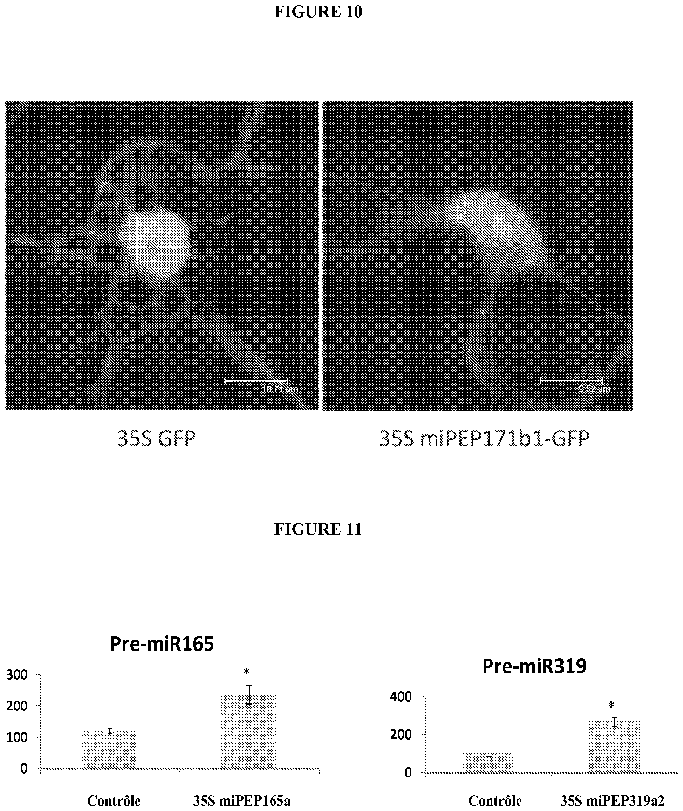

Les feuilles de tabac ont été transformées pour surexprimer le MtmiPEP171b1 de *Medicago truncatula* fusionné à une protéine fluorescente (GFP) (Figure 10). Les résultats obtenus indiquent que le miPEP est localisé dans de petits corps nucléaires.

### c) Identification de miPEPS à partir des bases de données

A partir des bases de données génomiques de plantes, il a été recherché la présence de cadres ouverts de lecture au sein des transcrits primaires de 70 miRs, nous avons identifié 101 miORFs susceptibles de coder un miPEP.

A l'heure actuelle, les AtmiPEP165a et AtmiPEP319a2, identifiés chez *Arabidopsis thaliana*, ont déjà été caractérisés. Les expériences réalisées chez la plante modèle du tabac ont permis de démontrer que la surexpression de AtmiORF165a ou de AtmiORF319a entraîne respectivement une augmentation de l'accumulation du AtmiR165a ou du AtmiR319a (Figure 11).

miR165a régule des facteurs de transcription comme *Revoluta*, *Phavoluta* et *Phabulosa.* Le miR319 régule des gènes de la famille TCP.

### EXEMPLE 3 - Caractérisation chez la plante modèle Arabidopsis thaliana

### Exemple 3A - AtmiPEP165a

Concernant AtmiPEP165a, il a été démontré *in vivo* chez *Arabidopsis thaliana* que le traitement par le AtmiPEP165a entraine un phénotype de croissance racinaire très accélérée (Figure 12).

Par ailleurs, le traitement des plantes avec des concentrations de plus en plus importantes de miPEP165a indique un effet dose-dépendant de l'accumulation du miR165a et de la régulation négative de ses gènes cibles (PHAVOLUTA : *PHV,* PHABOLUSA : *PHB* et REVOLUTA : *REV*) en fonction de la quantité de miPEP165A (voir figure 22).

### Exemple 3B - AtmiPEP164a

Concernant AtmiPEP164a, celui-ci a été synthétisé et a été utilisé pour rechercher une augmentation de l'accumulation du miR164a chez des racines d'*A. thaliana* traitées avec le peptide synthétique.

Les analyses par Northen blot indiquent que le traitement de la plante avec le peptide miPEP164a entraîne une augmentation de l'accumulation du miR164a (Figure 23).

Le même type de résultat a été obtenu par qRT-PCR (Figure 30).

Il a également été démontré *in vivo* chez *Arabidopsis thaliana* que le traitement de la plante avec le AtmiPEP164a augmente significativement la croissance de la plante (Figure 24).

### Exemple 3C - AtmiPEP165a

Concernant AtmiPEP165a, celui-ci a été synthétisé et a été utilisé pour rechercher une augmentation de l'accumulation du miR165a chez des racines d'*A*. *thaliana* traitées avec le peptide synthétique.

Les analyses par Northen blot indiquent que le traitement de la plante avec le peptide miPEP165a entraîne une augmentation de l'accumulation du miR165a (Figure 25).

Par ailleurs, pour déterminer le mode d'action des miPEPs vis-à-vis de l'accumulation de leurs propres miRs, l'expression du pri-miR165a a été analysée chez des plantes sauvages et chez des plantes transformées pour surexprimer le miPEP165a (Figure 35).

L'expression du pri-miR165a est favorisée chez les plantes surexprimant le miPEP165a (Figure 36A).

En présence de cordycepine, un inhibiteur de la synthèse d'ARN, la quantité de pri-miR165a est identique chez les plantes sauvages et chez les plantes transformées (Figure 36B), ce qui indique que le miPEP165a n'agit pas comme un stabilisateur de l'ARN, mais plutôt comme un activateur transcriptionnel du pri-miR165a.

De plus, l'expression du pri-miR165a été analysée chez des plantes sauvages (plantes Col-0) et chez des plantes mutées portant un allèle faible de la seconde grande sous-unité de l'ARN polymerase II (plantes *nrpb2-3*). Les résultats obtenus indiquent que les plantes mutées ne présentent pas d'augmentation de l'accumulation du pri-miR165 en réponse au miPEP165a, contrairement aux plantes sauvages. Ceci semble indiquer que le miPEP agit comme un régulateur transcriptionnel.

### Exemple 3D - AtmiPEP319a1

Concernant AtmiPEP319a1, celui-ci a également été synthétisé et utilisé pour rechercher une augmentation de l'accumulation du miR319a chez des racines d'*A*. *thaliana* traitées avec le peptide synthétique.

Les analyses par qRT-PCRindiquent que la surexpression du AtmiPEP319a1 entraîne une augmentation de l'accumulation du miR319a (Figures 26 et 31).

Il a également été démontré *in vivo* chez *Arabidopsis thaliana* que le traitement de la plante avec le AtmiPEP319a1 augmente significativement la croissance de la plante (Figure 27).

### Exemple 3E - AtmiPEP160b1

Concernant AtmiPEP160b1, il a été démontré *in vivo* chez *A. thaliana* que la surexpression de ce miPEP entraine une accumulation du AtmiR160b (Figure 29).

### Matériels et Méthodes

### Matériel biologique

La surface des graines de *M. truncatula* a été stérilisée et celles-ci ont été mises à germer sur des plaques d'agar pendant 5 jours à 4°C dans l'obscurité. Les jeunes pousses ont ensuite été cultivées sur des plaques carrées de 12 cm remplies de milieu Fahraeus sans nitrogène et contenant du phosphate 7,5 µM (Lauresergues et al., Plant J., 72(3):512-22, 2012). Les racines latérales ont été dénombrées chaque jour. En pots, les plantes ont été arrosées tous les deux jours avec du milieu Long Ashton modifié contenant peu de phosphore (Balzergue et al., Journal of experimental botany, (62)1049-1060, 2011).

Les peptides ont été synthétisés par Eurogentec ou Smartox-Biotech. Le MtmiPEP171b1 a été remis en suspension dans une solution d'eau 40%/ acétonitrile 50%/ acide acétique 10% (v/v/v), et les autres peptides ont été remis en suspension dans de l'eau.

L'arrosage des feuilles par pulvérisations avec les peptides a été réalisé en utilisant des solutions de peptides à différentes concentrations (0,01, 0,1, 1 µM), une première fois 12h avant prélèvement puis une deuxième fois 30min avant prélèvement

### Transcription inverse des microRNAs

L'ARN a été extrait en utilisant le réactif Tri-Reagent (MRC) selon les instructions du fabricant, à l'exception de la précipitation de l'ARN qui a été réalisée avec 3 volumes d'éthanol. La transcription inverse de l'ARN a été réalisée en utilisant l'amorce spécifique RTprimerl71b tige boucle en combinaison avec des hexamères pour effectuer la transcription inverse de l'ARN de haut poids moléculaire.

En bref, 1 µg de RNA a été ajouté à l'amorce tige boucle MIR171b (0,2 µM), l'héxamère (500 ng), le tampon RT (IX), l'enzyme SuperScript Reverse transcriptase (SSIII) (une unité), les dNTPs (0,2 mM chacun), le DTT (0,8 mM) dans un mélange réactionnel final de 25 µl. Pour effectuer la transcription inverse, une réaction de transcription inverse pulsée a été réalisée (40 répétitions du cycle suivant : 16°C pendant 2 minutes, 42°C pendant une minute et 50°C pendant une seconde, suivies d'une inactivation finale de la transcription inverse à 85°C pendant 5 minutes).

### Analyses par RT-PCR quantitative (qRT-PCR)

L'ARN total des racines de *M. truncatula* ou des feuilles de tabac a été extrait en utilisant le kit d'extraction RNeasy Plant Mini Kit (Qiagen). La transcription inverse a été réalisée en utilisant la transcriptase inverse SuperScript II (Invitrogen) à partir de 500 ng d'ARN total. Trois répétitions (n=3) ont été réalisées avec deux répétitions techniques chacune. Chaque expérience a été répétée de deux à trois fois. Les amplifications par qPCR ont été réalisées en utilisant un thermocycleur LightCycler 480 System (Roche Diagnostics) selon la méthode décrite dans Lauressergues et al. (Plant J., 72(3):512-22, 2012).

### Analyses statistiques

Les valeurs moyennes de l'expression relative des gènes ou de la production de racines latérales ont été analysées en utilisant le test de Student ou le test de Kruskal-Wallis. Les barres d'erreurs représentent l'écart type de la moyenne (SEM, Standard Error of the Mean). Les astérisques indiquent une différence significative (*p*<0,05).

### Constructions plasmidiques

Les fragments d'ADN d'intérêt ont été amplifiés avec la Pfu polymerase (Promega). Les fragments d'ADN ont été clonés à l'aide des enzymes XhoI et NotI dans un plasmide pPEX-DsRED pour une surexpression sous le contrôle du promoteur fort constitutif 35S, et à l'aide des enzymes KpnI-NcoI dans un plasmide pPEX GUS pour les gènes rapporteurs, selon la méthode décrite dans Combier et al. (Genes & Dev, 22: 1549-1559, 2008).

Pour les miPEPs 165a et 319a, les miORFs correspondants ont été clonés dans pBIN19 selon la méthode décrite dans Combier et al. (Genes & Dev, 22: 1549-1559, 2008).

### Transformation des plantes

Les plantes composites possédant des racines transformées avec *Agrobacterium Rhizogenes* ont été obtenues par la méthode décrite dans Boisson-Dernier et al. (Mol Plant-Microbe Interact, 18:1269-1276, 2005). Les racines transformées ont été vérifiées et sélectionnées par observations du DsRED avec une loupe binoculaire à fluorescence. Les racines témoins correspondent à des racines transformées avec *A*. *rhizogenes* ne contenant pas le vecteur pPEX-DsRED. La transformation des feuilles de tabac a été réalisée selon la méthode décrite dans Combier et al. (Genes & Dev, 22: 1549-1559, 2008).

### Northern blot

L'analyse par Northern blot a été réalisée selon le protocole décrit dans Lauressergues et al. Plant J, 72(3):512-22, 2012.

Les échantillons biologiques ont été homogénéisés dans un tampon contenant 0,1 M de NaCl, 2% de SDS, 50 mM de Tris-HCl (pH 9), 10 mM de EDTA (PH 8) et 20 mM de mercaptoéthanol, et l'ARN a été extrait 2 fois avec un mélange phénol/chloroforme et précipité à l'éthanol.

L'ARN a été chargé sur gel PAGE 15% et transféré sur une membrane de nylon (HybondNX, Amersham). L'ARN a été hybridé avec une sonde d'oligonucléotides, radioactive marquée à son extrémité, pour détecter l'ARN U6 ou pour le miR164a.

Les hybridations ont été réalisées à 55°c. Les signaux d'hybridation ont été quantifiés en utilisant un phophorimager (Fuji) et normalisés avec le signal de la sonde spécifique de l'ARN U6.

### Stabilité de l'ARN

Pour chaque condition, des plantes agées de 2 semaines et cultivées verticalement sur milieu MS solide ont été transférées dans des plaques 6 puits contenant 1 ml de milieu MS liquide. Après 16 heures d'incubation avec 1mM de miPEP, les plantes ont été traitées avec 100 µg/ml de cordycepine ou avec de l'eau (utilisée comme contrôle), et récoltées à différents moments pour extraire et quantifier l'ARN. Chaque expérience a été réalisée 3 fois.

### Marquage histochimiques

Le marquage avec GUS a été réalisée selon la méthode décrite dans Combier et al., (Genes & Dev, 22: 1549-1559, 2008). Les échantillons ont été observés avec un microscope (axiozoom).

### Immunolocalisation

Des racines ou des plantules de tissus de Medicago ont été fixés pendant 2 heures dans 4% de formol (v / v) à 50 mM de tampon phosphate (pH 7,2), puis inclus dans de l'agarose LMP 5% dans de l'eau (à bas point de fusion). Des coupes fines (100 um) ont été obtenues et placées dans du Pbi (tampon phosphate pour l'immunologie) sur des lames coatées avec du téflon, bloquées dans Pbi, 2% Tween et 1% d'albumine de sérum bovin pendant 2 heures (PbiT-BSA), puis marquées épendant une nuit (12 h) à 4 ° C avec l'anticorps primaire dilué dans du BSA-PbiT. Les coupes ont été lavées avec du PBiT et incubées à température ambiante pendant 2 h avec un anticorps secondaire dilué dans PbiT-BSA. Les lames ont ensuite été lavées dans du Pbi pendant 30 min et montées dans du citifluor (milieu de montage). Les anticorps primaires et les dilutions ont été les suivants: 1716a (1:500, v/v). L'anticorps secondaire était un anticorps de chèvre anti-IgG de lapin couplé à la sonde fluorescente Alexa Fluor 633 (Molecular Probes), et a été utilisé à une dilution de 1:1000 (v/v).

### Western blot

Un extrait total de protéines a été obtenu selon la méthode décrite dans Combier et al., (Genes & Dev, 22: 1549-1559, 2008) et séparé par SDS-PAGE. Le transfert a été réalisé dans un tampon phosphate pendant une nuit à 4°C, à 15V, puis la membrane a été incubée pendant 45 min à température ambiante dans une solution de glutaraldehyde 0,2% (v/v). Les anticorps primaires ont été utilisés à une dilution 1 : 1 000 (v/v) et des anticorps de chèvres anti-IgG de lapin conjugués à HRP ont été utilisés comme anticorps secondaires à une dilution 1 : 40 000 (v/v).

**Table 8. Liste des amorces**

| | |
|---|---|
| AtmiR160bq5 (SEQ ID NO : 429) | TCCCCAAATTCTTGACCAAA |
| AtmiR160bq3 (SEQ ID NO : 430) | TTGAGGGGAAAACATGAACC |
| AtmiR164aq5 (SEQ ID NO : 431) | ACGAAATCCGTCTCATTTGC |
| AtmiR164aq3 (SEQ ID NO : 432) | GGGTGAAGAGCTCATGTTGG |
| AtmiR165q5 (SEQ ID NO : 433) | TGAGGGGAATGTTGTCTGG |
| AtmiR165q3 (SEQ ID NO : 434) | GAAGCCTGGTCCGAGGATA |
| AtmiR319q5 (SEQ ID NO : 435) | CGAGTCGCCAAAATTCAAAC |
| AtmiR319q3 (SEQ ID NO : 436) | GCTCCCTTCAGTCCAATCAA |
| AtPHVq5 (SEQ ID NO : 437) | GCAACTGCAGTGGAATAGCA |
| AtPHVq3 (SEQ ID NO : 438) | GCGACCTTCATGGGTTCTAA |
| AtPHBq5 (SEQ ID NO : 439) | CTCAGCATCAGCAACGTGAT |
| AtPHBq3 (SEQ ID NO : 440) | AACTCTGCTAGGGCCTCCTC |
| AtREVq5 (SEQ ID NO : 441) | TCACAACTCCTCAGCATTCG |
| AtREVq3 (SEQ ID NO : 442) | ACCCAATCAACAGCAGTTCC |
| Atactine2F (SEQ ID NO : 443) | GGTAACATTGTGCTCAGTGG |
| Atactine2R (SEQ ID NO : 444) | CTCGGCCTTGGAGATCCACA |
| miORF319acla5 (SEQ ID NO : 445) | tcatcgATGAATATACATACATACCATCAT |
| miORF319abam3 (SEQ ID NO : 446) | |
| AtmiR319cla5 (SEQ ID NO : 447) | tcatcgAGAGAGAGCTTCCTTGAGTC |
| AtmiR319bam3 (SEQ ID NO : 448) | tccggatccAGAGGGAGCTCCCTTCAGT |
| AtmiR165cla5 (SEQ ID NO : 449) | tcatcgGTTGAGGGGAATGTTGTCTG |
| AtmiR165bam3 (SEQ ID NO : 450) | tccggatccGTTGGGGGGGATGAAGCC |
| AtmiR165muorf5cla (SEQ ID NO : 451) | tcatcgATGAGGGTTAAGCTATTTCAGT |
| AtmiR165muorf3bam (SEQ ID NO : 452) | tccggatccTAATATCCTCGATCCAGACAAC |
| miR171bq5 (SEQ ID NO : 453) | CGCCTCAATTTGAATACATGG |
| miR171bq3 (SEQ ID NO : 454) | ACGCGGCTCAATCAAACTAC |
| pri-miR171bq5 (SEQ ID NO : 455) | GCACTAGCTGGTTTCATTATTCC |
| pri-miR171bq3 (SEQ ID NO : 456) | TTGCAAAATTTGGAGAGCCTA |
| miR171eq5 (SEQ ID NO : 457) | AAGCGATGTTGGTGAGGTTC |
| miR171eq3 (SEQ ID NO : 458) | CGGCTCAATCTGAGATCGTAT |
| miR169dq5 (SEQ ID NO : 459) | GGGCGGTGAGATTAACAAAA |
| miR169dq3 (SEQ ID NO : 460) | CCTGCCGGAAATGAAACTAA |
| miORF171e2Cla5 (SEQ ID NO : 461) | tcatcgATGATGGTGTTTGGGAAGCC |
| miORF171e2bam3 (SEQ ID NO : 462) | tccggatccTACATGTAAATCCGTCTTCGG |
| miORF171b5'AGG (SEQ ID NO : 463) | AGGCTTCTTCATAGGCTCTCC |
| miR171e5'Xho (SEQ ID NO : 464) | |
| miR171e3'Not (SEQ ID NO : 465) | |
| 171b scramble F bis (SEQ ID NO : 466) | |
| 171b scramble R bis (SEQ ID NO : 467) | |
| PromiORF171b1 ATG BsaIR (SEQ ID NO : 468) | |
| PromiORF171b2 ATG BsaIR (SEQ ID NO : 469) | |
| PromiORF171b6 ATG BsaI R (SEQ ID NO : 470) | |
| 171b fusion GFP sans ATG BsaI R (SEQ ID NO : 471) | |
| Pro171b fusionTranscript GUSpPEX BsaI R (SEQ ID NO : 472) | |
| Pro171b fusionATG1 GUSpPEXsansATG BsaI R (SEQ ID NO : 473) | |
| Pro171b fusionTraduc GUSpPEX BsaI R (SEQ ID NO : 474) | |
| 171b NcoI F (SEQ ID NO : 475) | Gaattcctgcccatggttttcg |
| 171b NcoI R (SEQ ID NO : 476) | Cgaaaaccatgggcaggaattc |
| 171b AflII F (SEQ ID NO : 477) | cagtcccgacttaagctcaatac |
| 171b AflII R (SEQ ID NO : 478) | gtattgagcttaagtcgggactg |
| 171b génomiq BsaI F (SEQ ID NO : 479) | |
| 171b génomiq BsaI R (SEQ ID NO : 480) | |
| AthmiORF160b F (SEQ ID NO : 481) | TAGCATGTTTTCCCCTCAATGA |
| AthmiORF160b R (SEQ ID NO : 482) | cgtaTCATTGAGGGGAAAACAT |
| AthMIR160b BsaI F (SEQ ID NO: 483) | |
| AthMIR160b BsaI R (SEQ ID NO : 484) | |
| pro 165a BsaI F (SEQ ID NO :485) | aGGTCTCCAAATcatgaagcaggcagtaataacct |
| 165a muORF tagHA BsaI R (SEQ ID NO : 486) | |
| 165a fusion transcript BsaI R (SEQ ID NO : 487) | aGGTCTCgccatagtggcggagacgaagatg |
| 165a fusion traduc BsaI R (SEQ ID NO : 488) | aGGTCTCgccattaatatcctcgatccagacaac |
| pro 165a BsaI F (SEQ ID NO:489) | aGGTCTCCAAATtaaactgtcagtgcatggatgt |
| 165a fusion GTG BsaI R (SEQ ID NO : 490) | aGGTCTCgACACattcacaaatttttgttgtagagag |
| 165a fusion GTGmutGTT BsaI R (SEQ ID NO : 491) | aGGTCTCgACACattaacaaatttttgttgtagagag |
| 165a fusion CTG BsaI R (SEQ ID NO : 492) | aGGTCTCgACACtagcagattcacaaatttttgttg |
| 165a fusion ATG BsaI R (SEQ ID NO : 493) | aGGTCTCgACACcctcatgataatcgatcttagca |

### B : Analyse des miPEPs chez l'animal

### EXEMPLE 4 - Identification de candidats miPEPs chez la drosophile

### a) Identification de candidats miPEPs

Une première étude réalisée par RACE-PCR chez l'animal modèle *Drosophila melanogaster* montre l'existence de miRs qui sont exprimés au cours de l'embryogenèse, miR1 et miR8.

Comme chez les plantes, il a été identifié des miORFs dans chacun des deux miRs étudiés. Par exemple, le miR8, connu pour son rôle dans la régulation de la croissance des insectes, présente un miORF codant potentiellement le miPEP8.

Le DmmiR1 (identifié chez *Drosophila melanogaster*) présente quant à lui deux DmmiORFs codant potentiellement le DmmiPEPla et le DmmiPEP1b.

Une analyse phylogénétique montre la conservation évolutive de la présence des miORFs à travers la douzaine d'espèces de drosophile analysées, c'est à dire depuis leur divergence il y a plus de 60 millions d'années (Figure 13).

Les miPEPs identifiés chez la drosophile présentent par ailleurs plusieurs similitudes avec les miPEPs de plante. Si leur séquence primaire et donc leur taille évoluent rapidement entre espèces, on retrouve une taille réduite (de 32 à 104 aa), ainsi qu'une forte conservation pour une charge globale basique (pHi de 9,5 à 12) (Figure 14).

L'ensemble de ces résultats indiquent donc l'existence de miPEPs régulateurs, codés par les transcrits primaires des microRNAs, à travers un large spectre d'espèces eucaryotes. Ces peptides découverts représentent un réservoir encore inexploré de molécules naturelles, susceptibles de réguler une variété de fonctions biologiques fondamentales, aussi bien chez les végétaux que chez les animaux.

### b) Expression des miPEPS

Des cellules de *Drosphila melanogaster* ont été transfectées pour surexprimer le DmmiPEP8 (à partir de la séquence SEQ ID NO : 494) ou bien le DmmiPEP8 mt dont les codons d'initiation de traduction ont été mutés (à partir de la séquence SEQ ID NO : 495). Comme contrôle de transfection, un plasmide produisant la protéine moesin-RFP a été co-transfecté avec les plasmides codants les DmmiPEP8 et DmmiPEP 8 mt. On constate que les codons initiateurs présents dans la séquence DmmiPEP8 sont fonctionnels puisqu'ils permettent la synthèse d'une protéine de fusion miPEP::GFP visualisée par la GFP alors que les constructions mutées sur les ATG ne produisent quasiment plus de GFP.

Ces résultats suggèrent donc que l'ORF du miPEP est fonctionnel (Figure 38).

### Matériels et Méthodes

### Cellules de Drosophila melanogaster

Les cellules S2 sont cultivées dans un flask T75 dans 12mL de milieu Schneider's (GIBCO), contenant 1% de pénicilline 100U/mL et streptavidine 100mg/mL (Sigma) et 10% de sérum de veau foetal décomplémenté (30' à 56°C).

Les transfections transitoires sont réalisées à l'aide du kit de transfection FuGENE® HD (Roche), selon leurs recommandations. Classiquement, 1,5 millions de cellules S2, préalablement ensemmencées dans des plaques 6 puits (3ml de milieu par puit), sont transfectées avec 250 ng d'ADN plasmidique total. L'ADN est mis en contact avec le Fugene (3 µl) dans 100 µl d'OPTIMEM (GIBCO). Apres 20 minutes, le réactif de transfection formé est mis en contact avec les cellules dans le milieu de culture. L'ARN des cellules est extrait 66h après transfection.

### Clonage des fragments codant DmmiPEP8 et DmmiPEP8 muté

Les fragments d'ADN correspondant aux séquences du DmmiPEP8 (SEQ ID NO : 494) et du DmmiPEP8 muté sur les codons initiateurs (SEQ ID NO : 495) ont été amplifiés par PCR pour être clonés en phase avec la GFP dans un plasmide pUAS. Les cellules d'insecte S2 ont été co-transfectées par un mélange de plasmides (ratio 1/1/1, 300 ng au total) codant le DmmiPEP8::GFP ou sa version mutée DmmiPEP8 mt::GFP, un pACT-GAL4 permettant leur surexpression par le biais du facteur de transcription GAL4 produit sous le contrôle du promoteur actine et d'un vecteur d'expression codant la protéine moesin-RFP utilisée comme temoin de transfection, elle aussi placée sous le contrôle du promoteur actine. 48h post transfection, les cellules ont été fixées par ajout de formaldehyde dans le milieu de culture 4% (P/V) final pendant 10min. Les cellules ont ensuite été rincées 2X par du PBS1X et montées sur lames pour être observée au microscope (Leica SPE).

**Table 9. Séquences clonées**

| | |
|---|---|
| | SEQ ID NO: 494 |
| | SEQ ID NO: 495 |

### C : Caractérisation de miPEPs chez l'homme

### EXEMPLE 5 - Caractérisation du HsmiPEP155

Les fragments d'ADN d'intérêt (le HsmiPEP155 et le miPEP muté) ont été synthétisés ou amplifiés par PCR à l'aide d'amorces spécifiques, puis clonés à l'aide des enzymes XhoI et NotI dans un plasmide pUAS permettant leur surexpression par le biais du facteur de transcription GAL4 dont l'expression est contrôlée par un promoteur fort constitutif.

Les différentes constructions ont été réalisées soit par amplification PCR sur de l'ADN génomique de cellules HeLa, soit par RT-PCR sur des ARN totaux de cellules humaines L428. Les fragments PCR amplifiés sont digérés par les enzymes de restriction HindIII/EcoRI puis clonés dans le vecteur pcDNA3.1. La souche d'*Escherichia coli* DH5α est électroporée puis cultivée sur un milieu solide (2YT+agar+Ampicilline). L'ADN plasmidique de différents clones est alors préparé et séquencé pour vérification. Les constructions sont ensuite préparées à l'aide du QIAfilter Plasmid Midi kit (QIAGEN) et conservées à -20°C.

Les cellules HeLa (lignée tumorale établie, ATCC CCL-2.2) sont cultivées en plaque 6 puits dans du milieu complet [(DMEM (1x) +Glutamax +4.5g/L glucose sans pyruvate + 1x Pénicilline/Streptomycine + 1mM Na-pyruvate + 10% sérum de veau] et placées dans un incubateur à 37°C et 5% de CO2.

Les cellules sont transfectées lorsqu'elles sont à 50% de confluence. En début d'expérience, le milieu complet contenant les antibiotiques est remplacé par du milieu complet sans antibiotique.

Pour chaque puit, un mix A [250µl d'Optimem (+Glutamax) (Gibco) + 2µg d'ADN] et un mix B [250µl d'Optimem + 4µl de Lipofectamine 2000 (Invitrogen)] est préparé, on laisse 5' à température ambiante. Puis le mix B est mélangé dans le mix A goutte à goutte, et laissé à incuber à 25' à température ambiante. Le mélange est alors déposé dans le puit, goutte à goutte. 4-5 heures après, le milieu est changé et remplacé par du milieu complet avec antibiotiques. 48 heures après transfection, les cellules sont arrêtées. Le milieu est aspiré et éliminé, les cellules sont rincées au PBS 1X. Il est alors possible de garder les cellules à - 20°C ou d'extraire directement les ARN totaux.

Pour chaque puit, les ARNs sont extraits en déposant 1ml de Tri-Reagent (Euromedex) sur les cellules. On aspire et refoule plusieurs fois le Tri-reagent afin de lyser correctement les cellules, puis on le transfère dans un tube de 1,5 ml. On rajoute 0,2 ml de chloroforme saturé en eau. On mélange par vortex, puis on laisse 2 à 3 minutes à température ambiante. On centrifuge 5 minutes à 15300rpm et à 4°C. La phase aqueuse est précipitée avec 0,5 ml d'isopropanol après une incubation de 10 minutes à température ambiante et une centrifugation de 15 minutes à 15300rpm et à 4°C. Le surnageant est éliminé et le culot est rincé avec 1ml d'Ethanol 70% et centrifugation de 5 minutes à 15300rpm à 4°C. De nouveau on élimine le surnageant et le culot est séché quelques minutes à l'air.

Afin d'éliminer au mieux l'ADN génomique potentiellement restant, les ARNs sont traités à la DNase. Pour cela, le culot est resuspendu dans 170µl d'eau ultra pure, 20µl de tampon DNase 10x et 10µl de RQ1 RNase free-DNase et placé 30 minutes à 37°C. Puis, on rajoute 20µl de SDS10% et 5µl de protéinase K (20mg/ml) durant 20 minutes à 37°C.

Une dernière extraction phénol est réalisée avec 225 µl d'un mélange Phénol/H₂O/Chloroforme, et centrifugée pendant 5 minutes à 15300 rpm à 4°C.

La phase aqueuse est alors précipitée avec 20µl de Sodium Acetate 3M et 600µl d'Ethanol 100% pendant 20 minutes à -80°C. Puis, on centrifuge 15' à 4°C à 15300rpm. On élimine le surnageant. On rince le culot dans 1ml d'Ethanol 70%, on centrifuge 5' 15300rpm 4°C, on élimine de nouveau le surnageant et on laisse quelques minutes le culot sécher à l'air.

Le culot est ensuite repris dans 15-20 µl d'eau ultra pure et les ARNs sont dosés.

10-15µg d'ARN totaux sont ensuite analysés par Northern Blot sur un gel 15% acrylamide [solution d'acrylamide/bis-acrylamide 40%, ratio 19:1], 7M Urée en TBE 1x. La migration est réalisée à 400V, dans du TBE1x comme tampon de migration, après avoir pré-chauffé le gel. Les ARN sont ensuite électro-transférés sur une membrane de nylon Biodyne Plus 0.45µm, pendant 2heures, à 1V et 4°C dans une cuve de transfert. A la fin du transfert, la membrane est irradiée aux UV à 0,124 J/cm2. La membrane est ensuite pré-hybridée dans un tampon 5xSSPE, 1xDenhardt's, 1%SDS et 150ug/ml de d'ARNt de levure, 1heure à 50°C dans un four à hybridation. Puis, on rajoute la sonde nucléotidique marquée en 5' au γ-³²P-ATP (0.5 à 1.10⁶cpm/ml de tampon d'hybridation) et on hybride la nuit à 50°C. La membrane est alors lavée 2 fois dans du 0.1xSSPE/0.1%SDS à température ambiante et exposée dans une cassette d'autoradiographie contenant un écran BioMax HE (Kodak) et un film BioMax MS (Kodak), pour détecter un microARN, durant 24-48 heures, à -80°C.

## Revendications

1. Procédé de détection et d'identification d'un micropeptide (miPEP) codé par une séquence nucléotidique contenue dans la séquence du transcrit primaire d'un microARN,
comprenant :
- a) une étape de détection d'un cadre ouvert de lecture de 12 à 303 nucléotides contenu dans la séquence du transcrit primaire dudit microARN, puis
- b) une étape de comparaison entre :
• l'accumulation dudit microARN dans une cellule eucaryote déterminée exprimant ledit microARN,
en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et
• l'accumulation dudit microARN dans une cellule eucaryote de même type que la susdite cellule eucaryote déterminée exprimant ledit microARN,
en absence dudit peptide,
dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.

2. Procédé de détection et d'identification d'un miPEP selon la revendication 1, dans lequel la modulation de l'accumulation dudit microARN est une diminution ou une augmentation de l'accumulation dudit microARN, en particulier une augmentation.

3. Procédé de détection et d'identification d'un miPEP selon la revendication 1 ou 2, dans lequel la présence du peptide dans la cellule résulte :
- de l'introduction dans la cellule d'un acide nucléique codant ledit peptide, ou
- de l'introduction dans la cellule dudit peptide.

4. Procédé de détection et d'identification d'un miPEP selon l'une des revendications précédentes, dans lequel ledit cadre ouvert de lecture de l'étape a) est contenu dans la partie 5' ou 3' dudit transcrit primaire du microARN, de préférence dans la partie 5'.

5. Procédé de détection et d'identification d'un miPEP selon l'une des revendications précédentes, dans lequel ledit microARN est présent dans une cellule végétale sauvage ou dans une cellule animale sauvage, et dans lequel ladite cellule eucaryote utilisée à l'étape b) est une cellule végétale ou est une cellule animale.

6. Procédé de détection et d'identification d'un miPEP selon la revendication 5, dans lequel ladite cellule eucaryote utilisée à l'étape b) est une cellule végétale d'une plante crucifère, d'une plante légumineuse ou d'une plante solanacée.

7. Procédé de détection et d'identification d'un miPEP selon la revendication 5, dans lequel ladite cellule eucaryote utilisée à l'étape b) est une cellule animale choisi parmi une cellule humaine ou une cellule de drosophile.

8. MiPEP choisi parmi le groupe de peptides consistant en :
- SEQ ID NO : 1 à SEQ ID NO : 104 et SEQ ID NO : 355,
- SEQ ID NO : 375 à SEQ ID NO : 386, ou
- SEQ ID NO : 424.

9. Molécule d'acide nucléique codant un miPEP tel que défini selon la revendication 8, ladite molécule d'acide nucléique étant choisie parmi le groupe d'acides nucléiques consistant en :
- SEQ ID NO : 105 à SEQ ID NO : 208 et SEQ ID NO : 356,
- SEQ ID NO : 387 à SEQ ID NO : 399, ou
- SEQ ID NO : 425.

10. Utilisation d'au moins :
- un miPEP tel que défini selon la revendication 8,
- un acide nucléique selon la revendication 9 codant ledit miPEP, ou
- un vecteur contenant ledit acide nucléique,
pour moduler l'expression d'un gène dans une cellule eucaryote végétale déterminée,
ladite cellule eucaryote végétale déterminée étant capable d'exprimer un microARN, dont le transcrit primaire contient au moins une séquence nucléotidique codant ledit au moins un miPEP et dont l'accumulation est modulée par ledit au moins un miPEP,
l'expression dudit gène étant régulée par ledit microARN, ledit miPEP étant choisi parmi le groupe de peptides consistant en :
SEQ ID NO : 59, SEQ ID NO : 60, SEQ ID NO : 24, SEQ ID NO : 25, SEQ ID NO : 26, SEQ ID NO : 43, SEQ ID NO : 76,
ledit acide nucléique étant choisi parmi le groupe d'acides nucléiques consistant en :
SEQ ID NO : 163, SEQ ID NO : 164, SEQ ID NO : 128, SEQ ID NO : 129, SEQ ID NO : 130, SEQ ID NO : 147, SEQ ID NO : 180,
ledit microARN étant choisi parmi le groupe d'acides nucléiques consistant en :
SEQ ID NO : 319, SEQ ID NO : 297, SEQ ID NO : 305, SEQ ID NO : 331.

11. Procédé de modulation de l'expression d'un gène régulée par un microARN dans une cellule eucaryote végétale,
comprenant la mise en oeuvre d'une étape d'accumulation d'un miPEP dans ladite cellule eucaryote,
ledit miPEP étant choisi parmi le groupe de peptides consistant en : SEQ ID NO : 59, SEQ ID NO : 60, SEQ ID NO : 24, SEQ ID NO : 25, SEQ ID NO : 26, SEQ ID NO : 43, SEQ ID NO : 76
dans lequel, l'accumulation dudit miPEP dans ladite cellule eucaryote induit une modulation de l'expression dudit gène par rapport à l'expression dudit gène sans accumulation dudit miPEP.

12. Utilisation d'une composition comprenant au moins :
- un miPEP tel que défini selon la revendication 8, à l'exception des miPEP de séquences SEQ ID NO : 102, SEQ ID NO : 103, SEQ ID NO : 104 et SEQ ID NO : 355,
- un acide nucléique selon la revendication 9 codant ledit miPEP, à l'exception des acides nucléiques de séquences SEQ ID NO : 206, SEQ ID NO : 207, SEQ ID NO : 208 et SEQ ID NO : 356, ou
- un vecteur contenant ledit acide nucléique,
en tant qu'agent phytopharmaceutique,
- pour favoriser la croissance et/ou le développement des plantes,
- ou pour la modification des paramètres physiologiques de semences végétales
- ou pour prévenir ou traiter les maladies des plantes,
ladite composition étant appliquée par enrobage ou pelliculage sur lesdites semences végétales.

13. Utilisation d'une composition selon la revendication 12 pour la modulation des paramètres physiologiques d'une plante.

14. Utilisation d'une composition selon la revendication 13 pour la modulation des paramètres physiologiques d'une plante, lesdits paramètres étant choisis parmi la biomasse, la surface foliaire, la floraison et le calibre du fruit.

15. Utilisation d'une composition selon la revendication 12 pour la modification des paramètres physiologiques de semences végétales, lesdits paramètres étant choisis parmi la germination, l'implantation racinaire et la résistance au stress hydrique.

16. Utilisation d'une composition selon la revendication 12 pour favoriser la résistance aux maladies infectieuses.

17. Utilisation d'une composition comprenant au moins :
- un miPEP tel que défini selon la revendication 8 à l'exception des miPEP de séquences SEQ ID NO : 102, SEQ ID NO : 103, SEQ ID NO : 104 et SEQ ID NO : 355 ,
- un acide nucléique selon la revendication 9 codant ledit miPEP, à l'exception des acides nucléiques de séquences SEQ ID NO : 206, SEQ ID NO : 207, SEQ ID NO : 208 et SEQ ID NO : 356, ou
- un vecteur contenant ledit acide nucléique,
en tant qu'herbicide, pour éliminer les plantes ou ralentir leur croissance, ou
en tant que pesticide pour éliminer les organismes nuisibles des plantes ou susceptibles d'être classés comme tels.

18. Utilisation d'une composition selon la revendication 17, dans laquelle ledit pesticide est choisi parmi le groupe consistant en : un insecticide, un arachnicide, un limacide, et un rodonticide.

19. Utilisation d'une composition selon l'une quelconque des revendications 17 ou 18, dans laquelle ladite composition en tant que pesticide est appliquée sur une plante ou sur un support en contact avec la plante.

20. Composition comprenant en combinaison une quantité de semences d'une plante et une quantité d'un peptide dont la séquence comprend ou consiste en une séquence identique à celle d'un miPEP naturellement présent chez ladite plante,
ledit miPEP étant choisi parmi le groupe de peptides consistant en : SEQ ID NO : 59, SEQ ID NO : 60, SEQ ID NO : 24, SEQ ID NO : 25, SEQ ID NO : 26, SEQ ID NO : 43, SEQ ID NO : 76.

21. Composition selon la revendication 20, ladite composition étant formulée de façon à former une semence enrobée.

## Patentansprüche

1. Verfahren zum Nachweisen und Identifizieren eines Mikropeptids (miPEP), das von einer Nukleotidsequenz kodiert wird, die in der Sequenz des Primärtranskripts einer microRNA enthalten ist, umfassend:
- a) einen Schritt zum Nachweisen eines offenen Leserahmens von 12 bis 303 Nukleotiden, der in der Sequenz des Primärtranskripts der microRNA enthalten ist, dann
- b) einen Schritt zum Vergleichen:
• der Akkumulation der microRNA in einer spezifizierten eukaryotischen Zelle, die die microRNA
in Gegenwart eines Peptids exprimiert, das von einer Nukleotidsequenz kodiert wird, die in Bezug auf diejenige des offenen Leserahmens identisch oder degeneriert ist, wobei das Peptid in der Zelle unabhängig von der Transkription des Primärtranskripts der microRNA vorhanden ist, und
• der Akkumulation der microRNA in einer eukaryotischen Zelle des gleichen Typs wie die oben genannte spezifizierte eukaryotische Zelle, die die microRNA
in Abwesenheit des Peptids exprimiert,
wobei eine Modulation der Akkumulation der microRNA in Gegenwart des Peptids in Bezug auf die Akkumulation der microRNA in Abwesenheit des Peptids die Existenz eines Mikropeptids anzeigt, das von dem offenen Leserahmen kodiert wird.

2. Verfahren zum Nachweisen und Identifizieren eines miPEP nach Anspruch 1, wobei die Modulation der Akkumulation der microRNA eine Abnahme oder eine Zunahme der Akkumulation der microRNA, insbesondere eine Zunahme, ist.

3. Verfahren zum Nachweisen und Identifizieren eines miPEP nach Anspruch 1 oder 2, wobei die Gegenwart des Peptids in der Zelle aus
- der Einführung einer Nukleinsäure, die das Peptid kodiert, in die Zelle, oder
- der Einführung des Peptids in die Zelle resultiert.

4. Verfahren zum Nachweisen und Identifizieren eines miPEP nach einem der vorhergehenden Ansprüche, wobei der offene Leserahmen aus Schritt a) in dem 5'- oder 3'-Abschnitt des Primärtranskripts der microRNA, vorzugsweise im 5'-Abschnitt, enthalten ist.

5. Verfahren zum Nachweisen und Identifizieren eines miPEP nach einem der vorhergehenden Ansprüche, wobei die microRNA in einer Wildtyp-Pflanzenzelle oder in einer Wildtyp-Tierzelle vorhanden ist, und wobei die in Schritt b) verwendete eukaryotische Zelle eine Pflanzenzelle oder eine Tierzelle ist.

6. Verfahren zum Nachweisen und Identifizieren eines miPEP nach Anspruch 5, wobei die in Schritt b) verwendete eukaryotische Zelle eine Pflanzenzelle eines Kreuzblütlers, eines Hülsenfrüchtlers oder eines Nachtschattengewächses ist.

7. Verfahren zum Nachweisen und Identifizieren eines miPEP nach Anspruch 5, wobei die in Schritt b) verwendete eukaryotische Zelle eine Tierzelle ist, ausgewählt aus einer humanen Zelle oder einer Drosophila-Zelle.

8. MiPEP, ausgewählt aus der Gruppe von Peptiden, bestehend aus:
- SEQ ID NO: 1 bis SEQ ID NO: 104 und SEQ ID NO: 355,
- SEQ ID NO: 375 bis SEQ ID NO: 386, oder
- SEQ ID NO: 424.

9. Nukleinsäuremolekül, das ein miPEP kodiert, wie nach Anspruch 8 definiert, wobei das Nukleinsäuremolekül insbesondere ausgewählt ist aus der Gruppe von Nukleinsäuren, bestehend aus:
- SEQ ID NO: 105 bis SEQ ID NO: 208 und SEQ ID NO: 356,
- SEQ ID NO: 387 bis SEQ ID NO: 399, oder
- SEQ ID NO: 425.

10. Verwendung mindestens:
- eines miPEP wie nach Anspruch 8 definiert,
- einer Nukleinsäuresequenz nach Anspruch 9, die das miPEP kodiert, oder
- eines Vektors, der die Nukleinsäure enthält,
um die Expression eines Gens in einer spezifizierten eukaryotischen Pflanzenzelle zu modulieren,
wobei die spezifizierte eukaryotische Pflanzenzelle geeignet ist, eine microRNA zu exprimieren, deren Primärtranskript mindestens eine Nukleotidsequenz enthält, die das mindestens eine miPEP kodiert und deren Akkumulation von dem mindestens einen miPEP moduliert wird,
wobei die Expression des Gens von der microRNA reguliert wird, wobei das miPEP ausgewählt ist aus der Gruppe von Peptiden, bestehend aus:
SEQ ID NO: 59, SEQ ID NO: 60, SEQ ID NO: 24, SEQ ID NO: 25, SEQ ID NO: 26, SEQ ID NO: 43, SEQ ID NO: 76,
wobei die Nukleinsäure ausgewählt ist aus der Gruppe von Nukleinsäuren, bestehend aus:
SEQ ID NO: 163, SEQ ID NO: 164, SEQ ID NO: 128, SEQ ID NO: 129, SEQ ID NO: 130, SEQ ID NO: 147, SEQ ID NO: 180,
wobei die microRNA ausgewählt ist aus der Gruppe von Nukleinsäuren, bestehend aus:
SEQ ID NO: 319, SEQ ID NO: 297, SEQ ID NO: 305, SEQ ID NO: 331.

11. Verfahren zum Modulieren der Expression eines Gens, das von einer microRNA in einer eukaryotischen Pflanzenzelle reguliert wird,
umfassend das Durchführen eines Schritts zum Akkumulieren eines miPEP in der eukaryotischen Zelle, wobei das MiPEP ausgewählt ist aus der Gruppe von Peptiden, bestehend aus: SEQ ID NO: 59, SEQ ID NO: 60, SEQ ID NO: 24, SEQ ID NO: 25, SEQ ID NO: 26, SEQ ID NO: 43, SEQ ID NO: 76,
wobei die Akkumulation des miPEP in der eukaryotischen Zelle eine Modulation der Expression des Gens in Bezug auf die Expression des Gens ohne Akkumulation des miPEP induziert.

12. Verwendung einer Zusammensetzung, umfassend mindestens:
- ein miPEP wie nach Anspruch 8 definiert, außer miPEPs mit den Sequenzen SEQ ID NO: 102, SEQ ID NO: 103, SEQ ID NO: 104 und SEQ ID NO: 355,
- eine Nukleinsäure nach Anspruch 9, das das miPEP kodiert, außer Nukleinsäuren mit den Sequenzen SEQ ID NO: 206, SEQ ID NO: 207, SEQ ID NO: 208 und SEQ ID NO: 356, oder
- einen Vektor, enthaltend die Nukleinsäure, als phytopharmazeutisches Mittel,
- um das Wachstum und/oder die Entwicklung von Pflanzen zu fördern,
- oder zur Modifikation der physiologischen Parameter von Pflanzensamen,
- oder um Pflanzenkrankheiten zu verhindern oder zu behandeln,
wobei die Zusammensetzung durch Beschichten oder Filmbeschichtung dieser Pflanzensamen aufgebracht wird.

13. Verwendung einer Zusammensetzung nach Anspruch 12 zur Modulation der physiologischen Parameter einer Pflanze.

14. Verwendung einer Zusammensetzung nach Anspruch 13 zur Modulation der physiologischen Parameter einer Pflanze, wobei die Parameter ausgewählt sind aus der Biomasse, der Blattoberfläche, der Blüte und der Fruchtgröße.

15. Verwendung einer Zusammensetzung nach Anspruch 12 zur Modifikation der physiologischen Parameter von Pflanzensamen, wobei die Parameter ausgewählt sind aus der Keimung, der Etablierung des Wurzelsystems und der Resistenz gegen Trockenstress.

16. Verwendung einer Zusammensetzung nach Anspruch 12 zur Förderung der Resistenz gegen Infektionskrankheiten.

17. Verwendung einer Zusammensetzung, umfassend mindestens:
- ein miPEP wie nach Anspruch 8 definiert, außer miPEPs mit den Sequenzen SEQ ID NO: 102, SEQ ID NO: 103, SEQ ID NO: 104 und SEQ ID NO: 355,
- eine Nukleinsäure nach Anspruch 9, die das miPEP kodiert, außer die Nukleinsäuren mit den Sequenzen SEQ ID NO: 206, SEQ ID NO: 207, SEQ ID NO: 208 und SEQ ID NO: 356, oder
- einen Vektor, der die Nukleinsäure enthält,
als Herbizid, um Pflanzen zu beseitigen oder um ihr Wachstum zu verlangsamen, oder
als Pestizid zum Beseitigen von pflanzenschädlichen Organismen oder Organismen, die als solche eingestuft werden könnten.

18. Verwendung einer Zusammensetzung nach Anspruch 17, wobei das Pestizid ausgewählt ist aus der Gruppe bestehend aus: einem Insektizid, einem Arachnizid, einem Molluskizid und einem Rodentizid.

19. Verwendung einer Zusammensetzung nach einem der Ansprüche 17 oder 18, wobei die Zusammensetzung als Pestizid auf eine Pflanze oder auf einen Träger, der mit der Pflanze in Kontakt ist, aufgebracht wird.

20. Zusammensetzung, umfassend in Kombination eine Menge an Samen einer Pflanze und eine Menge eines Peptids, dessen Sequenz eine Sequenz umfasst oder aus dieser besteht, die mit der eines miPEP identisch ist, das natürlich in der Pflanze vorhanden ist,
wobei das MiPEP ausgewählt ist aus der Gruppe von Peptiden, bestehend aus: SEQ ID NO: 59, SEQ ID NO: 60, SEQ ID NO: 24, SEQ ID NO: 25, SEQ ID NO: 26, SEQ ID NO: 43, SEQ ID NO: 76.

21. Zusammensetzung nach Anspruch 20, wobei die Zusammensetzung derart formuliert ist, dass sie einen beschichteten Samen bildet.

## Claims

1. Process for detecting and identifying a micropeptide (miPEP) encoded by a nucleotide sequence contained in the sequence of the primary transcript of a microRNA, comprising:
a) a step of detecting an open reading frame from 12 to 303 nucleotides contained in the sequence of the primary transcript of said microRNA, then
- b) a step of comparison between:
• the accumulation of said microRNA in a specified eukaryotic cell expressing said microRNA,
in the presence of a peptide encoded by a nucleotide sequence that is identical or degenerate with respect to that of said open reading frame, said peptide being present in the cell independently of transcription of the primary transcript of said microRNA, and
• the accumulation of said microRNA in a eukaryotic cell of the same type as the aforesaid specified eukaryotic cell expressing said microRNA,
in the absence of said peptide,
in which a modulation of the accumulation of said microRNA in the presence of said peptide with respect to the accumulation of said microRNA in the absence of said peptide indicates the existence of a micropeptide encoded by said open reading frame.

2. Process for detecting and identifying a miPEP according to claim 1, in which the modulation of the accumulation of said microRNA is a decrease or an increase in the accumulation of said microRNA, in particular an increase.

3. Process for detecting and identifying a miPEP according to claim 1 or 2, in which the presence of the peptide in the cell results:
- from the introduction into the cell of a nucleic acid encoding said peptide, or
- from the introduction into the cell of said peptide.

4. Process for detecting and identifying a miPEP according to one of the preceding claims, in which said open reading frame in step a) is contained in the 5 'or 3' portion of said primary transcript of the microRNA, preferably in the 5 ' portion.

5. Process for detecting and identifying a miPEP according to one of the preceding claims, in which said microRNA is present in a wild-type plant cell or in a wild-type animal cell, and in which said eukaryotic cell used in step b) is a plant cell or is an animal cell.

6. Process for detecting and identifying a miPEP according to claim 5, in which said eukaryotic cell used in step b) is a plant cell of a cruciferous plant, of a leguminous plant or of a solanaceous plant.

7. Process for detecting and identifying a miPEP according to claim 5, in which said eukaryotic cell used in step b) is an animal cell selected from a human cell or Drosophila cell.

8. MiPEP selected from the group of peptides consisting of:
- SEQ ID NO: 1 to SEQ ID NO: 104 and SEQ ID NO: 355,
- SEQ ID NO: 375 to SEQ ID NO: 386, or
- SEQ ID NO: 424.

9. Nucleic acid molecule encoding a miPEP such as defined in claim 8, said nucleic acid molecule being selected from the group of nucleic acids consisting of:
- SEQ ID NO: 105 to SEQ ID NO: 208 and SEQ ID NO: 356,
- SEQ ID NO: 387 to SEQ ID NO: 399, or
- SEQ ID NO: 425.

10. Use of at least:
- a miPEP such as defined according to claim 8,
- a nucleic acid according to claim 9 encoding said miPEP, or
- a vector containing said nucleic acid,
to modulate the expression of a gene in a specified eukaryotic plant cell,
said specified eukaryotic plant cell being capable of expressing a microRNA, the primary transcript of which contains at least one nucleotide sequence encoding said at least one miPEP and the accumulation of which is modulated by the said at least one miPEP,
the expression of said gene being regulated by said microRNA, said miPEP being selected from the group of peptides consisting of:
SEQ ID NO: 59, SEQ ID NO: 60, SEQ ID NO: 24, SEQ ID NO: 25, SEQ ID NO: 26, SEQ ID NO: 43, SEQ ID NO: 76,
said nucleic acid being selected from the group of nucleic acids consisting of:
SEQ ID NO: 163, SEQ ID NO: 164, SEQ ID NO: 128, SEQ ID NO: 129, SEQ ID NO: 130, SEQ ID NO: 147, SEQ ID NO: 180,
said microRNA being selected from the group of nucleic acids consisting of:
SEQ ID NO: 319, SEQ ID NO: 297, SEQ ID NO: 305, SEQ ID NO: 331.

11. Process for modulating the expression of a gene regulated by a microRNA in a eukaryotic plant cell,
comprising carrying out a step of accumulation of a miPEP in said eukaryotic cell, said miPEP being selected from the group of peptides consisting of: SEQ ID NO: 59, SEQ ID NO: 60, SEQ ID NO: 24, SEQ ID NO: 25, SEQ ID NO: 26, SEQ ID NO: 43, SEQ ID NO: 76
in which the accumulation of said miPEP in said eukaryotic cell induces a modulation of the expression of said gene with respect to the expression of said gene without accumulation of said miPEP.

12. Use of a composition comprising at least:
- a miPEP such as defined according to claim 8, with the exception of miPEPs of sequences SEQ ID NO: 102, SEQ ID NO: 103, SEQ ID NO: 104 and SEQ ID NO: 355,
- a nucleic acid according to claim 9 encoding said miPEP, with the exception of nucleic acids of sequences SEQ ID NO: 206, SEQ ID NO: 207, SEQ ID NO: 208 and SEQ ID NO: 356, or
- a vector containing said nucleic acid,
as a phytopharmaceutical agent,
- for promoting the growth and/or the development of plants,
- or for modifying the physiological parameters of plant seeds
- or for preventing or treating plant diseases,
said composition being applied by coating or by film-coating on said plant seeds.

13. Use of a composition according to claim 12 for modulating the physiological parameters of a plant.

14. Use of a composition according to claim 13 for modulating the physiological parameters of a plant, said parameters being selected from the biomass, the foliar surface area, flowering and fruit size.

15. Use of a composition according to claim 12 for modulating the physiological parameters of plant seeds, said parameters being chosen from germination, root development and resistance to water stress.

16. Use of a composition according to claim 12 for promoting resistance to infectious diseases.

17. Use of a composition comprising at least:
- a miPEP such as defined according to claim 8 with the exception of miPEPs of sequences SEQ ID NO: 102, SEQ ID NO: 103, SEQ ID NO: 104 and SEQ ID NO: 355,
- a nucleic acid according to claim 9 encoding said miPEP, with the exception of nucleic acids of sequences SEQ ID NO: 206, SEQ ID NO: 207, SEQ ID NO: 208 and SEQ ID NO: 356, or
- a vector containing said nucleic acid,
as a herbicide, for eradicating plants or slowing their growth, or
as a pesticide for eradicating organisms harmful to plants or liable to be classified as such.

18. Use of a composition according to claim 17, wherein said pesticide is selected from the group consisting of: an insecticide, an arachnicide, a molluscicide, and a rodenticide.

19. Use of a composition according to any one of claims 17 or 18, wherein said composition as a pesticide is applied to a plant or to a support in contact with the plant.

20. A composition comprising in combination a quantity of seeds of a plant and a quantity of a peptide, the sequence of which comprises or consists of a sequence identical to that of a miPEP naturally present in said plant,
said miPEP being selected from the group of peptides consisting of: SEQ ID NO: 59, SEQ ID NO: 60, SEQ ID NO: 24, SEQ ID NO: 25, SEQ ID NO: 26, SEQ ID NO: 43, SEQ ID NO: 76.

21. The composition of claim 20, said composition being formulated so as to form a coated seed.


### SEQUENCE LISTING

<110> CENTRE NATIONAL DE LA RECHERCHE SCIENTIFIQUE - CNRS UNIVERSITE PAUL SABATIER
<120> MICROPEPTIDES ET LEUR UTILISATION POUR MODULER L'EXPRESSION DE GENES
<130> WOB 13 CK TLS IPEP
<150> FR 13/60727
   <151> 2013-10-31
<150> FR 14/55044
   <151> 2013-10-31
<160> 495
<170> PatentIn version 3.5
<210> 1
   <211> 33
   <212> PRT
   <213> Arabidopsis thaliana
<400> 1
   Met Phe Cys Ser Ile Gln Cys Val Ala Arg His Leu Phe Pro Leu His
   1 5 10 15
   Val Arg Glu Ile Lys Lys Ala Thr Arg Ala Ile Lys Lys Gly Lys Thr
   20 25 30
   Leu
<210> 2
   <211> 24
   <212> PRT
   <213> Arabidopsis thaliana
<400> 2
   Met Arg Arg Gln Thr Ser Val Pro Phe Ala Cys Lys Arg Asp Lys Glu
   1 5 10 15
   Ser Asp Lys Ser His Lys Glu Arg
   20
<210> 3
   <211> 37
   <212> PRT
   <213> Arabidopsis thaliana
<400> 3
   Met Val Met Phe Phe Leu Asp Leu Asp Lys Asn Pro Arg Phe Asp Leu
   1 5 10 15
   Leu Lys Gly Leu Lys Trp Asn Leu Phe Ser Ser His Ile Ser Pro Ser
   20 25 30
   Leu Pro Pro Ser Leu
   35
<210> 4
   <211> 11
   <212> PRT
   <213> Arabidopsis thaliana
<400> 4
   Met Lys Asp Asn Phe Pro Leu Leu Leu Arg Leu
   1 5 10
<210> 5
   <211> 4
   <212> PRT
   <213> Arabidopsis thaliana
<400> 5
   Met Ser Asp Asp
   1
<210> 6
   <211> 16
   <212> PRT
   <213> Arabidopsis thaliana
<400> 6
   Met Ile Tyr Ile Asn Lys Tyr Gly Ser Ile Ser Ala Val Glu Asp Asp
   1 5 10 15
<210> 7
   <211> 4
   <212> PRT
   <213> Arabidopsis thaliana
<400> 7
   Met Ser Gln Arg
   1
<210> 8
   <211> 16
   <212> PRT
   <213> Arabidopsis lyrata
<400> 8
   Met Thr Cys Pro Leu Leu Ser Leu Ser Phe Leu Leu Ser Lys Tyr Ile
   1 5 10 15
<210> 9
   <211> 16
   <212> PRT
   <213> Arabidopsis thaliana
<400> 9
   Met Thr Trp Pro Leu Leu Ser Leu Ser Phe Leu Leu Ser Lys Tyr Val
   1 5 10 15
<210> 10
   <211> 18
   <212> PRT
   <213> Capsella rubella
<400> 10
   Met Thr Cys Thr Leu Ser Ala Leu Ser Leu Ser Leu Asn Met Phe Arg
   1 5 10 15
   Val Asn
<210> 11
   <211> 5
   <212> PRT
   <213> Arabidopsis thaliana
<400> 11
   Met Phe Tyr Leu Ser
   1 5
<210> 12
   <211> 37
   <212> PRT
   <213> Arabidopsis thaliana
<400> 12
   Met Val Asn Thr Ser Ser Phe Phe Ile Ser Ser Phe Ile Leu Pro Leu
   1 5 10 15
   Val Leu Ser Glu Ser Asn Cys Leu Leu Phe Arg Thr Ile Tyr Lys Phe
   20 25 30
   Ser Met Val Leu Tyr
   35
<210> 13
   <211> 24
   <212> PRT
   <213> Arabidopsis thaliana
<400> 13
   Met Phe Cys Leu Leu Ile Pro Ile Phe Ser Phe Val Phe Ser Pro Asn
   1 5 10 15
   Arg His Leu Arg Leu Gln Glu Gln
   20
<210> 14
   <211> 5
   <212> PRT
   <213> Arabidopsis thaliana
<400> 14
   Met Phe Ser Pro Gln
   1 5
<210> 15
   <211> 23
   <212> PRT
   <213> Arabidopsis thaliana
<400> 15
   Met Lys Tyr Ile His Ile Leu Ile Leu Phe Lys Ser Arg Ser Thr Tyr
   1               5                   10                  15
   Lys Leu Ser Thr Asn His Ile
               20
<210> 16
   <211> 10
   <212> PRT
   <213> Arabidopsis thaliana
<400> 16
   Met Lys Ile Pro Leu Phe Leu Pro Lys Leu
   1               5                   10
<210> 17
   <211> 27
   <212> PRT
   <213> Arabidopsis thaliana
<400> 17
   Met Val Ser Gly Gln Glu Asp Ser Trp Leu Lys Leu Ser Ser Leu Cys
   1               5                   10                  15
   Phe Leu Phe Leu Ser Leu Leu Asp Ser Leu Ile
               20                  25
<210> 18
   <211> 34
   <212> PRT
   <213> Arabidopsis thaliana
<400> 18
   Met Phe Leu Leu Ile Phe Leu Arg Leu Ile Met Ile Cys Val Cys Ser
   1               5                   10                  15
   Ser Thr Asp Phe Leu Arg Ser Val Asn Tyr Phe Cys Leu Phe Ile Tyr
               20                  25                  30
   Asp Leu
<210> 19
   <211> 9
   <212> PRT
   <213> Arabidopsis thaliana
<400> 19
   Met Ser Thr Thr Gln Glu His Arg Ser
   1               5
<210> 20
   <211> 25
   <212> PRT
   <213> Arabidopsis thaliana
<400> 20
   Met Ile Leu Lys Cys Trp Ser Ser Arg Phe Leu Arg Val Ser Pro Tyr
   1               5                   10                  15
   Gln Asn Ala His Ser Leu Ser Leu Gly
               20                  25
<210> 21
   <211> 13
   <212> PRT
   <213> Arabidopsis lyrata
<400> 21
   Met Pro Leu Ala Val Ile Arg Gln Gly Ile Val Trp Pro
   1               5                   10
<210> 22
   <211> 24
   <212> PRT
   <213> Arabidopsis lyrata
<400> 22
   Met Pro Ser Trp His Asp Met Val Leu Leu Pro Tyr Val Lys His Thr
     1               5                   10                  15
   His Ala Asn Thr Arg His Ile Thr
               20
<210> 23
   <211> 8
   <212> PRT
   <213> Arabidopsis lyrata
<400> 23
   Met Thr Trp Phe Phe Cys Leu Thr
   1               5
<210> 24
   <211> 37
   <212> PRT
   <213> Arabidopsis thaliana
<400> 24
   Met Pro Ser Trp His Gly Met Val Leu Leu Pro Tyr Val Lys His Thr
   1               5                   10                  15
   His Ala Ser Thr His Thr His Thr His Asn Ile Tyr Gly Cys Ala Cys
               20                  25                  30
   Glu Leu Val Phe His
           35
<210> 25
   <211> 22
   <212> PRT
   <213> Arabidopsis thaliana
<400> 25
   Met Ala Trp Tyr Gly Ser Phe Ala Leu Arg Lys Thr His Ser Arg Gln
   1               5                   10                  15
   His Thr His Thr His Thr
               20
<210> 26
   <211> 8
   <212> PRT
   <213> Arabidopsis thaliana
<400> 26
   Met Val Trp Phe Phe Cys Leu Thr
   1               5
<210> 27
   <211> 7
   <212> PRT
   <213> Brassica rapa
<400> 27
   Met Met Ile Ile Leu Trp Lys
   1               5
<210> 28
   <211> 24
   <212> PRT
   <213> Brassica rapa
<400> 28
   Met Leu Trp Ala Lys Leu Val Ser Phe Ser Thr Leu His Ser Leu Val
   1               5                   10                  15
   Phe Leu Leu Ser Pro Ser Phe Ala
               20
<210> 29
   <211> 29
   <212> PRT
   <213> Brassica rapa
<400> 29
   Met Pro Ser Trp His Gly Ile Val Ile Leu Pro Phe Val Lys His Thr
   1               5                   10                  15
   His Ala Asn Ile His Tyr Ser Tyr Ser Cys Val Cys Ile
               20                  25
<210> 30
   <211> 34
   <212> PRT
   <213> Carica papaya
<400> 30
   Met Ile Ala Cys His Pro Tyr Leu Pro Phe Pro Leu Phe Leu Ser Leu
   1               5                   10                  15
   Thr Phe Tyr Ser Ile Phe Phe Ser Pro Ser Pro Pro Ser Pro Ser Leu
               20                  25                  30
   Pro Leu
<210> 31
   <211> 53
   <212> PRT
   <213> Carica papaya
<400> 31
   Met Pro Ser Leu Leu Ala Phe Ser Pro Phe Pro Phe Ser Asn Ile Leu
   1               5                   10                  15
   Leu Asn Leu Leu Leu Pro Leu Pro Pro Phe Pro Leu Ser Ala Ile Ile
               20                  25                  30
   Thr Ile Ile Lys Pro Leu Ser Leu Ser Leu Pro Leu Ser Leu Ser Leu
           35                  40                  45
   Ser Gly Phe Ser Val
       50
<210> 32
   <211> 15
   <212> PRT
   <213> Capsella rubella
<400> 32
   Met Glu Leu Lys Gly Leu Arg Thr Trp Gln Leu Leu Asp Lys Val
   1 5 10 15
<210> 33
   <211> 14
   <212> PRT
   <213> Capsella rubella
<400> 33
   Met Pro Ser Trp His Gly Met Ala Cys Phe Tyr Cys Leu Thr
   1 5 10
<210> 34
   <211> 24
   <212> PRT
   <213> Capsella rubella
<400> 34
   Met Ala Trp His Gly Met Phe Leu Leu Pro Tyr Val Lys His Thr His
   1 5 10 15
   Ala Asn Thr Tyr Ser Leu Tyr Met
   20
<210> 35
   <211> 35
   <212> PRT
   <213> Gossypium raimondii
<400> 35
   Met Met Arg Ser Arg Ile Leu Lys Phe Gln Tyr Arg Phe Gly Met Gly
   1 5 10 15
   Ile Gly Gly Arg Lys Gln Leu Lys Asn Gln Leu Cys Gln Ile Gln Gly
   20 25 30
   Arg Ile Ser
   35
<210> 36
   <211> 10
   <212> PRT
   <213> Gossypium raimondii
<400> 36
   Met Ser Asn Ser Arg Ser Tyr Gln Leu Lys
   1 5 10
<210> 37
   <211> 17
   <212> PRT
   <213> Gossypium raimondii
<400> 37
   Met Asn Glu Asp Leu Glu Ile Ser Thr Arg Lys Arg Thr Pro Gln Leu
   1               5                   10                  15
   Cys
<210> 38
   <211> 12
   <212> PRT
   <213> Medicago truncatula
<400> 38
   Met Pro Lys Phe Asp Ile Phe Phe Tyr Ile Phe Val
   1               5                   10
<210> 39
   <211> 83
   <212> PRT
   <213> Medicago truncatula
<400> 39
   Met Ser Tyr Ile Ser Leu Ser Pro Lys Leu Leu Pro Ile Asn Thr Lys
   1               5                   10                  15
   Pro Phe Pro Trp Leu Val Gln Phe Asn Phe Tyr Phe Ser Ser Asn Thr
               20                  25                  30
   Lys Cys Asn Lys Leu His Phe Leu Gly Glu Lys Leu Leu Val Gly Glu
           35                  40                  45
   Ala Gly His Val Gln Ile Leu Phe Leu Ile His Ser Leu Ile Met His
       50                  55                  60
   Ile Asn Ile Phe Cys Thr Cys Ser Pro Ser Pro Thr Arg Leu Pro His
   65                  70                  75                  80
   Pro Ser Leu
<210> 40
   <211> 20
   <212> PRT
   <213> Oryza sativa
<400> 40
   Met Gln Thr His Ser Asn Thr Pro Gln Ser Thr Tyr Ser Leu Ser Leu
   1               5                   10                  15
   Ser Leu Ser Glu
               20
<210> 41
   <211> 15
   <212> PRT
   <213> Oryza sativa
<400> 41
   Met Cys Val Cys Asp Ile Asn Met His Ser Met Leu Met Leu Leu
   1               5                   10                  15
<210> 42
   <211> 25
   <212> PRT
   <213> Arabidopsis lyrata
<400> 42
   Met Arg Ile Lys Leu Phe Gln Leu Arg Gly Met Leu Ser Gly Ser Arg
   1               5                   10                  15
   Ile Leu Tyr Ile Tyr Thr Cys Val Cys
               20                  25
<210> 43
   <211> 18
   <212> PRT
   <213> Arabidopsis thaliana
<400> 43 Ile Leu
<210> 44
   <211> 36
   <212> PRT
   <213> Brassica carinata
<400> 44
   Met Arg Met Lys Leu Phe Gln Leu Arg Gly Met Leu Ser Gly Ser Arg
   1               5                   10                  15
   Ile Leu Tyr Ile His Lys Tyr Val Tyr Met Leu Ile Gln Val Phe Asp
               20                  25                  30
   His Ile Cys Ile
           35
<210> 45
   <211> 27
   <212> PRT
   <213> Brassica juncea
<400> 45
   Met Arg Met Lys Leu Phe Gln Leu Arg Gly Met Leu Ser Gly Ser Arg
   1               5                   10                  15
   Ile Leu Tyr Ile His Lys Tyr Val Tyr Ile Cys
               20                  25
<210> 46
   <211> 36
   <212> PRT
   <213> Brassica napus
<400> 46
   Met Arg Met Lys Leu Phe Gln Leu Arg Gly Met Leu Ser Gly Ser Arg
   1               5                   10                  15
   Ile Leu Tyr Ile His Lys Tyr Val Tyr Met Ile Ile Gln Val Phe Asp
               20                  25                  30
   His Ile Cys Ile
           35
<210> 47
   <211> 36
   <212> PRT
   <213> Brassica oleracea
<400> 47
   Met Arg Met Lys Leu Phe Gln Leu Arg Gly Met Leu Ser Gly Ser Arg
   1               5                   10                  15
   Ile Leu Tyr Ile His Lys Tyr Val Tyr Met Leu Ile Gln Val Phe Asp
               20                  25                  30
   His Ile Cys Ile
           35
<210> 48
   <211> 27
   <212> PRT
   <213> Brassica rapa
<400> 48
   Met Arg Met Lys Leu Phe Gln Leu Arg Gly Met Leu Ser Gly Ser Arg
   1               5                   10                  15
   Ile Leu Tyr Ile His Lys Tyr Val Tyr Ile Cys
               20                  25
<210> 49
   <211> 19
   <212> PRT
   <213> Arabidopsis thaliana
<400> 49
   Met Leu Asp Leu Phe Arg Ser Asn Asn Arg Ile Glu Pro Ser Asp Phe
   1               5                   10                  15
   Arg Phe Asp
<210> 50
   <211> 4
   <212> PRT
   <213> Arabidopsis thaliana
<400> 50
   Met Arg Asp Arg
   1
<210> 51
   <211> 10
   <212> PRT
   <213> Arabidopsis thaliana
<400> 51
   Met Asn Arg Lys Ile Ser Leu Ser Leu Ser
   1               5                   10
<210> 52
   <211> 8
   <212> PRT
   <213> Arabidopsis thaliana
<400> 52
   Met Met Gly Cys Phe Val Gly Phe
   1               5
<210> 53
   <211> 8
   <212> PRT
   <213> Arabidopsis thaliana
<400> 53
   Met Gln Glu Glu Thr Tyr Glu Gly
   1               5
<210> 54
   <211> 59
   <212> PRT
   <213> Arabidopsis thaliana
<400> 54
   Met Pro His Thr Asn Leu Lys Asp Leu Phe Ile Phe Ser Pro Asn Val
   1               5                   10                  15
   Phe Phe Ser Phe Ala Ile Tyr Leu His Asn Ser Trp Asn Lys Asn Tyr
               20                  25                  30
   Ile His Lys Arg Glu Asn Phe His Asn Thr Ser Phe Ala Leu Ile Phe
           35                  40                  45
   Phe Phe Ser Ser Ile Met Ser Ile Asn Tyr Gly
       50                  55
<210> 55
   <211> 39
   <212> PRT
   <213> Arabidopsis thaliana
<400> 55
   Met Phe Phe Phe Arg Leu Leu Phe Ile Ser Thr Ile Leu Gly Thr Lys
   1               5                   10                  15
   Thr Thr Phe Thr Asn Glu Arg Ile Phe Thr Thr Pro Leu Leu Leu Ser
               20                  25                  30
   Phe Phe Phe Phe Arg Pro Leu
           35
<210> 56
   <211> 6
   <212> PRT
   <213> Arabidopsis thaliana
<400> 56
   Met Arg His Lys Glu Ser
   1 5
<210> 57
   <211> 34
   <212> PRT
   <213> Arabidopsis thaliana
<400> 57
   Met Asn Leu Leu Lys Lys Glu Arg Gln Arg Arg Arg Gln Arg Ser Ile
   1 5 10 15
   Gly Ser His Cys Ile Ala Ser Leu Val Leu Lys Asp Gly Tyr Met Lys
   20 25 30
   Lys Ile
<210> 58
   <211> 9
   <212> PRT
   <213> Arabidopsis thaliana
<400> 58
   Met Val Leu Ser Gly Lys Leu Thr Phe
   1 5
<210> 59
   <211> 20
   <212> PRT
   <213> Medicago truncatula
<400> 59
   Met Leu Leu His Arg Leu Ser Lys Phe Cys Lys Ile Glu Arg Asp Ile
   1 5 10 15
   Val Tyr Ile Ser
   20
<210> 60
   <211> 5
   <212> PRT
   <213> Medicago truncatula
<400> 60
   Met Lys Ile Glu Glu
   1               5
<210> 61
   <211> 34
   <212> PRT
   <213> Zea mays
<400> 61
   Met His Leu Pro Ser Thr Pro Ser Arg Pro Pro Pro Gln His Thr Ser
   1               5                   10                  15
   Leu Ser Phe Leu Gly Lys Glu Met Thr Lys Gly Thr Thr Thr Ala Cys
               20                  25                  30
   Phe Gly
<210> 62
   <211> 10
   <212> PRT
   <213> Arabidopsis thaliana
<400> 62
   Met Leu Ser Leu Ser His Phe His Ile Cys
   1               5                   10
<210> 63
   <211> 23
   <212> PRT
   <213> Medicago truncatula
<400> 63
   Met Met Val Phe Gly Lys Pro Lys Lys Ala Met Leu Val Arg Phe Asn
   1               5                   10                  15
   Pro Lys Thr Asp Leu His Val
               20
<210> 64
   <211> 10
   <212> PRT
   <213> Medicago truncatula
<400> 64
   Met Ala Ser Ala Ala Lys Val Tyr Met Ala
   1               5                   10
<210> 65
   <211> 6
   <212> PRT
   <213> Arabidopsis thaliana
<400> 65
   Met Ala Ser Lys Ile Trp
   1 5
<210> 66
   <211> 10
   <212> PRT
   <213> Arabidopsis thaliana
<400> 66
   Met Val Arg Phe Gln Leu Ser Ile Arg Asp
   1 5 10
<210> 67
   <211> 12
   <212> PRT
   <213> Arabidopsis thaliana
<400> 67
   Met Cys Thr Tyr Tyr Tyr Leu Ile Asn Lys Tyr Phe
   1 5 10
<210> 68
   <211> 11
   <212> PRT
   <213> Arabidopsis thaliana
<400> 68
   Met Phe Pro Ala Lys Trp Cys Arg Leu Glu Ser
   1 5 10
<210> 69
   <211> 22
   <212> PRT
   <213> Arabidopsis thaliana
<400> 69
   Met Gly Ser Leu Ser Leu Phe Lys Ser Gln Leu Glu Ile Leu Met Leu
   1 5 10 15
   Leu Leu Ser Leu Ser Lys
   20
<210> 70
   <211> 20
   <212> PRT
   <213> Arabidopsis thaliana
<400> 70
   Met Ser Val Tyr Ile His Val Pro Ile Ser Leu Asn Cys Phe Ser Pro
   1               5                   10                  15
   Lys Ser Ser Cys
               20
<210> 71
   <211> 11
   <212> PRT
   <213> Arabidopsis thaliana
<400> 71
   Met Gly Val Pro Asn Phe Arg Pro Arg Asn Arg
   1               5                   10
<210> 72
   <211> 23
   <212> PRT
   <213> Arabidopsis cebennensis
<400> 72
   Met Arg Ser Arg Val Ser Phe Phe Phe Phe Lys Ile Met Leu Phe Arg
   1               5                   10                  15
   Leu Leu Gly Tyr Arg Ser Met
               20
<210> 73
   <211> 44
   <212> PRT
   <213> Arabidopsis cebennensis
<400> 73
   Met His Thr Tyr Ile His Thr Ile Ser Asn Ile Ser Ser Ile Phe Phe
   1               5                   10                  15
   Cys Ser Lys Arg Ser Phe Ser Pro Phe Thr Tyr Ile Arg Ile Ile Val
               20                  25                  30
   Val Ile Asp Pro Phe Arg Ile Ala Leu Thr Phe Arg
           35                  40
<210> 74
   <211> 22
   <212> PRT
   <213> Arabidopsis halleri
<400> 74
   Met Arg Ser Arg Val Ser Leu Phe Leu Ser Phe Ser Ser Asn Phe Ala
   1               5                   10                  15
   Ala Tyr Ser Pro Arg Ser
               20
<210> 75
   <211> 48
   <212> PRT
   <213> Arabidospis lyrata
<400> 75
   Met His Thr Tyr Ile Pro Ser Ser Ser Phe Pro Ile Ser Asn Ile Ser
   1               5                   10                  15
   Ser Val Phe Phe Cys Tyr Lys Arg Ser Phe Ser Pro Tyr Thr Tyr Ile
               20                  25                  30
   Arg Ile Ile Val Val Ile Asp Pro Phe Arg Ile Ala Leu Thr Phe Arg
           35                  40                  45
<210> 76
   <211> 50
   <212> PRT
   <213> Arabidopsis thaliana
<400> 76
   Met Asn Ile His Thr Tyr His His Leu Leu Phe Pro Ser Leu Val Phe
   1               5                   10                  15
   His Gln Ser Ser Asp Val Pro Asn Ala Leu Ser Leu His Ile His Thr
               20                  25                  30
   Tyr Glu Tyr Ile Ile Val Val Ile Asp Pro Phe Arg Ile Thr Leu Ala
           35                  40                  45
   Phe Arg
       50
<210> 77
   <211> 19
   <212> PRT
   <213> Arabidopsis thaliana
<400> 77
   Met Phe Gln Thr Leu Tyr Leu Phe Ile Tyr Ile His Thr Asn Ile Leu
   1 5 10 15
   Leu Leu Ser
<210> 78
   <211> 45
   <212> PRT
   <213> Brassica rapa
<400> 78
   Met Phe Lys Leu Tyr Phe Ser Ala Ile Leu Ser Thr Gln Tyr Met His
   1 5 10 15
   Thr Tyr His His Arg Ile Ala Leu Ile Phe Leu Ser Ile Leu Tyr Pro
   20 25 30
   Ser Thr Asn Tyr Leu Met Ser Pro Ile Leu Asn Pro Thr
   35 40 45
<210> 79
   <211> 20
   <212> PRT
   <213> Carica papaya
<400> 79
   Met Lys Ile Lys Leu Gly Phe Ser Leu Ile Lys Ile Ile Ile Leu Leu
   1 5 10 15
   Asp Lys Asn Ser
   20
<210> 80
   <211> 21
   <212> PRT
   <213> Capsella rubella
<400> 80
   Met His Pro His Thr Tyr Ile His Ile Pro Ser Ser Ser Phe Leu Ile
   1 5 10 15
   Ser Ser Phe Cys Leu
   20
<210> 81
   <211> 45
   <212> PRT
   <213> Eucalyptus grandis
<400> 81
   Met Lys His Ile Gln Arg Trp Arg Tyr Gly Glu Thr Ser Gly Arg Gln
   1               5                   10                  15
   Gly Asp Trp Lys Arg Leu Glu Ile Lys Val His Ser Asn Pro Ser Leu
               20                  25                  30
   Lys Val Lys Lys Asn Thr Asn Asn Phe Ser Ser Ser Leu
           35                  40                  45
<210> 82
   <211> 28
   <212> PRT
   <213> Gossypium raimondii
<400> 82
   Met Ile His Phe Asn Leu Ser Gln Trp Arg Ala Ile Cys Met Ala Asn
   1               5                   10                  15
   Phe His Leu Thr Tyr Ser Phe Leu Phe Gly Val Leu
               20                  25
<210> 83
   <211> 21
   <212> PRT
   <213> Medicago truncatula
<400> 83
   Met His Val Tyr Leu Glu Leu Phe Met Val Ile Lys Gly Leu Gly Phe
   1               5                   10                  15
   Leu Leu Leu Val Lys
               20
<210> 84
   <211> 23
   <212> PRT
   <213> Oryza sativa
<400> 84
   Met Glu Met Ile Gln Arg Pro Cys Leu Ile Leu Lys Phe Phe Phe Lys
   1               5                   10                  15
   Leu Ser Thr Leu Tyr Ile Pro
               20
<210> 85
   <211> 38
   <212> PRT
   <213> Physcomitrella patens
<400> 85
   Met Phe His Arg Arg Arg Ser Ser Val Leu Leu Pro Pro Phe Gly Gln
   1               5                   10                  15
   Thr Gln Pro Asn Pro Arg Cys Leu Pro Asp Leu Arg Phe Pro Ser Cys
               20                  25                  30
   Phe Thr Pro Cys Thr Ala
           35
<210> 86
   <211> 42
   <212> PRT
   <213> Thellungiella halophila
<400> 86
   Met Thr Ile Cys Lys Val Ser Lys Ala Cys Phe Tyr Ala Gly Lys Ile
   1               5                   10                  15
   Glu Asn Ser Arg Leu Ile Lys Lys Ile Gly Ile Pro Lys Arg Glu Gly
               20                  25                  30
   Ala Pro Phe Ser Pro Ile Arg Glu Asn Gln
           35                  40
<210> 87
   <211> 37
   <212> PRT
   <213> Thellungiella halophila
<400> 87
   Met Glu Ile Gln Ile Lys Lys Lys Asn Leu Tyr Ile Met Asn Thr Gln
   1               5                   10                  15
   Lys Leu Pro Asn Leu Tyr Ile Tyr Ile Tyr Lys Tyr Val Phe Ile Lys
               20                  25                  30
   Leu Met Val Val Glu
           35
<210> 88
   <211> 43
   <212> PRT
   <213> Arabidopsis thaliana
<400> 88
   Met Val Pro Gln Ile Asn Leu Trp Ser Ser Arg Val Ile Leu Lys Ile
   1               5                   10                  15
   Arg Ile Asp Ser Ser Thr His Arg Glu Glu Asp His Cys Ile Gln Asn
   20 25 30
   His Lys His Gly Leu Ser Phe Ile Phe Ser Phe
   35 40
<210> 89
   <211> 16
   <212> PRT
   <213> Arabidopsis thaliana
<400> 89
   Met Ser Leu Gln Phe Tyr Glu Arg Val Ser Phe Lys Asn Thr Val Lys
   1 5 10 15
<210> 90
   <211> 12
   <212> PRT
   <213> Arabidopsis thaliana
<400> 90
   Met Thr Glu Gln Glu Glu Glu Ser Gln Met Ser Thr
   1 5 10
<210> 91
   <211> 39
   <212> PRT
   <213> Arabidopsis thaliana
<400> 91
   Met Tyr Leu Gln Tyr Ile Asp Asn Val Ile Ser Ile Tyr Ser Asn Asn
   1 5 10 15
   Arg Arg Val Gly Arg Met Phe Ser Arg Val Pro Leu Ser Thr Ser Leu
   20 25 30
   Glu Ile Gln Phe Phe Ile Lys
   35
<210> 92
   <211> 12
   <212> PRT
   <213> Arabidopsis thaliana
<400> 92
   Met Ser Lys Glu Ile Phe Phe Ser Pro Gly Phe Glu
   1 5 10
<210> 93
   <211> 44
   <212> PRT
   <213> Arabidopsis thaliana
<400> 93
   Met Arg Thr His Glu Gln Ser Thr Ala Ile Thr Thr Leu Arg His Cys
   1               5                   10                  15
   Tyr Ser Ser Arg Phe Met Cys Ser Gln Val Thr Pro Ala Glu Leu Phe
               20                  25                  30
   Leu Tyr Arg Pro Cys Phe Ile Asn Ala Val Ala Arg
           35                  40
<210> 94
   <211> 5
   <212> PRT
   <213> Arabidopsis thaliana
<400> 94
   Met Lys Arg Asn Met
   1               5
<210> 95
   <211> 5
   <212> PRT
   <213> Arabidopsis thaliana
<400> 95
   Met Gln Cys Glu Ile
   1               5
<210> 96
   <211> 4
   <212> PRT
   <213> Arabidopsis thaliana
<400> 96
   Met Phe Cys Ala
   1
<210> 97
   <211> 6
   <212> PRT
   <213> Arabidopsis thaliana
<400> 97
   Met Val Met Ala His His
   1               5
<210> 98
   <211> 54
   <212> PRT
   <213> Arabidopsis thaliana
<400> 98
   Met Met Lys Pro Arg Trp Asn Cys Ser Leu Tyr Gly Ile Thr Glu Trp
   1               5                   10                  15
   Thr Asn Asn Gln Asn Gln Lys Ser Lys Arg Lys Gly Arg Arg Lys Thr
               20                  25                  30
   Gln Ile Trp Arg Ile Gly Asp Arg Leu Asp Thr Val Glu Cys Ile Thr
           35                  40                  45
   Leu Met Leu Ser Ala Tyr
       50
<210> 99
   <211> 10
   <212> PRT
   <213> Arabidopsis thaliana
<400> 99
   Met Leu Leu Ile Ile Val Glu Leu Val Leu
   1               5                   10
<210> 100
   <211> 14
   <212> PRT
   <213> Arabidopsis thaliana
<400> 100
   Met Leu Cys Phe Asn Phe Arg Cys Val Arg Arg Phe Ala Glu
   1               5                   10
<210> 101
   <211> 36
   <212> PRT
   <213> Arabidopsis thaliana
<400> 101
   Met Tyr Thr Tyr Gln Leu Asp Asn Ser Phe Ser Trp Phe Leu Cys Thr
   1               5                   10                  15
   Arg Phe Cys Leu Tyr Arg Tyr Phe Leu Phe Asn Phe Arg Cys Phe Arg
               20                  25                  30
   Arg Phe Ser Glu
           35
<210> 102
   <211> 88
   <212> PRT
   <213> Drosophila melanogaster
<400> 102
   Met Trp Arg Glu Val Cys Ala Gln Lys Ser Gln Thr Lys Arg Arg Asn
   1               5                   10                  15
   Phe Ile Thr Gly Asn Gln Arg Arg Asn Lys Thr Lys Ala Asn Arg Lys
               20                  25                  30
   Ala Glu Thr Lys Gln Gln Lys Val Tyr Glu Phe Phe Val Gln Ala Arg
           35                  40                  45
   Glu Arg Cys Lys Thr Arg Lys Lys His Glu Lys Lys Thr Leu Lys Lys
       50                  55                  60
   Thr Lys Lys Ile Gln Asn Arg Tyr Arg Ala Val Ser Glu Asn Glu Trp
   65                  70                  75                  80
   Gly Lys Gly Phe Pro Ser His Ile
                   85
<210> 103
   <211> 36
   <212> PRT
   <213> Drosophila melanogaster
<400> 103
   Met Arg Thr Lys Lys Ser Asn Lys Lys Ala Gln Phe Tyr Tyr Gly Gln
   1               5                   10                  15
   Pro Thr Thr Lys Gln Asn Lys Ser Gln Pro Lys Ser Arg Asn Lys Ala
               20                  25                  30
   Ala Lys Ser Leu
           35
<210> 104
   <211> 71
   <212> PRT
   <213> Drosophila melanogaster
<400> 104
   Met Glu Pro Gly Phe Val Phe Val Leu Phe Pro Thr His Leu Ser Thr
   1               5                   10                  15
   Gln His Thr Gln Arg Glu Lys Ser Ile Leu Val Met Gly Leu Asn Leu
               20                  25                  30
   Gln Ser Ala Lys Gln Ser Asp Lys Gln Asn Ser Lys Glu Arg Lys Lys
           35                  40                  45
   Asn Thr Gln Ile Asn Ser Gln Arg Ile Pro Tyr Arg Gln Gly Gly Gln
       50                  55                  60
   Cys Ser Lys Val Leu Ser Pro
   65                  70
<210> 105
   <211> 92
   <212> DNA
   <213> Arabidopsis thaliana
<400> 105
   atgttctgtt caattcaatg cgtcgccaga catctgttcc ctttgcatgt aagagagata       60
   aagaaagcga caagagccat aaagaaaggt aa                                     92
<210> 106
   <211> 75
   <212> DNA
   <213> Arabidopsis thaliana
<400> 106
   atgcgtcgcc agacatctgt tccctttgca tgtaagagag ataaagaaag cgacaagagc       60
   cataaagaaa ggtaa                                                        75
<210> 107
   <211> 114
   <212> DNA
   <213> Arabidopsis thaliana
<400> 107
   atggttatgt tttttctcga tttagacaaa aaccctagat ttgatcttct aaagggtctc       60
   aaatggaatc tcttctcttc tcatatctct ccctctctcc ctccctctct ttga            114
<210> 108
   <211> 36
   <212> DNA
   <213> Arabidopsis thaliana
<400> 108
   atgaaggaca actttcctct tctccttcgg ttataa 36
<210> 109
   <211> 15
   <212> DNA
   <213> Arabidopsis thaliana
<400> 109
   atgagtgatg actga 15
<210> 110
   <211> 51
   <212> DNA
   <213> Arabidopsis thaliana
<400> 110
   atgatatata taaataaata tgggtcgata tcggctgtgg aggacgacta g 51
<210> 111
   <211> 15
   <212> DNA
   <213> Arabidopsis thaliana
<400> 111
   atgagccaaa gataa 15
<210> 112
   <211> 51
   <212> DNA
   <213> Arabidopsis lyrata
<400> 112
   atgacgtgtc ctcttctctc tctctctttc cttctctcta agtatattta g 51
<210> 113
   <211> 51
   <212> DNA
   <213> Arabidopsis thaliana
<400> 113
   atgacgtggc ctcttctctc tctctctttc cttctctcta agtatgttta g 51
<210> 114
   <211> 53
   <212> DNA
   <213> Capsella rubella
<400> 114
   atgacgtgta ctctctctgc tctatctctc tctctaaata tgtttagggt taa 53
<210> 115
   <211> 18
   <212> DNA
   <213> Arabidopsis thaliana
<400> 115
   atgttttatc tttcataa 18
<210> 116
   <211> 114
   <212> DNA
   <213> Arabidopsis thaliana
<400> 116
   atggttaata ctagtagctt tttcatttca agttttatcc ttccattggt tctttctgag 60
   tcaaattgtc tcctgtttcg aaccatatat aagttttcaa tggttttgta ttaa 114
<210> 117
   <211> 75
   <212> DNA
   <213> Arabidopsis thaliana
<400> 117
   atgttttgtt tgttgattcc catcttctct tttgtctttt caccaaatcg tcatttaagg 60
   cttcaagaac agtaa 75
<210> 118
   <211> 18
   <212> DNA
   <213> Arabidopsis thaliana
<400> 118
   atgttttccc ctcaatga 18
<210> 119
   <211> 72
   <212> DNA
   <213> Arabidopsis thaliana
<400> 119
   atgaaataca tacacatttt gattttattt aaatcaagat cgacgtataa gctatccacc 60
   aatcatattt aa 72
<210> 120
   <211> 33
   <212> DNA
   <213> Arabidopsis thaliana
<400> 120
   atgaaaattc cattgtttct gccgaagctt tga 33
<210> 121
   <211> 84
   <212> DNA
   <213> Arabidopsis thaliana
<400> 121
   atggtatctg gtcaagaaga ttcctggtta aaactttcat ctctctgttt cctttttctt 60
   tctttgttgg attcattaat ttga 84
<210> 122
   <211> 105
   <212> DNA
   <213> Arabidopsis thaliana
<400> 122
   atgtttcttt taatcttttt gagattaata atgatttgtg tttgttcatc aaccgatttt 60
   ctcagatctg tcaattattt ttgtttattt atttatgatt tatga 105
<210> 123
   <211> 30
   <212> DNA
   <213> Arabidopsis thaliana
<400> 123
   atgtccacta ctcaagagca taggtcttga 30
<210> 124
   <211> 78
   <212> DNA
   <213> Arabidopsis thaliana
<400> 124
   atgatactaa agtgctggag ttcccggttc ctgagagtga gtccatatca aaatgcgcat 60
   tcgttatcac ttggttga 78
<210> 125
   <211> 42
   <212> DNA
   <213> Arabidopsis lyrata
<400> 125
   atgcccttag cagttattag acaagggatt gtttggccct ag 42
<210> 126
   <211> 75
   <212> DNA
   <213> Arabidopsis lyrata
<400> 126
   atgccatcat ggcatgacat ggttcttttg ccttacgtaa aacacactca cgccaacaca 60
   cgccacataa cataa 75
<210> 127
   <211> 27
   <212> DNA
   <213> Arabidopsis lyrata
<400> 127
   atgacatggt tcttttgcct tacgtaa 27
<210> 128
   <211> 114
   <212> DNA
   <213> Arabidopsis thaliana
<400> 128
   atgccatcat ggcatggtat ggttcttttg ccttacgtaa aacacactca cgccagcaca 60
   cacacacaca cacataacat atacggatgt gcgtgtgagc tagtcttcca ttaa 114
<210> 129
   <211> 69
   <212> DNA
   <213> Arabidopsis thaliana
<400> 129
   atggcatggt atggttcttt tgccttacgt aaaacacact cacgccagca cacacacaca 60
   cacacataa 69
<210> 130
   <211> 27
   <212> DNA
   <213> Arabidopsis thaliana
<400> 130
   atggtatggt tcttttgcct tacgtaa 27
<210> 131
   <211> 24
   <212> DNA
   <213> Brassica rapa
<400> 131
   atgatgataa ttttgtggaa ataa 24
<210> 132
   <211> 75
   <212> DNA
   <213> Brassica rapa
<400> 132
   atgctttggg ccaagctagt ttcttttagc actcttcact cactagtttt tcttctcagc 60
   ccttcttttg cgtga 75
<210> 133
   <211> 120
   <212> DNA
   <213> Brassica rapa
<400> 133
   atgccatcat ggcatggcat tgtcattttg cctttcgtaa aacacactca cgccaacata 60
   cattattcat attcatgtgt atgtatatga atgccatcat ggcatatgcc atcatggcat 120
<210> 134
   <211> 105
   <212> DNA
   <213> Carica papaya
<400> 134
   atgattgcat gccatcccta cttgcctttt ccccttttcc tttctctaac attttactca 60
   atcttcttct ccccctcccc cccttccccc tctctgccat tataa 105
<210> 135
   <211> 162
   <212> DNA
   <213> Carica papaya
<400> 135
   atgccatccc tacttgcctt ttcccctttt cctttctcta acattttact caatcttctt 60
   ctccccctcc cccccttccc cctctctgcc attataacca taattaaacc tctctccctc 120
   tctctccctc tctctctctc tctctctggg ttctcagtat aa 162
<210> 136
   <211> 48
   <212> DNA
   <213> Capsella rubella
<400> 136
   atggaattaa aaggtttgag aacttggcag ttattagaca aggtatag 48
<210> 137
   <211> 45
   <212> DNA
   <213> Capsella rubella
<400> 137
   atgccatcat ggcatggcat ggcatgtttc tattgcctta cgtaa 45
<210> 138
   <211> 90
   <212> DNA
   <213> Capsella rubella
<400> 138
   atggcatggc atgtttctat tgccttacgt aaaacacact cacgccaaca catactcact 60
   atacatgtaa ataagtatgt gcgcgtgtga 90
<210> 139
   <211> 108
   <212> DNA
   <213> Gossypium raimondii
<400> 139
   atgatgagat caagaatttt aaagtttcaa tatagatttg gcatgggtat tggcggcaga 60
   aagcaattaa aaaaccagtt atgtcaaatt caaggtcgta tcagttaa 108
<210> 140
   <211> 33
   <212> DNA
   <213> Gossypium raimondii
<400> 140
   atgtcaaatt caaggtcgta tcagttaaaa tga 33
<210> 141
   <211> 54
   <212> DNA
   <213> Gossypium raimondii
<400> 141
   atgaatgaag atttagaaat ttcaacaagg aagaggaccc cacagctttg ttaa 54
<210> 142
   <211> 39
   <212> DNA
   <213> Medicago truncatula
<400> 142
   atgcccaaat ttgatatttt tttttatata tttgtatag 39
<210> 143
   <211> 231
   <212> DNA
   <213> Medicago truncatula
<400> 143
   atgtcatata tctctctctc tcctaagttg ctacctataa atactaagcc tttcccttgg 60
   ttggttcaat tcaacttcta cttctcatca aacacaaagt gcaataagct tcatttcctg 120
   ggtgagaagc tccttgttgg agaagcaggg cacgtgcaaa tcctctttct gattcattct 180
   ctcataatgc atatcaatat cttttgcacg tgctcccctt ctccaactag g 231
<210> 144
   <211> 63
   <212> DNA
   <213> Oryza sativa
<400> 144
   atgcaaaccc actccaacac tccacaatcc acatactctc tctctctctc tctctctgag 60
   tag 63
<210> 145
   <211> 48
   <212> DNA
   <213> Oryza sativa
<400> 145
   atgtgtgtgt gtgatatcaa tatgcattcg atgttgatgc tactgtag 48
<210> 146
   <211> 78
   <212> DNA
   <213> Arabidopsis lyrata
<400> 146
   atgagaatta agctatttca gttgagggga atgttgtctg gatcgaggat attatacata 60
   tatacatgtg tatgttga 78
<210> 147
   <211> 57
   <212> DNA
   <213> Arabidopsis thaliana
<400> 147
   atgagggtta agctatttca gttgagggga atgttgtctg gatcgaggat attatag 57
<210> 148
   <211> 111
   <212> DNA
   <213> Brassica carinata
<400> 148
   atgagaatga agctatttca gttgagggga atgttgtctg gatcgaggat attatatata 60
   cacaaatacg tatatatgtt aatacaagtg tttgatcata tatgtatata g 111
<210> 149
   <211> 84
   <212> DNA
   <213> Brassica juncea
<400> 149
   atgagaatga agctatttca gttgagggga atgttgtctg gatcgaggat attatatata 60
   cacaaatatg tatatatatg ttaa 84
<210> 150
   <211> 111
   <212> DNA
   <213> Brassica napus
<400> 150
   atgagaatga agctatttca gttgagggga atgttgtctg gatcgaggat attatatata 60
   cacaaatacg tatatatgat aatacaagtg tttgatcata tatgtatata g 111
<210> 151
   <211> 111
   <212> DNA
   <213> Brassica oleracea
<400> 151
   atgagaatga agctatttca gttgagggga atgttgtctg gatcgaggat attatatata 60
   cacaagtacg tatatatgtt aatacaagtg tttgatcata tatgtatata g 111
<210> 152
   <211> 84
   <212> DNA
   <213> Brassica rapa
<400> 152
   atgagaatga agctatttca gttgagggga atgttgtctg gatcgaggat attatatata 60
   cacaaatatg tatatatatg ttaa 84
<210> 153
   <211> 60
   <212> DNA
   <213> Arabidopsis thaliana
<400> 153
   atgttggatc tctttcgatc taacaatcga attgaacctt cagatttcag atttgattag 60
<210> 154
   <211> 15
   <212> DNA
   <213> Arabidopsis thaliana
<400> 154
   atgagagata gataa 15
<210> 155
   <211> 33
   <212> DNA
   <213> Arabidopsis thaliana
<400> 155
   atgaacagaa aaatctctct ttctctttct tga 33
<210> 156
   <211> 27
   <212> DNA
   <213> Arabidopsis thaliana
<400> 156
   atgatgggtt gttttgtggg attttaa 27
<210> 157
   <211> 27
   <212> DNA
   <213> Arabidopsis thaliana
<400> 157
   atgcaggagg aaacatatga ggggtga 27
<210> 158
   <211> 180
   <212> DNA
   <213> Arabidopsis thaliana
<400> 158
   atgccacata caaacttgaa agatctcttc atcttttctc caaatgtttt tttttcgttt 60
   gctatttatc tccacaattc ttggaacaaa aactacattc acaaacgaga gaattttcac 120
   aacacctctt ttgctctcat tttttttttt tcgtccatta tgagtattaa ttatggttag 180
<210> 159
   <211> 120
   <212> DNA
   <213> Arabidopsis thaliana
<400> 159
   atgttttttt ttcgtttgct atttatctcc acaattcttg gaacaaaaac tacattcaca 60
   aacgagagaa ttttcacaac acctcttttg ctctcatttt ttttttttcg tccattatga 120
<210> 160
   <211> 21
   <212> DNA
   <213> Arabidopsis thaliana
<400> 160
   atgagacata aagagagtta a 21
<210> 161
   <211> 105
   <212> DNA
   <213> Arabidopsis thaliana
<400> 161
   atgaacctcc tcaagaagga aagacagagg aggagacaaa gaagtatagg ttcacattgc        60
   atagccagtt tagttttgaa ggatggatat atgaaaaaaa tatga                       105
<210> 162
   <211> 30
   <212> DNA
   <213> Arabidopsis thaliana
<400> 162
   atggttctct ccggtaaatt aacattttag 30
<210> 163
   <211> 63
   <212> DNA
   <213> Medicago truncatula
<400> 163
   atgcttcttc ataggctctc caaattttgc aaaattgaaa gagacatagt atatatatct        60
   tag                                                                      63
<210> 164
   <211> 18
   <212> DNA
   <213> Medicago truncatula
<400> 164
   atgaagattg aagagtaa 18
<210> 165
   <211> 105
   <212> DNA
   <213> Zea mays
<400> 165
   atgcatctgc cttcaactcc ctctcgcccc ccaccccaac acacatctct ctcttttcta        60
   gggaaggaaa tgacgaaggg gacgacgacg gcatgcttcg gctag                       105
<210> 166
   <211> 33
   <212> DNA
   <213> Arabidopsis thaliana
<400> 166
   atgttgtctc tttctcattt tcatatctgc taa 33
<210> 167
   <211> 72
   <212> DNA
   <213> Medicago truncatula
<400> 167
   atgatggtgt ttgggaagcc gaaaaaagcg atgttggtga ggttcaatcc gaagacggat 60
   ttacatgtat ag 72
<210> 168
   <211> 33
   <212> DNA
   <213> Medicago truncatula
<400> 168
   atggcttcag ctgcaaaagt atacatggcg tga 33
<210> 169
   <211> 21
   <212> DNA
   <213> Arabidopsis thaliana
<400> 169
   atggcttcca agatctggta a 21
<210> 170
   <211> 33
   <212> DNA
   <213> Arabidopsis thaliana
<400> 170
   atggttaggt tccaactaag tatacgagat taa 33
<210> 171
   <211> 39
   <212> DNA
   <213> Arabidopsis thaliana
<400> 171
   atgtgtacgt actattatct cataaataaa tatttttaa 39
<210> 172
   <211> 36
   <212> DNA
   <213> Arabidopsis thaliana
<400> 172
   atgtttccag caaaatggtg ccgtcttgag tcttga 36
<210> 173
   <211> 69
   <212> DNA
   <213> Arabidopsis thaliana
<400> 173
   atgggatctc tctctttatt taaaagtcaa ttagagatct tgatgctact tctgtccctt 60
   tccaagtga 69
<210> 174
   <211> 63
   <212> DNA
   <213> Arabidopsis thaliana
<400> 174
   atgagtgtat atattcatgt acctatctct ctcaattgct tctcaccaaa atcatcttgc 60
   tga 63
<210> 175
   <211> 36
   <212> DNA
   <213> Arabidopsis thaliana
<400> 175
   atgggagttc ccaactttag acctcgaaac cgataa 36
<210> 176
   <211> 72
   <212> DNA
   <213> Arabidopsis cebennensis
<400> 176
   atgagatcta gggtttcttt ctttttcttc aaaatcatgc tttttcgctt gctaggttat 60
   agatccatgt aa 72
<210> 177
   <211> 134
   <212> DNA
   <213> Arabidopsis cebennensis
<400> 177
   atgcatacat acatacatac catctctaat atttcatcaa tcttcttttg ttccaaacgc 60
   ctttctctcc atttacatac atacgaatca ttgttgtcat agatccgttt agaattgctt 120
   taacttttag atga 134
<210> 178
   <211> 69
   <212> DNA
   <213> Arabidopsis halleri
<400> 178
   atgagatcta gggtttcttt gtttctttcg ttttcttcaa attttgctgc atattctcca 60
   agatcatga 69
<210> 179
   <211> 147
   <212> DNA
   <213> Arabidospis lyrata
<400> 179
   atgcatacat acataccatc atcatctttt cccatctcta atatttcatc agtcttcttt 60
   tgttacaaac gctctttctc gccatataca tacataagaa tcattgttgt catagatccg 120
   tttagaattg ctttaacttt tagatga 147
<210> 180
   <211> 153
   <212> DNA
   <213> Arabidopsis thaliana
<400> 180
   atgaatatac atacatacca tcatcttctt ttcccatctc tagtttttca tcaatcttct 60
   gatgttccaa acgctctatc tcttcatata catacatacg aatatattat tgttgtcata 120
   gatccattta gaatcacttt agcttttaga tga 153
<210> 181
   <211> 60
   <212> DNA
   <213> Arabidopsis thaliana
<400> 181
   atgttccaaa cgctctatct cttcatatac atacatacga atatattatt gttgtcatag 60
<210> 182
   <211> 138
   <212> DNA
   <213> Brassica rapa
<400> 182
   atgtttaagc tctacttctc agcaattctc tccacccaat acatgcatac ataccatcat 60
   cgtatcgctc taatttttct atcaatcttg tatccttcca caaattatct tatgtctccc 120
   attttaaatc ctacatag 138
<210> 183
   <211> 63
   <212> DNA
   <213> Carica papaya
<400> 183
   atgaagatta aattaggttt tagtcttatt aagattatta tattactaga caaaaacagt 60
   taa 63
<210> 184
   <211> 66
   <212> DNA
   <213> Capsella rubella
<400> 184
   atgcatccac atacatacat acatatacca tcatcttctt ttctcatctc tagtttttgt 60
   ttataa 66
<210> 185
   <211> 138
   <212> DNA
   <213> Eucalyptus grandis
<400> 185
   atgaagcata ttcaaaggtg gagatatggg gagacttccg gaaggcaagg ggattggaaa 60
   aggctcgaga tcaaagtgca tagcaaccct tcgctaaagg tgaaaaagaa tacgaataac 120
   ttcagtagct cactttaa 138
<210> 186
   <211> 87
   <212> DNA
   <213> Gossypium raimondii
<400> 186
   atgatccatt tcaacctgtc acagtggaga gcaatttgta tggctaattt ccatctcacc 60
   tattcttttc tgtttggggt tctctag 87
<210> 187
   <211> 66
   <212> DNA
   <213> Medicago truncatula
<400> 187
   atgcatgtat atcttgaatt gtttatggta ataaaggggt taggatttct ccttttggtg 60
   aagtga 66
<210> 188
   <211> 72
   <212> DNA
   <213> Oryza sativa
<400> 188
   atggaaatga tacaaaggcc gtgtttaatt ttaaaatttt ttttcaaact ttcaacactt 60
   tacatcccat aa 72
<210> 189
   <211> 117
   <212> DNA
   <213> Physcomitrella patens
<400> 189
   atgttccacc gtcggagatc ctcggtgctg ctacccccgt tcggccaaac ccaacccaac 60
   cctaggtgtc tgccggacct ccgcttcccc tcctgcttca ccccctgcac cgcttaa 117
<210> 190
   <211> 129
   <212> DNA
   <213> Thellungiella halophila
<400> 190
   atgacgatat gtaaagtaag caaggcatgt ttttatgcag ggaagattga aaattcaaga 60
   ttaatcaaga aaattggaat accaaaaaga gagggagctc ccttcagtcc aatcagagag 120
   aatcaatga 129
<210> 191
   <211> 114
   <212> DNA
   <213> Thellungiella halophila
<400> 191
   atggagattc aaattaaaaa gaaaaactta tatataatga atacacaaaa gctacctaat 60
   ctgtatatat atatatataa atatgtcttc attaaattaa tggtcgtgga atag 114
<210> 192
   <211> 132
   <212> DNA
   <213> Arabidopsis thaliana
<400> 192
   atggtacctc aaattaatct atggtcatct agggttatct tgaagattag aattgattct 60
   agcacgcaca gagaggaaga tcattgcatc cagaatcaca aacatggcct atcttttatc 120
   ttttcttttt ga 132
<210> 193
   <211> 51
   <212> DNA
   <213> Arabidopsis thaliana
<400> 193
   atgtctctcc aattttatga gagggtttcc ttcaagaaca cagtaaaata g 51
<210> 194
   <211> 39
   <212> DNA
   <213> Arabidopsis thaliana
<400> 194
   atgacagagc aagaagaaga aagtcaaatg tccacatga 39
<210> 195
   <211> 120
   <212> DNA
   <213> Arabidopsis thaliana
<400> 195
   atgtatctac aatatattga taatgtaata tctatatatt caaacaatcg tcgtgttggt 60
   cggatgtttt ctagagttcc tctgagcact tcattggaga tacaattttt tataaaatag 120
<210> 196
   <211> 39
   <212> DNA
   <213> Arabidopsis thaliana
<400> 196
   atgagcaagg agatattttt ttcccctggg tttgaatga 39
<210> 197
   <211> 133
   <212> DNA
   <213> Arabidopsis thaliana
<400> 197
   atgagaacac acgagcaatc aacggctata acgacgctac gtcattgtta cagctctcgt 60
   ttcatgtgtt ctcaggtcac ccctgctgag ctctttctct accgtccatg ttttatcaac 120
   gccgtggccc gtg 133
<210> 198
   <211> 18
   <212> DNA
   <213> Arabidopsis thaliana
<400> 198
   atgaagagaa acatgtaa 8
<210> 199
   <211> 18
   <212> DNA
   <213> Arabidopsis thaliana
<400> 199
   atgcaatgtg aaatatga 18
<210> 200
   <211> 15
   <212> DNA
   <213> Arabidopsis thaliana
<400> 200
   atgttttgtg cttga 15
<210> 201
   <211> 21
   <212> DNA
   <213> Arabidopsis thaliana
<400> 201
   atggtcatgg ctcatcatta g 21
<210> 202
   <211> 165
   <212> DNA
   <213> Arabidopsis thaliana
<400> 202
   atgatgaaac ctcgatggaa ctgctctctt tatggaatca cggaatggac aaataatcaa 60
   aatcagaaat cgaagcgaaa agggaggaga aaaacgcaga tttggaggat tggggacaga 120
   ttagatactg ttgaatgcat cactctaatg ctatcagcct attaa 165
<210> 203
   <211> 33
   <212> DNA
   <213> Arabidopsis thaliana
<400> 203
   atgctgctta tcatcgtgga gttggttctg taa 33
<210> 204
   <211> 45
   <212> DNA
   <213> Arabidopsis thaliana
<400> 204
   atgctttgtt tcaatttcag gtgcgttaga aggtttgcag agtag 45
<210> 205
   <211> 108
   <212> DNA
   <213> Arabidopsis thaliana
<400> 205
   atgtacacct accagcttga taactctttt tcgtggtttc tgtgtactcg tttctgtttg 60
   tacagatact tcttgttcaa tttcagatgc tttagaaggt tttcggag 108
<210> 206
   <211> 267
   <212> DNA
   <213> Drosophila melanogaster
<400> 206
   atgtggcgcg aagtatgcgc acaaaaaagt caaacaaaaa ggcgcaattt tattacgggc 60
   aaccaacgac gaaacaaaac aaaagccaac cgaaaagcag aaacaaagca gcaaaaagtt 120
   tatgaatttt ttgtgcaggc gcgtgaaaga tgcaaaacga gaaaaaaaca tgaaaaaaaa 180
   acattaaaaa aaacaaaaaa aatccaaaac agataccgag ctgtatccga aaacgagtgg 240
   ggaaaggggt ttcccagtca catataa 267
<210> 207
   <211> 111
   <212> DNA
   <213> Drosophila melanogaster
<400> 207
   atgcgcacaa aaaagtcaaa caaaaaggcg caattttatt acgggcaacc aacgacgaaa 60
   caaaacaaaa gccaaccgaa aagcagaaac aaagcagcaa aaagtttatg a 111
<210> 208
   <211> 216
   <212> DNA
   <213> Drosophila melanogaster
<400> 208
   atggagcctg gctttgtttt tgttttattt ccaacccact tgagcacaca gcacacacag 60
   agagaaaaat caatactcgt tatgggatta aatttacaaa gcgcaaagca aagcgacaaa 120
   caaaattcaa aagaaagaaa aaaaaacact caaataaact cacaaagaat tccttatcgc 180
   caagggggcc aatgttctaa ggttctttcg ccttga 216
<210> 209
   <211> 614
   <212> DNA
   <213> Arabidopsis thaliana
<400> 209
   attcattgtt cactctcaaa tctcaagttc attgccattt ttaggtctct ctataaattc 60
   aaatgttctg ttcaattcaa tgcgtcgcca gacatctgtt ccctttgcat gtaagagaga 120
   taaagaaagc gacaagagcc ataaagaaag gtaagactct ttgaaataga gagagataag 180
   gttttctctt atcttcttct catcagatct ttgtttcttt accctctttc tttctttttt 240
   ttgcttttta tggttatgtt ttttctcgat ttagacaaaa accctagatt tgatcttcta 300
   aagggtctca aatggaatct cttctcttct catatctctc cctctctccc tccctctctt 360
   tgattctttg tcttctccag ttaaaactca gatctaacac aaagcttaaa agattctcat 420
   cgtttcttgt tttctttgtt tcatcttgta gatctctgaa gttggactaa ttgtgaatga 480
   aagagttggg acaagagaaa cgcaaagaaa ctgacagaag agagtgagca cacaaaggca 540
   atttgcatat cattgcactt gcttctcttg cgtgctcact gctctttctg tcagattccg 600
   gtgctgatct cttt 614
<210> 210
   <211> 422
   <212> DNA
   <213> Arabidopsis thaliana
<400> 210
   ctctgccttt agttctttct tttttggtaa tatatttatt tttcgttacg atttggtcaa 60
   aaccctagat ttgttttcca aaagcatatc tgaaaatgaa ggacaacttt cctcttctcc 120
   ttcggttata aatattctct ccggttttgc ttgtttaacc taaaagcctc agatctaact 180
   ccaacacctt caaagtctgc ctcctttcca atcttctttc ttctgttcga tctctaatct 240
   cagaatttgt gtcggtaagg taaaggtgat aatgagtgat gactgatgag ggagttttgg 300
   gacaaatttt aagagaaacg catagaaact gacagaagag agtgagcaca caaaggcact 360
   ttgcatgttc gatgcatttg cttctcttgc gtgctcactg ctctatctgt cagattccgg 420
   ct 422
<210> 211
   <211> 268
   <212> DNA
   <213> Arabidopsis thaliana
<400> 211
   tcccacatcc aaagatagaa agatgtaagg tctagagtct tgttcttaat cccctaacag 60
   aacaatgata tatataaata aatatgggtc gatatcggct gtggaggacg actagctacg 120
   gtttcgagcc tggtcacatg cgtagagtgt gaaaggtaat taggaggtga cagaagagag 180
   tgagcacaca tggtggtttc ttgcatgctt ttttgattag ggtttcatgc ttgaagctat 240
   gtgtgcttac tctctctctg tcacccct 268
<210> 212
   <211> 328
   <212> DNA
   <213> Arabidopsis thaliana
<400> 212
   tcccacagcc aatgagccaa agataaagaa acacctatcc tataataatt tagagcaata 60
   tacctccata atggaacatc tatatatata aaggtatccg tatatctcta tatattatat 120
   tcattgagtt taaagtggct agggtttata gatgtatgtg atattaagag atatgaaaca 180
   tatttgtcga cggtttgagt ggtgaggaat tgatggtgac agaagagagt gagcacacat 240
   ggtggctttc ttgcatattt gaaggttcca tgcttgaagc tatgtgtgct cactctctat 300
   ccgtcacccc cttctctccc tctccctc 328
<210> 213
   <211> 592
   <212> DNA
   <213> Arabidopsis lyrata
<400> 213
   aaaaaatgac gtgtcctctt ctctctctct ctttccttct ctctaagtat atttagggtt 60
   aattattagg gttctttatc tctttcttca gtctttgaag tttcttcaat agctttaatt 120
   gaagtgattt acctctctgg gtgtttttag tatatatatc atgtacatga tcgaatttct 180
   ttctatccaa gttctcatca aaccttctca tgttttgaag agttaaaggc tttatagttt 240
   gcttaggtca gatccataac atactgtatt tgacaagttt ctttgtctca cgatagatct 300
   tggtctgacc aaaatgattt tctcgagaaa aaaaaagatg gaagtagagc tccttgaagt 360
   tcaaacgaga gttgagcagg gtaaagaaaa gctgctaagc tatggatccc ataagcccta 420
   atccttataa agaaaaaaaa ggatttggtt atatggcttg catatctcag gagctttaac 480
   ttgcccttta atggctttta ctcttctttg gattgaaggg agctctacat cttctttcac 540
   cttctctatt tttctttctt tattttctcc tctacagtaa tttatttgga tt 592
<210> 214
   <211> 599
   <212> DNA
   <213> Arabidopsis thaliana
<400> 214
   ttccaaaaca tgacgtggcc tcttctctct ctctctttcc ttctctctaa gtatgtttag      60
   ggttaataat tagggttcct cctctctttt gttctgtctt tatatctcct tcatagctct     120
   aatgtaagag atttacctct tttggtgttt ttgttaatcc acgttctcat caaaactttc     180
   tcattgtttt atgaagagtt aaaggtcttt acagtttgct tatgtcagat ccataatata     240
   tttgacaaga tactttgttt ttcgatagat cttgatctga cgatggaagt agagctcctt     300
   aaagttcaaa catgagttga gcagggtaaa gaaaagctgc taagctatgg atcccataag     360
   ccctaatcct tgtaaagtaa aaaaggattt ggttatatgg attgcatatc tcaggagctt     420
   taacttgccc tttaatggct tttactcttc tttggattga agggagctct acatcttctt     480
   tcaccttctc tattttttat ttttctttat ttctactcaa caattattta ttcggattca     540
   tctttaattt tccgttataa tttctttttg gtaaggatta ttcgctataa tttgagaat      599
<210> 215
   <211> 623
   <212> DNA
   <213> Capsella rubella
<400> 215
   ttccaacgaa tgacgtgtac tctctctgct ctatctctct ctctaaatat gtttagggtt      60
   aattagggtt cttcatctgt ctctctctct ctctctcttc agagtcttta tagcttcttc     120
   caagattttt aattgaaagt aatttacctc ttttggagtt ctgtacatat agaatatcag     180
   gagtcgtgtt tcttttttat caaggttctc atctaacctt tatagtattt tcattagttg     240
   ataaaggtct tcatagtttg cttagatcag atcttgtctt cgtcttttcg atagatcttg     300
   ttctgtccaa tatacagtga ttttatttcg agagcaaaaa agatgagagg tagagctcct     360
   tgaagttcaa acgagagttt agcagggtag agaaaagctg ctaagctatg gatcccataa     420
   gccctaatcc ttgttaatga taaaggattt ggttatatgg cttgcatatc tcaggagctt     480
   taacttgccc tttaattgct tttactcttc tttggattga agggagctct acatcttctt     540
   tgacttctct ctctattaag tctttcttta ttttcttctc tacaatagtt gttttggatc     600
   ggaagatctt taagtttccc tta                                             623
<210> 216
   <211> 612
   <212> DNA
   <213> Arabidopsis thaliana
<400> 216
   tttcactttt gttctcctcc tccctttttt tcttttcagg attcttcttt tctatgtttt 60
   atctttcata atagatctga taattttgat ttttcactat atatattatg gttaatacta 120
   gtagcttttt catttcaagt tttatccttc cattggttct ttctgagtca aattgtctcc 180
   tgtttcgaac catatataag ttttcaatgg ttttgtatta actcaagtat tcaacattat 240
   gtctctcttt ttcttgcttg gatctctaat gctgttcata ttttaaagca taggtttagg 300
   ttagatgcat gtaactgcca attaaaagaa ggtcaagagt tttttgattg tatgaatata 360
   tgagttagtc aaagcagatc cacacgatta tatagaaaaa caaaggaaga agaagaggaa 420
   gagctccttg aagttcaatg gagggtttag cagggtgaag taaagctgct aagctatgga 480
   tcccataagc cttatcaaat tcaatataat tgatgataag gtttttttta tggatgccat 540
   atctcaggag ctttcactta cccctttaat ggcttcactc ttctttggat tgaagggagc 600
   tcttcatctc tc 612
<210> 217
   <211> 515
   <212> DNA
   <213> Arabidopsis thaliana
<400> 217
   catcccaccc ttaattgttt tatataaacc atttctcctc ctctctccat caccttcaat 60
   ctctctcgat ctctctctgg atccccaatc tcacctccat gttttgtttg ttgattccca 120
   tcttctcttt tgtcttttca ccaaatcgtc atttaaggct tcaagaacag taaccccaat 180
   tcctccacaa gagggagaga aaacaaaaga tcttccaatt ccattctcgt acatgcaaat 240
   cacaatccat gccatagatt gtttctattc ctccttattt attgcttgta tctgttcatg 300
   catggaccag gtggagagag cattacttaa aaatagaatt agctatctgt tttaggcgaa 360
   ttagtttcct tacataacca tgtatatgtc atgacgcata tacatatgta gatgtatatg 420
   tattatatat gtatgcctgg ctccctgtat gccatatgct gagcccatcg agtatcgatg 480
   acctccgtgg atggcgtatg aggagccatg catat 515
<210> 218
   <211> 276
   <212> DNA
   <213> Arabidopsis thaliana
<400> 218
   actcataact ctccccaaat tcttgaccaa aaatatccgc cactttctct ctggttcatg      60
   ttttcccctc aatgaaatac atacacattt tgattttatt taaatcaaga tcgacgtata     120
   agctatccac caatcatatt taagggttcc cgtatacata tatactatat atatatatgg     180
   aataatagtc gtgcctggct ccctgtatgc cacaagaaaa catcgattta gtttcaaaat     240
   cgatcactag tggcgtacag agtagtcaag catgac                               276
<210> 219
   <211> 243
   <212> DNA
   <213> Arabidopsis thaliana
<400> 219
   ctctaactca tccttctctt ctatgaaaat tccattgttt ctgccgaagc tttgatcagt      60
   acttctcttt tgcttgatct cggtttttga ccagtttatt gcgtcgatca atgcattgaa     120
   agtgactaca tcggggttcc gatttttttt gttcttcata tgatgaagcg gaaacagtaa     180
   tcaaccctgg tttagtcact ttcactgcat taatcaatgc atttgtaaaa agagggaaaa     240
   gca                                                                   243
<210> 220
   <211> 537
   <212> DNA
   <213> Arabidopsis thaliana
<400> 220
   ctagaagaaa aaaccagatc tataaagttt gttattaaaa gatagagaga gaggagggat      60
   gtagtaggcc aataggcaaa tcagagaatc acaaatggta tctggtcaag aagattcctg     120
   gttaaaactt tcatctctct gtttcctttt tctttctttg ttggattcat taatttgaca     180
   tatctctatc atcacactga ttctctttct cccagtttgt ctgcagatgc atgtgtgtaa     240
   tctagggtat atgtttttgt ccatttggtt tcataaggca ataaagatcc agctatttac     300
   tacttgtggt atagattttg actgttgaat tttcagatct gatgtgtttc gtttgatccg     360
   attcggaaaa tttatgtttc gttgacattt tggagtttag ttggaagaag agtgagagtc     420
   gctggaggca gcggttcatc gatctcttcc tgtgaacaca ttaaaaatgt aaaagcatga     480
   atagatcgat aaacctctgc atccagcgtt tgcctcttgt atctttctta ttgactt        537
<210> 221
   <211> 261
   <212> DNA
   <213> Arabidopsis thaliana
<400> 221
   ctgcatctat ccacctctct ctgtaaattt atctaaatgt ttcttttaat ctttttgaga 60
   ttaataatga tttgtgtttg ttcatcaacc gattttctca gatctgtcaa ttatttttgt 120
   ttatttattt atgatttatg aatgaggaaa gagtgaagtc gctggaggca gcggttcatc 180
   gatcaattcc tgtgaatatt tatttttgtt tacaaaagca agaatcgatc gataaacctc 240
   tgcatccagc gctgcttgct c 261
<210> 222
   <211> 481
   <212> DNA
   <213> Arabidopsis thaliana
<400> 222
   tatcacagtt ctcatcaaat atttgaaagt atcaaacaaa aaaaggagag tgagaaaaat 60
   aaagagagag atagagagag atcatgtcca ctactcaaga gcataggtct tgattggtgg 120
   aagacaagta ccttagataa accgaccaaa acccggtgga taaaatcgag ttccaacctc 180
   ttcaacgaca acgatttcaa cactctcttc caggaacaac ttcctccagg cagatgatac 240
   taaagtgctg gagttcccgg ttcctgagag tgagtccata tcaaaatgcg cattcgttat 300
   cacttggttg aacccatttg gggatttaaa tttggaggtg aaatggaacg cgtaattgat 360
   gactcctacg tggaacctct tcttaggaag agcacggtcg aagaagtaac tgcgcagtgc 420
   ttaaatcgta gatgctaaag tcgttgaaga ggacttggaa cttcgatatt atcccccgtg 480
   t 481
<210> 223
   <211> 471
   <212> DNA
   <213> Arabidopsis lyrata
<400> 223
   agtagggttg gaaaattttt ttacattttt actctaaaat agaatagagt tggagatgcc 60
   cttagcagtt attagacaag ggattgtttg gccctagcga tcctctcttc actctctcac 120
   ttttgtagtt caacccttct tttgcgtgag atgccatcat ggcatgacat ggttcttttg 180
   ccttacgtaa aacacactca cgccaacaca cgccacataa cataaataaa ttatatatac 240
   atatacgtat gtgcgtgtga gtcttccatt aatgcaatct ttgggcctat atatatatac 300
   aaaccttcca taaccaaagt tatcatacta caaaagctct ctcgtacttg gaaatgcggg 360
   tgagaatctc catgttggag aagcagggca cgtgcaaacc aacaaacacg aaatccgtct 420
   catgtgtttt gcacgtactc cccttctcca acatgagctc ctgacccatt g 471
<210> 224
   <211> 466
   <212> DNA
   <213> Arabidopsis thaliana
<400> 224
   agacaagccc ccacactaaa aaaacagtaa tatggaataa aaaaaagctt tcaaaactta      60
   gcagttatta gacaaggtat tgtttggccc tagctagcga tcgtttagct ctcttcactc     120
   tctcactttt ttagttcaac ccttcttttg cgtgagatgc catcatggca tggtatggtt     180
   cttttgcctt acgtaaaaca cactcacgcc agcacacaca cacacacaca taacatatac     240
   ggatgtgcgt gtgagctagt cttccattaa tgcaatcttt gggcctatat atacaaacct     300
   ttccataacc aaagttctca tactacaaac gcccctcatg tgcttggaaa tgcgggtgag     360
   aatctccatg ttggagaagc agggcacgtg caaaccaaca aacacgaaat ccgtctcatt     420
   tgcttatttg cacgtactta acttctccaa catgagctct tcaccc                    466
<210> 225
   <211> 450
   <212> DNA
   <213> Brassica rapa
<400> 225
   agacaacccc acgttttaaa ataagaaatg atgataattt tgtggaaata aaagctagta      60
   tacttttgca ataattagac aaggtattga tgctttgggc caagctagtt tcttttagca     120
   ctcttcactc actagttttt cttctcagcc cttcttttgc gtgaaatgcc atcatggcat     180
   ggcattgtca ttttgccttt cgtaaaacac actcacgcca acatacatta ttcatattca     240
   tgtgtatgta tatgaatgtt ccattaatgc aatctttggg gcctatatat acgaagctta     300
   catcaccaaa gctctcatat tacaaaagct cacatatata cttggaaatg taggtgagaa     360
   cctccatgtt ggagaagcag ggcacgtgca aaccaaaaaa catgaaatct gtttcatatg     420
   ctttgcacgt gctcccctcc tccaacatga                                      450
<210> 226
   <211> 513
   <212> DNA
   <213> Carica papaya
<400> 226
   agacaacact cctctttgtt cccttcctca cgtatccact tttgaaattt gtaatttgtg 60
   tgcaccacca tgattgcatg ccatccctac ttgccttttc cccttttcct ttctctaaca 120
   ttttactcaa tcttcttctc cccctccccc ccttccccct ctctgccatt ataaccataa 180
   ttaaacctct ctccctctct ctccctctct ctctctctct ctctgggttc tcagtataaa 240
   tgcagctctg cttatacttc cacacctata tatatatacc tgacccttct tcacctcctt 300
   catccacctc ctccttcttc cccaaaactt tcttaactgt tctctgcata catatatatc 360
   cacatacata tatatatata tagagagaga gtgagacaga gaggttaccg aggcaattgg 420
   gtgagtagct ccctgttgga gaagcagggc acgtgcaaat tctccatggc tttcccctct 480
   ttgcacgtgc tccccttctc caacatgggt tcc 513
<210> 227
   <211> 464
   <212> DNA
   <213> Capsella rubella
<400> 227
   cggccacccc cacatttaac aagaaaaaaa ctgatggaat taaaaggttt gagaacttgg 60
   cagttattag acaaggtata gtttggccct agcttctttt aatttagctc tctccactct 120
   cacacttttc aactttcacc cttctcttgc gtgagtcgcg agatgccatc atggcatggc 180
   atggcatgtt tctattgcct tacgtaaaac acactcacgc caacacatac tcactataca 240
   tgtaaataag tatgtgcgcg tgtgagtctt ccatccatca atgcaatctt tggggctata 300
   tatatacaaa ccttttccat aaccaaagct ctcatataaa ctacaaaagg ctcacttggg 360
   aaatgcgggt gagaatctcc acgttggaga agcagggcac gtgcaaacca acaaacacga 420
   aaccctcctc atgtgctttg cacgtactcc ccttctccaa catg 464
<210> 228
   <211> 571
   <212> DNA
   <213> Gossypium raimondii
<400> 228
   gaaaacccaa gttcaggcta acaagttatc tgatgatgag atcaagaatt ttaaagtttc      60
   aatatagatt tggcatgggt attggcggca gaaagcaatt aaaaaaccag ttatgtcaaa     120
   ttcaaggtcg tatcagttaa aatgaatgaa gatttagaaa tttcaacaag gaagaggacc     180
   ccacagcttt gttaaattaa gtgtgtggtt tttataatta tcatctcgaa agtttcataa     240
   tatcaattag attaaaacat ctctgaattt cataattaca aaccagatag atagatacat     300
   gaaaacttag accccagaga tctgtcttta aagaatgccc acttctagac tcaatctcta     360
   ttactctctt tttttctctc tctctctctt cggaaaaact tgtatataaa taaatgacac     420
   tttctttgct ttctgcactc aactcatgaa cttgaaaagc tttacttgga tgggttggtt     480
   gggggtgagt atctcttgtt ggagaagcag ggcacgtgca agttcctatg tttaagtgaa     540
   ctttgcacgt gctccccttc tccaccgtga g                                    571
<210> 229
   <211> 592
   <212> DNA
   <213> Medicago truncatula
<400> 229
   gaagagaaaa aacctagtgt aaaatttgat atactcttta tgtatagtac gaatgttttt      60
   ttaaaaatta tgtaaaaaat gataaaataa taactaacta aattaacagt aaaattagaa     120
   aagtaaaata ctatgcccaa atttgatatt tttttttata tatttgtata gattattatt     180
   atttgatatg taaagtccaa ttaaaaattt gttttaacta agatttgaac taggttttct     240
   taaaagactc atcttttact tcaaatttat ttatcatttg aattcaatca ctttctaata     300
   ttattattat tatttccacc atactcattg cttctgccac gttactttag ttagatctct     360
   tatgtcatat atctctctct ctcctaagtt gctacctata aatactaagc ctttcccttg     420
   gttggttcaa ttcaacttct acttctcatc aaacacaaag tgcaataagc ttcatttcct     480
   gggtgagaag ctccttgttg gagaagcagg gcacgtgcaa atcctctttc tgattcattc     540
   tctcataatg catatcaata tcttttgcac gtgctcccct tctccaacta gg             592
<210> 230
   <211> 520
   <212> DNA
   <213> Oryza sativa
<400> 230
   atgcaaaccc actccaacac tccacaatcc acatactctc tctctctctc tctctctgag 60
   taggagtaca tgtgtgtgtg tgatatcaat atgcattcga tgttgatgct actgtagcca 120
   tcttgtggct atataaaccc agcaggcagc agcacagctt agctagagag ccatattgca 180
   tgcacactcg ctaatctctt ttctctactc tacttgcatt acaccacctc tgcattgcac 240
   ttcagttcat tcattccact gatgcatgga tcgatgttgc taccttcttc tcttctcctc 300
   atgcatccat gcatcgatct cacctagctt cttcctcatc ctctctcgat cgattacaag 360
   agaaaagtgt ttgctgttct tgctatcgat ctacaggtga gtaggttctt gttggagaag 420
   cagggtacgt gcaaaatgca caccggttgg tcgagctaat taacaagctc tgacgaccat 480
   ggtgatcgaa tgcacgtgct ccccttctcc accatggcct 520
<210> 231
   <211> 501
   <212> DNA
   <213> Arabidopsis lyrata
<400> 231
   ctagggttta ggaatgacga cttgtttctg ttgtgtctta ttaaaagccc atcttcgtct 60
   ccgccactca tcattccctc atcataacac catcatcacc attcaccaac ctctctctct 120
   ttctctctct cctctcgatc tacaacaaaa tgtgaatctg ctaagatcga ttatcatgag 180
   aattaagcta tttcagttga ggggaatgtt gtctggatcg aggatattat acatatatac 240
   atgtgtatgt tgatacatgt gatcatagag agtatcctcg gaccaggctt catccccccc 300
   aacatgttat tgcctctgat caccatatat atgtcgttac atttcatggt taattacttg 360
   cacaaatcac aaaagcttgg tttgtaactt tctatgacct tttttaatga ctttgaatct 420
   ttcatgcatg acttcttaag agtagattta cacatttgcg gatccgttta tgctttttgc 480
   ttttgtttcg tttatatata t 501
<210> 232
   <211> 484
   <212> DNA
   <213> Arabidopsis thaliana
<400> 232
   ctagggttta ggaatgacga cctgtttctg ttgtgtctta ttaaaagccc atcttcgtct      60
   ccgccactca tcattccctc atcataacac catcatcacc attcaccaac ctctctctct     120
   ctctcctcta tcactctcta caacaaaaat ttgtgaatct gctaagatcg attatcatga     180
   gggttaagct atttcagttg aggggaatgt tgtctggatc gaggatatta tagatatata     240
   catgtgtatg ttaatgattc aagtgatcat agagagtatc ctcggaccag gcttcatccc     300
   ccccaacatg ttattgcctc tgatcaccat ttattgttac attttttttt gttaattact     360
   tgcgcaaatt acaaaagctt ggtttttgtg atgactttga atctttcttg catggcttct     420
   taagagtaga tttacggatc cgtctatgct ttttgctttt tgtttcgttt atttgtattt     480
   aaac                                                                  484
<210> 233
   <211> 552
   <212> DNA
   <213> Brassica carinata
<400> 233
   gagatcaatg aaattatcct gccaaataaa acgtgtgacg tttattcaaa aatatatgca      60
   ttagatgctt tgatattaaa atatttcctt ttaaaagcta gctagggttt aggaatgacg     120
   agttgtgtct tattaaaagc ccttcttctc ctccgccact catcattccc tcatcataac     180
   accatcatca ccattcaccc acctctcctc tttctctctc tctctctctc tctctctctc     240
   tagaacaaca agtgagaatc tgctaaaata ttgtgactat tatcatgaga atgaagctat     300
   ttcagttgag gggaatgttg tctggatcga ggatattata tatacacaaa tacgtatata     360
   tgttaataca agtgtttgat catatatgta tatagattat tctcggacca ggcttcatcc     420
   cccctaacat gttattgcct ctgatcacca gattctatca actcttcgct tattattttg     480
   tcacaaacaa gtaataagct cataattttc tttgagtctt tcagcatcgt ttcattatgt     540
   ttttcgaatc cg                                                         552
<210> 234
   <211> 566
   <212> DNA
   <213> Brassica juncea
<400> 234
   gagatcaatg aaattatcct gccaaataaa acgtgtgacg tttattcaaa aatatatgca 60
   ttaaatgctt tgatattaaa atatttcctt ttaaaagcta gctagggttt aggaatgacg 120
   agttgtgtct tattaaaagc ccttcttctc ctccgccact catcattccc tcatcataac 180
   accatcatca ccattcatcc acctcttctc tcctctctct ctctctctct ctctctctct 240
   ctctctctct ctctagaaca acaagtgaga atctgctaaa atattgtgat tattatcatg 300
   agaatgaagc tatttcagtt gaggggaatg ttgtctggat cgaggatatt atatatacac 360
   aaatatgtat atatatgtta atatcagtgt ttgatcatat atatgtatat agattattct 420
   cggaccaggc ttcatccccc ctaacatgtt attgcctctg atcaccagat tctatcaact 480
   cttagcttat tatttgtcac aaacaagtaa taagctcaat aatgtctttg agtctttcag 540
   catcgtttca tatgttttcg aatccg 566
<210> 235
   <211> 549
   <212> DNA
   <213> Brassica napus
<400> 235
   gatatcaatg aaattatcct gccaaataaa acgtgtgacg tttattcaaa aatatatgct 60
   ttaaatgctt tcatattaaa atatttcctt ttaaaagcta gctagggttt aggaatgacg 120
   agttgtgtct tattaaaagc ccttcttctc ctccgccact catcattccc tcatcataac 180
   accatcatca ccattcaccc acctctcctc tttctctctc tctctctctc tctctctctc 240
   tagaacaaca agtgagaatc tgctaaaata ttgtgactat tatcatgaga atgaagctat 300
   ttcagttgag gggaatgttg tctggatcga ggatattata tatacacaaa tacgtatata 360
   tgataataca agtgtttgat catatatgta tatagattat tctcggacca ggcttcatcc 420
   cccctaacat gttattgcct ctgatcacca gattctatca actcttcgct tattatttgt 480
   cacaaacaag taataagctc aataatgtct ttgagtcttt cagcatcgtt tcatatgttt 540
   tcgaatccg 549
<210> 236
   <211> 543
   <212> DNA
   <213> Brassica oleracea
<400> 236
   gggatcaatg aaaattatcc tgccaaataa aaacgtgtga cgtttatcca aaaatatatg 60
   cattaaatgc tgtgatatga agtatttcct ttaaaagcta gctagggttt aggaattacg 120
   agttgtgttt tattaaaagc ccttcttctc ctccgccact catcattccc tcatcataac 180
   accatcatca ccattcaccc acctctcctc tttctctctc tctctctctc tctctagaac 240
   aacaagtgag aatctgctaa aatattgtga ctattatcat gagaatgaag ctatttcagt 300
   tgaggggaat gttgtctgga tcgaggatat tatatataca caagtacgta tatatgttaa 360
   tacaagtgtt tgatcatata tgtatataga ttattctcgg accaggcttc atccccccta 420
   acatgttatt gcctctgatc accagattct atcaactctt cgcttattat ttgtcacaaa 480
   caagtagtaa gctcaataat gtctttgagc ctttcagcat cgtttcatat gttttcgaat 540
   ccg 543
<210> 237
   <211> 566
   <212> DNA
   <213> Brassica rapa
<400> 237
   gagatcaatg aaattatcct gccaaataaa acgtgtgacg tttattcaaa aatatatgca 60
   ttaaatgctt tgatattaaa atatttcctt ttaaaagcta gctagggttt aggaatgacg 120
   agttgtgtct tattaaaagc ccttcttctc ctccgccact catcattccc tcatcataac 180
   accatcatca ccattcatcc acctcttctc tcctctctct ctctctctct ctctctctct 240
   ctctctctct ctctagaaca acaagtgaga atctgctaaa atattgtgat tattatcatg 300
   agaatgaagc tatttcagtt gaggggaatg ttgtctggat cgaggatatt atatatacac 360
   aaatatgtat atatatgtta atatcagtgt ttgatcatat atatgtatat agattattct 420
   cggaccaggc ttcatccccc ctaacatgtt attgcctctg atcaccagat tctatcaact 480
   cttagcttat tatttgtcac aaacaagtaa taagctcaat aatgtctttg agtctttcag 540
   catcgtttca tatgttttcg aatccg 566
<210> 238
   <211> 414
   <212> DNA
   <213> Arabidopsis thaliana
<400> 238
   catcatcacc actcacttat cttcttctcc atctctctct ctgcttctcc cttaatctta 60
   gccgggtctc gtgggggacg aacatagaaa gagagagata taaagatata tattcagaaa 120
   ccctagattc tataatttcg actgaaaaga aaaaggggct ttctcttttg aggggactgt 180
   tgtctggctc gaggactctg gctcgctcta ttcatgttgg atctctttcg atctaacaat 240
   cgaattgaac cttcagattt cagatttgat tagggtttta gcgtcttcgg accaggcttc 300
   attcccccca attgttgctc cctgtttact ccatatttct tccttctttt caaattaggg 360
   tttcagatcc agtgaatgaa cccttgttaa aggtttgatc tcttacctta cttt 414
<210> 239
   <211> 328
   <212> DNA
   <213> Arabidopsis thaliana
<400> 239
   tctcatcatt ctcttcatca tcaccacatt catctctctc tctctctctc tctctctctc 60
   tttctcttcc ttgatcttag ccggatctgt tgggggacga acacatgaga gatagataaa 120
   atataagaaa tttctcgaaa aaacctaata gaaaaaggtc tgtttcttaa agaagaagaa 180
   gaagaggatt taaagaggga tttttctttt gaggggactg ttgtctggct cgaggactct 240
   tattctaata caatctcatt tgaatacatt cagatctgat gattgattag ggttttagtg 300
   tcgtcggacc aggcttcatt ccccccaa 328
<210> 240
   <211> 192
   <212> DNA
   <213> Arabidopsis thaliana
<400> 240
   tgatgaacag aaaaatctct ctttctcttt cttgatctgc tacggtgaag tctatggtgc 60
   accggcatct gatgaagctg ccagcatgat ctaattagct ttctttatcc tttgttgtgt 120
   ttcatgacga tggttaagag atcagtctcg attagatcat gttcgcagtt tcacccgttg 180
   actgtcgcac cc 192
<210> 241
   <211> 846
   <212> DNA
   <213> Arabidopsis thaliana
<400> 241
   aaccacaaag taccgctgct attttctttt tacgtctttg tatttgcatc gtctaagaga        60
   atgatgggtt gttttgtggg attttaatgc aggaggaaac atatgagggg tgattaaggc       120
   aaaaacctta agatgtggtc atttagatac atggagtcaa actaagaatg gaccttggcg       180
   aaagcttctt cacggtcaag atttaaaatc aggtacgaca ctgtgtacgt gagagagaga       240
   gagagagaga gaaagagatt atagaaagaa agagatgtat cacaataaag gagtatattt       300
   agggtcacag gtggtggaga tataggtatg cagggccaag gctctaatct cttcatagcc       360
   ctattgattt tgtccctctc tctctctctt tcttcctctc ttagctgtat gcattatgat       420
   gcgtctttta attcactgtt tcaggcttct ttaattcgtg gtgtctctct cctttttacc      480
   caaccatctc ttaaaatttt taacatctgt tcctcaaatc ctctctcatc tctttctata       540
   agtatctata gcgcctctta aaccacaaag catcacctct gtcttctctc atctcctttc       600
   tgtattctct ttcattgcct tcacgtctgt tgcaatttct ccacttcttg agcttccgtt       660
   ttttacaatt attgatccgt caaatatgtg agatttgcac aacttgttgc tcaggtattt       720
   tgaagacaag tccacaaggg aacaagtgaa gctgccagca tgatctatct ttggttaaga       780
   gatgaatgtg gaaacatatt gcttaaaccc aagctaggtc atgctctgac agcctcactc       840
   cttcct                                                                  846
<210> 242
   <211> 571
   <212> DNA
   <213> Arabidopsis thaliana
<400> 242
   gagcaagaca atgccacata caaacttgaa agatctcttc atcttttctc caaatgtttt        60
   tttttcgttt gctatttatc tccacaattc ttggaacaaa aactacattc acaaacgaga       120
   gaattttcac aacacctctt ttgctctcat tttttttttt tcgtccatta tgagtattaa       180
   ttatggttag ggaatcttac agaatgaaaa tgaaggtgtg aatggattgt ctcatctaaa       240
   gccttgaatg tgggaaaaag gccattgttg ttcagccaag gatgacttgc cggtagcttg       300
   tattatgatt actctatatt cgatttatat tatggagatg atggtttata tatatttact       360
   tatctacata gttttagttg attttttttc gtacgtaata taatacgaaa aagtatttac       420
   ttatttatat atgtgtgttg gggcaagaag tgtaaccaag ctagcccggc aagtcatcta       480
   tggctatgca actgtctctt cctctcattc taggcttacg atgacacgta aaaaatccca       540
   aatatcacta atatgatatg aatatggatg a                                      571
<210> 243
   <211> 241
   <212> DNA
   <213> Arabidopsis thaliana
<400> 243
   aggcatgaga cataaagaga gttaaatata atgaagaaga gaggtctaat atggcgaaaa      60
   gagtcatgtt taatagccaa ggatgacttg cctgatcttt ttcacctcca tgattcaatt     120
   ttaagttcgt ggattttgga ttattatgcg tttaaaaggt ataataattt gagatcatgt     180
   tgaatcttgc gggttaggtt tcaggcagtc tctttggcta tcttgacatg ctttcttcat     240
   c                                                                     241
<210> 244
   <211> 689
   <212> DNA
   <213> Arabidopsis thaliana
<400> 244
   gaattttgat ttatgaacct cctcaagaag gaaagacaga ggaggagaca aagaagtata      60
   ggttcacatt gcatagccag tttagttttg aaggatggat atatgaaaaa aatatgaaga     120
   gagagaagag agaagaagag gaggattaaa gagggtgagg ccagcttttg tgctttggta     180
   gtagatgagg tttaaatgct ccataccttc catttccttc tctcttaccc taatttaatt     240
   cttcctctcc tttataactc cccacagaca ttctcacttc tcctcctcac acttcacatc     300
   aacacttctt tcttgttttt tcattttaca atgtttcctt tgatatccgc actttaagca     360
   tgagagagtc cctttgatat tggcctggtt cactcagatc ttacctgacc acacacgtag     420
   atatacatta ttctctctag attatctgat tgagccgcgc caatatctca gtactctctc     480
   gtctctattt tggactttgt ggtcttgtag atcgatttgt atgtgtgtgt tgaaatggag     540
   acaagtactt gtaacttctt tgttgttata ttgtttacct ataggctgat gtcataaact     600
   cttttgatct tgtttctaac ttccagattc ttgaaaaatc aagtcgtgtg tgtgtctcca     660
   tggaagcctt ttccatttct tcctttcca                                       689
<210> 245
   <211> 452
   <212> DNA
   <213> Arabidopsis thaliana
<400> 245
   actcataaac tttgctactg gccgcatttc tattttctcc ttcgattctt ctaattcgta        60
   ctttggtttc tgacgtccct aaaattttta gacagtaaga gtttctccag gatccgatgg       120
   ttctctccgg taaattaaca ttttagtgtc aatagtcatt tatacatatt tttatttcac       180
   tttttgtttt gtttattggt tttctggagc taagtggaga ttatagtcga acaagagtgg       240
   ttttatgcaa ggtaacgcga gatattagtg cggttcaatc aaatagtcgt cctcttaact       300
   catggagaac ggtgttgttc gattgagccg tgccaatatc acgcggtaaa ccaaaaatgg       360
   caaagatagt tattataacc ttaaaggtat gtatcattat cgttttattg tttcaatttt       420
   gattaatggc tttgatattt catttttttt tt                                     452
<210> 246
   <211> 767
   <212> DNA
   <213> Medicago truncatula
<400> 246
   attggtcaaa catacataca gtagcactag ctggtttcat tattccacta tgcttcttca        60
   taggctctcc aaattttgca aaattgaaag agacatagta tatatatctt agcaaggaga       120
   aattcaggat attgaggatg aagattgaag agtaatcagt gatgaagaaa gcaagcaagg       180
   tattggcgcg cctcaatttg aatacatggc tataaaaatg catcatatca gccatgtagt       240
   ttgattgagc cgcgtcaata tcttgtttcc atctccaaat ttaccaatct catcaaatca       300
   aattaacacc acaatcaagg ctttcattta atgcagtcaa aataggttga ccttatcatc       360
   gaagaaattg ttttctcatt cctatcgaag ttggacttgc cgaaaatgct cgaaagcatg       420
   tgttttagtt cgacaggcga aaaagttacc gaaggacaat ttggttgtgg ttcggataag       480
   atcaagcaac ggatattttc aagacacgtt cgaaattcaa atcaaatgga taagtatcgt       540
   tagtttactg cagttatagt tttaaattca aatctaggca gttgtttcta tttgtataaa       600
   tagtagtttt tccctaggga aaaggggtcg caattcaatc atacaaaaaa cttacaatca       660
   aattatccgc atggaagaga gaaacgagtc acaagttgca atgtatgaac atgtgtacca       720
   atttacattc aatcagtaca atttaagttc atttccataa aaaaaaa                     767
<210> 247
   <211> 854
   <212> DNA
   <213> Zea mays
<400> 247
   accagggtta ggtatccatc cacacagcaa tgcatctgcc ttcaactccc tctcgccccc      60
   caccccaaca cacatctctc tcttttctag ggaaggaaat gacgaagggg acgacgacgg     120
   catgcttcgg ctagctcgtt ggtgctagga caagggcgga ggtattggcg cgcctcaatc     180
   cgaaggcgtg gctgatagat tggcgcggca gccatgttct tggattgagc cgcgtcaata     240
   tctccccttg cctgtcccgt acctagctag cttgcttgcc tcactgatcg atgtcgtccc     300
   tatttcatgg agaagctgat gattgattat tctcacaagc aagaactgtc tgatctgttg     360
   cctgcatcga tcaggatcta tatgctggag agttcacaag aacatggaca gaactcgctt     420
   caacaaccga tcaatcgatt gattaggtat gtacctacct catatgcctc agctcttcgt     480
   tatggatttc ttcaaccgaa gggtcagtaa gctcttggtt ccatgccact gcgtgaacta     540
   agcgttcaca aaatccgttc cmcggcatga accaagcact caaaatcgca tgcagcatct     600
   ttcgtttcaa aaaaaaattg acttytgaaa acaatagatg aatcagtttc aaacatatat     660
   gattatccat tttctcaacc gggaatttat atctcgttgg gatgcaaaac cgttcagtag     720
   taaaactact ccacgagtat aaactgtttc agttatttta ctattaatta gttacccgta     780
   tgctgttatg gtttctatat atctataagt aaaccttact taaataagat agttatacaa     840
   aaaaaaaaaa aaaa                                                       854
<210> 248
   <211> 660
   <212> DNA
   <213> Arabidopsis thaliana
<400> 248
   caagaaaaaa cattgaaata gctcatgttg tctctttctc attttcatat ctgctaaaaa      60
   aagaaccgtg ttttctaaac tggtttaacg gtaagtacct gtctctagta acttacctat     120
   caatttgttc caatcattta cttgctttga cttatttggt ttccttttgt tttgtttttc     180
   tttaatatgt ggatggagtt tggtgtaata agcaactgaa gagtcgatga gcgcactatc     240
   ggacatcaaa tacgagatat tggtgcggtt caatcagaaa accgtactct tttgttttaa     300
   agatcggttt atttgattga gccgtgccaa tatcacgcgt ttaaatagtt taaagattct     360
   atgttagttg atgtgatcaa tcaaggtatg aatctatatc aattctctta tgcatagttt     420
   tatatttaca gagatgaggt attatcaatg tctatcgtcg aggatcacgc tcttacttat     480
   gttatatttc tatataattt tattaattag ttttctaaaa gagaaggaca atttaaaatt     540
   attttaaaga gtttttttta agtagttttg ttttcatgtt tatcttctgc aggctctgaa     600
   gttaggatag taacaagaaa aaagacagaa aaaaagaaga aaattcatat acattcgtga     660
<210> 249
   <211> 225
   <212> DNA
   <213> Medicago truncatula
<400> 249
   gaataagtga atattatcga tatttatatc atatatcaac ttttcttctg tgcttgcttg 60
   caaatttgca attaagcttt tttgatctta tgtaagagaa tattattgat gatggtgttt 120
   gggaagccga aaaaagcgat gttggtgagg ttcaatccga agacggattt acatgtatag 180
   agttgtaaaa tacgatctca gattgagccg cgccaatatc acttt 225
<210> 250
   <211> 367
   <212> DNA
   <213> Medicago truncatula
<400> 250
   ccacaaaact ataactagct agaagcttta atcgccttat ttattataat aataataata 60
   aatatggctt cagctgcaaa agtatacatg gcgtgatatt gatccggctc atctatatct 120
   tcaagttcaa tcatccatat tcatatcaat ttcagacgag ccgaatcaat atcactcttg 180
   tttgcttcat tgcatattaa ttatatactt catttataag ttatagtttg ccatatatat 240
   attagattga ttctgcagaa gtagacagga gtggtgttgt ttctgctcat cttattaaat 300
   aatgaatgaa tgaatgacat ttgcttactt ataagacgag ccgaatcaat atcactccag 360
   tacacct 367
<210> 251
   <211> 712
   <212> DNA
   <213> Arabidopsis thaliana
<400> 251
   ctctctctct ctctctctct catctgtgtt ctagatctca ccaggtcttt ctctggttaa 60
   tatatggctt ccaagatctg gtaatatgtt ataaatacgt catacttaag cttttttcaa 120
   atcaaaaata gaaatttgtg ggtttgtctc gttttactat tttagcagta tatattaaga 180
   agttcagatg ttattcgatc atctgttttt gcttcccctc tgccatcttt atcttttagg 240
   gtttcaattc tttttcactt ttttcttctg gtttggagat ggttaggttc caactaagta 300
   tacgagatta aatttgacat cttagttact tcaaaattcc ttcaatcaaa acaagtcatc 360
   tcgactattc cgccatgttt gtatatacat atttatatat tatatatatg aaggtacgag 420
   tttctagtgt ctataaatta agaaggttaa gtaccatata gatgatattt gttaagtagt 480
   aagtcactca aagtttgagt ttgggtttga gtttgagttt gagtttgagt ttgagagaca 540
   aaagattact acaagaagat tgttaaacaa aaatggaaga ctaatttccg gagccacggt 600
   cgttgttggc tgctgtggca tcatcaagat tcacatctgt tgatggacgg tggtgattca 660
   ctctccacaa agttctctat gaaaatgaga atcttgatga tgctgcatcg gc 712
<210> 252
   <211> 714
   <212> DNA
   <213> Arabidopsis thaliana
<400> 252
   acttgcacct ctcactccct ttctctaact agtcttgtgt gcacccattt atgtgtacgt 60
   actattatct cataaataaa tatttttaaa attagatgca tttattgata tgaaaaagtt 120
   acaagattag tttgttgtgt gtgagacttt ggatcgacag atcgaaaaat taactaaccg 180
   gtcagtattg aatatcaact attatatgct ccatgcattc gcttatagtt tcacacaatt 240
   tgttttcttc acggtctaaa atcagaagat tccatatatt ttcttatgac gtaaaaggac 300
   cacttataag ttgacacgtc agcccttgga ttcgtgaggt ttttctctct acttcaccta 360
   tctacttttc ctcatatccc actgcttttc tccttcttgt tcttgttttt ctcgtttttt 420
   tcttcttctt ctccaagaaa atagagatcg aaaagattag atctattttg tgtagcaaga 480
   aattatcatt ttcgtttctt cattcatata ttgttctatt atgttgtaca ataatagata 540
   ctcgatctct tgtgcgtgcg taaattttat acaagttgtc ggcggatcca tggaagaaag 600
   ctcatctgtc gttgtttgta ggcgcagcac cattaagatt cacatggaaa ttgataaata 660
   ccctaaatta gggttttgat atgtatatga gaatcttgat gatgctgcat caac 714
<210> 253
   <211> 324
   <212> DNA
   <213> Arabidopsis thaliana
<400> 253
   tcaccaaata ggctcttctt tatcgcttca tatatataaa agtctacatc tatctctttc 60
   taggtcacta gctagactct agattaagga ttgaaattag ggtttcatgt ttccagcaaa 120
   atggtgccgt cttgagtctt gaaaagatcc aagacaaaac caaatcacta catacatccc 180
   tatcatcaac cagctactgt tcgctgttgg agcatcatca agattcacaa atcatcaagt 240
   attcgtgtaa ataaacccat ttatgattag atttttgatg tatgtatgag aatcttgatg 300
   atgctgcagc tgcaatcagt ggct 324
<210> 254
   <211> 531
   <212> DNA
   <213> Arabidopsis thaliana
<400> 254
   tgtcatattg agaactcttt agcctttggc ttctgttcct gacacttgta tagtgaagtg 60
   ggcttgtgtt atatagatgg gatctctctc tttatttaaa agtcaattag agatcttgat 120
   gctacttctg tccctttcca agtgatttta cgtcgaccaa ctagcttttt tcatatgagt 180
   gtatatattc atgtacctat ctctctcaat tgcttctcac caaaatcatc ttgctgattc 240
   atttgctgtc tgaatcctct tgctttcctc tttgcttttt catttgttga tttaaaccat 300
   gggagttccc aactttagac ctcgaaaccg ataaggatct ttctctgcgg ttgaaatagc 360
   taggttctcg atgaataggc tagcctttgg tggatgttat cagccagtag tcgcagatgc 420
   agcaccatta agattcacaa gagatgtggt tccctttgct ttcgcctctc gatccgcaga 480
   aaagggttcc ttatcgagtg ggaatcttga tgatgctgca tcagcaaata c 531
<210> 255
   <211> 618
   <212> DNA
   <213> Arabidopsis cebennensis
<400> 255
   ttgtatccat agtgtatttc ctcgcatcta ccatccattt tctacgcctc tctctctctc 60
   tttctccatc aaatcttgtt ttgttcaaac tctctctctc atcaattctc tccatacaat 120
   acatgcatac atacatacat accatctcta atatttcatc aatcttcttt tgttccaaac 180
   gctctttctc tccatttaca tacatacgaa tcattgttgt catagatccg tttagaattg 240
   ctttaacttt tagatgagat ctagggtttc tttctttttc ttcaaaatca tgctttttcg 300
   cttgctaggt tatagatcca tgtaagttta gagtagatgt acacacacac gctcggacac 360
   ttattaaata catgttgata cacttaatac tcgctgtttt gaattgatgt tgtaggaata 420
   tataaatgta gagagagctt ccttgagtcc attcacaggt cggatatgat ccaattagct 480
   tccgactcat tcatccaaat accgagtcgc caaaattcaa attagactcg ttaaatgaat 540
   gaatgatgcg gtagacaaat tggatcattg attctctttg attggactga agggagctcc 600
   ctctctcttc tgtattcc 618
<210> 256
   <211> 660
   <212> DNA
   <213> Arabidopsis halleri
<400> 256
   ttgtatccat agtgtatttc ctcgcatcta ccatccattt tctacgcctc actctctctt 60
   tctccatcaa atcttgtttt gatcaaactc tctctctctc tctctcatca attgtctcca 120
   tacaatacat acataccatc atctttccca tctctaatat ttcatcaatc ttcttttgtt 180
   caaacgctct tcctctccat atacatatac atacatacga atcacattgg tgtcatagat 240
   ccgtttagaa ttgctttaac ttttagatga gatctagggt ttctttgttt ctttcgtttt 300
   cttcaaattt tgctgcatat tctccaagat catgattttt cgcttgctag gttatagatc 360
   catgcaaata tagagtagat ttacacacac acacgctcgg acacttatta catacatgtt 420
   gatacactta atactcgctg tttttaattg atgttgtagg aatatatata tgtagagaga 480
   gcttccttga gtccattcac aggtcgtgat atgatccaat tagcttccga ctcattcatc 540
   caaataccga gtcgccaaaa ttcgaactag actcgttaaa tgaatgaatg atgcggtaga 600
   caaattggat cattgattct ctttgattgg actgaaggga gctccctctc tcttctgtat 660
<210> 257
   <211> 632
   <212> DNA
   <213> Arabidospis lyrata
<400> 257
   ttgtatccat agtgtatttc ctcgcatcta ccatccattt tctacgcctc tctctttctc 60
   catcaaatct tgttttgttc caactctctc tctcatcaat tcattccata caatacatgc 120
   atacatacat accatcatca tcttttccca tctctaatat ttcatcagtc ttcttttgtt 180
   acaaacgctc tttctcgcca tatacataca taagaatcat tgttgtcata gatccgttta 240
   gaattgcttt aacttttaga tgagatctag ggtttctttc tttttcttca atttttgctg 300
   catattcttc aaaatcatga tttttcgctt gctaggttat agatccatgc aaatatagag 360
   tagatgtaca cacattcacg ctcggacact tattacatac atgttgatac acttaatact 420
   cgctgttttg aatggatgtt gtaggaatat atatgtagag agagcttcct tgagtccatt 480
   cacaggtcgt gatatgatcc aattagcttc cgactcattc atccaaatac cgagtcgcca 540
   aaattcgaac tagactcgtt aaatgaatga atgatgcggt agacaaattg gatcattgat 600
   tctctttgat tggactgaag ggagctccct ct 632
<210> 258
   <211> 646
   <212> DNA
   <213> Arabidopsis thaliana
<400> 258
   ttgtatccgc agtgtatttc ctcgcatcta ccatcccttt tctacgcctc tctccctctc 60
   tctctttctc catcaaatct tgttttgttc aaactctctc tctctcatct attctctcca 120
   tacaatacat gaatatacat acataccatc atcttctttt cccatctcta gtttttcatc 180
   aatcttctga tgttccaaac gctctatctc ttcatataca tacatacgaa tatattattg 240
   ttgtcataga tccatttaga atcactttag cttttagatg agatctaggg tttctttgtt 300
   ttctttcaaa ttttgttgca tattcttcta aatcatggtt tttcgcttgc taggttatag 360
   atccatgcaa atatggagta gatgtacaaa cacacgctcg gacgcatatt acacatgttc 420
   atacacttaa tactcgctgt tttgaattga tgttttagga atatatatgt agagagagct 480
   tccttgagtc cattcacagg tcgtgatatg attcaattag cttccgactc attcatccaa 540
   ataccgagtc gccaaaattc aaactagact cgttaaatga atgaatgatg cggtagacaa 600
   attggatcat tgattctctt tgattggact gaagggagct ccctct 646
<210> 259
   <211> 660
   <212> DNA
   <213> Brassica rapa
<400> 259
   ttgtatccat tgtgtatttc cttgcatcca tcaataaatt ttatgttacg cctctctatt 60
   atttctctct acatcacact gtcttatgtt taagctctac ttctcagcaa ttctctccac 120
   ccaatacatg catacatacc atcatcgtat cgctctaatt tttctatcaa tcttgtatcc 180
   ttccacaaat tatcttatgt ctcccatttt aaatcctaca tagatccaca catacgaatt 240
   attcttgtct gaagatccat ccatttacga ttgctttaac ttttacatga gatctagggc 300
   ttctttattt ttcttcaaat cttgctgcat atatttcaag atcatgcttt tcggcttgct 360
   agggttctag atccatggat gtatagcgta catacataca cgcactaatt catacctgta 420
   gtttgtacgg agaacatcat aaaatatcac tgtttggaat taatcgtgta ggaaatatag 480
   ataggtaggg agcttccttt agtccattca cagatcatga tatgatccaa ttagcttccg 540
   actcattcat ccaaataccg agtcacgaaa atttcaactt agactcgtta aatgaatgaa 600
   tgatgcggta aacaaattgg atcattgatt ctctctgatt ggactgaagg gagctccctc 660
<210> 260
   <211> 932
   <212> DNA
   <213> Carica papaya
<400> 260
   ctatatccct gtcaaatagt acttggtttt gttttagcca caaatcttct ggtcttgcaa 60
   agttccttct gatctctccc cctttctcat tttttcctcc tcttatatat ggatcatcaa 120
   tttgctgtac acacacacac atttactgta gtgataatta gctagctaat ttgttagtat 180
   gtaaattaga tcccaagtac cctgttatat ttttttaggc ttatcctatg catacctgat 240
   agtacaagaa cttagtttgt aattaggtac ttggtagtag ggttagatta attactgtct 300
   tgaaagagaa cttatccaac aaatagagct atgaagatta aattaggttt tagtcttatt 360
   aagattatta tattactaga caaaaacagt taaatttttt taattgggta attaggtact 420
   tagcaatagg gttagattaa ttactgtttt gaaagagaac ctacctacaa atagagttga 480
   aatgattatg ttttagtctt actaagattg tcatatttct ggaaaaaaac aaatcttgaa 540
   acagataatt cagatagtca tgatcaatgg aaaaaacatc atgggtgtgt gcttaattaa 600
   gctaatatat atatatatga agatataatg ttatgcacac tagctatgaa tttgtaagaa 660
   taatgaagga taaagatgat atatttagat gttataagtg taagtaaggt ggaatgggtt 720
   gatgggtagt agtagtagta gtagagatga ttggtggaga gagcttcctt cagcccactc 780
   atggatgggt atgaaggggt agaagtagct gccgactcat tcattcagcc actcagtatg 840
   taaactcgtc ccactgttga ctgtatgaat gatgcgggag atatttttac atccatcttt 900
   ctctgtgctt ggactgaagg gagctccttc tt 932
<210> 261
   <211> 702
   <212> DNA
   <213> Capsella rubella
<400> 261
   ttgtatccat agtgtatttc ctcgcatcta ccatctacta ttttctacgc ctctctctct      60
   ttatccctct atctctttct tcatcaaatc ttcttttgtt caaagtctct ctcatcattt     120
   ttctctatac acatacatgc atccacatac atacatacat ataccatcat cttcttttct     180
   catctctagt ttttgtttat aaattttgtt ccaaggatct gtatctctcc aataaagata     240
   catacaaatt attgttgtca tagatctatt agaattgctt taacttttat atgagatcta     300
   ggatttcttc cttttctttc aaaatttgct gcatattttt caaaatcatg atttatcgct     360
   tgctaggttc tagatccatg caaatttaga gtatttttac acacacacac gcttggacac     420
   aagtacatac atgtagtttt cttttatgtg gtgaaaagta cataacatgt agtttatagt     480
   tactagtcgc tatataattt aaaattgatg ttataggaat atatgtacgg agagagcttc     540
   cttgagtcca ttcacaggtc gtgatatgat ccaattagct tccgactcat tcatccaaat     600
   accgagtctc accaaaattg gaacaagact tgttaaatga atgaatgatg cggtagacaa     660
   attggatcat tgattctctt tgattggact gaagggagct cc                        702
<210> 262
   <211> 767
   <212> DNA
   <213> Eucalyptus grandis
<400> 262
   tcgtttccca tccccatttc atagaataat gccaccaaac aaagaagcat tagctcaaag 60
   actaattacc atctgtttta ttgatagata cgtgcgaaac ggtgattgtt ttttcccaaa 120
   taagaaacca aaatgaagca tattcaaagg tggagatatg gggagacttc cggaaggcaa 180
   ggggattgga aaaggctcga gatcaaagtg catagcaacc cttcgctaaa ggtgaaaaag 240
   aatacgaata acttcagtag ctcactttaa attccgaaac attaaacaaa tcaaatctcc 300
   ctcgccctcc ttgcctcctc tctttaccta tataaagcca ccgccccttc aatgaaatcc 360
   acgagtggaa ggtcacagta tagtagggtc ctgcaaaggg agagcgagag cggctccact 420
   gtctacctat aagcagttcc tttcttttgt ttacatgtct gttgcacctc accgagtttt 480
   tctattctct ttcctctggt tggttagcag atttctcagg ggacttccct cctcccttga 540
   ggatccttct cttgaagcga tatgtctcga atgggtaaga gagagagagg aagggagctt 600
   tcttcagtcc acccatggga cgtgttgggt tttaattagc tgccgactca ttcatccaaa 660
   taccgagcga gagcaagtaa cagagctccg taaatgaatg gatgatgcgg gagtcttgtt 720
   gattcccaag ctttccgtga ttggactgaa gggagctccc tctatct 767
<210> 263
   <211> 612
   <212> DNA
   <213> Gossypium raimondii
<400> 263
   gcgtatcctt ccacttacgg attcaccata gctgctatag cccgagtttg cttgtcataa 60
   tagaaataaa ctaagggaga aaaaagctct ccactctcgc ctttttcttc ttcgagtctc 120
   tctctttgac tggcctttgt gcaaagatct tcctttttta aacagtcgct ttctttactc 180
   tccctttccc ttctttctct taattgctaa agcagcttcc acttccactc actttaaccc 240
   atgatccatt tcaacctgtc acagtggaga gcaatttgta tggctaattt ccatctcacc 300
   tattcttttc tgtttggggt tctctagtct agggttgcat gacatgagag acatggctct 360
   tttttttttt tttcagttca caggtgttta tatatgttgt tgtgttattt tgtcttaaag 420
   ctttgtgatt gatgatctga taggtaagag agagctttct tcagtccact catgggatgg 480
   ggatggggtt taattagctg ccgactcatt catccaaata ctgtgttaca aaacccagta 540
   aatgagtgaa tgatgcggga gacaaattga atcctaatct tcctgtgctt ggactgaagg 600
   gagctccctc cc 612
<210> 264
   <211> 675
   <212> DNA
   <213> Medicago truncatula
<400> 264
   atttatccaa tcatgttgct ctcacttctt ttcagcagtt ctgttgatat caccctttta     60
   gttgaagctt tggattcatg actttatgag atggtaaatc tttaaattaa gtttaagtat     120
   gcacttgatt tgtttatata atttgtttat ttagatttta aaccctaagt tgactttttt     180
   aatttgattt aaattatgat atgattgtta tttggcttac catggtcata atttagggtt     240
   tagaagatgc atgtatatct tgaattgttt atggtaataa aggggttagg atttctcctt     300
   ttggtgaagt gagaaaatct cataattttg ttctgaaggt agtttttaag atttagggtt     360
   atgggttctt tgtttgaatg ctttttcaag tctttttcaa tgatatttgc ctagatctgt     420
   tttaattttg aattaaaatt ctgggtttgg attaatatta ttgaagatta ttattaatta     480
   atttattaga aatagatgaa gagagcttcc ttcagtccac tcatggaagg gtaaggggtt     540
   tgaatttacc tgctgactca ttgattcaaa cacaatagac aattatgggg ttatgctatt     600
   gtgaattgtg tgaatgatgc aggaggtgaa tttcttcctt ttcttctttg cttggactga     660
   agggagtctc ccttt                                                      675
<210> 265
   <211> 866
   <212> DNA
   <213> Oryza sativa
<400> 265
   ttatatctga cgcgttgtaa tcctgtttaa ttagggcttt gcccatttct tttgacccct      60
   tccggacatt cgctagttgg aaccttgttt tactcctagc agtgtactgt gtagtactta     120
   ttacgagcaa acgtaaaaat aaataaatgg aaatgataca aaggccgtgt ttaattttaa     180
   aatttttttt caaactttca acactttaca tcccataaaa agcttactat acatacaaac     240
   ttccaacctt ttcgtcacat cgtatctaat ttcaactaaa cttttaattt tagcgtgaac     300
   taaacacagc cgaagcccgg ccacattctc actattttta ttcattttat catgcctgtg     360
   atgtcacgcc ttggccctat ttaataggcc ttctccattt ctctccatat gatgtcttct     420
   cttctctatc cctcttgcca tcttctatct tccctcttgc acccatcttt gtgataactt     480
   ctactagctc ctctcctact accagtcata ccactctcac aaatcctcca agatccgcat     540
   ggggagaagc tccaaaagtt tcgtggttag tttaatttca tgcttgtttg ctgccgtttt     600
   tcatgttgat ctgatcttaa tatatgtaga ctgctgttaa catattcttt taatttgatg     660
   gaagaagcga tcgatggatg gaagagagcg atccttcagt ccactcatgg gcggtgctag     720
   ggtcgaatta gctgccgact cattcaccca caatgccaag caagaaacgc ttgagatagc     780
   gaagcttagc agatgagtga atgaagcggg agagtaacgt tccgatctcg cgccgtcttt     840
   gcttggactg aagggtgctc cctcct                                          866
<210> 266
   <211> 801
   <212> DNA
   <213> Physcomitrella patens
<400> 266
   tgggatccac acgagtaatt attccctctc tcatgcatca caacttggac ttgcccagct      60
   tttacctctc tccatgttcc accgtcggag atcctcggtg ctgctacccc cgttcggcca     120
   aacccaaccc aaccctaggt gtctgccgga cctccgcttc ccctcctgct tcaccccctg     180
   caccgcttaa atttgcacct cgtagtattt atctatgccc catttcagat gacttgcatg     240
   acacccatct ggattcgtct ccaggcgcct tgtcgtatct attcgctatc agtctttctt     300
   tcagtctgtc tttctgccaa gcacaccgtg ctggtggtgg tgctatcagt cgaagcagtc     360
   gccgaagggt ccttgtatcc agccctcacc ggagacagtt cgcttcgggg tggggagaac     420
   tgttgagacc gtcgatgttg catgcagcag cactggcgaa ggtgcttctg atactgggta     480
   ttccagcctc gccgctcggt gcactgcagg tacgtggtta catgacacta ctgtgtgggt     540
   agtctgggca caagtagatc gacatgccag atttggcccg tatgcgtgta tgtgcggtta     600
   tgttcccgtt tcgattgcgg ataccttgat tgtggagctc cgtttcggtc caatagtggc     660
   tgcgacggaa ggtggtcccg ctgccgaatc acacgtccgg gttgcttatc ggggcagggc     720
   cccgatacgg tatccgaacg tttgtcccgg gaactggtcg accttccgcc cggcgtctct     780
   tggactgaag ggagctccac t                                               801
<210> 267
   <211> 819
   <212> DNA
   <213> Thellungiella halophila
<400> 267
   ttttatacaa ataatgttcg ataacactaa accctagcca tccaactaat agacaaaacc      60
   ctacttgtaa tttacaaccg caaattccca gagaacagag taactacgag agagagatgg     120
   agattcaaat taaaaagaaa aacttatata taatgaatac acaaaagcta cctaatctgt     180
   atatatatat atataaatat gtcttcatta aattaatggt cgtggaatag aaaaaggaaa     240
   acctaatttg atcgctaggg cttatcagag taaagatggt taaccttcaa aagatgacta     300
   attaaccggg gagataatta aaagattaaa tacgccaaca gagagttaag agataccaga     360
   tttaaattcc acaatttggt catgttcttc ttcacgtatt catgacgatg tctgaattat     420
   agagaaaccc aaaatataaa atgttaattt taccagacat ttacatacca ataactctat     480
   gacgatatgt aaagtaagca aggcatgttt ttatgcaggg aagattgaaa attcaagatt     540
   aatcaagaaa attggaatac caaaaagaga gggagctccc ttcagtccaa tcagagagaa     600
   tcaatgaccc aatttgtcta ccgcatcatt cattcattta acaagtctag ctcgaattct     660
   tggtgactcg gtatttggat gaatgagtcg gaagctaatt ggatcatatc acgacctatg     720
   aatggactca aggaagctct ctacaaatgt attcctacca catcaaccca aatatagtga 780
   ttacagatgc tgttctcact gtagactaca tttacgttt 819
<210> 268
   <211> 503
   <212> DNA
   <213> Arabidopsis thaliana
<400> 268
   agacatctct tcttctctca tctctctttt cttctctctt ttcctcacat aaactctctt 60
   tttttactat taaatccata tggtacctca aattaatcta tggtcatcta gggttatctt 120
   gaagattaga attgattcta gcacgcacag agaggaagat cattgcatcc agaatcacaa 180
   acatggccta tcttttatct tttctttttg atctaagtca ctgttttatg ctatatatag 240
   tataatcaaa ttctttacat gtgcttgtat gtatgcgtat atatagtaac ggaattgtta 300
   atatgcttat agatgttgag ttggtggagg aagagagctt tcttcggtcc actcatggag 360
   taatatgtga gatttaattg actctcgact cattcatcca aataccaaat gaaagaattt 420
   gttctcatat ggtaaatgaa tgaatgatgc gagagacaaa ttgagtcttc acttctctat 480
   gcttggactg aagggagctc cct 503
<210> 269
   <211> 365
   <212> DNA
   <213> Arabidopsis thaliana
<400> 269
   tcttattcca tcacaatcat ctagggtttt aagccaagct tatatagccc gtcataaaga 60
   gaactcatct gcctctctct caataccaat aaatatcacc accgtccttc tctcctatca 120
   ctattcaatc tatcgcaaac tcctttatgt ctctccaatt ttatgagagg gtttccttca 180
   agaacacagt aaaatagatt ggatctttaa acttttgttc cttttcatga gggtttgaca 240
   aagattttct tacagtcatc tttggcattc tgtccacctc cttctataca tatatgcatg 300
   tgtatatata tatgcgtttc gtgtgaaaga aggaggtggg tatactgcca atagagatct 360
   gttag 365
<210> 270
   <211> 134
   <212> DNA
   <213> Arabidopsis thaliana
<400> 270
   ttgtatcatg acagagcaag aagaagaaag tcaaatgtcc acatgagttc cctttaacgc 60
   ttcattgttg aatactcaaa gccacattgg tttgtatata acactgaagt gtttgggggg 120
   actcttggtg tcat 134
<210> 271
   <211> 229
   <212> DNA
   <213> Arabidopsis thaliana
<400> 271
   tttcaaaccc taacactctt ataaaccgat tcgccaaaat gtatctacaa tatattgata 60
   atgtaatatc tatatattca aacaatcgtc gtgttggtcg gatgttttct agagttcctc 120
   tgagcacttc attggagata caatttttta taaaatagtt ttctactgaa gtgtttgggg 180
   gaactcccgg gctgattcgg tattttaaat tcagtagact agctagctg 229
<210> 272
   <211> 220
   <212> DNA
   <213> Arabidopsis thaliana
<400> 272
   tggtaataga aatgagcaag gagatatttt tttcccctgg gtttgaatga acatcattga 60
   gtgcatcgtt gatgtaattt tacttatttt attccattgt tgaattaatt aaagaagtat 120
   atatcagcgt tgcattcaat tatgtttttc taattttcag gaaatacaaa aaaaatgaaa 180
   aaaaaaaatc acttaaaaga ccttgagagt tcttttgact 220
<210> 273
   <211> 225
   <212> DNA
   <213> Arabidopsis thaliana
<400> 273
   ggatatcgaa actcaaactg taacagtcct tttattactg gtttagaaga tagataaata 60
   ttgttaaggt agtggatctc gacagggttg atatgagaac acacgagcaa tcaacggcta 120
   taacgacgct acgtcattgt tacagctctc gtttcatgtg ttctcaggtc acccctgctg 180
   agctctttct ctaccgtcca tgttttatca acgccgtggc ccgtg 225
<210> 274
   <211> 165
   <212> DNA
   <213> Arabidopsis thaliana
<400> 274
   tcttatagag atgaagagaa acatgtaaac tcactagttt tagggcgcct ctccattggc 60
   aggtccttta cttccaaata tacacataca tatatgaata tcgaaaattt ccgatgatcg 120
   atttataaat gacctgccaa aggagagttg ccctgaaact ggttc 165
<210> 275
   <211> 187
   <212> DNA
   <213> Arabidopsis thaliana
<400> 275
   caataactca aaatgcaatg tgaaatatga agaatatatt aaatagtagt gaagatgcat 60
   gtttatgaag acagagagat aatgtatggt tggattactg ggcgaatact cctatggcag 120
   atcgcattgg ctagatatgc aagtaaaatg cttctctgcc aaaggagatt tgccccgcaa 180
   ttcatcc 187
<210> 276
   <211> 235
   <212> DNA
   <213> Arabidopsis thaliana
<400> 276
   atttaggtct ctcttcttct tcttcttttt cttcttgagc gccggcgaaa aaagtctctg 60
   tgagaaaaag atacgacgat tgtcattaga agagtcgtat tacatgtttt gtgcttgaat 120
   ctaattcaac aggctttatg taagagattc tttaacaatt cctataatct ttgttgttgg 180
   attagattca cgcacaaact cgtaatctgt cttttcgatt tttaccagat ctgtc 235
<210> 277
   <211> 944
   <212> DNA
   <213> Arabidopsis thaliana
<400> 277
   aattatatcc atggtcatgg ctcatcatta gtcgcactgc tctccttttc tcaaagttta 60
   aattcgacat ttggtaaaat gatgaaacct cgatggaact gctctcttta tggaatcacg 120
   gaatggacaa ataatcaaaa tcagaaatcg aagcgaaaag ggaggagaaa aacgcagatt 180
   tggaggattg gggacagatt agatactgtt gaatgcatca ctctaatgct atcagcctat 240
   taatagcgtc ctatattttc gaagactttt aatgtttagg gttatggatt tttcgagcga 300
   agcatggaga gatgttgaat tggatactat aggatttggt acaacacata catatgttct 360
   gcttctgcaa aactaacata tcaagttcag agaaaccagt aagtcgttga atattttatt 420
   atccattcaa cgctttcttc ttttggatca tgtcttgttt gcttgaccac ttcttcttgc 480
   ttaagaggat ggacaatata taaaaactgg agccttcttt ttctatgaat gcttatcatc 540
   gcggagttga tctgttcaat tcacctgcca ttggatgctt tttttatata tacttcactg 600
   ttcaatttca gatgctttag aaggtttgcg gagtagctag agaatctggt atcttcagtt 660
   cttcaatttc agctacttgg tatcagcttc gtcattgtat atcaacacat tcttaatata 720
   taatactact ttttcatcca ttaaacccct tacaatgtcg agtaaacgaa gcatctgtcc 780
   cctggtattg tcttcgagct tggtgttttt ttctagccaa ctccaagttc tcgagttgat 840
   cattgtttgt attcttgaga cattatttgg ggacgagatg ttttgttgac tcgatataag 900
   aaggggcttt atggaagaaa ttgtagtatt atatatcgag agtg 944
<210> 278
   <211> 420
   <212> DNA
   <213> Arabidopsis thaliana
<400> 278
   ctataaatgc tgcttatcat cgtggagttg gttctgtaaa catttgaaaa ttctgaacag 60
   tttcacctgc cattggatgc tttgtttcaa tttcaggtgc gttagaaggt ttgcagagta 120
   gctagagaat ctcgtatctt cactttctgc tacttggtat cagcttcgtc actttatatc 180
   aacacattct taatatacaa tactactttt tcatccatta atccccttac aatgtcgagt 240
   aaacgaagca tctgtcccct ggtattgtct tcgagcttgg tgtgtttttc tagccagccc 300
   caagttctcg agttgatcat tgtttgtatt ctgacacatt atttggggac gagatgtttt 360
   gttgactcga tataagaagg ggctttatgg aagaaattgt agtattatat attgagaatg 420
<210> 279
   <211> 383
   <212> DNA
   <213> Arabidopsis thaliana
<400> 279
   tagtataacc gctgatgtac acctaccagc ttgataactc tttttcgtgg tttctgtgta      60
   ctcgtttctg tttgtacaga tacttcttgt tcaatttcag atgctttaga aggttttcgg     120
   agtggctaga gatctgttat ctgtatgaac agctacttgg tatcagcttc gtcattttat     180
   caacacattc ttaatataca atacttcttt ttcatgcatt aagcccctta caatgtcgag     240
   taaacaaagc atgtgtccgc taatattgtc ttcgagcttg gtatttttgt attctgatac     300
   ggtatttggg gacgacatct tttgttgact cgatataaga agggggtttg tggaagaaat     360
   tgtagtatta tatatcaaga atg                                             383
<210> 280
   <211> 2164
   <212> DNA
   <213> Drosophila melanogaster
<400> 280
   tttgtggaac acattcgacc cactgaaaaa ttgatataat ttaatgaaag tgcataaaaa      60
   tggtggacag tgcattaaac tgagcattga acacaaaggc cgctcagcaa attgctaatt     120
   aaaattcacg attgccattt cacctgacac gttgacgatt ttcattacaa ttcgattatg     180
   tttcgttgca gggaatttta aatgttaatt gccaagaatg tttcaacaaa ttcatttctc     240
   attaatgtgt cttttcattt aattttatgt tgtatgagct gcacgagaaa tgagttgtac     300
   ttttagttcg acggcagagt catgaatgtt cggcaaagaa tgtaataata actatcctct     360
   ttagacaaat atagatacaa atctatcaga ttctaaaagt agaataatca attaatcaga      420
   aagctaaaaa taaataggca tatttatatt ttaatgcgga tttttgaagt tcaacgggag      480
   aaatgaatcc tttttaccag ccacaggcgc aatttgcaac agaaagtgta gcagaagtac      540
   tcctcgaata tttccctgct ccaggagtca tccatgtggt ttcgaggcac acatttgaca      600
   aactcatgcc ccgctatttg ttgtaaaaac acaatcgcac acatggccgc atttcggcga      660
   cttccagaga gcggtacact taaggcggcc tgggaaacgc ctgcaatctg ctggtcgcga      720
   actgcagatt gcatccatgt gccaggcgac catgcgacca tgtgaccatg tgcccgcccg      780
   acgcctcgca gcccacatcc tgcccatcga gggcacaact cagcgtgggt attgccgctc      840
   cggctgcttc aagtaggtaa aaaccgagaa gattgaggat gaatgtatga gtatgagaaa      900
   atactcggcg gaacatatgc tgccgggctt gacctgaccc tgcctcatgt gtgggtctcc      960
   gatttaattt taggcaccta tataaacgcg tgtttacact gcagccagaa cacagtcgcc     1020
   gtcttcagtt cgcgccgtca actcctcgat cgatcgatcg catcgtctcg gatcgaatag     1080
   agctgggctt ctgctccgga gctacatcgc cgtacttgtc ggacgagtgt ggtgatgaaa     1140
   agtcgcttag tccgggattc ctgccagatc tctaagggat gagctggcat cccaggctgg     1200
   ccatgtggcg cgaagtatgc gcacaaaaaa gtcaaacaaa aaggcgcaat tttattacgg     1260
   gcaaccaacg acgaaacaaa acaaaagcca accgaaaagc agaaacaaag cagcaaaaag     1320
   tttatgaatt ttttgtgcag gcgcgtgaaa gatgcaaaac gagaaaaaaa catgaaaaaa     1380
   aaacattaaa aaaaacaaaa aaaatccaaa acagataccg agctgtatcc gaaaacgagt     1440
   ggggaaaggg gtttcccagt cacatataaa cacacttcag tgcgcttaaa aattgcttta     1500
   ttgcagttgg actataaaaa cgcacggcag cgaacaccgc acaacaaaaa ggacgagcag     1560
   aagtgggcaa ataaaacgaa agctcttaaa cgaaaaacag gaaaatttgc atgccacaaa     1620
   aataagcata aggatttgcc gcgcacaaag tagaagcaaa aaggaattgc ccaaatgcag     1680
   ccacaaaaga ctgtggcaaa tgttttgcag cttgcccctt tttccctgca attaccgtca     1740
   gtcgttgtca ttattcagca gattatatgg ttttgcttat tccggaccac catcatcatc     1800
   atcattatca tcatcttcgg taagttagac aatcccataa aaaactgtcc aagtgagtag     1860
   tgccaccaaa agttagccgc gttgtggaaa atccaaaaca aagaccatcc catattcagc     1920
   ctttgagagt tccatgcttc cttgcattca atagttatat tcaagcatat ggaatgtaaa     1980
   gaagtatgga gcgaaatctg gcgagacatc ggagttgaaa ctaaaactga aatttgattg     2040
   aaacagaagt agaaccgtaa tgaaatgaat gaaatattaa cccgtttcta caatccctga     2100
   ataaaattat taattaatta tagagcgggc taattttaca atatatattg attttttttt     2160
   gaag                                                                  2164
<210> 281
   <211> 919
   <212> DNA
   <213> Drosophila melanogaster
<400> 281
   attctttttt ggtgctcgat cgtgacggtt tgctcgcgct ctccgctgcg ccgctctttc        60
   cgttgcatat gtgtgcgggc gttattgtgc atgtttccgg tggccgaaaa aaaatagtaa      120
   aataaaatat agaaaacaga aaccaagaat aataacagcc atacgataaa cagtgtgcca      180
   atgtgtgtgt ctgtgtgtgt gtgcatctcg cgtaacaaca ataattgcat ttatcggatg      240
   gcgccagctt caatttaatt ataaataaca tgttcaactt tttatactat tttccctgcg      300
   tcaaagtggg cgttgcaact gcccccggaa aatcacgcgc cccggttcaa agttaaagtt      360
   tgctgggtaa cgcacacaca cacacacaca atcactcaca cgcggtcaca cgcacatttc      420
   aataaactaa tggagcctgg ctttgttttt gttttatttc caacccactt gagcacacag      480
   cacacacaga gagaaaaatc aatactcgtt atgggattaa atttacaaag cgcaaagcaa      540
   agcgacaaac aaaattcaaa agaaagaaaa aaaaacactc aaataaactc acaaagaatt      600
   ccttatcgcc aagggggcca atgttctaag gttctttcgc cttgagaact ttgagcttcc      660
   tctggcaaag gagattataa tgtacaaata atgttgcaat aaccagttga aaccaatgga      720
   ataccgaatc ttgctaatta gcaaggacat ctgttcacat cttaccgggc agcattagat      780
   cctttttata actctaatac tgtcaggtaa agatgtcgtc cgtgtcctta accttcagta      840
   ccaccaacag cagcagcagc accaaaaaaa aaaaaaaaaa aatgcgtaaa aatccaaaca      900
   aatcataaaa gtcgaagga                                                   919
<210> 282
   <211> 20
   <212> RNA
   <213> Arabidopsis thaliana
<400> 282
   ugacagaaga gagugagcac 20
<210> 283
   <211> 20
   <212> RNA
   <213> Arabidopsis thaliana
<400> 283
   ugacagaaga gagugagcac 20
<210> 284
   <211> 20
   <212> RNA
   <213> Arabidopsis thaliana
<400> 284
   ugacagaaga gagugagcac 20
<210> 285
   <211> 20
   <212> RNA
   <213> Arabidopsis thaliana
<400> 285
   ugacagaaga gagugagcac 20
<210> 286
   <211> 21
   <212> RNA
   <213> Arabidopsis lyrata
<400> 286
   uuuggauuga agggagcucu a 21
<210> 287
   <211> 21
   <212> RNA
   <213> Arabidopsis thaliana
<400> 287
   uuuggauuga agggagcucu a 21
<210> 288
   <211> 21
   <212> RNA
   <213> Capsella rubella
<400> 288
   uuuggauuga agggagcucu a 21
<210> 289
   <211> 21
   <212> RNA
   <213> Arabidopsis thaliana
<400> 289
   uuuggauuga agggagcucu u 21
<210> 290
   <211> 21
   <212> RNA
   <213> Arabidopsis thaliana
<400> 290
   ugccuggcuc ccuguaugcc a 21
<210> 291
   <211> 21
   <212> RNA
   <213> Arabidopsis thaliana
<400> 291
   ugccuggcuc ccuguaugcc a 21
<210> 292
   <211> 21
   <212> RNA
   <213> Arabidopsis thaliana
<400> 292
   ucaaugcauu gaaagugacu a 21
<210> 293
   <211> 21
   <212> RNA
   <213> Arabidopsis thaliana
<400> 293
   ucgauaaacc ucugcaucca g 21
<210> 294
   <211> 21
   <212> RNA
   <213> Arabidopsis thaliana
<400> 294
   ucgauaaacc ucugcaucca g 21
<210> 295
   <211> 24
   <212> RNA
   <213> Arabidopsis thaliana
<400> 295
   uugaagagga cuuggaacuu cgau 24
<210> 296
   <211> 21
   <212> RNA
   <213> Arabidopsis lyrata
<400> 296
   uggagaagca gggcacgugc a 21
<210> 297
   <211> 21
   <212> RNA
   <213> Arabidopsis thaliana
<400> 297
   uggagaagca gggcacgugc a 21
<210> 298
   <211> 21
   <212> RNA
   <213> Brassica rapa
<400> 298
   uggagaagca gggcacgugc a 21
<210> 299
   <211> 21
   <212> RNA
   <213> Carica papaya
<400> 299
   uggagaagca gggcacgugc a 21
<210> 300
   <211> 21
   <212> RNA
   <213> Capsella rubella
<400> 300
   uggagaagca gggcacgugc a 21
<210> 301
   <211> 21
   <212> RNA
   <213> Gossypium raimondii
<400> 301
   uggagaagca gggcacgugc a 21
<210> 302
   <211> 21
   <212> RNA
   <213> Medicago truncatula
<400> 302
   uggagaagca gggcacgugc a 21
<210> 303
   <211> 21
   <212> RNA
   <213> Oryza sativa
<400> 303
   uggagaagca ggguacgugc a 21
<210> 304
   <211> 21
   <212> RNA
   <213> Arabidopsis lyrata
<400> 304
   ucggaccagg cuucaucccc c 21
<210> 305
   <211> 21
   <212> RNA
   <213> Arabidopsis thaliana
<400> 305
   ucggaccagg cuucaucccc c 21
<210> 306
   <211> 21
   <212> RNA
   <213> Brassica carinata
<400> 306
   ucggaccagg cuucaucccc c 21
<210> 307
   <211> 21
   <212> RNA
   <213> Brassica juncea
<400> 307
   ucggaccagg cuucaucccc c 21
<210> 308
   <211> 21
   <212> RNA
   <213> Brassica napus
<400> 308
   ucggaccagg cuucaucccc c 21
<210> 309
   <211> 21
   <212> RNA
   <213> Brassica oleracea
<400> 309
   ucggaccagg cuucaucccc c 21
<210> 310
   <211> 21
   <212> RNA
   <213> Brassica rapa
<400> 310
   ucggaccagg cuucaucccc c 21
<210> 311
   <211> 21
   <212> RNA
   <213> Arabidopsis thaliana
<400> 311
   ucggaccagg cuucauuccc c 21
<210> 312
   <211> 21
   <212> RNA
   <213> Arabidopsis thaliana
<400> 312
   ucggaccagg cuucauuccc c 21
<210> 313
   <211> 21
   <212> RNA
   <213> Arabidopsis thaliana
<400> 313
   ugaagcugcc agcaugaucu a 21
<210> 314
   <211> 21
   <212> RNA
   <213> Arabidopsis thaliana
<400> 314
   ugaagcugcc agcaugaucu a 21
<210> 315
   <211> 21
   <212> RNA
   <213> Arabidopsis thaliana
<400> 315
   cagccaagga ugacuugccg g 21
<210> 316
   <211> 21
   <212> RNA
   <213> Arabidopsis thaliana
<400> 316
   uagccaagga ugacuugccu g 21
<210> 317
   <211> 21
   <212> RNA
   <213> Arabidopsis thaliana
<400> 317
   ugauugagcc gcgccaauau c 21
<210> 318
   <211> 21
   <212> RNA
   <213> Arabidopsis thaliana
<400> 318
   uugagccgug ccaauaucac g 21
<210> 319
   <211> 21
   <212> RNA
   <213> Medicago truncatula
<400> 319
   ugauugagcc gcgucaauau c 21
<210> 320
   <211> 21
   <212> RNA
   <213> Zea mays
<400> 320
   ggauugagcc gcgucaauau c 21
<210> 321
   <211> 21
   <212> RNA
   <213> Arabidopsis thaliana
<400> 321
   uugagccgug ccaauaucac g 21
<210> 322
   <211> 21
   <212> RNA
   <213> Medicago truncatula
<400> 322
   agauugagcc gcgccaauau c 21
<210> 323
   <211> 21
   <212> RNA
   <213> Medicago truncatula
<400> 323
   cgagccgaau caauaucacu c 21
<210> 324
   <211> 21
   <212> RNA
   <213> Arabidopsis thaliana
<400> 324
   agaaucuuga ugaugcugca u 21
<210> 325
   <211> 20
   <212> RNA
   <213> Arabidopsis thaliana
<400> 325
   gcagcaccau uaagauucac 20
<210> 326
   <211> 21
   <212> RNA
   <213> Arabidopsis thaliana
<400> 326
   agaaucuuga ugaugcugca g 21
<210> 327
   <211> 21
   <212> RNA
   <213> Arabidopsis thaliana
<400> 327
   ggaaucuuga ugaugcugca u 21
<210> 328
   <211> 21
   <212> RNA
   <213> Arabidopsis cebennensis
<400> 328
   uuggacugaa gggagcuccc u 21
<210> 329
   <211> 21
   <212> RNA
   <213> Arabidopsis halleri
<400> 329
   uuggacugaa gggagcuccc u 21
<210> 330
   <211> 21
   <212> RNA
   <213> Arabidospis lyrata
<400> 330
   uuggacugaa gggagcuccc u 21
<210> 331
   <211> 21
   <212> RNA
   <213> Arabidopsis thaliana
<400> 331
   uuggacugaa gggagcuccc u 21
<210> 332
   <211> 21
   <212> RNA
   <213> Brassica rapa
<400> 332
   uuggacugaa gggagcuccc u 21
<210> 333
   <211> 21
   <212> RNA
   <213> Carica papaya
<400> 333
   uuggacugaa gggagcuccu u 21
<210> 334
   <211> 19
   <212> RNA
   <213> Capsella rubella
<400> 334
   uuggacugaa gggagcucc 19
<210> 335
   <211> 21
   <212> RNA
   <213> Eucalyptus grandis
<400> 335
   uuggacugaa gggagcuccc u 21
<210> 336
   <211> 21
   <212> RNA
   <213> Gossypium raimondii
<400> 336
   uuggacugaa gggagcuccc u 21
<210> 337
   <211> 22
   <212> RNA
   <213> Medicago truncatula
<400> 337
   uuggacugaa gggagucucc cu 22
<210> 338
   <211> 21
   <212> RNA
   <213> Oryza sativa
<400> 338
   uuggacugaa gggugcuccc u 21
<210> 339
   <211> 20
   <212> RNA
   <213> Physcomitrella patens
<400> 339
   cuuggacuga agggagcucc 20
<210> 340
   <211> 20
   <212> RNA
   <213> Thellungiella halophila
<400> 340
   uggacucaag gaagcucucu 20
<210> 341
   <211> 21
   <212> RNA
   <213> Arabidopsis thaliana
<400> 341
   uuggacugaa gggagcuccc u 21
<210> 342
   <211> 20
   <212> RNA
   <213> Arabidopsis thaliana
<400> 342
   uuggcauucu guccaccucc 20
<210> 343
   <211> 21
   <212> RNA
   <213> Arabidopsis thaliana
<400> 343
   cugaaguguu uggggggacu c 21
<210> 344
   <211> 21
   <212> RNA
   <213> Arabidopsis thaliana
<400> 344
   cugaaguguu ugggggaacu c 21
<210> 345
   <211> 21
   <212> RNA
   <213> Arabidopsis thaliana
<400> 345
   ucauugagug caucguugau g 21
<210> 346
   <211> 21
   <212> RNA
   <213> Arabidopsis thaliana
<400> 346
   uguguucuca ggucaccccu g 21
<210> 347
   <211> 21
   <212> RNA
   <213> Arabidopsis thaliana
<400> 347
   ugccaaagga gaguugcccu g 21
<210> 348
   <211> 21
   <212> RNA
   <213> Arabidopsis thaliana
<400> 348
   ugccaaagga gauuugcccc g 21
<210> 349
   <211> 21
   <212> RNA
   <213> Arabidopsis thaliana
<400> 349
   uuagauucac gcacaaacuc g 21
<210> 350
   <211> 22
   <212> RNA
   <213> Arabidopsis thaliana
<400> 350
   uuggggacga gauguuuugu ug 22
<210> 351
   <211> 22
   <212> RNA
   <213> Arabidopsis thaliana
<400> 351
   uuggggacga gauguuuugu ug 22
<210> 352
   <211> 21
   <212> RNA
   <213> Arabidopsis thaliana
<400> 352
   ccccuuacaa ugucgaguaa a 21
<210> 353
   <211> 22
   <212> RNA
   <213> Drosophila melanogaster
<400> 353
   uggaauguaa agaaguaugg ag 22
<210> 354
   <211> 23
   <212> RNA
   <213> Drosophila melanogaster
<400> 354
   uaauacuguc agguaaagau guc 23
<210> 355
   <211> 17
   <212> PRT
   <213> Homo sapiens
<400> 355 Val
<210> 356
   <211> 53
   <212> DNA
   <213> Homo sapiens
<400> 356
   tggagatggc tctaatggtg gcacaaacca ggaaggggaa atctgtggtt taa 53
<210> 357
   <211> 1500
   <212> DNA
   <213> Homo sapiens
<400> 357
   gccgagcccg ggcccagcgc cgcctgcagc ctcgggaagg gagcggatag cggagccccg      60
   agccgcccgc agagcaagcg cggggaacca aggagacgct cctggcactg cagataactt     120
   gtctgcattt caagaacaac ctaccagaga ccttacctgt caccttggct ctcccaccca     180
   atggagatgg ctctaatggt ggcacaaacc aggaagggga aatctgtggt ttaaattctt     240
   tatgcctcat cctctgagtg ctgaaggctt gctgtaggct gtatgctgtt aatgctaatc     300
   gtgatagggg tttttgcctc caactgactc ctacatatta gcattaacag tgtatgatgc     360
   ctgttactag cattcacatg gaacaaattg ctgccgtggg aggatgacaa agaagcatga     420
   gtcaccctgc tggataaact tagacttcag gctttatcat ttttcaatct gttaatcata     480
   atctggtcac tgggatgttc aaccttaaac taagttttga aagtaaggtt atttaaaaga     540
   tttatcagta gtatcctaaa tgcaaacatt ttcatttaaa tgtcaagccc atgtttgttt     600
   ttatcattaa cagaaaatat attcatgtca ttcttaattg caggttttgg cttgttcatt     660
   ataatgttca taaacacctt tgattcaact gttagaaatg tgggctaaac acaaatttct     720
   ataatatttt tgtagttaaa aattagaagg actactaacc tccagttata tcatggattg     780
   tctggcaacg ttttttaaaa gatttagaaa ctggtacttt cccccaggta acgattttct     840
   gttcaggcaa cttcagttta aaattaatac ttttatttga ctcttaaagg gaaactgaaa     900
   ggctatgaag ctgaattttt ttaatgaaat atttttaaca gttagcaggg taaataacat     960
   ctgacagcta atgagatatt ttttccatac aagataaaaa gatttaatca aaaaatttca    1020
   tatttgaaat gaagtcccaa atctaggttc aagttcaata gcttagccac ataatacggt    1080
   tgtgcgagca gagaatctac ctttccactt ctaagcctgt ttcttcctcc atatggggat    1140
   aatactttac aaggttgttg tgaggcttag atgagataga gaattattcc ataagataat    1200
   caagtgctac attaatgtta tagttagatt aatccaagaa ctagtcaccc tactttatta    1260
   gagaagagaa aagctaatga tttgatttgc agaatattta aggtttggat ttctatgcag    1320
   tttttctaaa taaccatcac ttacaaatat gtaaccaaac gtaattgtta gtatatttaa    1380
   tgtaaacttg ttttaacaac tcttctcaac attttgtcca ggttattcac tgtaaccaaa    1440
   taaatctcat gagtctttag ttgatttaaa ataaaaaaaa aaaaaaaaaa aaaaaaaaaa    1500
<210> 358
   <211> 23
   <212> RNA
   <213> Homo sapiens
<400> 358
   uaauacuguc agguaaagau guc 23
<210> 359
   <211> 337
   <212> DNA
   <213> Medicago truncatula
<400> 359
   ttagggtttt cagctcatgg taataaaaat gtcatctaat gtcttgcatg tgggaatgag    60
   gtcatatatg cagccaagga tgacttgccg gcgagcctct ttcgatactt ttatgacata   120
   attaatcatg tggatagcca aggtactaaa ctcactttgc actaaaacaa atatttttgc   180
   tttagtgcaa acttagttta ggcgcttcgc aacggctagt caaatgtcct agttccaatg   240
   tgattggttg tccggcaagt cgtctctggc tacgtaaagg cctccttttt tcatgctaga   300
   tttttgatga tttgatatag ccacacatat tttggaa                            337
<210> 360
   <211> 121
   <212> DNA
   <213> Medicago truncatula
<400> 360
   aagaggcaga gagagtaatg cagccaagga tgacttgccg acaacattgg cgaatgttca    60
   tgtgatttct gcctcattgt gccggcaagt tgtccttggc tatgttagtc tctcatcttc   120
   t                                                                   121
<210> 361
   <211> 114
   <212> DNA
   <213> Medicago truncatula
<400> 361
   tgaattcccc tccgcttttt gatgttggct tgtctcaatc aaatcaaagt tcttgaaatt    60
   tgagttcttt agtctgattg agtcgtgcca atatcatatt aagcgataaa agtc         114
<210> 362
   <211> 367
   <212> DNA
   <213> Medicago truncatula
<400> 362
   ccacaaaact ataactagct agaagcttta atcgccttat ttattataat aataataata 60
   aatatggctt cagctgcaaa agtatacatg gcgtgatatt gatccggctc atctatatct 120
   tcaagttcaa tcatccatat tcatatcaat ttcagacgag ccgaatcaat atcactcttg 180
   tttgcttcat tgcatattaa ttatatactt catttataag ttatagtttg ccatatatat 240
   attagattga ttctgcagaa gtagacagga gtggtgttgt ttctgctcat cttattaaat 300
   aatgaatgaa tgaatgacat ttgcttactt ataagacgag ccgaatcaat atcactccag 360
   tacacct 367
<210> 363
   <211> 135
   <212> DNA
   <213> Medicago truncatula
<400> 363
   aactgcaact tgaggaggca tccaaaggga tcgcattgat cctataatat ttcaacttta 60
   gtcactttaa ttttctctca tataatactt aattgggatc atgccatccc tttggatttc 120
   tcctttagta gctac 135
<210> 364
   <211> 119
   <212> DNA
   <213> Medicago truncatula
<400> 364
   aggcatccaa agggatcgca ttgatcccaa atctaattaa gtccctagct acttaattaa 60
   caacttaatt tccttaatat ctcataatat ttgggatcat gctatccctt tggattcat 119
<210> 365
   <211> 93
   <212> DNA
   <213> Medicago truncatula
<400> 365
   tgcttttcca cagctttctt gaacttcttt cgtatcttaa atctgttttc aagattaaaa 60
   gtcctagaag ctcaagaaag ctgtgggaga ata 93
<210> 366
   <211> 95
   <212> DNA
   <213> Medicago truncatula
<400> 366
   tattcttcca cagctttctt gaactgcatc caaattgagt tcctttgcat tgccatggcc 60
   attgttttgc ggttcaataa agctgtggga agata 95
<210> 367
   <211> 21
   <212> RNA
   <213> Medicago truncatula
<400> 367
   cagccaagga ugacuugccg g 21
<210> 368
   <211> 21
   <212> RNA
   <213> Medicago truncatula
<400> 368
   cagccaagga ugacuugccg a 21
<210> 369
   <211> 21
   <212> RNA
   <213> Medicago truncatula
<400> 369
   ugauugaguc gugccaauau c 21
<210> 370
   <211> 20
   <212> RNA
   <213> Medicago truncatula
<400> 370
   gagccgaauc aauaucacuc 20
<210> 371
   <211> 21
   <212> RNA
   <213> Medicago truncatula
<400> 371
   uccaaaggga ucgcauugau c 21
<210> 372
   <211> 21
   <212> RNA
   <213> Medicago truncatula
<400> 372
   uccaaaggga ucgcauugau c 21
<210> 373
   <211> 21
   <212> RNA
   <213> Medicago truncatula
<400> 373
   uuccacagcu uucuugaacu u 21
<210> 374
   <211> 21
   <212> RNA
   <213> Medicago truncatula
<400> 374
   uuccacagcu uucuugaacu g 21
<210> 375
   <211> 15
   <212> PRT
   <213> Arabidopsis thaliana
<400> 375
   Met Met Leu His Ile Thr His Arg Phe Glu Ser Asp Val Gly Cys
   1               5                   10                  15
<210> 376
   <211> 4
   <212> PRT
   <213> Arabidopsis thaliana
<400> 376
   Met Leu Tyr Val
   1
<210> 377
   <211> 14
   <212> PRT
   <213> Arabidopsis thaliana
<400> 377
   Met Phe Met Arg Arg Gly Leu Val Tyr Asn Asn Ile Tyr Ile
   1               5                   10
<210> 378
   <211> 17
   <212> PRT
   <213> Arabidopsis thaliana
<400> 378
   Met Met Lys Val Cys Asp Glu Gln Asp Gly Glu Ala Gly His Val His
   1               5                   10                  15
   Tyr
<210> 379
   <211> 28
   <212> PRT
   <213> Arabidopsis thaliana
<400> 379
   Met Lys Lys Arg Ile Thr Arg Ile Asn Leu Glu Glu Gln Ile Lys Lys
   1               5                   10                  15
   Thr Leu Asp Asp Ser Arg Thr Arg Leu His Ser Pro
               20                  25
<210> 380
   <211> 10
   <212> PRT
   <213> Arabidopsis thaliana
<400> 380
   Met Lys Lys Ile Gly Ser Ile Asp Ser Phe
   1               5                   10
<210> 381
   <211> 6
   <212> PRT
   <213> Arabidopsis thaliana
<400> 381
   Met Thr Cys Arg Phe Lys
   1               5
<210> 382
   <211> 11
   <212> PRT
   <213> Arabidopsis thaliana
<400> 382
   Met Lys Asn Glu Asn Leu Cys Gly Ser Gln Gly
   1               5                   10
<210> 383
   <211> 45
   <212> PRT
   <213> Arabidopsis thaliana
<400> 383
   Met Lys Cys Met Met Lys Lys Arg Gly Leu Thr Trp Arg Lys Ala Ser
   1               5                   10                  15
   Cys Leu Val Ala Lys Asp Asp Leu Pro Asp Leu Phe Arg Leu His Asp
               20                  25                  30
   Ser Ile Ser Asn Ser Cys Ile Leu Asp Tyr Tyr Thr Phe
           35                  40                  45
<210> 384
   <211> 7
   <212> PRT
   <213> Arabidopsis thaliana
<400> 384
   Met Phe Pro Arg Glu Ser Leu
   1 5
<210> 385
   <211> 29
   <212> PRT
   <213> Arabidopsis thaliana
<400> 385
   Met Thr Leu Ser Val Phe Phe His Ser Phe Leu Glu Leu Gln Asn Phe
   1 5 10 15
   Phe Arg Phe Phe Phe Phe Ser Phe Asp Ile Ser Tyr Ala
   20 25
<210> 386
   <211> 23
   <212> PRT
   <213> Arabidopsis thaliana
<400> 386
   Met Ser Leu Ala Lys Gly Glu Leu Pro Cys His Cys Phe Arg Leu Asn
   1 5 10 15
   Thr Val Tyr Asn Arg Phe Cys
   20
<210> 387
   <211> 48
   <212> DNA
   <213> Arabidopsis thaliana
<400> 387
   atgatgttgc atatcacaca taggtttgag agtgatgttg gttgttga 48
<210> 388
   <211> 15
   <212> DNA
   <213> Arabidopsis thaliana
<400> 388
   atgctgtatg tatag 15
<210> 389
   <211> 45
   <212> DNA
   <213> Arabidopsis thaliana
<400> 389
   atgttcatgc gtagaggttt ggtatacaac aatatataca tataa 45
<210> 390
   <211> 54
   <212> DNA
   <213> Arabidopsis thaliana
<400> 390
   atgatgaagg tgtgtgatga gcaagatgga gaagcagggc acgtgcatta ctag 54
<210> 391
   <211> 87
   <212> DNA
   <213> Arabidopsis thaliana
<400> 391
   atgaagaaga gaatcactcg aattaatttg gaagaacaaa ttaagaaaac cctagatgat 60
   tctcggacca ggcttcattc cccctaa 87
<210> 392
   <211> 33
   <212> DNA
   <213> Arabidopsis thaliana
<400> 392
   atgaagaaga tcggtagtat tgattcattt taa 33
<210> 393
   <211> 21
   <212> DNA
   <213> Arabidopsis thaliana
<400> 393
   atgacttgcc gatttaaatg a 21
<210> 394
   <211> 12
   <212> DNA
   <213> Arabidopsis thaliana
<400> 394
   atggtgacat ga 12
<210> 395
   <211> 36
   <212> DNA
   <213> Arabidopsis thaliana
<400> 395
   atgaagaatg agaacttgtg tggtagccaa ggatga 36
<210> 396
   <211> 138
   <212> DNA
   <213> Arabidopsis thaliana
<400> 396
   atgaagtgta tgatgaagaa gagaggtcta acatggcgga aagcgtcatg tttagtagcc        60
   aaggatgact tgcctgatct ttttcgcctc cacgattcaa tttcaaattc atgcattttg       120
   gattattata ccttttaa                                                     138
<210> 397
   <211> 24
   <212> DNA
   <213> Arabidopsis thaliana
<400> 397
   atgtttccga gagagtccct ctga 24
<210> 398
   <211> 90
   <212> DNA
   <213> Arabidopsis thaliana
<400> 398
   atgaccctct ctgtattctt ccacagcttt cttgaactgc aaaacttctt cagatttttt        60
   tttttttctt ttgatatctc ttacgcataa                                         90
<210> 399
   <211> 72
   <212> DNA
   <213> Arabidopsis thaliana
<400> 399
   atgtcacttg ccaaaggaga gttgccctgt cactgcttcc gcttaaacac agtctataac        60
   cggttctgct aa                                                            72
<210> 400
   <211> 331
   <212> DNA
   <213> Arabidopsis thaliana
<400> 400
   ctttgtcact tcatacactc cctattgtct atatatatat atatacttac acatattcaa        60
   acattataat acttaattac acatacatac tttatgatgt tgcatatcac acataggttt       120
   gagagtgatg ttggttgttg acagaagata gagagcacta aggatgacat gcaagtacat 180
   acatatatat catcacaccg catgtggatg ataaaatatg tataacaaat tcaaagaaag 240
   agagggagag aaagagagag aacctgcatc tctactcttt tgtgctctct atacttctgt 300
   caccaccttt atctcttctt ctctctaacc t 331
<210> 401
   <211> 304
   <212> DNA
   <213> Arabidopsis thaliana
<400> 401
   atttactctt caccgccctc tctctatata tagtctctat cctcacatat tatatatcaa 60
   accgcaagaa tgctgtatgt atagtggagg gtgatagtgt ggttgctgac agaagataga 120
   gagcactaag gatgctatgc aaaacagaca cagatatgtg tttctaattg tatttcatac 180
   tttaacctca aagttgatat aaaaaaagaa agaaagatag aagagctaga agactatctg 240
   catctctatt cctatgtgct ctctatgctt ctgtcatcac ctttctttct ctatttctct 300
   ctac 304
<210> 402
   <211> 245
   <212> DNA
   <213> Arabidopsis thaliana
<400> 402
   aaccaaaact cttcaacatt tctctctgac tacttcattt cctcttccca acagttaaaa 60
   aaagttctga ttcgattcaa gccaagatcc acgtataaag atatgttcat gcgtagaggt 120
   ttggtataca acaatatata catataatag tttgtcgtta tgcctggctc cctgtatgcc 180
   acgagtggat accgattttg gttttaaaat cggctgccgg tggcgtacaa ggagtcaagc 240
   atgac 245
<210> 403
   <211> 240
   <212> DNA
   <213> Arabidopsis thaliana
<400> 403
   atacattctc tctttctctc tctctctctc tctcatcccg gcccagttat gtggtcggag 60
   agaatgatga aggtgtgtga tgagcaagat ggagaagcag ggcacgtgca ttactagctc 120
   atatatacac tctcaccaca aatgcgtgta tatatgcgga attttgtgat atagatgtgt 180
   gtgtgtgttg agtgtgatga tatggatgag ttagttcttc atgtgcccat cttcaccatc 240
<210> 404
   <211> 280
   <212> DNA
   <213> Arabidopsis thaliana
<400> 404
   tcacacatac ctttctttct cttcttcttc ttacgaaaag tttcatcaca ttcacattat 60
   ctttaacttt ggtctctttt cttttttgtc tcttttctct tcttgataac gtggttctag 120
   tcttgattaa ttcattgttg tgcgatttag tgttgagagg attgttgtct ggctcgaggt 180
   catgaagaag agaatcactc gaattaattt ggaagaacaa attaagaaaa ccctagatga 240
   ttctcggacc aggcttcatt ccccctaacc tacttatcgc 280
<210> 405
   <211> 179
   <212> DNA
   <213> Arabidopsis thaliana
<400> 405
   atttagcttc ttcttcttct tcttcttctg tctacttaca taaagttatc cttgctttgg 60
   tttagggttg agaggaatat tgtctggctc gaggtcatga agaagatcgg tagtattgat 120
   tcattttaaa gagtgaaatc cctaaatgat tctcggacca ggcttcattc cccccaacc 179
<210> 406
   <211> 376
   <212> DNA
   <213> Arabidopsis thaliana
<400> 406
   tagtattcat aagcaccaaa acaaatatgt agagatctcc tcttccattc tctattgtta 60
   ctttcgagaa gaaacataca aaacacatac atttttcttt tgtttgtggt tttcatatat 120
   acaagtgggt atagctagtg aaacgcgaat gtgacgaaag tagtgtgcag ccaaggatga 180
   cttgccgatt taaatgatct ttctttatac tctattaaga caatttagtt tcaaactttt 240
   tttttttttt ttttttgaag gattcaggaa gaaattagga tatattattc cgtataaaat 300
   acaagatata taaaaccaaa aagaaaaagt aacatgatcg gcaagttgtc cttggctaca 360
   cgttactttg tgtcgc 376
<210> 407
   <211> 77
   <212> DNA
   <213> Arabidopsis thaliana
<400> 407
   actcatcaac aacctcttca taaatacata aatcatataa gagaaaatgg tgacatgaag 60
   aatgagaact tgtgtgg 77
<210> 408
   <211> 248
   <212> DNA
   <213> Arabidopsis thaliana
<400> 408
   aggcaaaaac atatagagag taatgaagtg tatgatgaag aagagaggtc taacatggcg 60
   gaaagcgtca tgtttagtag ccaaggatga cttgcctgat ctttttcgcc tccacgattc 120
   aatttcaaat tcatgcattt tggattatta taccttttaa agtataatag gtcaaatatc 180
   atgttgaatc ttgcgggtta ggtttcaggc agtctctttg gctatcttga catgcttttt 240
   ccatccat 248
<210> 409
   <211> 187
   <212> DNA
   <213> Arabidopsis thaliana
<400> 409
   attcactccc ttcttcttct taatctcctt acagttacag acattctctc acttgcgttc 60
   ttgtttcttt tacaaaacag atacactatg tttccgagag agtccctctg atattggcct 120
   ggttcactca gattctcttt tactaactca tctgattgag ccgtgtcaat atctcagtcc 180
   tctctcg 187
<210> 410
   <211> 246
   <212> DNA
   <213> Arabidopsis thaliana
<400> 410
   tctcacaact tcaacttccc tctttctcta tattacgctt ttgcccctca ctccctcttt 60
   ccacaattag ggtttcgtct gctctacatg accctctctg tattcttcca cagctttctt 120
   gaactgcaaa acttcttcag attttttttt ttttcttttg atatctctta cgcataaaat 180
   agtgattttc ttcatatctc tgctcgattg atttgcggtt caataaagct gtgggaagat 240
   acagac 246
<210> 411
   <211> 631
   <212> DNA
   <213> Arabidopsis thaliana
<400> 411
   gaataaccaa ccagccttct ctcaaagcaa accaaaaaga aaaaccaaca ttgaaagagg      60
   aagttacgat aagcggagca gtaatagggc atctttctat tggcaggcga cttggctatt     120
   tgtatctttt gtgttcttga ctattggcta tgtcacttgc caaaggagag ttgccctgtc     180
   actgcttccg cttaaacaca gtctataacc ggttctgcta atatcaatcc ttcttttgga     240
   catgtccaaa gccgagattg attgatagag aattggtctc tctggctaca aaactagtgc     300
   ggttctctcg atttaagttt taatagcatt cactttgcac attgcatctt tcacatcaaa     360
   tttccatttc atcaaccatc taaacctctt tgttagcttt gatataagca acgatctaaa     420
   gtctaaaaac cattaatcct ctgaaaaaaa agacaatttc gatggttcta ttatgtttct     480
   ccaatgcaga aattgtatcg tctgaattat agtagatttt ttctagacta aagtgtaaac     540
   caagacgaat ctgcactaac aagacacacc aatagacttt acagagaaag gttacgagtt     600
   ttgaaaatat taacggacca tagtcatcgc g                                    631
<210> 412
   <211> 21
   <212> RNA
   <213> Arabidopsis thaliana
<400> 412
   uugacagaag auagagagca c 21
<210> 413
   <211> 20
   <212> RNA
   <213> Arabidopsis thaliana
<400> 413
   ugacagaaga uagagagcac 20
<210> 414
   <211> 21
   <212> RNA
   <213> Arabidopsis thaliana
<400> 414
   ugccuggcuc ccuguaugcc a 21
<210> 415
   <211> 21
   <212> RNA
   <213> Arabidopsis thaliana
<400> 415
   uggagaagca gggcacgugc a 21
<210> 416
   <211> 21
   <212> RNA
   <213> Arabidopsis thaliana
<400> 416
   ucggaccagg cuucauuccc c 21
<210> 417
   <211> 21
   <212> RNA
   <213> Arabidopsis thaliana
<400> 417
   ucggaccagg cuucauuccc c 21
<210> 418
   <211> 21
   <212> RNA
   <213> Arabidopsis thaliana
<400> 418
   cagccaagga ugacuugccg a 21
<210> 419
   <211> 21
   <212> RNA
   <213> Arabidopsis thaliana
<400> 419
   uagccaagga ugacuugccu g 21
<210> 420
   <211> 21
   <212> RNA
   <213> Arabidopsis thaliana
<400> 420
   uagccaagga ugacuugccu g 21
<210> 421
   <211> 21
   <212> RNA
   <213> Arabidopsis thaliana
<400> 421
   ugauugagcc gugucaauau c 21
<210> 422
   <211> 21
   <212> RNA
   <213> Arabidopsis thaliana
<400> 422
   uuccacagcu uucuugaacu g 21
<210> 423
   <211> 21
   <212> RNA
   <213> Arabidopsis thaliana
<400> 423
   ugccaaagga gaguugcccu g 21
<210> 424
   <211> 13
   <212> PRT
   <213> Medicago truncatula
<400> 424
   Met Val Lys Glu Ser Phe Met Glu Arg Leu Lys Val Arg
   1 5 10
<210> 425
   <211> 42
   <212> DNA
   <213> Medicago truncatula
<400> 425
   atggtcaaag agtctttcat ggagaggttg aaagtgagat ga 42
<210> 426
   <211> 291
   <212> DNA
   <213> Medicago truncatula
<400> 426
   tggataagtt aatttatggt caaagagtct ttcatggaga ggttgaaagt gagatgaagc 60
   caaggatgac ttgccggtat aatagtaatt tgccacaaat ctagatagct attagctatg 120
   tttggatggg cggtgagatt aacaaaatta cagcagcatt gtgattttgt tgatgcttta 180
   aagtgtagtt tttatcaaaa ttacagtggt tcactgtaat tatgagaatc tcaccgtcaa 240
   tctaaatatg catttagttt catttccggc aggtcatcct tcggctatat t 291
<210> 427
   <211> 21
   <212> RNA
   <213> Medicago truncatula
<400> 427
   aagccaagga ugacuugccg g 21
<210> 428
   <211> 20
   <212> PRT
   <213> Artificial Sequence
<220>
   <223> Primer
<400> 428
   Leu Ile Val Ser His Leu Tyr Ser Glu Lys Phe Asp Cys Met Arg Lys
   1               5                   10                  15
   Ile Leu Arg Ile
               20
<210> 429
   <211> 20
   <212> DNA
   <213> Artificial Sequence
<220>
   <223> Primer
<400> 429
   tccccaaatt cttgaccaaa 20
<210> 430
   <211> 20
   <212> DNA
   <213> Artificial Sequence
<220>
   <223> Primer
<400> 430
   ttgaggggaa aacatgaacc 20
<210> 431
   <211> 20
   <212> DNA
   <213> Artificial Sequence
<220>
   <223> Primer
<400> 431
   acgaaatccg tctcatttgc 20
<210> 432
   <211> 20
   <212> DNA
   <213> Artificial Sequence
<220>
   <223> Primer
<400> 432
   gggtgaagag ctcatgttgg 20
<210> 433
   <211> 19
   <212> DNA
   <213> Artificial Sequence
<220>
   <223> Primer
<400> 433
   tgaggggaat gttgtctgg 19
<210> 434
   <211> 19
   <212> DNA
   <213> Artificial Sequence
<220>
   <223> Primer
<400> 434
   gaagcctggt ccgaggata 19
<210> 435
   <211> 20
   <212> DNA
   <213> Artificial Sequence
<220>
   <223> Primer
<400> 435
   cgagtcgcca aaattcaaac 20
<210> 436
   <211> 20
   <212> DNA
   <213> Artificial Sequence
<220>
   <223> Primer
<400> 436
   gctcccttca gtccaatcaa 20
<210> 437
   <211> 20
   <212> DNA
   <213> Artificial Sequence
<220>
   <223> Primer
<400> 437
   gcaactgcag tggaatagca 20
<210> 438
   <211> 20
   <212> DNA
   <213> Artificial Sequence
<220>
   <223> Primer
<400> 438
   gcgaccttca tgggttctaa 20
<210> 439
   <211> 20
   <212> DNA
   <213> Artificial Sequence
<220>
   <223> Primer
<400> 439
   ctcagcatca gcaacgtgat 20
<210> 440
   <211> 20
   <212> DNA
   <213> Artificial Sequence
<220>
   <223> Primer
<400> 440
   aactctgcta gggcctcctc 20
<210> 441
   <211> 20
   <212> DNA
   <213> Artificial Sequence
<220>
   <223> Primer
<400> 441
   tcacaactcc tcagcattcg 20
<210> 442
   <211> 20
   <212> DNA
   <213> Artificial Sequence
<220>
   <223> Primer
<400> 442
   acccaatcaa cagcagttcc 20
<210> 443
   <211> 20
   <212> DNA
   <213> Artificial Sequence
<220>
   <223> Primer
<400> 443
   ggtaacattg tgctcagtgg 20
<210> 444
   <211> 20
   <212> DNA
   <213> Artificial Sequence
<220>
   <223> Primer
<400> 444
   ctcggccttg gagatccaca 20
<210> 445
   <211> 30
   <212> DNA
   <213> Artificial Sequence
<220>
   <223> Primer
<400> 445
   tcatcgatga atatacatac ataccatcat 30
<210> 446
   <211> 33
   <212> DNA
   <213> Artificial Sequence
<220>
   <223> Primer
<400> 446
   tccggatcct ctaaaagcta aagtgattct aaa 33
<210> 447
   <211> 26
   <212> DNA
   <213> Artificial Sequence
<220>
   <223> Primer
<400> 447
   tcatcgagag agagcttcct tgagtc 26
<210> 448
   <211> 28
   <212> DNA
   <213> Artificial Sequence
<220>
   <223> Primer
<400> 448
   tccggatcca gagggagctc ccttcagt 28
<210> 449
   <211> 26
   <212> DNA
   <213> Artificial Sequence
<220>
   <223> Primer
<400> 449
   tcatcggttg aggggaatgt tgtctg 26
<210> 450
   <211> 27
   <212> DNA
   <213> Artificial Sequence
<220>
   <223> Primer
<400> 450
   tccggatccg ttggggggga tgaagcc 27
<210> 451
   <211> 28
   <212> DNA
   <213> Artificial Sequence
<220>
   <223> Primer
<400> 451
   tcatcgatga gggttaagct atttcagt 28
<210> 452
   <211> 31
   <212> DNA
   <213> Artificial Sequence
<220>
   <223> Primer
<400> 452
   tccggatcct aatatcctcg atccagacaa c 31
<210> 453
   <211> 21
   <212> DNA
   <213> Artificial Sequence
<220>
   <223> Primer
<400> 453
   cgcctcaatt tgaatacatg g 21
<210> 454
   <211> 20
   <212> DNA
   <213> Artificial Sequence
<220>
   <223> Primer
<400> 454
   acgcggctca atcaaactac 20
<210> 455
   <211> 23
   <212> DNA
   <213> Artificial Sequence
<220>
   <223> Primer
<400> 455
   gcactagctg gtttcattat tcc 23
<210> 456
   <211> 21
   <212> DNA
   <213> Artificial Sequence
<220>
   <223> Primer
<400> 456
   ttgcaaaatt tggagagcct a 21
<210> 457
   <211> 20
   <212> DNA
   <213> Artificial Sequence
<220>
   <223> Primer
<400> 457
   aagcgatgtt ggtgaggttc 20
<210> 458
   <211> 21
   <212> DNA
   <213> Artificial Sequence
<220>
   <223> Primer
<400> 458
   cggctcaatc tgagatcgta t 21
<210> 459
   <211> 20
   <212> DNA
   <213> Artificial Sequence
<220>
   <223> Primer
<400> 459
   gggcggtgag attaacaaaa 20
<210> 460
   <211> 20
   <212> DNA
   <213> Artificial Sequence
<220>
   <223> Primer
<400> 460
   cctgccggaa atgaaactaa 20
<210> 461
   <211> 26
   <212> DNA
   <213> Artificial Sequence
<220>
   <223> Primer
<400> 461
   tcatcgatga tggtgtttgg gaagcc 26
<210> 462
   <211> 30
   <212> DNA
   <213> Artificial Sequence
<220>
   <223> Primer
<400> 462
   tccggatcct acatgtaaat ccgtcttcgg 30
<210> 463
   <211> 21
   <212> DNA
   <213> Artificial Sequence
<220>
   <223> Primer
<400> 463
   aggcttcttc ataggctctc c 21
<210> 464
   <211> 39
   <212> DNA
   <213> Artificial Sequence
<220>
   <223> Primer
<400> 464
   tcagtccgct cgaggaataa gtgaatatta tcgatattt 39
<210> 465
   <211> 38
   <212> DNA
   <213> Artificial Sequence
<220>
   <223> Primer
<400> 465
   aaggaaaaaa gcggccgcaa agtgatattg gcgcggct 38
<210> 466
   <211> 67
   <212> DNA
   <213> Artificial Sequence
<220>
   <223> Primer
<400> 466
   tagcatgtta ttacaccgct taagtaagtt ctgtaagata gagcgcgata ttgtttacat 60
   tagctag 67
<210> 467
   <211> 67
   <212> DNA
   <213> Artificial Sequence
<220>
   <223> Primer
<400> 467
   cgtactagct aatgtaaaca atatcgcgct ctatcttaca gaacttactt aagcggtgta 60
   ataacat 67
<210> 468
   <211> 38
   <212> DNA
   <213> Artificial Sequence
<220>
   <223> Primer
<400> 468
   aaaaggtctc gccataagca tagtggaata atgaaacc 38
<210> 469
   <211> 37
   <212> DNA
   <213> Artificial Sequence
<220>
   <223> Primer
<400> 469
   aaaaggtctc gccatttcca tacctgaatt tctcctt 37
<210> 470
   <211> 38
   <212> DNA
   <213> Artificial Sequence
<220>
   <223> Primer
<400> 470
   aaaaggtctc gccatcttca tcctcaatat cctaattc 38
<210> 471
   <211> 44
   <212> DNA
   <213> Artificial Sequence
<220>
   <223> Primer
<400> 471
   aggtctcgac acagatatat atactatgtc tctttcaatt ttgc 44
<210> 472
   <211> 35
   <212> DNA
   <213> Artificial Sequence
<220>
   <223> Primer
<400> 472
   aaaggtctcg ccatgaccat tttcccacga atatg 35
<210> 473
   <211> 36
   <212> DNA
   <213> Artificial Sequence
<220>
   <223> Primer
<400> 473
   aaggtctcga cacaagcata gtggaataat gaaacc 36
<210> 474
   <211> 38
   <212> DNA
   <213> Artificial Sequence
<220>
   <223> Primer
<400> 474
   aaggtctcga cacagatata tatactatgt ctctttca 38
<210> 475
   <211> 22
   <212> DNA
   <213> Artificial Sequence
<220>
   <223> Primer
<400> 475
   gaattcctgc ccatggtttt cg 22
<210> 476
   <211> 22
   <212> DNA
   <213> Artificial Sequence
<220>
   <223> Primer
<400> 476
   cgaaaaccat gggcaggaat tc 22
<210> 477
   <211> 23
   <212> DNA
   <213> Artificial Sequence
<220>
   <223> Primer
<400> 477
   cagtcccgac ttaagctcaa tac 23
<210> 478
   <211> 23
   <212> DNA
   <213> Artificial Sequence
<220>
   <223> Primer
<400> 478
   gtattgagct taagtcggga ctg 23
<210> 479
   <211> 43
   <212> DNA
   <213> Artificial Sequence
<220>
   <223> Primer
<400> 479
   aaaggtctcc tagcttggtc aaacatacat acagtagcac tag 43
<210> 480
   <211> 46
   <212> DNA
   <213> Artificial Sequence
<220>
   <223> Primer
<400> 480
   aaaggtctcg cgtaaaattg tactgattga atgtaaattg gtacac 46
<210> 481
   <211> 22
   <212> DNA
   <213> Artificial Sequence
<220>
   <223> Primer
<400> 481
   tagcatgttt tcccctcaat ga 22
<210> 482
   <211> 22
   <212> DNA
   <213> Artificial Sequence
<220>
   <223> Primer
<400> 482
   cgtatcattg aggggaaaac at 22
<210> 483
   <211> 36
   <212> DNA
   <213> Artificial Sequence
<220>
   <223> Primer
<400> 483
   aaaggtctcc tagcactcat aactctcccc aaattc 36
<210> 484
   <211> 37
   <212> DNA
   <213> Artificial Sequence
<220>
   <223> Primer
<400> 484
   aaaggtctcg cgtagaaaga atgttcgaaa aacaatg 37
<210> 485
   <211> 35
   <212> DNA
   <213> Artificial Sequence
<220>
   <223> Primer
<400> 485
   aggtctccaa atcatgaagc aggcagtaat aacct 35
<210> 486
   <211> 64
   <212> DNA
   <213> Artificial Sequence
<220>
   <223> Primer
<400> 486
   aggtctcgcg tactaagcgt aatctggaac atcgtatggg tataatatcc tcgatccaga 60
   caac 64
<210> 487
   <211> 31
   <212> DNA
   <213> Artificial Sequence
<220>
   <223> Primer
<400> 487
   aggtctcgcc atagtggcgg agacgaagat g 31
<210> 488
   <211> 34
   <212> DNA
   <213> Artificial Sequence
<220>
   <223> Primer
<400> 488
   aggtctcgcc attaatatcc tcgatccaga caac 34
<210> 489
   <211> 34
   <212> DNA
   <213> Artificial Sequence
<220>
   <223> Primer
<400> 489
   aggtctccaa attaaactgt cagtgcatgg atgt 34
<210> 490
   <211> 37
   <212> DNA
   <213> Artificial Sequence
<220>
   <223> Primer
<400> 490
   aggtctcgac acattcacaa atttttgttg tagagag 37
<210> 491
   <211> 37
   <212> DNA
   <213> Artificial Sequence
<220>
   <223> Primer
<400> 491
   aggtctcgac acattaacaa atttttgttg tagagag 37
<210> 492
   <211> 36
   <212> DNA
   <213> Artificial Sequence
<220>
   <223> Primer
<400> 492
   aggtctcgac actagcagat tcacaaattt ttgttg 36
<210> 493
   <211> 35
   <212> DNA
   <213> Artificial Sequence
<220>
   <223> Primer
<400> 493
   aggtctcgac accctcatga taatcgatct tagca 35
<210> 494
   <211> 272
   <212> DNA
   <213> Drosophila melanogaster
<400> 494
   cgcacacaca cacacacaca atcactcaca cgcggtcaca cgcacatttc aataaactaa 60
   tggagcctgg ctttgttttt gttttatttc caacccactt gagcacacag cacacacaga 120
   gagaaaaatc aatactcgtt atgggattaa atttacaaag cgcaaagcaa agcgacaaac 180
   aaaattcaaa agaaagaaaa aaaaacactc aaataaactc acaaagaatt ccttatcgcc 240
   aagggggcca atgttctaag gttctttcgc ct 272
<210> 495
   <211> 272
   <212> DNA
   <213> Artificial sequence
<220>
   <223> Mutated sequence
<400> 495
   cgcacacaca cacacacaca atcactcaca cgcggtcaca cgcacatttc aataaactaa      60
   gtgagcctgg ctttgttttt gttttatttc caacccactt aagcacacag cacacacaga     120
   gagaaaaatc aatactcgtt agtggattaa atttacaaag cgcaaagcaa agcgacaaac     180
   aaaattcaaa agaaagaaaa aaaaacactc aaataaactc acaaagaatt ccttatcgcc     240
   aagggggcca atgttctaag gttctttcgc ct                                   272